# EUROPEAN PATENT SPECIFICATION

(11) **EP 2 158 199 B1**
(45) Date of publication and mention of the grant of the patent: **17.11.2010**
(21) Application number: 08762541.4
(22) Date of filing: 11.06.2008
(51) Int. Cl.: C07D 417/08

(54) **PIPERIDINE COMPOUNDS AND USES THEREOF**
PIPERIDINVERBINDUNGEN UND ANWENDUNGEN DAVON
COMPOSÉS DE PIPÉRIDINE ET LEURS UTILISATIONS

(30) Priority: 12.06.2007 US 943440 P
(43) Date of publication of application: 03.03.2010
(73) Proprietor: AstraZeneca AB, 151 85 Södertälje (SE)
(72) Inventor: BASARAB, Gregory, Waltham, Massachusetts 02451 (US); HILL, Pamela, Waltham, Massachusetts 02451 (US); ZHOU, Fei, Waltham, Massachusetts 02451 (US)
(86) International application number: PCT/GB2008/050431
(87) International publication number: WO 2008/152418

(56) References cited:
- WO-A-2005/026149
- WO-A-2006/087543

## Description

The present invention relates to compounds which demonstrate antibacterial activity, processes for their preparation, pharmaceutical compositions containing them as the active ingredient, to their use as medicaments and to their use in the manufacture of medicaments for use in the treatment of bacterial infections in warm-blooded animals such as humans. In particular this invention relates to compounds useful for the treatment of bacterial infections in warm-blooded animals such as humans, more particularly to the use of these compounds in the manufacture of medicaments for use in the treatment of bacterial infections in warm-blooded animals such as humans.

The international microbiological community continues to express serious concern that the evolution of antibiotic resistance could result in strains against which currently available antibacterial agents will be ineffective. In general, bacterial pathogens may be classified as either Gram-positive or Gram-negative pathogens. Antibiotic compounds with effective activity against both Gram-positive and Gram-negative pathogens are generally regarded as having a broad spectrum of activity. The compounds of the present invention are regarded as effective against both Gram-positive and certain Gram-negative pathogens.

Gram-positive pathogens, for example Staphylococci, Enterococci, Streptococci and mycobacteria, are particularly important because of the development of resistant strains which arc both difficult to treat and difficult to eradicate from the hospital environment once established. Examples of such strains are methicillin resistant *staphylococcus aureus* (MRSA), methicillin resistant coagulase negative staphylococci (MRCNS), penicillin resistant *Streptococcus pneumoniae* and multiple resistant *Enterococcus faecium.*

The preferred clinically effective antibiotic for treatment of last resort of such resistant Gram-positive pathogens is vancomycin. Vancomycin is a glycopeptide and is associated with various toxicities, including nephrotoxicity. Furthermore, and most importantly, antibacterial resistance to vancomycin and other glycopeptides is also appearing. This resistance is increasing at a steady rate rendering these agents less and less effective in the treatment of Gram-positive pathogens. There is also now increasing resistance appearing towards agents such as β-lactams, quinolones and macrolides used for the treatment of upper respiratory tract infections, also caused by certain Gram negative strains including *H. influenzae* and *M. catarrhalis.*

Consequently, in order to overcome the threat of widespread multi-drug resistant organisms, there is an on-going need to develop new antibiotics, particularly those with either a novel mechanism of action and/or containing new pharmacophoric groups.

Deoxyribonucleic acid (DNA) gyrase is a member of the type II family of topoisomerases that control the topological state of DNA in cells (Champoux, J. J.; 2001. Ann. Rev. Biochem. 70: 369-413). Type II topoisomerases use the free energy from adenosine triphosphate (ATP) hydrolysis to alter the topology of DNA by introducing transient double-stranded breaks in the DNA, catalyzing strand passage through the break and reseating the DNA. DNA gyrase is an essential and conserved enzyme in bacteria and is unique among topoisomerases in its ability to introduce negative supercoils into DNA. The enzyme consists of two subunits, encoded by *gyrA* and *gyrB*, forming an A₂B₂ tetrameric complex. The A subunit of gyrase (GyrA) is involved in DNA breakage and resealing and contains a conserved tyrosine residue that forms the transient covalent link to DNA during strand passage. The B subunit (GyrB) catalyzes the hydrolysis of ATP and interacts with the A subunit to translate the free energy from hydrolysis to the conformational change in the enzyme that enables strand-passage and DNA resealing.

Another conserved and essential type II topoisomerase in bacteria, called topoisomerase IV, is primarily responsible for separating the linked closed circular bacterial chromosomes produced in replication. This enzyme is closely related to DNA gyrase and has a similar tetrameric structure formed from subunits homologous to Gyr A and to Gyr B. The overall sequence identity between gyrase and topoisomerase IV in different bacterial species is high. Therefore, compounds that target bacterial type II topoisomerases have the potential to inhibit two targets in cells, DNA gyrase and topoisomerase IV; as is the case for existing quinolone antibacterials (Maxwell, A. 1997, Trends Microbiol. 5: 102-109).

DNA gyrase is a well-validated target of antibacterials, including the quinolones and the coumarins. The quinolones (*e*.*g*. ciprofloxacin) are broad-spectrum antibacterials that inhibit the DNA breakage and reunion activity of the enzyme and trap the GyrA subunit covalently complexed with DNA (Drlica, K., and X. Zhao, 1997, Microbiol. Molec. Biol. Rev. 61: 377-392). Members of this class of antibacterials also inhibit topoisomerase IV and as a result, the primary target of these compounds varies among species. Although the quinolones are successful antibacterials, resistance generated by mutations in the target (DNA gyrase and topoisomerase IV) is becoming an increasing problem in several organisms, including *S. aureus* and *Streptococcus pneumoniae* (Hooper, D. C., 2002, The Lancet Infectious Diseases 2: 530-538). In addition, quinolones, as a chemical class, suffer from toxic side effects, including arthropathy that prevents their use in children (Lipsky, B. A. and Baker, C. A., 1999, Clin. Infect. Dis. 28: 352-364). Furthermore, the potential for cardiotoxicity, as predicted by prolongation of the QT_{c} interval, has been cited as a toxicity concern for quinolones.

There are several known natural product inhibitors of DNA gyrase that compete with ATP for binding the GyrB subunit (Maxwell, A. and Lawson, D.M. 2003, Curr. Topics in Med. Chem. 3: 283-303). The coumarins are natural products isolated from *Streptomyces spp*., examples of which are novobiocin, chlorobiocin and coumermycin A1. Although these compounds are potent inhibitors of DNA gyrase, their therapeutic utility is limited due to toxicity in eukaryotes and poor penetration in Gram-negative bacteria (Maxwell, A. 1997, Trends Microbiol. 5: 102-109). Another natural product class of compounds that targets the GyrB subunit is the cyclothialidines, which are isolated from *Streptomyces filipensis* (Watanabe, J. et al 1994, J. Antibiot. 47: 32-36). Despite potent activity against DNA gyrase, cyclothialidine is a poor antibacterial agent showing activity only against some eubacterial species (Nakada, N, 1993, Antimicrob. Agents Chemother. 37: 2656-2661).

Synthetic inhibitors that target the B subunit of DNA gyrase and topoisomeraseIV are known in the art. For example, coumarin-containing compounds are described in patent application number WO 99/35155, 5,6-bicyclic heteroaromatic compounds are described in patent application WO 02/060879, and pyrazole compounds are described in patent application WO 01/52845 (US 6,608,087). AstraZeneca has also published certain applications describing anti-bacterial compounds: WO2005/026149, WO2006/087544, WO2006/087548, WO2006/087543, WO2006/092599 and WO2006/092608.

We have discovered a new class of compounds which are useful for inhibiting DNA gyrase and / or topoisomerase IV.

According to the present invention there is provided a compound of formula **(I)**: 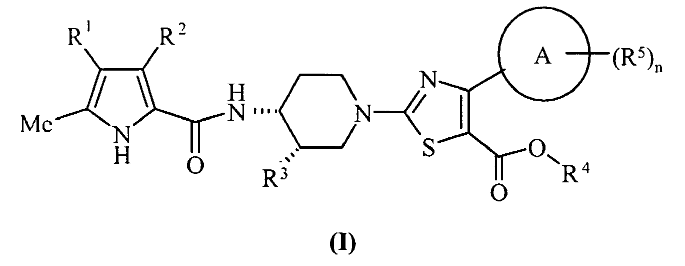
**R¹** is chloro or cyano;
**R²** is hydrogen, chloro or cyano;
**R³** is fluoro, methyl, methoxy, ethoxy, propoxy, allyloxy and benzyloxy;
**R⁴** is hydrogen or C₁₋₄alkyl;
**Ring A** is carbocyclyl or heterocyclyl; wherein if said heterocyclyl contains an -NH-moiety that nitrogen may be optionally substituted by a group selected from R⁶; and wherein if said heterocyclyl contains an -N= moiety that nitrogen may form a quaternary compound with a methyl group;
**R⁵** is a substituent on carbon and is selected from halo, nitro, cyano, hydroxy, amino, carboxy, carbamoyl, mercapto, sulphamoyl, sulfo, formyl, ureido, hydroxyiminomethyl, *N*-hydroxyformamido, hydrazinocarbonyl, *N*-hydroxyethanimidoyl, amino(hydroxyimino)methyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N,N*-(C₁₋₄alkyl)₂amino, C₁₋₄alkanoylamino, *N*-(C₁₋₄alkyl)carbamoyl, *N,N*-(C₁₋₄alkyl)₂carbamoyl, *N*-(C₁₋₄alkoxy)carbamoyl, *N'*-(C₁₋₄alkyl)ureido, *N,N'*-(C₁₋₄alkyl)₂ureido, *N*-(C₁₋₄alkyl)-*N*-(C₁₋₄alkoxy)carbamoyl, C₁₋₄alkylS(O)ₐ wherein a is 0 to 2, C₁₋₄alkoxycarbonyl, C₁₋₄alkoxycarbonylamino, *N*-(C₁₋₄alkyl)sulphamoyl, *N,N*-(C₁₋₄alkyl)₂sulphamoyl, C₁₋₄alkylsulphonylamino, C₁₋₄alkylsulphonylaminocarbonyl, *N'*-(C₁₋₄alkyl)hydrazinocarbonyl, *N',N'*-(C₁₋₄alkyl)₂hydrazinocarbonyl, carbocyclyl-R⁷- or heterocyclyl-R⁸-; wherein R⁵ may be optionally substituted on carbon by one or more R⁹; and wherein if said heterocyclyl contains an -NH- moiety that nitrogen may be optionally substituted by a group selected from R¹⁰;
**n** is 0, 1, 2, or 3;
**R⁹** is selected from halo, nitro, cyano, hydroxy, amino, carboxy, carbamoyl, mercapto, sulphamoyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N,N*-(C₁₋₄alkyl)₂amino, C₁₋₄alkanoylamino, *N*-(C₁₋₄alkyl)carbamoyl, *N,N*-(C₁₋₄alkyl)₂carbamoyl, C₁₋₄alkylS(O)ₐ wherein a is 0 to 2, C₁₋₄alkoxycarbonyl, *N*-(C₁₋₄alkyl)sulphamoyl, *N,N*-(C₁₋₄alkyl)₂sulphamoyl, C₁₋₄alkylsulphonylamino, C₁₋₄-alkoxycarbonylamino, carbocyclyl-R¹¹- or heterocyclyl-R¹²-; wherein R⁹ may be optionally substituted on carbon by one or more R¹³; and wherein if said heterocyclyl contains an -NH- moiety that nitrogen may be optionally substituted by a group selected from R¹⁴;
**R⁶**, **R¹⁰** and **R¹⁴** are independently selected from C₁₋₄alkyl, C₁₋₄alkanoyl, C₁₋₄alkylsulphonyl, C₁₋₄alkoxycarbonyl, carbamoyl, *N*-(C₁₋₄alkyl)carbamoyl, *N,N*-(C₁₋₄alkyl)carbamoyl, benzyl, benzyloxycarbonyl, benzoyl and phenylsulphonyl; wherein R⁶, R¹⁰ and R¹⁴ may be independently optionally substituted on carbon by a group selected from R²⁰;
**R⁷**, **R⁸**, **R¹¹** and **R¹²** are independently selected from a direct bond, -O-, -N(R¹⁵)-, -C(O)-, -N(R¹⁶)C(O)-, -C(O)N(R¹⁷)-, -S(O)ₚ-, -SO₂N(R¹⁸)- or -N(R¹⁹)SO₂-; wherein R¹⁵, R¹⁶, R¹⁷, R¹⁸ and R¹⁹ are independently selected from hydrogen or C₁₋₄alkyl and p is 0-2;
**R¹³** and **R²⁰** are independently selected from halo, nitro, cyano, hydroxy, trifluoromethoxy, trifluoromethyl, amino, carboxy, carbamoyl, mercapto, sulphamoyl, methyl, ethyl, ethenyl, ethynyl, methoxy, ethoxy, 2-trimethylsilylethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl or *N*-methyl-*N*-ethylsulphamoyl;
or a pharmaceutically acceptable salt thereof.

In an alternative embodiment, the present invention there is provided a compound of formula **(Ia)**: 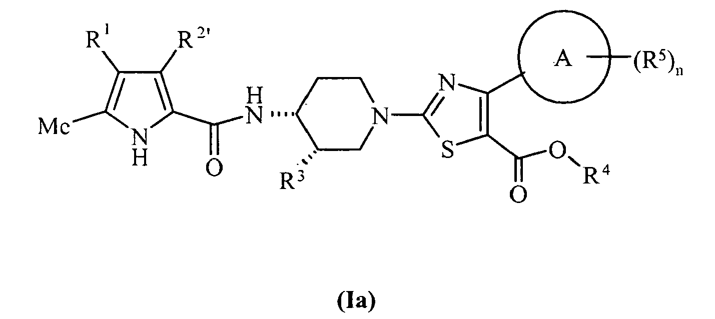 wherein:
**R¹** is chloro or cyano;
**R^{2'}** is hydrogen, chloro, cyano or methyl;
**R³** is fluoro, methyl, methoxy, ethoxy, propoxy, allyloxy and benzyloxy;
**R⁴** is hydrogen or C₁₋₄alkyl;
**Ring A** is carbocyclyl or heterocyclyl; wherein if said heterocyclyl contains an -NH-moiety that nitrogen may be optionally substituted by a group selected from R⁶; and wherein if said heterocyclyl contains an -N= moiety that nitrogen may form a quaternary compound with a methyl group;
**R⁵** is a substituent on carbon and is selected from halo, nitro, cyano, hydroxy, amino, carboxy, carbamoyl, mercapto, sulphamoyl, sulfo, formyl, ureido, hydroxyiminomethyl, *N*-hydroxyformamido, hydrazinocarbonyl, *N*-hydroxyethanimidoyl, amino(hydroxyimino)methyl, C₁₋₄alkyl, C₁₋₄alkenyl, C₂₋₄alkynyl, C₁₋₄alkoxy, C₁₋₄alkanoyl, C₁₋₄alkanoyloxy, *N*-(C₁₋₄alkyl)amino, *N,N*-(C₁₋₄alkyl)₂amino, C₁₋₄alkanoylamino, *N*-(C₁₋₄alkyl)carbamoyl, *N,N*-(C₁₋₄alkyl)₂carbamoyl, *N*-(C₁₋₄alkoxy)carbamoyl, *N'*-(C₁₋₄alkyl)ureido, *N',N'*-(C₁₋₄alkyl)₂ureido, *N*-(C₁₋₄alkyl)-*N*-(C₁₋₄alkoxy)carbamoyl, C₁₋₄alkylS(O)ₐ wherein a is 0 to 2, C₁₋₄alkoxycarbonyl, C₁₋₄alkoxycarbonylamino, *N*-(C₁₋₄alkyl)sulphamoyl, *N,N*-(C₁₋₄alkyl)₂sulphamoyl, C₁₋₄alkylsulphonylamino, C₁₋₄alkylsulphonylaminocarbonyl, *N'*-(C₁₋₄alkyl)hydrazinocarbonyl, *N',N'*-(C₁₋₄alkyl)₂hydrazinocarbonyl, carbocyclyl-R⁷- or heterocyclyl-R⁸-; wherein R⁵ may be optionally substituted on carbon by one or more R⁹; and wherein if said heterocyclyl contains an -NH- moiety that nitrogen may be optionally substituted by a group selected from R¹⁰;
**n** is 0, 1, 2, or 3;
**R⁹** is selected from halo, nitro, cyano, hydroxy, amino, carboxy, carbamoyl, mercapto, sulphamoyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N,N*-(C₁₋₄alkyl)₂amino, C₁₋₄alkanoylamino, *N*-(C₁₋₄alkyl)carbamoyl, *N,N*-(C₁₋₄alkyl)₂carbamoyl, C₁₋₄alkylS(O)ₐ wherein a is 0 to 2, C₁₋₄alkoxycarbonyl, *N*-(C₁₋₄alkyl)sulphamoyl, *N,N*-(C₁₋₄alkyl)₂sulphamoyl, C₁₋₄alkylsulphonylamino, C₁₋₄alkoxycarbonylamino, carbocyclyl-R¹¹- or heterocyclyl-R¹²-; wherein R⁹ may be optionally substituted on carbon by one or more R¹³; and wherein if said heterocyclyl contains an -NH- moiety that nitrogen may be optionally substituted by a group selected from R¹⁴;
**R⁶**, **R¹⁰** and **R¹⁴** are independently selected from C₁₋₄alkyl, C₁₋₄alkanoyl, C₁₋₄alkylsulphonyl, C₁₋₄alkoxycarbonyl, carbamoyl, *N*-(C₁₋₄alkyl)carbamoyl, *N,N*-(C₁₋₄alkyl)carbamoyl, benzyl, benzyloxycarbonyl, benzoyl and phenylsulphonyl; wherein R⁶, R¹⁰ and R¹⁴ may be independently optionally substituted on carbon by a group selected from R²⁰;
**R⁷**, **R⁸**, **R¹¹** and **R¹²** are independently selected from a direct bond, -O-, -N(R¹⁵)-, -C(O)-, -N(R¹⁶)C(O)-, -C(O)N(R¹⁷)-, -S(O)ₚ-, -SO₂N(R¹⁸)- or -N(R¹⁹)SO₂-; wherein R¹⁵, R¹⁶, R¹⁷, R¹⁸ and R¹⁹ are independently selected from hydrogen or C₁₋₄alkyl and p is 0-2;
**R¹³** and **R²⁰** are independently selected from halo, nitro, cyano, hydroxy, trifluoromethoxy, trifluoromethyl, amino, carboxy, carbamoyl, mercapto, sulphamoyl, methyl, ethyl, ethenyl, ethynyl, methoxy, ethoxy, 2-trimethylsilylethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N,N*-dimethylcarbamoyl, *N,N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N*-ethylsulphamoyl, *N,N*-dimethylsulphamoyl, *N,N*-diethylsulphamoyl or *N*-methyl-*N*-ethylsulphamoyl;
or a pharmaceutically acceptable salt thereof.

In this specification the term alkyl includes both straight and branched chain alkyl groups. For example, "C₁₋₄alkyl" includes methyl, ethyl, propyl, isopropyl and *t*-butyl. However references to individual alkyl groups such as propyl are specific for the straight chain version only. An analogous convention applies to other generic terms.

Where optional substituents are chosen from one or more groups it is to be understood that this definition includes all substituents being chosen from one of the specified groups or the substituents being chosen from two or more of the specified groups.

A "heterocyclyl" is a saturated, partially saturated or unsaturated, mono or bicyclic ring containing 4-12 atoms of which at least one atom is chosen from nitrogen, sulphur or oxygen, which may, unless otherwise specified, be carbon or nitrogen linked, wherein a -CH₂-group can optionally be replaced by a -C(O)- and a ring sulphur atom may be optionally oxidised to form the S-oxide(s). In one aspect of the invention a "heterocyclyl" is a saturated, partially saturated or unsaturated, monocyclic ring containing 5 or 6 atoms of which at least one atom is chosen from nitrogen, sulphur or oxygen, it may, unless otherwise specified, be carbon or nitrogen linked, a -CH₂- group can optionally be replaced by a -C(O)-and a ring sulphur atom may be optionally oxidised to form the S-oxides. In a further aspect of the invention a "heterocyclyl" is an unsaturated, carbon-linked, monocyclic ring containing 5 or 6 atoms of which at least one atom is chosen from nitrogen, sulphur or oxygen. Examples and suitable values of the term "heterocyclyl" are morpholino, piperidyl, pyridyl, pyranyl, pyrrolyl, pyrazolyl, isothiazolyl, indolyl, quinolyl, thienyl, 1,3-benzodioxolyl, thiadiazolyl, piperazinyl, thiazolidinyl, pyrrolidinyl, thiomorpholino, pyrrolinyl, homopiperazinyl, 3,5-dioxapiperidinyl, tetrahydropyranyl, imidazolyl, pyrimidyl, pyrazinyl, pyridazinyl, isoxazolyl, *N*-methylpyrrolyl, 4-pyridone, 1-isoquinolone, 2-pyrrolidone, 4-thiazolidone, pyridine-*N*-oxide and quinoline-*N*-oxide. Further examples and suitable values of the term "heterocyclyl" are imidazolyl, 1,2,4-oxadiazolyl, 1,3,4-oxadiazolyl, pyrazolyl, 1,2,4-triazolyl, pyridyl, benzothiazolyl, isoxazolyl, pyrazinyl, pyrimidinyl and thiazolyl.

A "carbocyclyl" is a saturated, partially saturated or unsaturated, mono or bicyclic carbon ring that contains 3-12 atoms; wherein a -CH₂- group can optionally be replaced by a -C(O)-. Particularly "carbocyclyl" is a monocyclic ring containing 5 or 6 atoms or a bicyclic ring containing 9 or 10 atoms. Suitable values for "carbocyclyl" include cyclopropyl, cyclobutyl, 1-oxocyclopentyl, cyclopentyl, cyclopentenyl, cyclohexyl, cyclohexenyl, phenyl, naphthyl, tetralinyl, indanyl or 1-oxoindanyl. A particular example of "carbocyclyl" is phenyl.

An example of "C₁₋₄alkanoyloxy" is acetoxy. Examples of "C₁₋₄alkoxycarbonyl" are methoxycarbonyl, ethoxycarbonyl, *n*- and *t*-butoxycarbonyl. Examples of "C₁₋₄alkoxycarbonylamino" are methoxycarbonylamino, ethoxycarbonylamino, *n*- and *t*-butoxycarbonylamino. Examples of "C₁₋₄alkoxy" are methoxy, ethoxy and propoxy. Examples of "C₁₋₄alkanoylamino" are formamido, acetamido and propionylamino. Examples of "C₁₋₄alkylS(O)ₐ wherein a is 0 to 2" are methylthio, ethylthio, methylsulphinyl, ethylsulphinyl, mesyl and ethylsulphonyl. Examples of "C₁₋₄alkanoyl" are propionyl and acetyl. Examples of "*N*-(C₁₋₄alkyl)amino" are methylamino and ethylamino. Examples of "*N,N*-(C₁₋₄alkyl)₂amino" are di-*N*-methylamino, di-(*N*-ethyl)amino and *N*-ethyl-*N*-methylamino. Examples of "C₂₋₄alkenyl" are vinyl, allyl and I-propenyl. Examples of "C₂₋₄alkynyl" are ethynyl, 1-propynyl and 2-propynyl. Examples of "*N*-(C₁₋₄alkyl)sulphamoyl" are *N*-(methyl)sulphamoyl and *N*-(ethyl)sulphamoyl. Examples of "*N,N*-(C₁₋₄alkyl)₂sulphamoyl" are *N,N*-(dimethyl)sulphamoyl and *N*-(methyl)-*N*-(ethyl)sulphamoyl. Examples of "*N*-(C₁₋₄alkyl)carbamoyl" are methylaminocarbonyl and ethylaminocarbonyl. Examples of "*N,N*-(C₁₋₄alkyl)₂carbamoyl" are dimethylaminocarbonyl and methylethylaminocarbonyl. Examples of "*N*-(C₁₋₄alkoxy)carbamoyl" are methoxyaminocarbonyl and isopropoxyaminocarbonyl. Examples of "*N*-(C₁₋₄alkyl)-*N*-(C₁₋₄alkoxy)carbamoyl" are *N*-methyl-*N*-methoxyaminocarbonyl and *N*-methyl-*N*-ethoxyaminocarbonyl. Examples of "*N'*-(C₁₋₄alkyl)ureido" are *N'*-methylureido and *N'*-isopropylureido. Examples of "*N',N'*-(C₁₋₄alkyl)₂ureido" are *N'N'*-dimethylureido and *N'*-methyl-*N'*-isopropylureido. Examples of "*N'*-(C₁₋₄alkyl)hydrazinocarbonyl" are *N'*-methylhydrazinocarbonyl and *N'*-isopropylhydrazinocarbonyl. Examples of "*N,N'*-(C₁₋₄alkyl)₂hydrazinocarbonyl" are *N'N'*-dimethylhydrazinocarbonyl and *N'*-methyl-*N'*-isopropylhydrazinocarbonyl. Examples of "C₁₋₄alkylsulphonylamino" are methylsulphonylamino, isopropylsulphonylamino and *t*-butylsulphonylamino. Examples of "C₁₋₄alkylsulphonylaminocarbonyl" are methylsulphonylaminocarbonyl, isopropylsulphonylaminocarbonyl and *t*-butylsulphonylaminocarbonyl. Examples of "C₁₋₄alkylsulphonyl" are methylsulphonyl, isopropylsulphonyl and *t*-butylsulphonyl.

A compound of formula (I) or **(Ia)** may form stable acid or basic salts, and in such cases administration of a compound as a salt may be appropriate, and pharmaceutically acceptable salts may be made by conventional methods such as those described following.

Suitable pharmaceutically-acceptable salts include acid addition salts such as methanesulfonate, tosylate, α-glycerophosphate, fumarate, hydrochloride, citrate, maleate, tartrate and hydrobromide. Also suitable are salts formed with phosphoric and sulfuric acid. In another aspect suitable salts are base salts such as an alkali metal salt for example sodium, an alkaline earth metal salt for example calcium or magnesium, an organic amine salt for example triethylamine, morpholine, *N*-methylpiperidine, *N*-ethylpiperidine, procaine, dibenzylamine, *N,N*-dibenzylethylamine, tris-(2-hydroxyethyl)amine, *N*-methyl d-glucamine and amino acids such as lysine. There may be more than one cation or anion depending on the number of charged functions and the valency of the cations or anions. In one aspect of the invention the pharmaceutically-acceptable salt is the sodium salt.

However, to facilitate isolation of the salt during preparation, salts which are less soluble in the chosen solvent may be utilised whether pharmaceutically-acceptable or not.

Within the present invention it is to be understood that a compound of the formula **(I)** or **(Ia)** or a salt thereof may exhibit the phenomenon of tautomerism and that the formulae drawings within this specification can represent only one of the possible tautomeric forms. It is to be understood that the invention encompasses any tautomeric form which inhibits DNA gyrase and or topoisomerase IV and is not to be limited merely to any one tautomeric form utilised within the formulae drawings. The formulae drawings within this specification can represent only one of the possible tautomeric forms and it is to be understood that the specification encompasses all possible tautomeric forms of the compounds drawn not just those forms which it has been possible to show graphically herein. The same applies to compound names.

It will be appreciated by those skilled in the art that in addition to the two asymmetric carbons drawn in formula **(I)** or **(Ia)**, compounds of formula **(I)** or **(Ia)** may contain additional asymmetrically substituted carbon(s) and sulphur atom(s), and accordingly may exist in, and be isolated in, as far as those additional asymmetrically substituted carbon(s) and sulphur atom(s) arc concerned, optically-active and racemic forms at those positions. It is to be understood that the present invention encompasses any racemic, optically-active, polymorphic or stereoisomeric form, or mixtures thereof, at any additional asymmetrically substituted carbon(s) and sulphur atom(s), which possesses properties useful in the inhibition of DNA gyrase and / or topoisomerase IV.

Optically-active forms may be prepared by procedures known in the art for example, by resolution of the racemic form by recrystallization techniques, by synthesis from optically-active starting materials, by chiral synthesis, by enzymatic resolution, by biotransformation, or by chromatographic separation using a chiral stationary phase.

Some compounds may exhibit polymorphism. It is to be understood that the present invention encompasses any polymorphic form, or mixtures thereof, which form possesses properties useful in the inhibition of DNA gyrase and / or topoisomerase IV

It is also to be understood that certain compounds of the formula (I) or **(Ia)** and salts thereof can exist in solvated as well as unsolvated forms such as, for example, hydrated forms. It is to be understood that the invention encompasses all such solvated forms which inhibit DNA gyrase and / or topoisomerase IV.

There follow particular and suitable values for certain substituents and groups referred to in this specification. These values may be used where appropriate with any of the definitions and embodiments disclosed hereinbefore, or hereinafter. For the avoidance of doubt each stated species represents a particular and independent aspect of this invention.
R¹ is chloro.
R¹ is cyano;
R² is hydrogen.
R² is chloro
R² is cyano.
R² is hydrogen or chloro.
R^{2'} is hydrogen.
R^{2'} is chloro
R^{2'} is cyano.
R^{2'} is hydrogen or chloro.
R³ is fluoro.
R³ is methyl.
R³ is methoxy.
R³ is ethoxy.
R³ is propoxy.
R³ is allyloxy.
R³ is benzyloxy.
R³ is fluoro or methoxy.
R⁴ is hydrogen.
R⁴ is C₁₋₄alkyl.
R⁴ is hydrogen, methyl or ethyl.
R⁴ is methyl or ethyl.
R⁴ is methyl.
R⁴ is ethyl.
Ring A is carbocyclyl.
Ring A is heterocyclyl; wherein if said heterocyclyl contains an -NH- moiety that nitrogen may be optionally substituted by a group selected from R⁶; and wherein if said heterocyclyl contains an -N= moiety that nitrogen may form a quaternary compound with a methyl group; wherein:
R⁶ is C₁₋₄alkyl; wherein R⁶ may be optionally substituted on carbon by a group selected from R²⁰; and
R²⁰ is selected from methoxy or 2-trimethylsilylethoxy.
Ring A is heterocyclyl; wherein if said heterocyclyl contains an -NH- moiety that nitrogen may be optionally substituted by a group selected from R⁶; and wherein if said heterocyclyl contains an -N= moiety that nitrogen may form a quaternary compound with a methyl group; wherein:
R⁶ is C₁₋₄alkyl; wherein R⁶ may be optionally substituted on carbon by a group selected from R²⁰; and
R²⁰ is selected from methoxy or ethoxy.
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl group; wherein
R⁶ is methyl or ethyl; wherein R⁶ may be optionally substituted on carbon by a group selected from R²⁰; and
R²⁰ is selected from methoxy or 2-trimethylsilylethoxy.
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl group; wherein
R⁶ is methyl or ethyl; wherein R⁶ may be optionally substituted on carbon by a group selected from R²⁰; and
R²⁰ is selected from methoxy or ethoxy.
Ring A is 1-(2-methoxyethyl)imidazol-2-yl, 1-(2-trimethylsilylethoxymethyl)imidazol-2-yl, 1-(methoxymethyl)imidazol-2-yl, 1,2,4-oxadiazol-5-yl, 1,3,4-oxadiazol-2-yl, 1,3-dimethylimidazol-2-yl, 1H-imidazol-2-yl, 1-methylimidazol-4-yl, 2H-pyrazol-3-yl, 2-methyl-1,2,4-triazol-3-yl, 2-pyridyl, benzothiazol-2-yl, isoxazol-5-yl, pyrazin-2-yl, pyrimidin-2-yl, pyrimidin-4-yl, thiazol-2-yl or thiazol-4-yl.
Ring A is 1-(2-methoxyethyl)imidazol-2-yl.
Ring A is 1-(2-trimethylsilylethoxymethyl)imidazol-2-yl.
Ring A is 1-(methoxymethyl)imidazol-2-yl.
Ring A is 1,2,4-oxadiazol-5-yl.
Ring A is 1,3,4-oxadiazol-2-yl.
Ring A is 1,3-dimethylimidazol-2-yl.
Ring A is 1H-imidazol-2-yl.
Ring A is 1-methylimidazol-4-yl.
Ring A is 2H-pyrazol-3-yl.
Ring A is 2-methyl-1,2,4-triazol-3-yl.
Ring A is 2-pyridyl.
Ring A is benzothiazol-2-yl.
Ring A is isoxazol-5-yl.
Ring A is pyrazin-2-yl.
Ring A is pyrimidin-2-yl.
Ring A is pyrimidin-4-yl.
Ring A is thiazol-2-yl.
Ring A is thiazol-4-yl.
R⁵ is a substituent on carbon and is selected from amino, C₁₋₄alkyl, C₁₋₄alkoxy, *N*-(C₁₋₄alkyl)amino or *N,N*-(C₁₋₄alkyl)₂amino.
R⁵ is a substituent on carbon and is selected from amino, methyl, methoxy, methylamio or dimethylamino.
n is 0-2.
n is 0.
n is 1
n is 2.
Ring A, R⁵ and n together form 1H-imidazol-2-yl, 2,6-bis(dimethylamino)pyrimidin-4-yl, 2-pyridyl, 2H-pyrazol-3-yl, pyrimidin-4-yl, isoxazol-5-yl, 1-methylimidazol-4-yl, pyrazin-2-yl, 2-aminothiazol-4-yl, 2-dimethylaminothiazol-4-yl, 2-methylaminothiazol-4-yl, 1,3-dimethylimidazol-2-yl, 1,4,5-trimethylimidazol-2-yl, 4,6-dimethoxypyrimidin-2-yl, 3-methyl-1,2,4-oxadiazol-5-yl, benzothiazol-2-yl, 4-methoxypyrimidin-2-yl, pyrimidin-2-yl, 1-methylimidazol-2-yl, 1-(2-methoxyethyl)imidazol-2-yl, 1-(methoxymethyl)imidazol-2-yl, 1-(2-trimethylsilylethoxymethyl)imidazol-2-yl, thiazol-2-yl, 2-methyl-1,2,4-triazol-3-yl, 5-methyl-1,3,4-oxadiazol-2-yl or 1,3,4-oxadiazol-2-yl.
Ring A, R⁵ and n together form R⁵ and n together form 1H-imidazol-2-yl.
Ring A, R⁵ and n together form 2,6-bis(dimethylamino)pyrimidin-4-yl.
Ring A, R⁵ and n together form 2-pyridyl.
Ring A, R⁵ and n together form 2H-pyrazol-3-yl.
Ring A, R⁵ and n together form pyrimidin-4-yl.
Ring A, R⁵ and n together form isoxazol-5-yl.
Ring A, R⁵ and n together form 1-methylimidazol-4-yl.
Ring A, R⁵ and n together form pyrazin-2-yl.
Ring A, R⁵ and n together form 2-aminothiazol-4-yl.
Ring A, R⁵ and n together form 2-dimethylaminothiazol-4-yl.
Ring A, R⁵ and n together form 2-methylaminothiazol-4-yl.
Ring A, R⁵ and n together form 1,3-dimethylimidazol-2-yl.
Ring A, R⁵ and n together form 1,4,5-trimethylimidazol-2-yl.
Ring A, R⁵ and n together form 4,6-dimethoxypyrimidin-2-yl.
Ring A, R⁵ and n together form 3-methyl-1,2,4-oxadiazol-5-yl.
Ring A, R⁵ and n together form benzothiazol-2-yl.
Ring A, R⁵ and n together form 4-methoxypyrimidin-2-yl.
Ring A, R⁵ and n together form pyrimidin-2-yl.
Ring A, R⁵ and n together form 1-methylimidazol-2-yl.
Ring A, R⁵ and n together form 1-(2-methoxyethyl)imidazol-2-yl.
Ring A, R⁵ and n together form 1-(methoxymethyl)imidazol-2-yl.
Ring A, R⁵ and n together form 1-(2-trimethylsilylethoxymethyl)imidazol-2-yl.
Ring A, R⁵ and n together form thiazol-2-yl.
Ring A, R⁵ and n together form 2-methyl-1,2,4-triazol-3-yl.
Ring A, R⁵ and n together form 5-methyl-1,3,4-oxadiazol-2-yl.
Ring A, R⁵ and n together form 1,3,4-oxadiazol-2-yl.

Therefore in a further aspect of the invention there is provided a compound of formula **(I)** (as depicted above) wherein:
R¹ is chloro or cyano;
R² is hydrogen or chloro;
R³ is fluoro or methoxy;
R⁴ is hydrogen or C₁₋₄alkyl;
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl group;
R⁶ is methyl or ethyl; wherein R⁶ may be optionally substituted on carbon by a group selected from R²⁰;
R²⁰ is selected from methoxy or 2-trimethylsilylethoxy;
R⁵ is a substituent on carbon and is selected from amino, C₁₋₄alkyl, C₁₋₄alkoxy, *N*-(C₁₋₄alkyl)amino or *N,N*-(C₁₋₄alkyl)₂amino; and
n is 0-2;
or a pharmaceutically acceptable salt thereof.

Therefore in a further aspect of the invention there is provided a compound of formula **(I)** (as depicted above) wherein:
R¹ is chloro or cyano;
R² is hydrogen or chloro;
R³ is fluoro or methoxy;
R⁴ is hydrogen or C₁₋₄alkyl;
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl group;
R⁶ is methyl or ethyl; wherein R⁶ may be optionally substituted on carbon by a group selected from R²⁰;
R²⁰ is selected from methoxy or ethoxy;
R⁵ is a substituent on carbon and is selected from amino, C₁₋₄alkyl, C₁₋₄alkoxy, *N*-(C₁₋₄alkyl)amino or *N,N*-(C₁₋₄alkyl)₂amino; and
n is 0-2;
or a pharmaceutically acceptable salt thereof.

Therefore in a further aspect of the invention there is provided a compound of formula **(I)** (as depicted above) wherein:
R¹ is chloro or cyano;
R² is hydrogen or chloro;
R³ is fluoro or methoxy;
R⁴ is hydrogen, methyl or ethyl;
Ring A is 1-(2-methoxyethyl)imidazol-2-yl, 1-(2-trimethylsilylethoxymethyl)imidazol-2-yl, 1-(methoxymethyl)imidazol-2-yl, 1,2,4-oxadiazol-5-yl, 1,3,4-oxadiazol-2-yl, 1,3-dimethylimidazol-2-yl, 1H-imidazol-2-yl, 1-methylimidazol-4-yl, 2H-pyrazol-3-yl, 2-methyl-1,2,4-triazol-3-yl, 2-pyridyl, benzothiazol-2-yl, isoxazol-5-yl, pyrazin-2-yl, pyrimidin-2-yl, pyrimidin-4-yl, thiazol-2-yl or thiazol-4-yl;
R⁵ is a substituent on carbon and is selected from amino, methyl, methoxy, methylamio or dimethylamino;
n is 0-2;
or a pharmaceutically acceptable salt thereof.

Therefore in a further aspect of the invention there is provided a compound of formula **(I)** (as depicted above) wherein:
R¹ and R² are chloro;
R³ is methoxy;
R⁴ is hydrogen;
Ring A is 2-methyl-1,2,4-triazol-3-yl or pyrimidin-2-yl;
n is 0:
or a pharmaceutically acceptable salt thereof.

Therefor in a further aspect of the invention there is provided a compound of formula **(Ia)** (as depicted above) wherein:
R¹ is chloro or cyano;
R^{2'} is hydrogen or chloro;
R³ is fluoro or methoxy;
R⁴ is hydrogen or C₁₋₄alkyl;
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl group;
R⁶ is methyl or ethyl; wherein R⁶ may be optionally substituted on carbon by a group selected from R²⁰;
R²⁰ is selected from methoxy or 2-trimethylsilylethoxy;
R⁵ is a substituent on carbon and is selected from amino, C₁₋₄alkyl, C₁₋₄alkoxy, *N*-(C₁₋₄alkyl)amino or *N,N*-(C₁₋₄alkyl)₂amino; and
n is 0-2;
or a pharmaceutically acceptable salt thereof.

Therefore in a further aspect of the invention there is provided a compound of formula **(Ia)** (as depicted above) wherein:
R¹ is chloro or cyano:
R^{2'} is hydrogen or chloro;
R³ is fluoro or methoxy;
R⁴ is hydrogen or C₁₋₄alkyl;
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl group;
R⁶ is methyl or ethyl; wherein R⁶ may be optionally substituted on carbon by a group selected from R²⁰;
R²⁰ is selected from methoxy or ethoxy;
R⁵ is a substituent on carbon and is selected from amino, C₁₋₄alkyl, C₁₋₄alkoxy, *N*-(C₁₋₄alkyl)amino or *N,N*-(C₁₋₄alkyl)₂amino; and
n is 0-2;
or a pharmaceutically acceptable salt thereof.

Therefore in a further aspect of the invention there is provided a compound of formula **(Ia)** (as depicted above) wherein:
R¹ is chloro or cyano;
R^{2'} is hydrogen or chloro;
R³ is fluoro or methoxy;
R⁴ is hydrogen, methyl or ethyl;
Ring A is 1-(2-methoxyethyl)imidazol-2-yl, 1-(2-trimethylsilylethoxymethyl)imidazol-2-yl, 1-(methoxymethyl)imidazol-2-yl, 1,2,4-oxadiazol-5-yl, 1,3,4-oxadiazol-2-yl, 1,3-dimethylimidazol-2-yl, 1H-imidazol-2-yl, 1-methylimidazol-4-yl, 2H-pyrazol-3-yl, 2-methyl-1,2,4-triazol-3-yl, 2-pyridyl, benzothiazol-2-yl, isoxazol-5-yl, pyrazin-2-yl, pyrimidin-2-yl, pyrimidin-4-yl, thiazol-2-yl or thiazol-4-yl;
R⁵ is a substituent on carbon and is selected from amino, methyl, methoxy, methylamio or dimethylamino;
n is 0-2;
or a pharmaceutically acceptable salt thereof.

Therefore in a further aspect of the invention there is provided a compound of formula **(Ia)** (as depicted above) wherein:
R¹ and R^{2'} are chloro;
R³ is methoxy;
R⁴ is hydrogen;
Ring A is 2-methyl-1,2,4-triazol-3-yl or pyrimidin-2-yl;
n is 0;
or a pharmaceutically acceptable salt thereof.

Particular compounds of the invention are the compounds of the Examples, each of which provides a further independent aspect of the invention. In further aspects, the present invention also comprises any two or more compounds of the Examples.

In one embodiment of the invention are provided compounds of formula **(I)** or **(Ia),** in an alternative embodiment are provided pharmaceutically-acceptable salts of compounds of formula **(I)** or **(Ia).**

In a further aspect the present invention provides a process for preparing a compound of formula **(I)** or **(Ia),** or a pharmaceutically-acceptable salt thereof.

Thus, the present invention also provides that the compounds of the formula **(I)** or **(Ia)** and pharmaceutically-acceptable salts thereof, can be prepared by a process as follows (wherein the variables are as defined above unless otherwise stated):
*Process a)* reacting a compound of formula **(II)** or **(II'):** 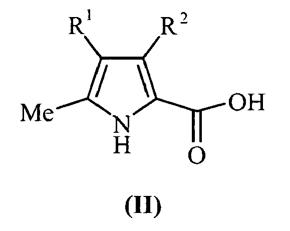 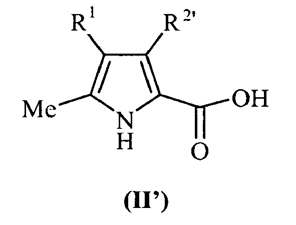 or an activated acid derivative thereof; with a compound of formula **(III):** 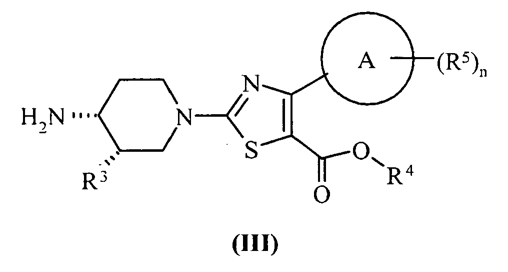 or
*Process b)* reacting a compound of formula **(IV)** or **(IV'):** 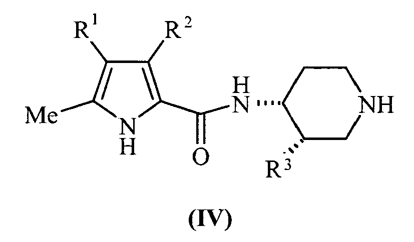 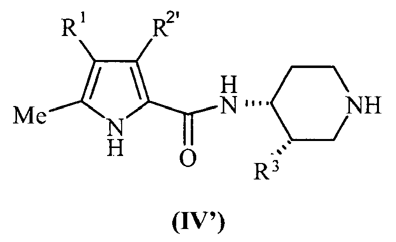 with a compound of formula **(V):** 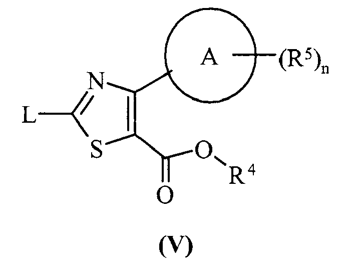 wherein L is a displaceable group; or
*Process c)* for compounds of formula **(I)** or **(Ia)** wherein R⁴ is C₁₋₄alkyl; reacting a compound of formula **(I)** or **(Ia)** which is a compound of formula **(VI)** or **(VI'):** 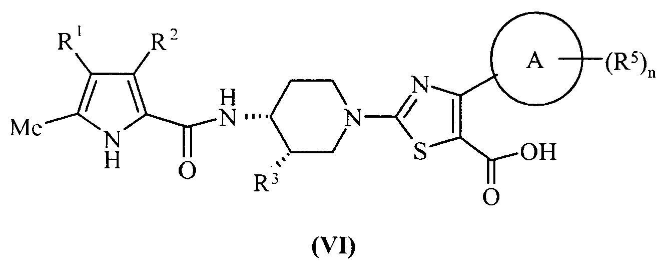 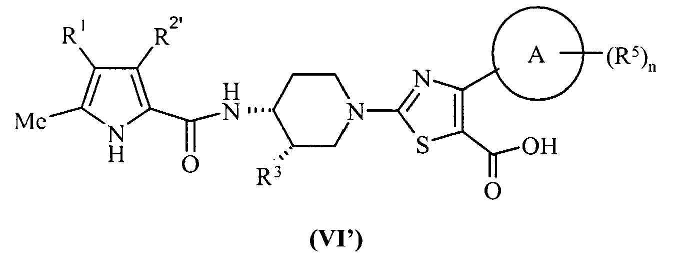 with a compound of formula **(VII):**

   R^{4a} -OH **(VII)**

   wherein R^{4a} is C₁₋₄alkyl;
   or
*Process d)* for compounds of formula **(I)** or **(Ia)** wherein R⁴ is hydrogen; deprotecting a compound of formula **(VIII)** or **(VIII'):** 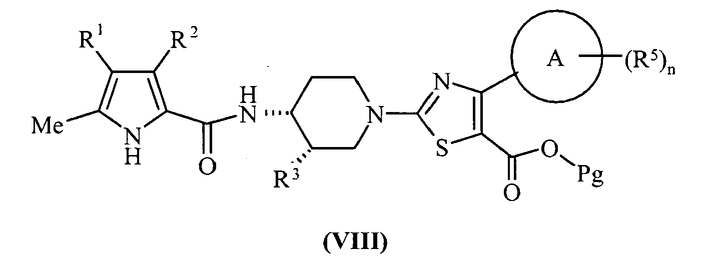 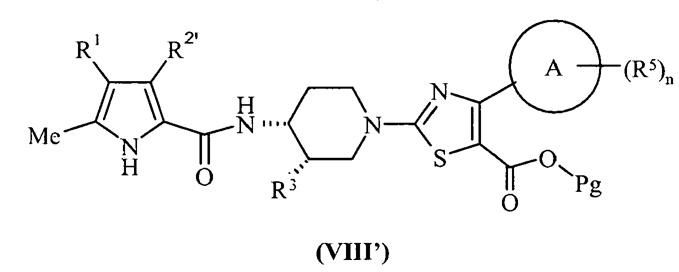
wherein Pg is a carboxylic acid protecting group; and thereafter if necessary:
i) converting a compound of the formula **(I)** or **(Ia)** into another compound of the formula (I) or **(Ia)**;
ii) removing any protecting groups;
iii) forming a pharmaceutically acceptable salt; and/or
iv) chirally purifying the compound of formula **(I)** or **(Ia).**

L is a displaceable group. Suitable values for L include halo, for example chloro and bromo, pentafluorophenoxy and 2,5-oxopyrrolidin-1-yloxy.

Pg is a carboxylic acid protecting group. Suitable values for Pg are defined herein below.

Specific reaction conditions for the above reaction are as follows.

*Process a)* Compounds of formula **(II)** or **(II')** and **(III)** may be coupled together in the presence of a suitable coupling reagent. Standard peptide coupling reagents known in the art can be employed as suitable coupling reagents, or for example carbonyldiimidazole and dicyclohexyl-carbodiimide, optionally in the presence of a catalyst such as dimethylaminopyridine or 4-pyrrolidinopyridine, optionally in the presence of a base for example triethylamine, pyridine, or 2,6-di-*alkyl*-pyridines such as 2,6-lutidine or 2,6-di-*tert*-butylpyridine. Suitable solvents include dimethylacetamide, dichloromethane, benzene, tetrahydrofuran and dimethylformamide. The coupling reaction may conveniently be performed at a temperature in the range of-40 to 40°C.

Suitable activated acid derivatives include acid halides, for example acid chlorides, and active esters, for example pentafluorophenyl esters. The reaction of these types of compounds with amines is well known in the art, for example they may be reacted in the presence of a base, such as those described above, and in a suitable solvent, such as those described above. The reaction may conveniently be performed at a temperature in the range of -40 to 40°C.

Compounds of formula **(III)** may be prepared according to *Schema 1*: 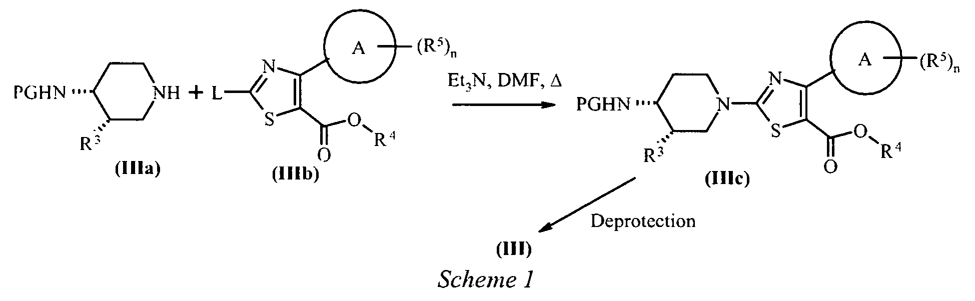 wherein PG is a nitrogen protecting group such as those defined herein below; and L is a displaceable group such as those defined herein above.

Compounds of formula **(II)** or **(II')** are commercially available compounds, or they are known in the literature, or they are prepared by standard processes known in the art. *Process b)* Compounds of formula **(IV)** or **(IV')** and **(V)** in a suitable solvent such as dimethylformamide or N-methylpyrrolindine and optionally in the presence of a base such as triethylamine or diisopropylamine are heated together at a temperature range between 50 to 100°C.

Compounds of formula **(IV)** may be prepared according to *Schema 2*: 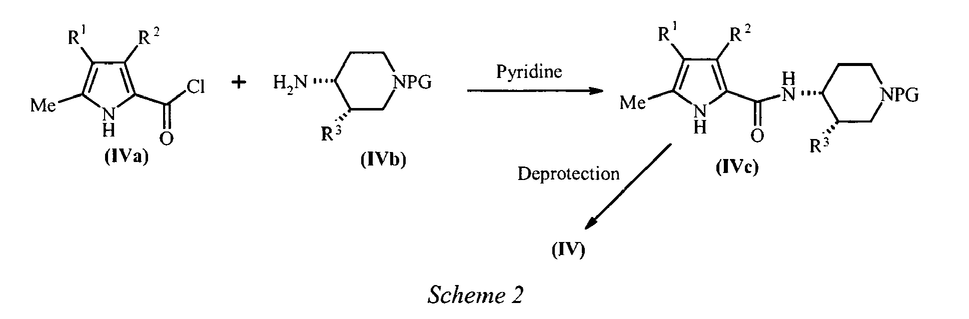 wherein PG is a nitrogen protecting group such as those defined herein below. Compounds of formula **(IV')** can be prepared by an analogous procedure.

Compounds of formula **(V)** may be prepared according to *Schema 3*: 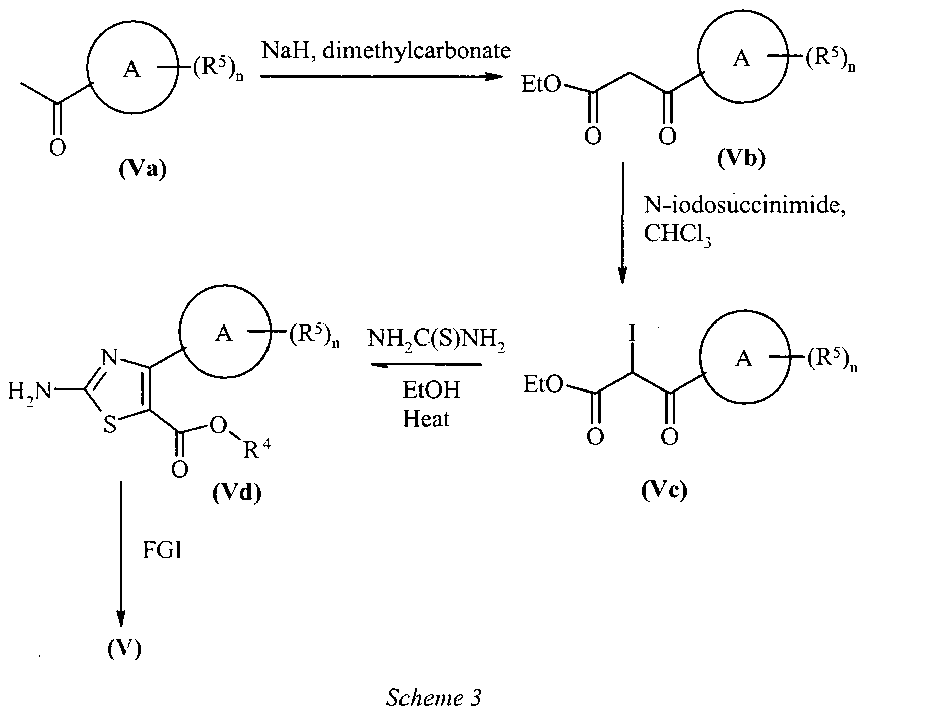 Wherein FGI is functional group interconversion of the NH₂ group to the required "L". *Process c*) Compounds of formula **(VI)** or **(VI')** and **(VII)** in a suitable solvent such as methanol, ethanol, or tetrahydrofuran in the presence of a base such as sodium hydroxide, lithium hydroxide, or barium hydroxide are reacted at a temperature range of 25 to 100 °C.

Compounds of formula **(VI)** or **(VI')** may be prepared by a suitable modification of the reactions described herein to make a compound of formula **(I)** or **(Ia)** wherein R⁴ is hydrogen.

Compounds of formula **(VII)** are commercially available compounds, or they are known in the literature, or they are prepared by standard processes known in the art.

### Process d) Suitable deprotection conditions are described hereinbelow.

Compounds of formula **(VIII)** or **(VIII')** may be prepared by a suitable modification of the reactions described herein to make a compound of formula **(I)** or **(Ia)**.

The formation of a pharnlaceutically-acceptable salt is within the skill of an ordinary organic chemist using standard techniques.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. The reagents used to introduce such ring substituents are either commercially available or are made by processes known in the art.

Introduction of substituents into a ring may convert one compound of the formula **(I)** or **(Ia)** into another compound of the formula **(I)** or **(Ia)**. Such reactions and modifications include, for example, introduction of a substituent by means of an aromatic substitution reaction, reduction of substituents, alkylation of substituents, oxidation of substituents, esterification of substituents, amidation of substituents, formation of heteroaryl rings. The reagents and reaction conditions for such procedures are well known in the chemical art. Particular examples of aromatic substitution reactions include the introduction of alkoxides, diazotization reactions followed by introduction of thiol group, alcohol group, halogen group. Examples of modifications include; oxidation of alkylthio to alkylsulphinyl or alkylsulphonyl.

The skilled organic chemist will be able to use and adapt the information contained and referenced within the above references, and accompanying Examples therein and also the Examples herein, to obtain necessary starting materials, and products. If not commercially available, the necessary starting materials for the procedures such as those described above may be made by procedures which are selected from standard organic chemical techniques, techniques which are analogous to the synthesis of known, structurally similar compounds, or techniques which are analogous to the above described procedure or the procedures described in the examples. It is noted that many of the starting materials for synthetic methods as described above arc commercially available and/or widely reported in the scientific literature, or could be made from commercially available compounds using adaptations of processes reported in the scientific literature. The reader is further referred to Advanced Organic Chemistry, 4th Edition, by Jerry March, published by John Wiley & Sons 1992, for general guidance on reaction conditions and reagents.

It will also be appreciated that in some of the reactions mentioned herein it may be necessary/desirable to protect any sensitive groups in compounds. The instances where protection is necessary or desirable are known to those skilled in the art, as are suitable methods for such protection. Conventional protecting groups may be used in accordance with standard practice (for illustration see T.W. Greene, Protective Groups in Organic Synthesis, John Wiley and Sons, 1991).

Examples of a suitable protecting group for a hydroxy group is, for example, an acyl group, for example an alkanoyl group such as acetyl, an aroyl group, for example benzoyl, a silyl group such as trimethylsilyl or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively a silyl group such as trimethylsilyl may be removed, for example, by fluoride or by aqueous acid; or an arylmethyl group such as a benzyl group may be removed, for example, by hydrogenation in the presence of a catalyst such as palladium-on-carbon.

A suitable protecting group for an amino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for examples, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine or 2-hydroxyethylamine, or with hydrazine.

A suitable protecting group for a carboxy group is, for example, an esterifying group, for example a methyl or an ethyl group which may be removed, for example, by hydrolysis with a base such as sodium hydroxide, or for example a t-butyl group which may be removed, for example, by treatment with an acid, for example an organic acid such as trifluoroacetic acid, or for example a benzyl group which may be removed, for example, by hydrogenation over a catalyst such as palladium-on-carbon, or for example, an allyl group which may be removed, for example, by use of a palladium catalyst such as palladium acetate.

The protecting groups may be removed at any convenient stage in the synthesis using conventional techniques well known in the chemical art, or they may be removed during a later reaction step or work-up.

Optically active forms of a compound of the invention may be obtained by carrying out one of the above procedures using an optically active starting material (formed, for example, by asymmetric induction of a suitable reaction step), or by resolution of a racemic form of the compound or intermediate using a standard procedure, or by chromatographic separation of diastereoisomers (when produced). Enzymatic techniques may also be useful for the preparation of optically active compounds and/or intermediates.

Similarly, when a pure regioisomer of a compound of the invention is required, it may be obtained by carrying out one of the above procedures using a pure regioisomer as a starting material, or by resolution of a mixture of the regioisomers or intermediates using a standard procedure.

### Enzyme Potency Testing Methods

*E.coli* GyrB ATPase Inhibition Activity: Compounds can be tested for inhibition of *E. coli* GyrB ATPase activity using an ammonium molybdate/malachite green-based phosphate detection assay (Lanzetta, P. A., L. J. Alvarez, P. S. Reinach, and O. A. Candia, 1979, 100: 95-97). Assays can be performed in multiwell plates in 30µl reactions containing: 50 mM Hepes buffer pH 7.5, 75 mM ammonium acetate, 8.0 mM magnesium chloride, 0.5 mM ethylenediaminetetraacetic acid, 5% glycerol, 1 mM 1,4-Dithio-DL-threitol, 200 nM bovine serum albumin, 1.6 µg/ml sheared salmon sperm DNA, 400 pM *E. coli* GyrA, 400 pM *E. coli* GyrB, 250 µM ATP, and compound in dimethylsulfoxide. Reactions can be quenched with 30 µl of ammonium molybdate/malachite green detection reagent containing 1.2 mM malachite green hydrochloride, 8.5 mM ammonium molybdate tetrahydrate, and 1 M hydrochloric acid. Plates can be read in an absorbance plate reader at 650 nm and percent inhibition values are calculated using dimethylsulfoxide (2%)-containing reactions as 0% inhibition and EDTA-containing (2.4 µM) reactions as 100% inhibition controls. An IC₅₀ measurement of compound potency for each compound can be determined from reactions performed in the presence of 10 different compound concentrations.

*E*. *coli* Topoisomerase IV ATPase Inhibition Activity: Compounds can be tested for inhibition of *E*. *coli* topoisomerase IV ATPase activity as described above for *E*. *coli* GyrB except the 30µl reactions contained the following: 20 mM TRIS buffer pH 8, 50 mM ammonium acetate, 8 mM magnesium chloride, 5% glycerol, 5 mM 1,4-Dithio-DL-threitol, 0.005% Brij-35, 5 µg/ml sheared salmon sperm DNA, 500 pM *E. coli* ParC, 500 pM *E. coli* ParE, 160 µM ATP, and compound in dimethylsulfoxide. An IC₅₀ measurement of compound potency for each compound can be determined from reactions performed in the presence of 10 different compound concentrations.

Compounds of the invention were tested in an assay substantially similar to the assay described above for measuring the inhibition of *E.coli* GyrB ATPase and *E. coli* Topoisomerase IV ATPase. In general, the compounds of the invention have IC₅₀ values of <200µg/ml in one or both assays.

*S. aureus* GyrB ATPase Inhibition Activity: Compounds may be tested for inhibition of *S. aureus* GyrB ATPase activity using an ammonium molybdate/malachite green-based phosphate detection assay (Lanzetta, P. A., L. J. Alvarez, P. S. Reinach, and O. A. Candia, 1979, 100: 95-97). Assays can be performed in multiwell plates in 30µl reactions containing: 50 mM Hepes buffer pH 7.5, 75 mM ammonium acetate, 8.0 mM magnesium chloride, 0.5 mM ethylenediaminetetraacetic acid, 5% glycerol, 1.0 mM 1,4-Dithio-DL-threitol, 200 nM bovine serum albumin, 1.0 µg/ml sheared salmon sperm DNA, 250 pM *E. coli* GyrA, 250 pM *S. aureus* GyrB, 250 µM ATP, and compound in dimethylsulfoxide. Reactions can be quenched with 30 µl of ammonium molybdate/malachite green detection reagent containing 1.2 mM malachite green hydrochloride, 8.5 mM ammonium molybdate tetrahydrate, and 1 M hydrochloric acid. Plates can be read in an absorbance plate reader at 650 nm and percent inhibition values can be calculated using dimethylsulfoxide (2%)-containing reactions as 0% inhibition and EDTA-containing (2.4 µM) reactions as 100% inhibition controls. An IC₅₀ measurement of compound potency for each compound can be determined from reactions performed in the presence of 10 different compound concentrations.

Compounds of the invention were tested in an assay substantially similar to the assay described above for measuring the inhibition of *S. aureus* GyrB ATPase. Percent inhibition of *S. aureus* GyrB ATPase at a compound concentration of 1 µM (unless otherwise noted) is disclosed in the following table:

| Compound # | % Inhibition |
|---|---|
| 1 | 99.2 |
| 2 | 101.0 |
| 3 | 98.6 |
| 4 | 97.7 |
| 5 | 99.4 |
| 6 | 99.9 |
| 7 | 97.1 |
| 8 | 105.9 |
| 9 | 102.1 |
| 10 | 101.3 |
| 11 | 92.0 |
| 12 | 104.9 |
| 13 | 100.7 |
| 14 | 98.3 |
| 15 | 100.7 |
| 16 | 100.5 |
| 17 | 97.7 |
| 18 | 100.6 |
| 19 | 101.9 |
| 20 | 98.6 |
| 21 | 99.1 |
| 22 | 101.0 |
| 23 | 100.4 |
| 24 | 65.9 |
| 25 | 98.3 |
| 26 | 97.4 |
| 27 | 99.9 |
| 28 | 87.1 |
| 29 | 101.0 |
| 30 | 101.8 |
| 31 | 99.3 |
| 32 | 99.9 |
| 33 | 101.3 |
| 34 | 99.6 |
| 35 | 96.6 |
| 36 | 99.6 |
| 37 | 101.0 |
| 38 | 97.3 |
| 39 | 100.7 |
| 40 | 100.7 |
| 41 | 103.7 |
| 42 | 99.9 |
| 43 | 102.0 |
| 44 | 99.6 |
| 45 | 100.3 |
| 46 | 101.5 |
| 47 | 100.6 |
| 48 | 100.2 |
| 49 | 112.5 |
| 50 | 97.0 |
| 51 | No data available |
| 52 | 101.1 |
| 53 | 100.4 |
| 54 | 97.8 |
| 55 | 97.9 |
| 56 | 93.9 |
| 57 | No data available |
| 58 | 99.5 |
| 59 | 100.0 |
| 60 | 103.6 |
| 61 | 94.7 |
| 62 | 99.9 |
| 63 | 99.8 |
| 64 | 96.2 |
| 65 | 98.3 |
| 66 | 100.6 |
| 67 | 75.2 |
| 68 | 97.0 |
| 69 | 100.1 |
| 70 | 99.5 |
| 71 | 101.1 |
| 72 | 101.2 |
| 73 | 99.9 |
| 74 | 100.5 |
| 75 | 93.1 |
| 76 | 99.2 |
| 77 | 100.4 |
| 78 | 100.8 |
| 79 | 100.7 |
| 80 | 99.7 |
| 81 | 100.5 |
| 82 | 102.9 |
| 83 | 102.0 |
| 84 | 102.7 |
| 85 | 102.1 |
| 86 | 99.9 |
| 87 | 71.0 |
| 88 | No data available |
| 89 | No data available |
| 90 | 100.7 |
| 91 | 96.5 |
| 92 | 100.3 |
| 93 | 99.3 |
| 94 | 106.2 |
| 95 | No data available |
| 96 | 90.2 |
| 97 | 91.5 |
| 98 | 84.4 |
| 99 | 101.9 |
| 100 | 111.7 |
| 101 | 93.1 |
| 102 | 81.4 |
| 103 | 85.7 |
| 104 | 86.4 |
| 105 | 103.3 |
| 106 | 100.9 |
| 107 | No data available |
| 108 | 86.6 |
| 109 | 101.0 |
| 110 | No data available |
| 111 | 104.9 |
| 112 | 81.1 |
| 113 | 92.9 |
| 114 | 82.9 |
| 115 | 87.5 |
| 116 | 101.1 |
| 117 | 108.5 |
| 118 | 92.1 |
| 119 | 79.4 |
| 120 | 103.5 |
| 121 | 103.2 |
| 122 | 102.9 |
| 123 | 103.5 |
| 124 | 103.7 |
| 125 | 103.4 |
| 126 | No data available |
| 127 | No data available |
| 128 | No data available |
| 129 | No data available |
| 130 | No data available |
| 131 | No data available |
| 132 | No data available |
| 133 | No data available |
| 134 | No data available |
| 135 | No data available |
| 136 | No data available |
| 137 | No data available |
| 138 | No data available |
| 139 | No data available |
| 140 | No data available |
| 141 | 116.0 |
| 142 | No data available |
| 143 | 98.8 |
| 144 | 113.9 |
| 145 | 112.6 |
| 146 | 115.9 |
| 147 | No data available |
| 148 | 99.9 |
| 149 | 103.3 |
| 150 | 101.6 |
| 151 | 114.8 |
| 152 | No data available |
| 153 | 98.6 |
| 154 | 112.0 |
| 155 | 109.6 |
| 156 | No data available |
| 157 | No data available |
| 158 | No data available |
| 159 | 31.0 |
| 160 | 4.5 |
| 161 | 14.0* |
| 162 | No data available |
| 163 | No data available |
| 164 | No data available |
| 165 | No data available |
| 166 | No data available |
| 167 | No data available |
| 168 | No data available |
| 169 | No data available |
| 170 | No data available |
| 171 | No data available |
| 172 | No data available |
| 173 | No data available |

| | |
|---|---|
| *Note: Compound was tested at a concentration of 5 µM. | |

### Bacterial Susceptibility Testing Methods

Compounds may be tested for antimicrobial activity by susceptibility testing in liquid media. Compounds may be dissolved in dimethylsulfoxide and tested in 10 doubling dilutions in the susceptibility assays. The organisms used in the assay may be grown overnight on suitable agar media and then suspended in a liquid medium appropriate for the growth of the organism. The suspension can be a 0.5 McFarland and a further 1 in 10 dilution can be made into the same liquid medium to prepare the final organism suspension in 100 µL. Plates can be incubated under appropriate conditions at 37 °C for 24 hrs prior to reading. The Minimum Inhibitory Concentration (MIC) may be determined as the lowest drug concentration able to reduce growth by 80% or more.

In an assay comparable to the above, Example 11 had an MIC of 0.06 µg/ml against *Streptococcus pneumoniae.*

According to a further feature of the invention there is provided a compound of the formula **(I)** or **(Ia)**, or a pharmaceutically-acceptable salt thereof for use in a method of treatment of the human or animal body by therapy.

We have found that compounds of the present invention inhibit bacterial DNA gyrase and / or topoisomerase IV and are therefore of interest for their antibacterial effects. In one aspect of the invention the compounds of the invention inhibit bacterial DNA gyrase and are therefore of interest for their antibacterial effects. In one aspect of the invention, the compounds of the invention inhibit topoisomerase IV and are therefore of interest for their antibacterial effects. In one aspect of the invention the compounds of the invention inhibit both DNA gyrase and topoisomerase IV and are therefore of interest for their antibacterial effects.

It is expected that the compounds of the present invention will be useful in treating bacterial infections. In one aspect of the invention "infection" or "bacterial infection" refers to a gynecological infection. In one aspect of the invention "infection" or "bacterial infection" refers to a respiratory tract infection (RTI). In one aspect of the invention "infection" or "bacterial infection" refers to a sexually transmitted disease. In one aspect of the invention "infection" or "bacterial infection" refers to a urinary tract infection. In one aspect of the invention "infection" or "bacterial infection" refers to acute exacerbation of chronic bronchitis (ACEB). In one aspect of the invention "infection" or "bacterial infection" refers to acute otitis media. In one aspect of the invention "infection" or "bacterial infection" refers to acute sinusitis. In one aspect of the invention "infection" or "bacterial infection" refers to an infection caused by drug resistant bacteria. In one aspect of the invention "infection" or "bacterial infection" refers to catheter-related sepsis. In one aspect of the invention "infection" or "bacterial infection" refers to chancroid. In one aspect of the invention "infection" or "bacterial infection" refers to chlamydia. In one aspect of the invention "infection" or "bacterial infection" refers to community-acquired pneumoniae (CAP). In one aspect of the invention "infection" or "bacterial infection" refers to complicated skin and skin structure infection. In one aspect of the invention "infection" or "bacterial infection" refers to uncomplicated skin and skin structure infection. In one aspect of the invention "infection" or "bacterial infection" refers to endocarditis. In one aspect of the invention "infection" or "bacterial infection" refers to febrile neutropenia. In one aspect of the invention "infection" or "bacterial infection" refers to gonococcal cervicitis. In one aspect of the invention "infection" or "bacterial infection" refers to gonococcal urethritis. In one aspect of the invention "infection" or "bacterial infection" refers to hospital-acquired pneumonia (HAP). In one aspect of the invention "infection" or "bacterial infection" refers to osteomyelitis. In one aspect of the invention "infection" or "bacterial infection" refers to sepsis. In one aspect of the invention "infection" or "bacterial infection" refers to syphilis.

In one aspect of the invention an "infection" or "bacterial infection" refers to an infection caused by *Acinetobacter baumanii.* In one aspect of the invention an "infection" or "bacterial infection" refers to an infection caused by *Acinetobacter haemolyticus.* In one aspect of the invention an "infection" or "bacterial infection" refers to an infection caused by *Acinetobacter junii.* In one aspect of the invention an "infection" or "bacterial infection" refers to an infection caused by *Acinetobacter johnsonii.* In one aspect of the invention an "infection" or "bacterial infection" refers to an infection caused by *Acinetobacter lwoffi.* In one aspect of the invention an "infection" or "bacterial infection" refers to an infection caused by *Bacteroides bivius.* In one aspect of the invention an "infection" or "bacterial infection" refers to an infection caused by *Bacteroides fragilis.* In one aspect of the invention an "infection" or "bacterial infection" refers to an infection caused by *Burkholderia cepacia.* In one aspect of the invention an "infection" or "bacterial infection" refers to an infection caused by *Campylobacter jejuni.* In one aspect of the invention an "infection" or "bacterial infection" refers to an infection caused by *Chlamydia pneumoniae.* In one aspect of the invention an "infection" or "bacterial infection" refers to an infection caused by *Chlamydia urealyticus.* In one aspect of the invention an "infection" or "bacterial infection" refers to an infection caused by *Chlamydophila pneumoniae.* In one aspect of the invention an "infection" or "bacterial infection" refers to an infection caused by *Clostridium difficili.* In one aspect of the invention an "infection" or "bacterial infection" refers to an infection caused by *Enterobacter aerogenes.* In one aspect of the invention an "infection" or "bacterial infection" refers to an infection caused by *Enterobacter cloacae.* In one aspect of the invention an "infection" or "bacterial infection" refers to an infection caused by *Enterococcus faecalis.* In one aspect of the invention an "infection" or "bacterial infection" refers to an infection caused by *Enterococcus faecium.* In one aspect of the invention an "infection" or "bacterial infection" refers to an infection caused by *Escherichia coli.* In one aspect of the invention an "infection" or "bacterial infection" refers to an infection caused by *Gardnerella vaginalis.* In one aspect of the invention an "infection" or "bacterial infection" refers to an infection caused by *Haemophilus parainfluenzae.* In one aspect of the invention an "infection" or "bacterial infection" refers to an infection caused by *Haemophilus influenzae.* In one aspect of the invention an "infection" or "bacterial infection" refers to an infection caused by *Helicobacter pylori.* In one aspect of the invention an "infection" or "bacterial infection" refers to an infection caused by *Klebsiella pneumoniae.* In one aspect of the invention an "infection" or "bacterial infection" refers to an infection caused by *Legionella pneumophila.* In one aspect of the invention an "infection" or "bacterial infection" refers to an infection caused by Methicillin-resistant *Staphylococcus aureus.* In one aspect of the invention an "infection" or "bacterial infection" refers to an infection caused by Methicillin-susceptible *Staphylococcus aureus.* In one aspect of the invention an "infection" or "bacterial infection" refers to an infection caused by *Moraxella catarrhalis.* In one aspect of the invention an "infection" or "bacterial infection" refers to an infection caused by *Morganella morganii.* In one aspect of the invention an "infection" or "bacterial infection" refers to an infection caused by *Mycoplasma pneumoniae.* In one aspect of the invention an "infection" or "bacterial infection" refers to an infection caused by *Neisseria gonorrhoeae.* In one aspect of the invention an "infection" or "bacterial infection" refers to an infection caused by Penicillin-resistant *Streptococcus pneumoniae.* In one aspect of the invention an "infection" or "bacterial infection" refers to an infection caused by Penicillin-susceptible *Streptococcus pneumoniae.* In one aspect of the invention an "infection" or "bacterial infection" refers to an infection caused by *Peptostreptococcus magnus.* In one aspect of the invention an "infection" or "bacterial infection" refers to an infection caused by *Peptostreptococcus micros.* In one aspect of the invention an "infection" or "bacterial infection" refers to an infection caused by *Peptostreptococcus anaerobius.* In one aspect of the invention an "infection" or "bacterial infection" refers to an infection caused by *Peptostreptococcus asaccharolyticus.* In one aspect of the invention an "infection" or "bacterial infection" refers to an infection caused by *Peptostreptococcus prevotii.* In one aspect of the invention an "infection" or "bacterial infection" refers to an infection caused by *Peptostreptococcus tetradius.* In one aspect of the invention an "infection" or "bacterial infection" refers to an infection caused by *Peptostreptococcus vaginalis.* In one aspect of the invention an "infection" or "bacterial infection" refers to an infection caused by *Proteus mirabilis.* In one aspect of the invention an "infection" or "bacterial infection" refers to an infection caused by *Pseudomonas aeruginosa.* In one aspect of the invention an "infection" or "bacterial infection" refers to an infection caused by Quinolone-Resistant *Staphylococcus aureus.* In one aspect of the invention an "infection" or "bacterial infection" refers to an infection caused by Quinolone-Resistant *Staphylococcus epidermis.* In one aspect of the invention an "infection" or "bacterial infection" refers to an infection caused by *Salmonella typhi.* In one aspect of the invention an "infection" or "bacterial infection" refers to an infection caused by *Salmonella paratyphi.* In one aspect of the invention an "infection" or "bacterial infection" refers to an infection caused by *Salmonella enteritidis.* In one aspect of the invention an "infection" or "bacterial infection" refers to an infection caused by *Salmonella typhimurium.* In one aspect of the invention an "infection" or "bacterial infection" refers to an infection caused by *Serratia macescens.* In one aspect of the invention an "infection" or "bacterial infection" refers to an infection caused by *Staphylococcus aureus.* In one aspect of the invention an "infection" or "bacterial infection" refers to an infection caused by *Staphylococcus epidermidis.* In one aspect of the invention an "infection" or "bacterial infection" refers to an infection caused by *Staphylococcus saprophyticus.* In one aspect of the invention an "infection" or "bacterial infection" refers to an infection caused by *Streptoccocus agalactiae.* In one aspect of the invention an "infection" or "bacterial infection" refers to an infection caused by *Streptococcus agalactiae.* In one aspect of the invention an "infection" or "bacterial infection" refers to an infection caused by *Streptococcus pneumoniae.* In one aspect of the invention an "infection" or "bacterial infection" refers to an infection caused by *Streptococcus pyogenes.* In one aspect of the invention an "infection" or "bacterial infection" refers to an infection caused by *Stenotrophomonas maltophilia.* In one aspect of the invention an "infection" or "bacterial infection" refers to an infection caused by *Ureaplasma urealyticum.* In one aspect of the invention an "infection" or "bacterial infection" refers to an infection caused by Vancomycin-Resistant *Enterococcus faecium.* In one aspect of the invention an "infection" or "bacterial infection" refers to an infection caused by Vancomycin-Resistant *Enterococcus faecalis.* In one aspect of the invention an "infection" or "bacterial infection" refers to an infection caused by Vancomycin-Resistant *Staphylococcus aureus.* In one aspect of the invention an "infection" or "bacterial infection" refers to an infection caused by Vancomycin-Resistant *Staphylococcus epidermis.*

In one aspect of the invention an "infection" or "bacterial infection" refers to an infection caused by Acinetobacter spp.. In one aspect of the invention an "infection" or "bacterial infection" refers to an infection caused by Bacteroides spp.. In one aspect of the invention an "infection" or "bacterial infection" refers to an infection caused by Burkholderia spp.. In one aspect of the invention an "infection" or "bacterial infection" refers to an infection caused by Campylobacter spp.. In one aspect of the invention an "infection" or "bacterial infection" refers to an infection caused by Chlamydia spp.. In one aspect of the invention an "infection" or "bacterial infection" refers to an infection caused by Chlamydophila spp.. In one aspect of the invention an "infection" or "bacterial infection" refers to an infection caused by Clostridium spp.. In one aspect of the invention an "infection" or "bacterial infection" refers to an infection caused by Enterobacter spp.. In one aspect of the invention an "infection" or "bacterial infection" refers to an infection caused by Enterococcus spp.. In one aspect of the invention an "infection" or "bacterial infection" refers to an infection caused by Escherichia spp.. In one aspect of the invention an "infection" or "bacterial infection" refers to an infection caused by Gardnerella spp.. In one aspect of the invention an "infection" or "bacterial infection" refers to an infection caused by Haemophilus spp.. In one aspect of the invention an "infection" or "bacterial infection" refers to an infection caused by Helicobacter spp.. In one aspect of the invention an "infection" or "bacterial infection" refers to an infection caused by Klebsiella spp.. In one aspect of the invention an "infection" or "bacterial infection" refers to an infection caused by Legionella spp.. In one aspect of the invention an "infection" or "bacterial infection" refers to an infection caused by Moraxella spp.. In one aspect of the invention an "infection" or "bacterial infection" refers to an infection caused by Morganella spp.. In one aspect of the invention an "infection" or "bacterial infection" refers to an infection caused by Mycoplasma spp.. In one aspect of the invention an "infection" or "bacterial infection" refers to an infection caused by Neisseria spp.. In one aspect of the invention an "infection" or "bacterial infection" refers to an infection caused by Peptostreptococcus spp.. In one aspect of the invention an "infection" or **bacterial infection" refers to an infection caused by Proteus spp.. In one aspect of the invention an "infection" or "bacterial infection" refers to an infection caused by Pseudomonas spp.. In one aspect of the invention an "infection" or "bacterial infection" refers to an infection caused by Salmonella spp.. In one aspect of the invention an "infection" or "bacterial infection" refers to an infection caused by Serratia spp.. In one aspect of the invention an "infection" or "bacterial infection" refers to an infection caused by Staphylococcus spp.. In one aspect of the invention an "infection" or "bacterial infection" refers to an infection caused by Streptoccocus spp.. In one aspect of the invention an "infection" or "bacterial infection" refers to an infection caused by Stenotrophomonas spp.. In one aspect of the invention an "infection" or "bacterial infection" refers to an infection caused by Ureaplasma spp.. In one aspect of the invention an "infection" or "bacterial infection" refers to an infection caused by aerobes. In one aspect of the invention an "infection" or "bacterial infection" refers to an infection caused by obligate anaerobes. In one aspect of the invention an "infection" or "bacterial infection" refers to an infection caused by facultative anaerobes. In one aspect of the invention an "infection" or "bacterial infection" refers to an infection caused by gram-positive bacteria. In one aspect of the invention an "infection" or "bacterial infection" refers to an infection caused by gram-negative bacteria. In one aspect of the invention an "infection" or "bacterial infection" refers to an infection caused by gram-variable bacteria. In one aspect of the invention an "infection" or "bacterial infection" refers to an infection caused by atypical respiratory pathogens.

According to a further feature of the present invention there is provided a method for producing an antibacterial effect in a warm blooded animal, such as man, in need of such treatment, which comprises administering to said animal an effective amount of a compound of the present invention, or a pharmaceutically-acceptable salt thereof.

According to a further feature of the invention there is provided a method for inhibition of bacterial DNA gyrase and / or topoisomerase IV in a warm-blooded animal, such as a human being, in need of such treatment which comprises administering to said animal an effective amount of a compound of formula **(I)** or **(Ia)** or a pharmaceutically acceptable salt thereof as defined hereinbefore.

According to a further feature of the invention there is provided a method of treating a bacterial infection in a warm-blooded animal, such as a human being, in need of such treatment which comprises administering to said animal an effective amount of a compound of formula **(I)** or **(Ia)** or a pharmaceutically acceptable salt thereof as defined hereinbefore.

According to a further feature of the invention there is provided a method of treating a bacterial infection selected from a gynecological infection, a respiratory tract infection (RTI), a sexually transmitted disease, a urinary tract infection, acute exacerbation of chronic bronchitis (ACEB), acute otitis media , acute sinusitis, an infection caused by drug resistant bacteria, catheter-related sepsis, chancroid, chlamydia, community-acquired pneumoniae (CAP), complicated skin and skin structure infection, uncomplicated skin and skin structure infection, endocarditis, febrile neutropenia, gonococcal cervicitis, gonococcal urethritis, hospital-acquired pneumonia (HAP), osteomyelitis, sepsis and /or syphilis in a warm-blooded animal, such as a human being, in need of such treatment which comprises administering to said animal an effective amount of a compound of formula **(I)** or **(Ia)** or a pharmaceutically acceptable salt thereof as defined hereinbefore.

A further feature of the present invention is a compound of formula **(I)** or **(Ia)** and pharmaceutically acceptable salts thereof for use as a medicament. Suitably the medicament is an antibacterial agent.

According to a further aspect of the invention there is provided the use of a compound of formula **(I)** or **(Ia),** or a pharmaceutically acceptable salt thereof in the manufacture of a medicament for the production of an anti-bacterial effect in a warm-blooded animal such as a human being.

According to a further aspect of the invention there is provided the use of a compound of formula **(I)** or **(Ia),** or a pharmaceutically acceptable salt thereof in the manufacture of a medicament for the inhibition of bacterial DNA gyrase and / or topoisomerase IV in a warm-blooded animal such as a human being.

Thus according to a further aspect of the invention there is provided the use of a compound of formula **(I)** or **(Ia),** or a pharmaceutically acceptable salt thereof in the manufacture of a medicament for the treatment of a bacterial infection in a warm-blooded animal such as a human being.

Thus according to a further aspect of the invention there is provided the use of a compound of formula **(I)** or **(Ia),** or a pharmaceutically acceptable salt thereof in the manufacture of a medicament for the treatment of a bacterial infection selected from a gynecological infection, a respiratory tract infection (RTI), a sexually transmitted disease, a urinary tract infection, acute exacerbation of chronic bronchitis (ACEB), acute otitis media , acute sinusitis, an infection caused by drug resistant bacteria, catheter-related sepsis, chancroid, chlamydia, community-acquired pneumoniae (CAP), complicated skin and skin structure infection, uncomplicated skin and skin structure infection, endocarditis, febrile neutropenia, gonococcal cervicitis, gonococcal urethritis, hospital-acquired pneumonia (HAP), osteomyelitis, sepsis and / or syphilis in a warm-blooded animal such as a human being.

According to a further aspect of the invention there is provided a compound of formula **(I)** or **(Ia),** or a pharmaceutically acceptable salt thereof for use in the production of an anti-bacterial effect in a warm-blooded animal such as a human being.

According to a further aspect of the invention there is provided a compound of formula **(I)** or **(Ia),** or a pharmaceutically acceptable salt thereof for use in inhibition of bacterial DNA gyrase and / or topoisomerase IV in a warm-blooded animal such as a human being.

Thus according to a further aspect of the invention there is provided a compound of formula **(I)** or **(Ia),** or a pharmaceutically acceptable salt thereof for use in the treatment of a bacterial infection in a warm-blooded animal such as a human being.

Thus according to a further aspect of the invention there is provided a compound of formula **(I)** or **(Ia)**, or a pharmaceutically acceptable salt thereof for use in the treatment of a bacterial infection selected from a gynecological infection, a respiratory tract infection (RTI), a sexually transmitted disease, a urinary tract infection, acute exacerbation of chronic bronchitis (ACEB), acute otitis media , acute sinusitis, an infection caused by drug resistant bacteria, catheter-related sepsis, chancroid, chlamydia, community-acquired pneumoniae (CAP), complicated skin and skin structure infection, uncomplicated skin and skin structure infection, endocarditis, febrile neutropenia, gonococcal cervicitis, gonococcal urethritis, hospital-acquired pneumonia (HAP), osteomyelitis, sepsis and / or syphilis in a warm-blooded animal such as a human being.

In order to use a compound of the formula **(I)** or **(Ia)** or a pharmaceutically-acceptable salt thereof, for the therapeutic (including prophylactic) treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula **(I)** or **(Ia)** or a pharmaceutically-acccptable salt thereof, and a pharmaceutically-acceptable diluent or carrier.

According to a further aspect of the invention there is provided a pharmaceutical composition which comprises a compound of formula **(I)** or **(Ia)** as defined hereinbefore or a pharmaceutically acceptable salt thereof, in association with a pharmaceutically acceptable excipient or carrier for use in producing an anti-bacterial effect in a warm-blooded animal, such as a human being.

According to a further aspect of the invention there is provided a pharmaceutical composition which comprises a compound of formula **(I)** or **(Ia)** as defined hereinbefore or a pharmaceutically acceptable salt thereof, in association with a pharmaceutically acceptable excipient or carrier for use in inhibition of bacterial DNA gyrase and / or topoisomerase IV in a warm-blooded animal, such as a human being.

According to a further aspect of the invention there is provided a pharmaceutical composition which comprises a compound of formula **(I)** or **(Ia)** as defined hereinbefore or a pharmaceutically acceptable salt thereof, in association with a pharmaceutically acceptable excipient or carrier for use in the treatment of a bacterial infection in a warm-blooded animal, such as a human being.

According to a further aspect of the invention there is provided a pharmaceutical composition which comprises a compound of formula **(I)** or **(Ia)** as defined hereinbefore or a pharmaceutically acceptable salt thereof, in association with a pharmaceutically acceptable excipient or carrier for use in the treatment of a gynecological infection, a respiratory tract infection (RTI), a sexually transmitted disease, a urinary tract infection, acute exacerbation of chronic bronchitis (ACEB), acute otitis media, acute sinusitis, an infection caused by drug resistant bacteria, catheter-related sepsis, chancroid, chlamydia, community-acquired pneumoniae (CAP), complicated skin and skin structure infection, uncomplicated skin and skin structure infection, endocarditis, febrile neutropenia, gonococcal cervicitis, gonococcal urethritis, hospital-acquired pneumonia (HAP), osteomyelitis, sepsis and/or syphilis in a warm-blooded animal, such as a human being.

The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing).

The compositions of the invention may be obtained by conventional procedures using conventional pharmaceutical excipients, well known in the art. Thus, compositions intended for oral use may contain, for example, one or more colouring, sweetening, flavouring and/or preservative agents.

Suitable pharmaceutically acceptable excipients for a tablet formulation include, for example, inert diluents such as lactose, sodium carbonate, calcium phosphate or calcium carbonate, granulating and disintegrating agents such as corn starch or algenic acid; binding agents such as starch; lubricating agents such as magnesium stearate, stearic acid or talc; preservative agents such as ethyl or propyl p-hydroxybenzoate, and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate or kaolin, or as soft gelatin capsules in which the active ingredient is mixed with water or an oil such as peanut oil, liquid paraffin, or olive oil.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with long chain aliphatic alcohols, for example heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as ethyl or propyl p-hydroxybenzoate, anti-oxidants (such as ascorbic acid), colouring agents, flavouring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavouring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavouring and colouring agents, may also be present.

The pharmaceutical compositions of the invention may also be in the form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavouring and preservative agents.

Syrups and elixirs may be formulated with sweetening agents such as glycerol, propylene glycol, sorbitol, aspartame or sucrose, and may also contain a demulcent, preservative, flavouring and/or colouring agent.

The pharmaceutical compositions may also be in the form of a sterile injectable aqueous or oily suspension, which may be formulated according to known procedures using one or more of the appropriate dispersing or wetting agents and suspending agents, which have been mentioned above. A sterile injectable preparation may also be a sterile injectable solution or suspension in a non-toxic parenterally-acceptable diluent or solvent, for example a solution in 1,3-butanediol.

Compositions for administration by inhalation may be in the form of a conventional pressurised aerosol arranged to dispense the active ingredient either as an aerosol containing finely divided solid or liquid droplets. Conventional aerosol propellants such as volatile fluorinated hydrocarbons or hydrocarbons may be used and the aerosol device is conveniently arranged to dispense a metered quantity of active ingredient.

For further information on formulation the reader is referred to Chapter 25.2 in Volume 5 of Comprehensive Medicinal Chemistry (Corwin Hansch; Chairman of Editorial Board), Pergamon Press 1990.

The amount of active ingredient that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition. Dosage unit forms will generally contain about 1 mg to about 500 mg of an active ingredient. For further information on Routes of Administration and Dosage Regimes the reader is referred to Chapter 25.3 in Volume 5 or Comprehensive Medicinal Chemistry (Corwin Hansch; Chairman of Editorial Board), Pergamon Press 1990.

As stated above the size of the dose required for the therapeutic or prophylactic treatment of a particular disease state will necessarily be varied depending on the host treated, the route of administration and the severity of the illness being treated. In one aspect of the invention a daily dose in the range of 1-50 mg/kg is employed. However the daily dose will necessarily be varied depending upon the host treated, the particular route of administration, and the severity of the illness being treated. Accordingly the optimum dosage may be determined by the practitioner who is treating any particular patient.

In addition to its use in therapeutic medicine, compounds of formula **(I)** or **(Ia)** and their pharmaceutically acceptable salts are also useful as pharmacological tools in the development and standardisation of in-vitro and in-vi*vo* test systems for the evaluation of the effects of inhibitors of DNA gyrase and / or topoisomerase IV in laboratory animals such as cats, dogs, rabbits, monkeys, rats and mice, as part of the search for new therapeutic agents.

In the above other, pharmaceutical composition, process, method, use and medicament manufacture features, the alternative and particular embodiments of the compounds of the invention described herein also apply.

### Combinations

The compounds of the invention described herein may be applied as a sole therapy or may involve, in addition to a compound of the invention, one or more other substances and/or treatments. Such conjoint treatment may be achieved by way of the simultaneous, sequential or separate administration of the individual components of the treatment. Where the administration is sequential or separate, the delay in administering the second component should not be such as to lose the beneficial effect of the combination. Suitable classes and substances may be selected from one or more of the following:
i) other antibacterial agents for example macrolides e.g. erythromycin, azithromycin or clarithromycin: quinolones e.g. ciprofloxacin or levofloxacin; β-lactams e.g. penicillins e.g. amoxicillin or piperacillin; cephalosporins e.g. ceftriaxone or ceftazidime; carbapenems, e.g. meropenem or imipenem etc; aminoglycosides e.g. gentamicin or tobramycin; or oxazolidinones; and/or
ii) anti-infective agents for example, an antifungal triazole e.g. or amphotericin; and/or
iii) biological protein therapeutics for example antibodies, cytokines, bactericidal/permeability-increasing protein (BPI) products; and/or
iv) efflux pump inhibitors.

Therefore, in a further aspect of the invention there is provided a compound of the formula **(I)** or **(Ia),** or a pharmaceutically acceptable salt thereof and a chemotherapeutic agent selected from:
i) one or more additional antibacterial agents; and/or
ii) one or more anti-infective agents; and/or
iii) biological protein therapeutics for example antibodies, cytokines, bactericidal/permeability-increasing protein (BPI) products; and/or
iv) one or more efflux pump inhibitors.

### Examples

The invention is now illustrated but not limited by the following Examples in which unless otherwise stated :-
(i) evaporations were carried out by rotary evaporation in-vacuo and work-up procedures were carried out after removal of residual solids by filtration;
(ii) operations were generally carried out at ambient temperature, that is typically in the range 18-26 °C and without exclusion of air unless otherwise stated, or unless the skilled person would otherwise work under an inert atmosphere;
(iii) column chromatography (by the flash procedure) was used to purify compounds and was performed on Merck Kieselgel silica (Art. 9385) unless otherwise stated;
(iv) yields arc given for illustration only and are not necessarily the maximum attainable;
(v) the structure of the end-products of the invention were generally confirmed by NMR and mass spectral techniques; proton magnetic resonance spectra is quoted and was generally determined in DMSO-d₆ unless otherwise stated using a Bruker DRX-300 spectrometer operating at a field strength of 300 MHz. Chemical shifts are reported in parts per million downfield from tetramethysilane as an internal standard (δ scale) and peak multiplicities are shown thus: s, singlet; d, doublet; AB or dd, doublet of doublets; dt, doublet of triplets; dm, doublet of multiplets; t, triplet, m, multiplet; br, broad;
(vi) fast-atom bombardment (FAB) mass spectral data were generally obtained using a Platform spectrometer (supplied by Micromass) run in electrospray and, where appropriate, either positive ion data or negative ion data were collected or using Agilent 1100series LC/MSD equipped with Sedex 75ELSD, run in atmospheric pressure chemical ionisation mode and, where appropriate, either positive ion data or negative ion data were collected; mass spectra were run with an electron energy of 70 electron volts in the chemical ionization (CI) mode using a direct exposure probe; where indicated ionization was effected by electron impact (EI), fast atom bombardment (FAB) or electrospray (ES); values for m/z are given; generally, only ions which indicate the parent mass are reported;
(vii) each intermediate was generally purified to the standard required for the subsequent stage and was characterised in sufficient detail to confirm that the assigned structure was correct; purity was assessed by high pressure liquid chromatography, thin layer chromatography, or NMR and identity was determined by infra-red spectroscopy (IR), mass spectroscopy or NMR spectroscopy as appropriate;
(vii) the following abbreviations may be used:
   DMF is *N,N*-dimethylformamide;
   SM is starting material;
   DMSO is dimethylsulfoxide;
   CDCl₃ is deuterated chloroform;
   MS is mass spectroscopy;
   EtOAc is ethyl acetate;
   THF is tetrahydrofuran;
   MeOH is methanol;
   TFA is trifluoroacetic acid;
   EtOH is ethanol;
   DCM is dichloromethane;
   HATU is N-[(dimethylamino)-1H,2,3-triazolo[4,5-b-]pyridin-1-ylmethylene]-N-methylmethanaminium hexafluorophosphate N-oxide;
   DIEA is diisopropyl ethyl amine; and
(viii) temperatures are quoted as °C.

### Example 1

### 2-((3S,4R)-4-{[3,4-Dichloro-5-methyl-1H-pyrrol-2-yl)carbonyl]amino}-3-methoxypiperidin-1-yl)-4-pyrimidin-2-yl-1,3-thiazole-5-carboxylic acid

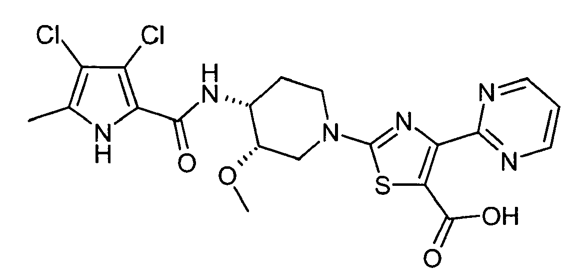

To a suspension of ethyl 2-((3*S*,4*R*)-4-{[(3,4-dichloro-5-methyl-1*H*-pyrrol-2-yl)carbonyl]amino}-3-methoxypiperidin-1-yl)-4-pyrimidin-2-yl-1,3-thiazole-5-carboxylate (Example 43; 0.04 g, 0.074 mmol) in MeOH was added lithium hydroxide (0.11 ml, 0.22 mmol, 2N). The reaction was heated in the microwave for 30 mins at 100 °C. The crude reaction was diluted with water and acidified with 1N HCl. The resulting precipitate was filtered, washed with water, and dried (0.021 g). MS (ES) (M+H)⁺: 511 for C₂₀H₂₀Cl₂N₆O₄S; NMR: 1.75 (m, 2H), 2.17 (s, 3H), 3.38 (s, 3H), 4.04 (s, 1H), 4.26 (m, 2H), 7.18 (d, 1H), 7.63 (d, 1H), 8.98 (s, 1H), 12.15 (s, 1H).

### Examples 2-42

The following Examples were prepared by the procedure described in Example 1 from the starting materials (SM) indicated.

| **Ex** | **Compound** | **Data** | **SM** |
|---|---|---|---|
| **2** | 2-((3S,4R)-4-{[(3,4-Dichloro-5-methyl-1H-pyrrol-2-yl)carbonyl]amino}-3-methoxypiperidin-1-yl)-4-pyrimidin-4-yl-1,3-thiazole-5-carboxylic acid | MS (ES) (M+H)⁺: 511 for C₂₀H₂₀Cl₂N₆O₄S | Example 81 |
| | | NMR: 1.72 (m, 2H), 2.12 (s, 3H), 3.27 (m, 3H), 3.33 (s, 3H), 3.42 (s, 1H), 3.53 (m, 1H), 4.24 (m, 1H), 7.09 (d, 1H), 8.21 (d, 1H), 9.07 (d, 1H), 9.36 (s, 1H), 12.10 (s, 1H) | |
| | 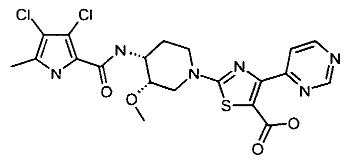 | | |
| **3** | 2'-((3S,4R)-4-{[(3,4-Dichloro-5-methyl-1H-pyrro1-2-yl)carbonyl]amino}-3-methoxypiperidin-1-yl)-2,4'-bi-1,3-thiazole-5'-carboxylic acid | MS (ES) (M+H)⁺: 516 for C₁₉H₁₉Cl₂N₅O₄S₂ | Example 44 |
| | | NMR: 1.72 (m, 2H), 2.12 (s, 3H), 3.32 (m, 1H), 3.38 (s, 3H), 3.54 (s, 1H), 3.86 (m, 1H), 4.25 (m, 1H), 7.18 (d, 1H), 7.80 (d, 2H), 12.21 (s, 1H) | |
| | 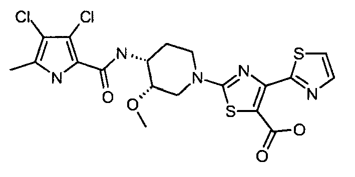 | | |
| **4** | 2-((3S,4R)-4-{[(3,4-Dichloro-5-methyl-1H-pyrrol-2-yl)carbonyl]amino}-3-fluoropiperidin-1-yl)-4-pyrimidin-2-yl-1,3-thiazole-5-carboxylic acid | MS (ES) (M+H)⁺: 499 for C₁₉H₁₇Cl₂FN₆O₃S | Example 45 |
| | | NMR: 1.88 (m, 2H), 2.20 (s, 3H), 3.57 (m, 1H), 4.39 (m, 3H), 4.91 (d, 1H), 7.28 (d, 1H), 7.65 (t, 1H), 9.00 (d, 2H), 12.11 (s, 1H), 15.08 (s, 1H) | |
| | 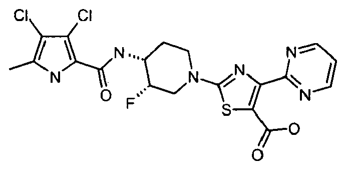 | | |
| **5** | 2-((3S,4R)-4-{[(3,4-Dichloro-5-methyl-1H-pyrrol-2-yl)carbonyl]amino}-3-fluoropiperidin-1-yl)-4-pyrimidin-4-yl-1,3-thiazole-5-carboxylic acid | MS (ES) (M+H)⁺: 499 for C₁₉H₁₇Cl₂FN₆O₃S | Example 46 |
| | | NMR: 1.83 (m, 2H), 2.13 (s, 3H), 3.53 (m, 2H), 4.06 (m, 1H), 4.39 (m, 2H), 4.85 (d, 1H), 7.24 (d, 1H), 8.19 (t, 1H), 9.07 (d, 1H), 9.36 (s, 1H), 12.05 (s, 1H) | |
| | 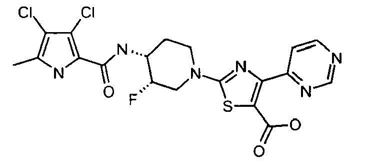 | | |
| **6** | 2'-((3S,4R)-4-{[(3,4-Dichloro-5-methyl-1H-pyrrol-2-yl)carbonyl]amino}-3-fluoropiperidin-1-yl)-2,4'-bi-1,3-thiazole-5'-carboxylic acid | MS (ES) (M+H)⁺: 504 for C₁₈H₁₆Cl₂FN₅O₃S₂ | Example 47 |
| | | NMR: 1.89 (m, 2H), 2.20 (s, 3H), 4.09 (m, 2H), 4.40 (m, 3H), 4.92 (d, 1H), 7.32 (d, 1H), 8.15 (m, 2H), 12.13 (s, 1H) | |
| | 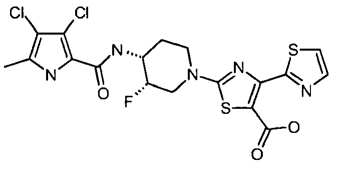 | | |
| **7** | 2-((3S,4R)-4-{[(3,4-Dichloro-5-methyl-1H-pyrrol-2-yl)carbonyl]amino}-3-methoxypiperidin-1-yl)-4-pyridin-2-yl-1,3-thiazole-5-carboxylic acid | MS (ES) (M+H)⁺: 510 for C₂₁H₂₁Cl₂N₅O₄S | Example 48 |
| | | NMR: 1.80 (m, 2H), 2.19 (s, 3H), 3.40 (s, 5H), 3.59 (s, 1H), 4.04 (m, 1H), 4.29 (m, 1H), 4.46 (m, 1H), 7.17 (d, 1H), 7.71 (t, 1H), 8.25 (t, 1H), 8.43 (d, 1H), 8.76 (s, 1H), 12.17 (s, 1H) | |
| | 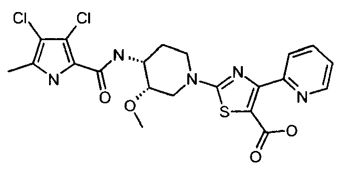 | | |
| **8** | 2-((3S,4R)-4-{[(3,4-Dichloro-5-methyl-1H-pyrrol-2-yl)carbonyl]amino}-3-fluoropiperidin-1-yl)-4-pyridin-2-yl-1,3-thiazole-5-carboxylic acid | MS (ES) (M+H)⁺: 498 for C₂₀H₁₈Cl₂FN₅O₃S | Example 49 |
| | | NMR: 1.90 (m, 2H), 2.20 (s, 3H), 3.40 (s, 1H), 3.59 (m, 1H), 4.12 (m, 1H), 4.45 (m, 2H), 4.93 (d, 1H), 7.29 (d, 1H), 7.72 (t, 1H), 8.26 (t, 1H), 8.43 (d, 1H), 8.76 (s, 1H), 12.11 (s, 1H) | |
| | 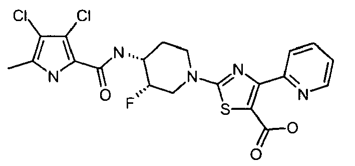 | | |
| **9** | 4-[2,6-Bis(dimethylamino)pyrimidin-4- yl]-2-((3S,4R)-4-{[(3,4-dichloro-5-methyl-1H-pyrrol-2-yl)carbonyl]amino}-3-methoxypiperidin-1-yl)-1,3-thiazole-5-carboxylic acid | MS (ES) (M+H)⁺: 597 for C₂₄H₃₀Cl₂N₈O₄S | Example 50 |
| | | NMR: 1.70 (m, 2H), 2.12 (s, 3H), 3.09 (s, 12H), 3.28 (s, 3H), 3.32 (s, 2H), 3.49 (s, 1H), 3.79 (m, 1H), 4.19 (m, 1H), 4.37 (m, 1H), 6.83 (s, 1H), 7.09 (d, 1H), 12.09 (s, 1H) | |
| | 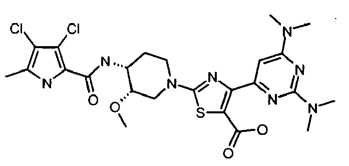 | | |
| **10** | 4-[2,6-Bis(dimethylamino)pyrimidin-4- yl]-2-((3S,4R)-4-{[(3,4-dichloro-5-methyl-1H-pyrrol-2-yl)carbonyl]amino}-3-fluoropipcridin-1-yl)-1,3-thiazole-5-carboxylic acid | MS (ES) (M+H)⁺: 585 for C₂₃H₂₇Cl₂FN₈O₃S | Example 51 |
| | | NMR: 1.88 (m, 2H), 2.20 (s, 3H), 3.35 (s, 12H), 3.51 (m, 1H), 4.05 (m, 1H), 4.38 (m, 2H), 4.89 (d, 1H), 6.90 (s, 1H), 7.28 (d, 1H), 12.12 (s, 1H) | |
| | 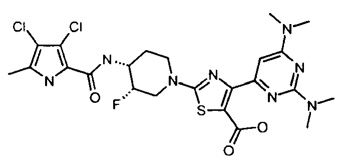 | | |
| **11** | 2-((3S,4R)-4-{[(3,4-Dichloro-5-methyl-1H-pyrrol-2-yl)carbonyl]amino}-3-methoxypiperidin-1-yl)-4-(4,6-dimethoxypyrimidin-2-yl)-1,3-thiazole-5-carboxylic acid | MS (ES) (M+H)⁺: 571 for C₂₂H₂₄Cl₂N₆O₆S | Example 52 |
| | | NMR: 1.70 (m, 2H), 2.12 (s, 3H), 3.27 (s, 6H), 3.49 (m, 1H), 3.84 (s, 6H), 4.22 (m, 2H), 6.29 (s, 1H), 7.09 (d, 1H), 12.10 (s, 1H), 13.80 (s, 1H) | |
| | 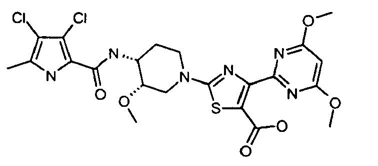 | | |
| **12** | 2-((3S,4R)-4-{[(3,4-Dichloro-5-methyl-1H-pyrrol-2-yl)carbonyl]amino}-3-methoxypiperidin-1-yl)-4-pyrazin-2-yl-1,3-thiazole-5-carboxylic acid | MS (ES) (M+H)⁺: 511 for C₂₀H₂₀Cl₂N₆O₄S | Example 53 |
| | | NMR: 1.78 (m, 2H), 2.17 (s, 3H), 3.33 (s, 3H), 3.39 (s, 3H), 4.01 (m, 1H), 4.26 (m, 1H), 4.27 (m, 1H), 7.15 (d, 1H), 8.77 (s, 1H), 8.83 (s, 1H), 9.34 (s, 1H), 12.16 (s, 1H), 15.29 (s, 1H) | |
| | 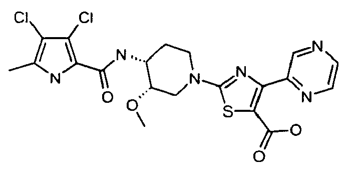 | | |
| **13** | 4-(1,3-Benzothiazol-2-yl)-2-((3S,4R)-4-{[(3,4-dichloro-5-methyl-1H-pyrrol-2-yl)carbonyl]amino}-3-methoxypiperidin-1-yl)-1,3-thiazole-5-carboxylic acid | MS (ES) (M+H)⁺: 566 for C₂₃H₂₁Cl₂N₅O₄S₂ | Example 54 |
| | | NMR: 1.80 (m, 2H), 2.17 (s, 3H), 3.35 (m, 3H), 3.42 (s, 3H), 3.61 (s, 1H), 4.29 (m, 1H), 7.16 (d, 1H), 7.64 (m, 2H), 8.13 (d, 1H), 8.27 (d, 1H), 12.16 (s, 1H), | |
| | 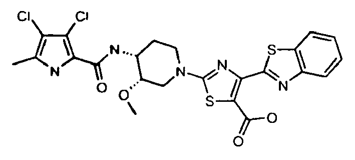 | | |
| **14** | 2-((3S,4R)-4-{[(3,4-Dichloro-5-methyl-1H-pyrrol-2-yl)carbonyl]amino}-3-methoxypiperidin-1-yl)-4-pyrazin-2-yl-1,3-thiazole-5-carboxylic acid | MS (ES) (M+H)⁺: 499 for C₁₉H₁₇Cl₂FN₆O₃S | Example 55 |
| | | NMR: 1.89 (m, 2H), 2.20 (s, 3H), 3.36 (m, 1H), 3.59 (dd, 1H), 4.14 (m, 1H), 4.44 (m, 2H), 4.92 (d, 1H), 7.29 (d, 1H), 8.78 (s, 1H), 8.83 (s, 1H), 9.31 (s, 1H), 12.12 (s, 1H) | |
| | 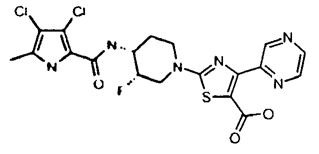 | | |
| **15** | 4-(1,3-Benzothiazol-2-yl)-2-((3S,4R)-4-{[(3,4-dichloro-5-methyl-1H-pyrrol-2-yl)carbonyl]amino}-3-fluoropiperidin-1-yl)-1,3-thiazole-5-carboxylic acid | MS (ES) (M+H)⁺: 554 for C₂₂H₁₈Cl₂FN₅O₃S₂ | Example 56 |
| | | NMR: 1.86 (m, 2H), 2.13 (s, 3H), 3.28 (m, 1H), 3.59 (dd, 1H), 4.08 (m, 1H), 4.37 (m, 2H), 4.89 (d, 1H), 7.23 (d, 1H), 7.58 (m, 2H), 8.08 (d, 2H), 8.21 (d, 1H), 8.83 (s, 1H), 9.31 (s, 1H), 12.05 (s, 1H), 15.99 (s, 1H) | |
| | 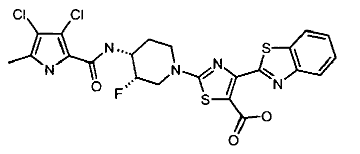 | | |
| **16** | 2-((3S,4R)-4-{[(3,4-Dichloro-5-methyl-1H-pyrrol-2-yl)carbonyl]amino}-3-methoxypiperidin-1-yl)-4-(1-{[2-(trimethylsilyl)ethoxy]methyl}-1H-imidazol-2-yl)-1,3-thiazole-5-carboxylic acid | MS (ES) (M+H)⁺: 630 for C₂₅H₃₄Cl₂N₆O₅SSi | Example 57 |
| | | NMR: -0.13 (s, 9H), 0.82 (m, 2H), 1.84 (m, 2H), 2.23 (s, 3H), 3.43 (m, 4H), 3.61 (m, 2H), 4.05 (m, 1H), 4.30 (m, 2H), 6.04 (m, 2H), 7.15 (d, 1H), 7.43 (s, 1H), 7.76 (s, 1H), 12.27 (s, 1H). | |
| | 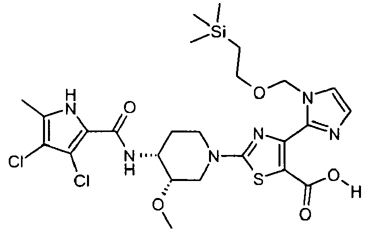 | | |
| **17** | 2-((3S,4R)-4-{[(3,4-Dichloro-5-methyl-1H-pyrrol-2-yl)carbonyl]amino}-3-methoxypiperidin-1-yl)-4-[1-(methoxymethyl)-1H-imidazol-2-yl]-1,3-thiazole-5-carboxylic acid | MS (ES) (M+H)⁺: 543 for C₂₁H₂₄Cl₂N₆O₅S | Example 58 |
| | | NMR: 1.83 (m, 2H), 2.26 (s, 3H), 3.34 (s, 3H), 3.44 (m, 4H), 3.68 (m, 1H), 4.02 (m, 1H), 4.34 (m, 2H), 6.01 (s, 2H), 7.22 (d, 1H), 7.42 (s, 1H), 7.74 (s, 1H), 12.22 (s, 1H). | |
| | 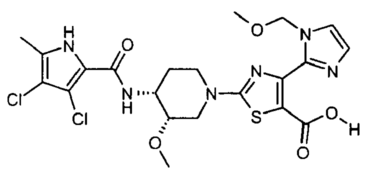 | | |
| **18** | 2-((3S,4R)-4-{[(3,4-Dichloro-5-methyl-1H-pyrrol-2-yl)carbonyl]amino}-3-methoxypiperidin-1-yl)-4-(1H-imidazol-2-yl)-1,3-thiazole-5-carboxylic acid | MS (ES) (M+H)⁺: 499 for C₁₉H₂₀Cl₂N₆O₄S | Example 86 |
| | | NMR: 1.64 (m, 2H), 2.26 (s, 3H), 3.48 (m, 4H), 3.66 (m, 1H), 4.00 (m, 1H), 4.32 (m, 2H), 4.54 (m, 1H), 7.25 (d, 1H), 7.41 (s, 2H), 12.25 (s, 1H). | |
| | 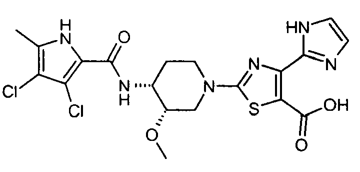 | | |
| **19** | 2-((3S,4R)-4-{[(3,4-Dichloro-5-methyl-1H-pyrrol-2-yl)carbonyl]amino}-3-fluoropiperidin-1-yl)-4-[1-(methoxymethyl)-1H-imidazol-2-yl]-1,3-thiazole-5-carboxylic acid | MS (ES) (M+H)⁺: 531 for C₂₀H₂₁Cl₂FN₆O₄S | Example 59 |
| | | NMR: 1.73 (m, 2H), 2.24 (s, 3H), 3.44 (m, 4H), 3.71 (s, 3H), 3.70 (dd, 1H), 4.03 (m, 1H), 4.44 (m, 2H), 5.05 (d, 1H), 6.02 (s, 2H), 7.31 (d, 1H), 7.43 (s, 1H), 7.72 (s, 2H), 12.13 (s, 1H). | |
| | 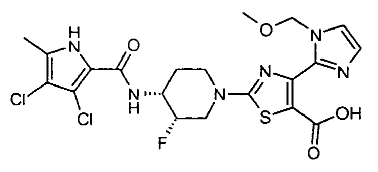 | | |
| **20** | 2-((3S,4R)-4-{[(3,4-Dichloro-5-methyl-1H-pyrrol-2-yl)carbonyl]amino}-3-fluoropiperidin-1-yl)-4-(1H-imidazol-2-yl)-1,3-thiazole-5-carboxylic acid | MS (ES) (M+H)⁺: 487 for C₁₈H₁₇Cl₂FN₆O₃S | Example 87 |
| | | NMR: 1.91 (m, 2H), 2.21 (s, 3H), 3.15-3.82 (m, 4H), 4.14 (m, 1H), 4.23-4.44 (m, 2H), 5.02 (d, 1H), 7.32 (d, 1H), 7.45 (s, 2H), 12.11 (s, 1H). | |
| | 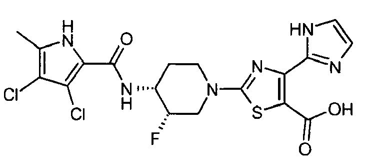 | | |
| **21** | 2-((3S,4R)-4-{[(3,4-Dichloro-5-methyl-1H-pyrrol-2-yl)carbonyl]amino}-3-methoxypiperidin-1-yl)-4-(1-methyl-1H-imidazol-2-yl)-1,3-thiazole-5-carboxylic acid | MS (ES) (M+H)⁺: 513 for C₂₀H₂₂Cl₂N₆O₄S | Example 88 |
| | | NMR: 1.82 (m, 2H), 2.22 (s, 3H), 3.42 (s, 3H), 3.63 (m, 1H), 4.03 (m, 1H), 4.12 (s, 3H), 4.31 (m, 2H), 7.24 (d, 1H), 7.34 (s, 1H), 7.53 (s, 1H), 12.22 (s, 1H). | |
| | 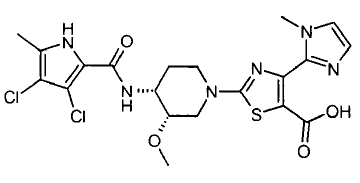 | | |
| **22** | 2-[5-Carboxy-2-((3S,4R)-4-{[(3,4-dichloro-5-methyl-1H-pyrrol-2-yl)carbonyl]amino}-3-methoxypiperidin-1-yl)-1,3-thiazol-4-yl]-1,3-dimethyl-1H-imidazol-3-ium trifluoroacetate | MS (ES) (M+H)⁺: 527 for C₂₁H₂₅Cl₂N₆O₄S | Example 89 |
| | | NMR: 1.81 (m, 2H), 2.24 (s, 3H), 3.42 (m, 4H), 3.62 (m, 1H), 3.73 (s, 6H), 4.01 (m, 3H), 4.34 (m, 2H), 7.27 (d, 1H), 7.95 (s, 2H), 12.24 (s, 1H). | |
| | 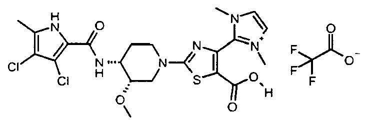 | | |
| **23** | 2-((3S,4R)-4-{[(3,4-Dichloro-5-methyl-1H-pyrrol-2-yl)carbonyl]amino}-3-methoxypiperidin-1-yl)-4-(1H-pyrazol-5-yl)-1,3-thiazole-5-carboxylic acid | MS (ES) (M+H)⁺: 499 for C₁₉H₂₀Cl₂N₆O₄S | Example 62 |
| | | NMR: 1.82 (m, 2H), 2.22 (s, 3H), 3.34 (m, 4H), 3.62 (m, 1H), 4.16 (m, 1H), 4.32 (m, 1H), 4.54 (m, 1H), 7.21 (d, 1H), 7.70 (s, 1H), 7.96 (s, 1H), 12.23 (s, 1H), 13.75 (s, 1H). | |
| | 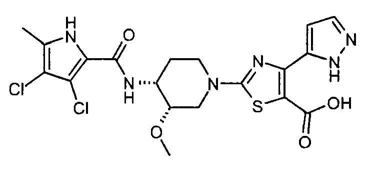 | | |
| **24** | 2-((3S,4R)-4-{[(3,4-Dichloro-5-methyl-1H-pyrrol-2-yl)carbonyl]amino}-3-fluoropiperidin-1-yl)-4-(1H-pyrazol-5-yl)-1,3-thiazole-5-carboxylic acid | MS (ES) (M+H)⁺: 487 for C₁₈H₁₇Cl₂FN₆O₃S | Example 63 |
| | | NMR: 1.91 (m, 2H), 2.22 (s, 3H), 3.64 (dd, 1H), 3.74 (m, 1H), 4.35 (m, 1H), 4.43 (m, 1H), 5.03 (d, 1H), 6.85 (s, 1H), 7.34 (d, 1H), 8.02 (s, 1H), 12.10 (s, 1H), 13.83 (s, 1H). | |
| | 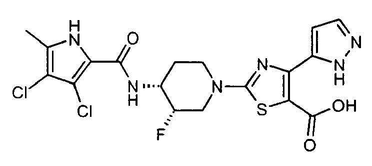 | | |
| **25** | 2-((3S,4R)-4-{[(3,4-Dichloro-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylic acid | MS (ES) (M+H)⁺: 557 for C₂₂H₂₆Cl₂N₆O₅S | Example 64 |
| | | NMR: 1.84 (m, 2H), 2.21 (s, 3H), 3.22 (s, 3H), 3.43 (m, 4H), 3.62 (m, 1H), 3.74 (m, 2H), 4.03 (m, 1H), 4.35 (m, 2H), 4.82 (m, 2H), 7.26 (d, 1H), 7.30 (s, 1H), 7.54 (s, 1H), 12.22 (s, 1H). | |
| | 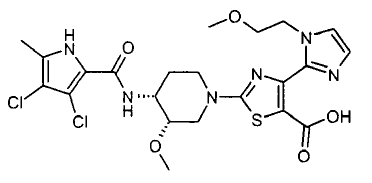 | | |
| **26** | 2-((3*S*,4*R*)-4-{[(3,4-Dichloro-5-methyl-1*H*-pyrrol-2-yl)carbonyl]amino}-3-fluoropiperidin-1-yl)-4-[1-(2-methoxyethyl)-1*H*-imidazol-2-yl]-1,3-thiazole-5-carboxylic acid | MS (ES) (M+H)⁺: 545 for C₂₁H₂₃Cl₂FN₆O₄S | Example 65 |
| | | NMR: 1.92 (m, 2H), 2.24 (s, 3H), 3.22 (s, 3H), 3.53 (m, 1H), 3.65 (m, 1H), 3.73 (s, 3H), 4.04 (m, 1H), 4.35 (m, 2H), 4.82 (m, 2H), 5.01 (d, 1H), 7.31 (m, 2H), 7.34 (s, 1H), 7.54 (s, 1H), 12.10 (s, 1H). | |
| | 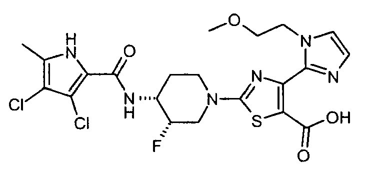 | | |
| **27** | 2-((3S,4R)-4-{[(3,4-Dichloro-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylic acid | MS (ES) (M+H)⁺: 514 for C₁₉H₂₁Cl₂N₇O₄S | Example 66 |
| | | NMR: 1.82 (m, 2H), 2.23 (s, 3H), 3.33-3.42 (m, 4H), 3.61 (m, 1H), 4.04 (m, 1H), 4.14 (s, 3H), 4.34 (m, 2H), 7.23 (d, 1H), 8.25 (s, 1H), 12.21 (s, 1H). 15.45, (s, 1H). | |
| | 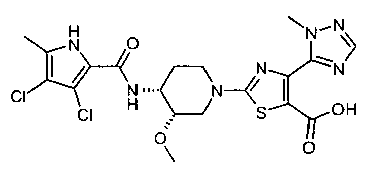 | | |
| **28** | 2-((3S,4R)-4-{[(3,4-Dichloro-5-methyl-1H-pyrrol-2-yl)carbonyl]amino}-3-fluoropiperidin-1-yl)-4-(1-methyl-1H-1,2,4-triazol-5-yl)-1,3-thiazole-5-carboxylic acid | MS (ES) (M+H)⁺: 502 for C₁₈H₁₈Cl₂FN₇O₃S | Example 67 |
| | | NMR: 1.91 (m, 2H), 2.22 (s, 3H), 3.32-3.45 (m, 4H), 3.61 (dd, 1H), 4.11 (m, 4H), 4.32-4.51 (m, 2H), 5.00 (d, 1H), 7.35 (d, 1H), 8.22 (s, 1H), 12.13 (s, 1H). | |
| | 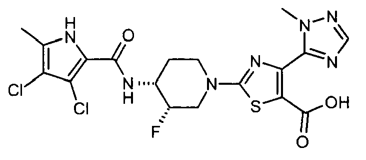 | | |
| **29** | 2-((3S,4R)-4-{[(3-Chloro-4-cyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylic acid | MS (ES) (M+H)⁺: 505 for C₂₀H₂₁ClN₈O₄S | Example 80 |
| | | NMR: 1.82 (m, 2H), 2.32 (s, 3H), 3.34-3.35 (m, 4H), 3.61 (m, 1H), 4.02 (m, 1H), 4.11 (s, 3H), 4.33 (m, 2H), 7.35 (d, 1H), 8.25 (s, 1H), 12.72 (s, 1H), 15.42 (s, 1H). | |
| | 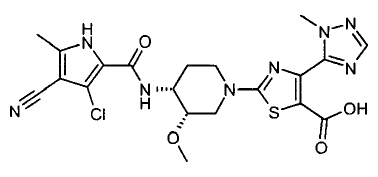 | | |
| **30** | 2-((3S,4R)-4-{[(3-Chloro-4-cyano-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylic acid | MS (ES) (M+H)⁺: 549 for C₂₃H₂₆ClN₇O₅S | Example 69 |
| | | NMR: 1.82 (m, 2H), 2.33 (s, 3H), 3.22 (s, 3H), 3.34 (m, 4H), 3.63 (m, 1H), 3.73(m, 2H), 3.95 (m, 1H), 4.33 (m, 2H), 4.82 (m, 2H), 7.31 (m, 2H), 7.51 (s, 1H), 12.70 (s, 1H). | |
| | 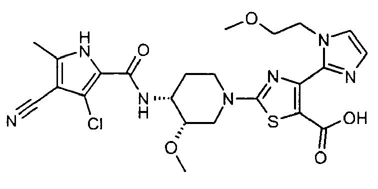 | | |
| **31** | 2-((3S,4R)-4-{[(4-Cyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylic acid | MS (ES) (M+H)⁺: 471 for C₂₀H₂₂N₈O₄S | Example 68 |
| | | NMR: 1.57 (m, 1H), 1.83 (m, 1H), 2.34 (s, 3H), 3.34 (m, 4H), 3.62 (m, 1H), 4.04 (m, 1H), 4.09 (s, 3H), 4.23-4.34 (m, 2H), 7.20 (s, 1H), 8.03 (d, 1H), 8.25 (s, 1H), 12.29 (s, 1H), 15.43 (s, 1H). | |
| | 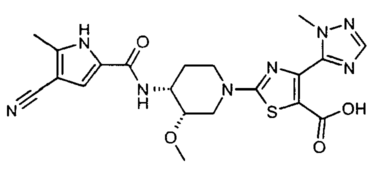 | | |
| **32** | 2-(3S,4R)-4-{[(3,4-Dichloro-5-methyl-1H-pyrrol-2-yl)carbonyl]amino}-3-methoxypiperidin-1-yl)-4-(1,4,5-trimethyl-1H-imidazol-2-yl)-1,3-thiazole-5-carboxylic acid | MS (ES) (M+H)⁺: 541 for C₂₂H₂₆Cl₂N₆O₄S | Example 70 |
| | | NMR: 1.81 (m, 2H), 2.24 (s, 9H), 3.34 (m, 4H), 3.64 (m, 1H), 3.65 (s, 3H), 4.01 (m, 1H), 4.05 (s, 3H), 4.28 (m, 2H), 7.17 (d, 1H), 12.23 (s, 1H). | |
| | 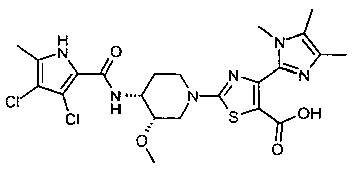 | | |
| **33** | 2'-Amino-2-((3S,4R)-4-{[(3,4-dichloro-5-methyl-1H-pyrrol-2-yl)carbonyl]amino}-3-methoxypiperidin-1-yl)-4,4'-bi-1,3- thiazole-5-carboxylic acid | MS (ES) (M+H)⁺: 529 for C₁₉H₂₀Cl₂N₆O₄S₂ | Example 71 |
| | | NMR: 1.78 (m, 2H), 2.21 (s, 3H), 3.33 (m, 4H), 3.51 (m, 1H), 3.67 (s, 3H), 4.02 (m, 1H), 4.28 (m, 2H), 7.15 (d, 1H), 7.25 (s; 2H), 8.11 (s, 2H), 12.15 (s, 1H). | |
| | 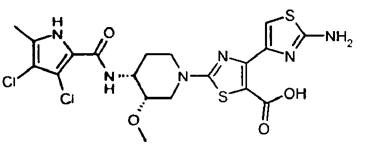 | | |
| **34** | 2-((3S,4R)-4-{[(3,4-Dichloro-5-methyl-1H-pyrrol-2-yl)carbonyl]amino}-3-methoxypiperdin-1-yl)-2'-(methylamino)-4,4'-bi-1,3-thiazole-5-carboxylic acid | MS (ES) (M+H)⁺: 543 for C₂₀H₂₂Cl₂N₆O₄S₂ | Example 72 |
| | | NMR: 1.78 (m, 2H), 2.17 (s, 3H), 2.81 (s, 3H), 3.22-3.44 (m, 4H), 3.51 (m, 1H), 3.88 (m, 1H), 4.22 (m, 2H), 7.22 (m, 1H), 7.51 (m, 1H). | |
| | 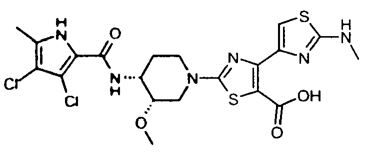 | | |
| **35** | 2-((3S,4R)-4-{[(3,4-Dichloro-5-methyl-1H-pyrrol-2-yl)carbonyl]amino}-3-methoxypiperidin-1-yl)-2'-(dimethylamino)-4,4'-bi-1,3-thiazole-5-carboxylic acid | MS (ES) (M+H)⁺: 560 for C₂₁H₂₄Cl₂N₆O₄S₂ | Example 73 |
| | | NMR: 1.75 (m, 2H), 2.21 (s, 3H), 3.16 (s, 6H), 3.28-3.43 (m, 4H), 3.55 (m, 1H), 3.95 (m, 1H), 4.32 (m, 2H), 7.18 (d, 1H), 7.43 (s, 1H), 12.21 (s, 1H). | |
| | 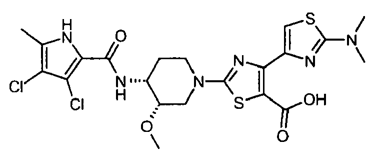 | | |
| **36** | 2'-Amino-2-((3S,4R)-4-{[(3,4-dichloro-5-methyl-1H-pyrrol-2-yl)carbonyl]amino}-3-fluoropiperidin-1-yl)-4,4'-bi-1,3-thiazole-5-carboxylic acid | MS (ES) (M+H)⁺: 519 for C₁₈H₁₇Cl₂FN₆O₃S₂ | Example 74 |
| | | NMR: 1.81 (m, 2H), 2.21 (s, 3H), 3.15 (d, 1H), 3.35 (m, 4H), 3.86 (m, 1H), 4.22 (m, 2H), 4.91 (d, 1H), 7.02 (m, 1H), 7.64 (m, 2H). | |
| | 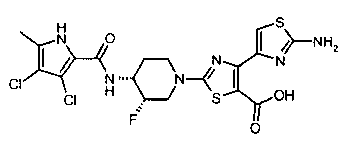 | | |
| **37** | 2-((3S,4R)-4-{[(3,4-Dichloro-5-methyl-1H-pyrrol-2-yl)carbonyl]amino}-3-(luoropipcridin-1-yl)-2'-(mcthylamino)-4,4'-bi-1,3-thiazole-5-carboxylic acid | MS (ES) (M+H)⁺: 533 for C₁₉H₁₉Cl₂FN₆O₃S₂ | Example 75 |
| | | NMR: 1.81 (m, 2H), 2.22 (s, 3H), 2.92 (m, 3H), 3.32 (m, 4H), 3.88 (m, 1H), 4.29 (m, 2H), 4.91 (d, 1H), 7.25-7.46 (m, 2H). | |
| | 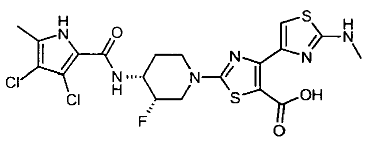 | | |
| **38** | 2-((3S,4R)-4-{[(3,4-Dichloro-5-methyl-1H-pyrrol-2-yl)carbonyl]amino}-3-fluoropiperidin-1-yl)-2'-(dimethylamino)-4,4'-bi-1,3-thiazole-5-carboxylic acid | MS (ES) (M+H)⁺: 547 for C₂₀H₂₁Cl₂FN₆O₃S₂ | Example 76 |
| | | NMR: 1.91 (m, 2H), 2.22 (s, 3H), 3.14 (s, 6H), 3.34 (m, 4H), 3.61 (dd, 1H), 4.08 (m, 1H), 4.42 (m, 2H), 5.01 (d, 1H), 7.27 (d, 1H), 7.39 (s, 1H), 12.14 (s, 1H). | |
| | 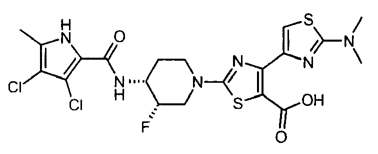 | | |
| **39** | 2-((3S,4R)-4-{[(3,4-Dichloro-5-methyl-1H-pyrrol-2-yl)carbonyl]amino}-3-fluoropiperidin-1-yl)-4-(1-methyl-1H-imidazol-2-yl)-1,3-thiazole-5-carboxylic acid | MS (ES) (M+H)⁺: 501 for C₁₉H₁₉Cl₂FN₆O₃S | Example 79 |
| | | NMR: 1.91 (m, 2H), 2.22 (s, 3H), 3.23 (m, 4H), 3.58 (m, 1H), 4.10 (m, 4H), 4.42 (m, 2H), 5.01 (d, 1H), 7.29 (m, 2H), 7.50 (s, 1H), 12.14 (s, 1H). | |
| | 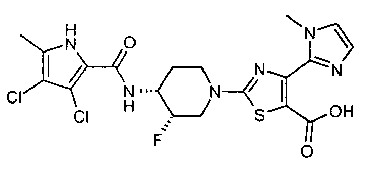 | | |
| **40** | 2-((3S,4R)-4-{[(3,4-Dichloro-5-methyl-1H-pyrrol-2-yl)carbonyl]amino}-3-methoxypiperidin-1-yl)-4-(1-methyl-1H-imidazol-4-yl)-1,3-thiazole-5-carboxylic acid | MS (ES) (M+H)⁺: 513 for C₂₀H₂₂Cl₂N₆O₄S | Example 77 |
| | | NMR: 1.81 (m, 2H), 2.22 (s, 3H), 3.41 (m, 4H), 3.62 (m, 1H), 3.77 (s, 3H), 4.02 (m, 1H), 4.18-4.41 (m, 2H), 7.23 (m, 1H), 7.81 (s, 1H), 8.10 (s, 1H), 12.27 (s, 1H). | |
| | 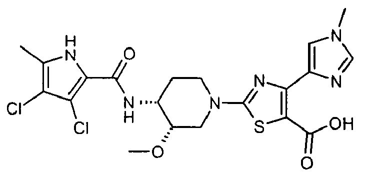 | | |
| **41** | 2-((3S,4R)-4-{[(3,4-Dichloro-5-methyl-1H-pyrrol-2-yl)carbonyl]amino}-3-fluoropiperidin-1-yl)-4-(1-methyl-1H-imidazol-4-yl)-1,3-thiazole-5-carboxylic acid | MS (ES) (M+H)⁺: 502 for C₂₁H₂₃Cl₂FN₆O₃S | Example 78 |
| | | NMR: 1.91 (m, 2H), 2.22 (s, 3H), 3.34 (m, 4H), 3.57 (m, 1H), 3.81 (s, 3H), 4.04 (m, 1H), 4.25-4.41 (m, 2H), 5.01 (d, 1H), 7.28 (d, 1H), 7.79(s, 1H), 8.20 (s, 1H), 12.13 (s, 1H). | |
| | 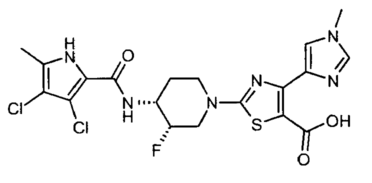 | | |
| **42** | 2-((3*S*,4*R*)-4-{[(3,4-Dichloro-5-methyl-1*H*-pyrrol-2-yl)carbonyl]amino}-3-methoxypiperidin-1-yl)-4-(4-methoxypyrimidin-2-yl)-1,3-thiazole-5-carboxylic acid | MS (ES) (M+H)⁺: 542 for C₂₁H₂₂Cl₂N₆O₅S | Example 82 |
| | | NMR: 1.78 (m, 2H), 2.19 (s, 3H), 3.35 (m, 3H), 3.42 (s, 3H), 3.57 (m, 1H), 3.99 (s, 3H), 4.30 (m, 1H), 4.43 (m, 1H), 7.08 (d, 1H), 7.18 (d, 1H), 8.69 (d, 1H), 12.17 (br, 1H). | |
| | 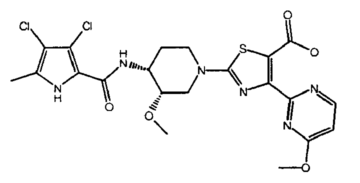 | | |

### Example 43

### Ethyl 2-((3S,4R)-4-{[(3,4-dichloro-5-methyl-1H-pyrrol-2-yl)carbonyl]amino}-3-methoxypiperidin-1-yl)-4-pyrimidin-2-yl-1,3-thiazole-5-carboxylate

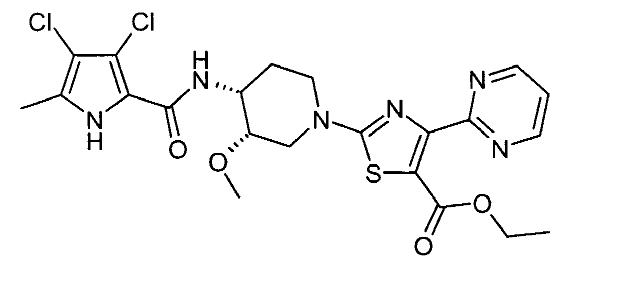

A suspension of 3,4-dichloro-*N*-[(3*S*,4*R*)-3-methoxypiperidin-4-yl]-5-methyl-1*H-*pyrrole-2-carboxamide hydrochloride (WO2006087543, 0.08 g, 0.23 mmol), ethyl 2-chloro-4-pyrimidin-2-yl-1,3-thiazole-5-carboxylate (Intermediate 1; 0.057 g, 0.21 mmol), and solid sodium bicarbonate (0.056 g, 0.69 mmol) in DMF (3 ml) was heated in the microwave for 30 mins at 100 °C. LCMS indicates conversion of starting material to product. The crude reaction mixture was added dropwise to a pH 4 citric acid buffer. The resulting solid was filtered, washed with water and dried (0.057 g). MS (ES) (M+H)⁺: 539 for C₂₂H₂₄Cl₂N6O₄S; NMR: 0.092 (t, 3H), 1.69 (m, 2H), 2.12 (s, 3H), 3.31 (m, 5H), 3.50 (s, 1H), 3.91 (q, 2H), 4.19 (m, 2H), 7.09 (d, 1H), 7.49 (t, 1H), 8.80 (d, 1H), 12.10 (s, 1H)

### Examples 44-85

The following Examples were prepared by the procedure described in Example 43 from the starting materials (SM) indicated.

| **Ex** | **Compound** | **Data** | **SM** |
|---|---|---|---|
| **44** | Methyl 2'-((3S,4R)-4-{[(3,4-dichloro-5-methyl-1H-pyrrol-2-yl)carbonyl]amino}-3-methoxypiperidin-1-yl)-2,4'-bi-1,3-thiazole-5'-carboxylate | MS (ES) (M+H)⁺: 530 for C₂₀H₂₁Cl₂N₅O₄S₂ | 3,4-dichloro-N-[(3S,4R)-3-methoxypiperidi n-4-yl]-5-methyl-1H-pyrrole-2-carboxamide hydrochloride and Intermediate 3 |
| | | NMR: 1.76 (m, 2H), 2.17 (s, 3H), 3.32 (m, 3H), 3.38 (s, 3H), 3.57 (s, 1H), 3.69 (s, 3H), 4.28 (m, 2H), 7.15 (d, 1H), 7.90 (s, 1H), 7.94 (s, 1H), 12.15 (s, 1H) | |
| | 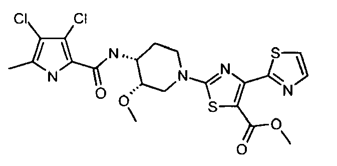 | | |
| **45** | Ethyl 2-((3S,4R)-4-{[(3,4-dichloro-5-methyl-1H-pyrrol-2-yl)carbonyl]amino}-3-fluoropiperidin-1-yl)-4-pyrimidin-2-yl-1,3-thiazole-5-carboxylate | MS (ES) (M+H)⁺: 527 for C₂₁H₂₁Cl₂FN₆O₃S | 3,4-dichloro-N-[(3S,4R)-3-fluoropiperidin-4-yl]-5-methyl-1H-pyrrole-2-carboxamide hydrochloride (WO2006087543 ) and Intermediate 1 |
| | | NMR: 0.98 (t, 3H), 1.86 (m, 2H), 2.18 (s, 3H), 3.57 (m, 1H), 3.97 (m, 3H), 4.33 (m, 2H), 4.88 (d, 1H), 7.29 (d, 1H), 7.55 (t, 1H), 8.86 (d, 2H), 12.10 (s, 1H) | |
| | 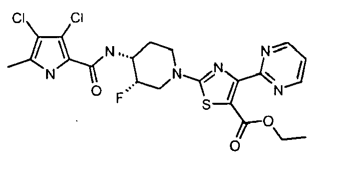 | | |
| **46** | Ethyl 2-((3S,4R)-4-{[(3,4-dichloro-5-methyl-1H-pyrrol-2-yl)carbonyl]amino}-3-fluoropiperidin-1-yl)-4-pyrimidin-4-yl-1,3-thiazole-5-carboxylate | MS (ES) (M+H)⁺: 527 for C₂₁H₂₁Cl₂FN₆O₃S | 3,4-dichloro-N-[(3S,4R)-3-fluoropiperidin-4-yl]-5-methyl-1H-pyrrole-2-carboxamide hydrochloride and Intermediate 2 |
| | | NMR: 1.03 (t, 3H), 1.81 (m, 2H), 2.12 (s, 3H), 3.51 (m, 1H), 3.65 (m, 1H), 4.01 (m, 2H), 4.28 (m, 2H), 4.83 (d, 1H), 7.20 (d, 1H), 7.67 (d, 1H), 8.83 (d, 1H), 9.17 (s, 1H), 12.04 (s, 1H) | |
| | 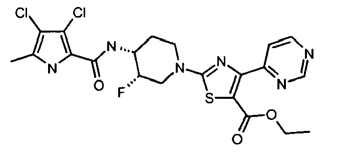 | | |
| **47** | Methyl 2'-((3S,4R)-4-{[(3,4-dichloro-5-methyl-1H-pyrrol-2-yl)carbonyl]amino}-3-fluoropiperidin-1-yl)-2,4'-bi-1,3-thiazole-5'-carboxylate | MS (ES) (M+H)⁺: 518 for C₁₉H₁₈Cl₂FN₅O₃S₂ | 3,4-dichloro-N-[(3S,4R)-3-fluoropiperidin-4-yl]-5-methyl-1H-pyrrole-2-carboxamide hydrochloride and Intermediate 3 |
| | | NMR: 1.82 (m, 2H), 2.13 (s, 3H), 3.65 (s, 3H), 3.96 (m, 1H), 4.29 (m, 2H), 4.85 (d, 1H), 7.22 (d, 1H), 7.86 (d, 2H), 12.04 (s, 1H) | |
| | 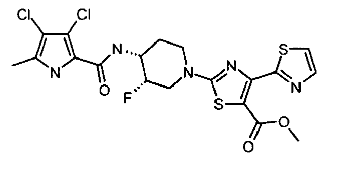 | | |
| **48** | Ethyl 2-((3S,4R)-4-{[(3,4-dichloro-5-methyl-1H-pyrrol-2-yl)carbonyl]amino}-3-methoxypiperidin-1-yl)-4-pyridin-2-yl-1,3-thiazole-5-carboxylate | MS (ES) (M+H)⁺: 538 for C₂₃H₂₅Cl₂N₅O₄S | 3,4-dichloro-N-[(3S,4R)-3-methoxypiperidi n-4-yl]-5-methyl-1H-pyrrole-2-carboxamide hydrochloride and Intermediate 13 |
| | | NMR: 1.06 (t, 3H), 1.76 (m, 2H), 2.19 (s, 3H), 3.29 (m, 2H), 3.39 (s, 4H), 3.56 (m, 1H), 4.03 (q, 2H), 4.26 (m, 2H), 7.16 (d, 1H), 7.40 (dd, 1H), 7.55 (d, 1H), 8.58 (d, 1H), 12.17 (s, 1H) | |
| | 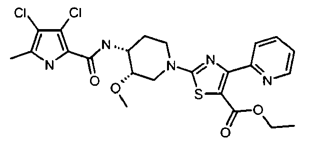 | | |
| **49** | Ethyl 2-((3S,4R)-4-{[(3,4-dichloro-5-methyl-1H-pyrrol-2-yl)carbonyl]amino}-3-fluoropiperidin-1-yl)-4-pyridin-2-yl-1,3-thiazole-5-carboxylate | MS (ES) (M+H)⁺: 526 for C₂₂H₂₂Cl₂FN₅O₃S | 3,4-dichloro-N-[(3S,4R)-3-fluoropiperidin-4-yl]-5-methyl-1H-pyrrole-2-carboxamide hydrochloride and Intermediate 13 |
| | | NMR: 1.07 (t, 3H), 1.87 (m, 2H), 2.19 (s, 3H), 3.32 (m, 2H), 3.61 (m, 1H), 4.04 (q, 2H), 4.35 (m, 2H), 4.89 (d, 1H), 7.28 (d, 1H), 7.43 (dd, 1H), 7.59 (d, 1H), 7.81 (t, 1H), 8.59 (d, 1H), 12.11 (s, 1H) | |
| | 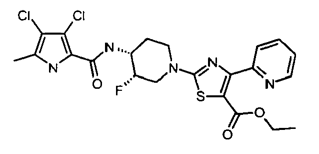 | | |
| **50** | Ethyl 4-[2,6-bis(dimethylamino)pyrimidin-4-yl]-2-((3S,4R)-4-{[(3,4-dichloro-5-methyl-1H-pyrrol-2-yl)carbonyl]amino}-3-methoxypiperidin-1-yl)-1,3-thiazole-5-carboxylate | MS (ES) (M+H)⁺: 625 for C₂₆H₃₄Cl₂N₈O₄S | 3,4-dichloro-N-[(3S,4R)-3-methoxypiperidi n-4-yl]-5-methyl-1 H-pyrrole-2-carboxamide hydrochloride and Intermediate 4 |
| | | NMR: 1.13 (t, 3H), 1.76 (m, 2H), 2.17 (s, 3H), 3.08 (s, 12H), 3.32 (m, 3H), 3.95 (m, 1H), 4.13 (q, 2H), 4.27 (m, 2H), 6.26 (s, 1H), 7.14 (d, 1H), 12.16 (s, 1H) | |
| | 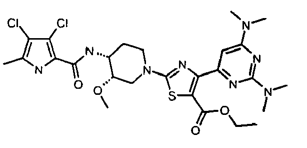 | | |
| **51** | Ethyl 4-[2,6-bis(dimethylamino)pyrimidin-4-yl]-2-((3S,4R)-4-{[(3,4-dichloro-5-methyl-1H-pyrrol-2-yl)carbonyl]amino}-3-fluoropiperidin-1-yl)-1,3-thiazole-5-carboxylate | MS (ES) (M+H)⁺: 613 for C₂₅H₃₁Cl₂FN₈O₃S | 3,4-dichloro-N-[(3S,4R)-3-fluoropiperidin-4-yl]-5-methyl-1H-pyrrole-2-carboxamide hydrochloride and Intermediate 4 |
| | | NMR: 1.07 (t, 3H), 1.79 (m, 2H), 2.13 (s, 3H), 3.00 (s, 12H), 3.27 (m, 1H), 3.48 (m, 1H), 4.03 (q, 2H), 4.19 (m, 2H), 4.83 (d. 1H), 6.03 (s, 1H), 7.22 (d, 1H), 12.05 (s, 1H) | |
| | 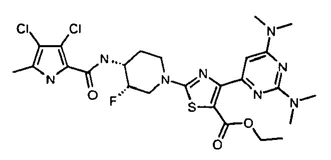 | | |
| **52** | Ethyl 2-((3S,4R)-4-{[(3,4-dichloro-5-methyl-1H-pyrrol-2-yl)carbonyl]amino}-3-methoxypiperidin-1-yl)-4-(4,6-dimethoxypyrimidin-2-yl)-1,3-thiazole-5-carboxylate | MS (ES) (M+H)⁺: 599 for C₂₄H₂₈Cl₂N₆O₆S | 3,4-dichloro-N-[(3S,4R)-3-methoxypiperidin-4-yl]-5-methyl-1H-pyrrole-2-carboxamide hydrochloride and Intermediate 5 |
| | | NMR: 0.96 (t, 3H), 1.70 (m, 2H), 2.12 (s, 3H), 3.27 (s, 6H), 3.79 (m, 6H), 3.95 (q, 2H), 4.22 (m, 2H), 6.23 (s, 1H), 7.09 (d, 1H), 12.10 (s, 1H) | |
| | 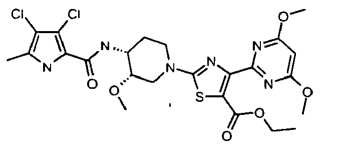 | | |
| **53** | Ethyl 2-((3S,4R)-4-{[(3,4-dichloro-5-methyl-1H-pyrrol-2-yl)carbonyl]amino}-3-methoxypiperidin-1-yl)-4-pyrazin-2-yl-1,3-thiazole-5-carboxylate | MS (ES) (M+H)⁺: 539 for C₂₂H₂₄Cl₂N₆O₄S | 3,4-dichloro-N-[(3S,4R)-3-methoxypiperidin-4-yl]-5-methyl-1H-pyrrole-2-carboxamide hydrochloride and Intermediate 6 |
| | | NMR: 1.08 (t, 3H), 1.77 (m, 2H), 2.19 (s, 3H), 3.33 (s, 3H), 3.39 (s, 3H), 3.57 (s, 1H), 4.06 (q, 2H), 4.11 (m, 2H), 7.16 (d, 1H), 8.67 (d, 2H), 8.84 (s, 1H), 12.16 (s, 1H) | |
| | 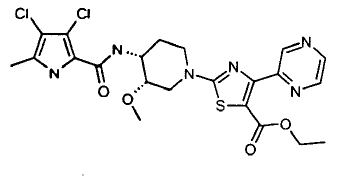 | | |
| **54** | Ethyl 4-(1,3-benzothiazol-2-yl)-2-((3S,4R)-4-{[(3,4-dichloro-5-methyl-1H-pyrrol-2-yl)carbonyl]amino}-3-methoxypiperidin-1-yl)-1,3-thiazole-5-carboxylate | MS (ES) (M+H)⁺: 594 for C₂₅H₂₅Cl₂N₅O₄S₂ | 3,4-dichloro-N-[(3S,4R)-3-methoxypiperidin-4-yl]-5-methyl-1H-pyrrole-2-carboxamide hydrochloride and Intermediate 7 |
| | | NMR: 1.17 (t, 3H), 1.78 (m, 2H), 2.17 (s, 3H), 3.32 (s, 3H), 3.41 (s, 5H), 4.17 (q, 2H), 4.44 (m, 1H), 7.15 (d, 1H), 7.57 (m, 2H), 8.10 (d, 1H), 8.14 (d, 1H), 12.15 (s, 1H) | |
| | 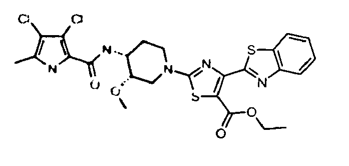 | | |
| **55** | Ethyl 2-((3S,4R)-4-{[(3,4-dichloro-5-methyl-1H-pyrrol-2-yl)carbonyl]amino}-3-fluoropiperidin-1-yl)-4-pyrazin-2-yl-1,3-thiazole-5-carboxylate | MS (ES) (M+H)⁺: 527 for C₂₁H₂₁Cl₂FN₆O₃S | 3,4-dichloro-N-[(3S,4R)-3-fluoropiperidin-4-yl]-5-methyl-1H-pyrrole-2-carboxamide hydrochloride and Intermediate 6 |
| | | NMR: 1.17 (t, 3H), 1.78 (m, 2H), 2.17 (s, 3H), 3.32 (s, 3H), 3.41 (s, 5H), 4.17 (q, 2H), 4.44 (m, 1H), 7.15 (d, 1H), 7.57 (m, 2H), 8.10 (d, 1H), 8.14 (d, 1H), 12.15 (s, 1H) | |
| | 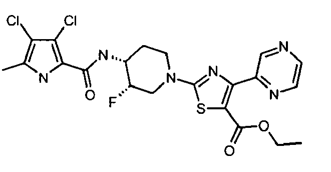 | | |
| **56** | Ethyl 4-(1,3-benzothiazol-2-yl)-2-((3S,4R)-4-{[(3,4-dichloro-5-methyl-1H-pyrrol-2-yl)carbonyl]amino-3-fluoropiperidin-1-yl)-1,3-thiazole-5-carboxylate | MS (ES) (M+H)⁺: 582 for C₂₄H₂₂Cl₂FN₅O₃S₂ | 3,4-dichloro-N-[(3S,4R)-3-fluoropiperidin-4-yl]-5-methyl-1H-pyrrole-2-carboxamide hydrochloride and Intermediate 7 |
| | | NMR: 1.12 (t, 3H), 1.84 (m, 2H), 2.13 (s, 3H), 3.27 (s, 3H), 3.55 (dd, 1H), 3.98 (m, 1H), 4.14 (q, 2H), 4.27 (m, 2H), 4.86 (d, 1H), 7.23 (d, 1H), 7.45 (m, 2H), 8.00 (d, 1H), 8.09 (d, 1H), 12.04 (s, 1H) | |
| | 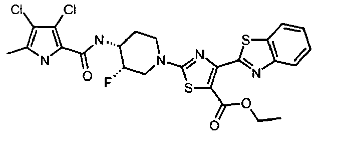 | | |
| **57** | Methyl 2-((3S,4R)-4-{[(3,4-dichloro-5-methyl-1H-pyrrol-2-yl)carbonyl]amino}-3-methoxypiperidin-1-yl)-4-(1-{[2-(trimethylsilyl)ethoxy]methyl}-1H-imidazol-2-yl)-1,3-thiazole-5-carboxylate | MS (ES) (M+H)⁺: 643 for C₂₆H₃₆Cl₂N₆O₅SSi | Intermediate 14 and 3,4-dichloro-N-[(3S,4R)-3-methoxypiperidi n-4-yl]-5-methyl-1H-pyrrole-2-carboxamide hydrochloride |
| | 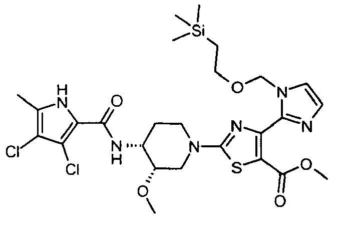 | | |
| **58** | Methyl 2-((3S,4R)-4-{[(3,4-dichloro-5-melhyl-1H-pyrrol-2-yl)carbonyl]amino}-3-methoxypiperidin-1-yl)-4-[1-(methoxymethyl)-1H-imidazol-2-yl]-1,3-thiazole-5-carboxylate | MS (ES) (M+H)⁺: 557 for C₂₂H₂₆Cl₂N₆O₅S | Intermediate 15 and 3,4-dichloro-N-[(3S,4R)-3-methoxypiperidin-4-yl]-5-methyl-1H-pyrrole-2-carboxamide hydrochloride |
| | | NMR: 1.81 (m, 2H), 2.23 (s, 3H), 3.14 (s, 3H), 3.43 (m, 4H), 3.58 (m, 1H), 3.65 (s, 3H), 4.02 (m, 1H), 4.34 (m, 2H), 5.26 (s, 2H), 7.15 (d, 1H), 7.39 (s, 2H), 12.22 (s, 1H). | |
| | 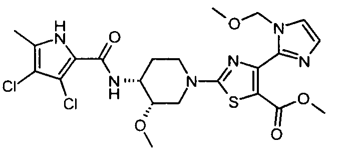 | | |
| **59** | Methyl 2-((3S,4R)-4-{[(3,4-dichloro-5-methyl-1H-pyrrol-2-yl)carbonyl]amino}-3-fluoropiperidin-1-yl)-4-[1-(methoxymethyl)-1H-imidazol-2-yl]-1,3-thiazole-5-carboxylate | MS (ES) (M+H)⁺: 545 for C₂₁H₂₃Cl₂FN₆O₄S | Intermediate 15 and 3,4-dichloro-N-[(3S,4R)-3-fluoropiperidin-4-yl]-5-methyl-1H-pyrrole-2- carboxamide hydrochloride |
| | | NMR: 1.91 (m, 2H), 2.22 (s, 3H), 3.11 (s, 3H), 3.42 (m, 4H), 3.48 (m, 1H), 3.61 (s, 3H), 3.66 (m, 1H), 4.02 (m, 1H), 4.33-4.46 (m, 1H), 5.04 (d, 1H), 5.18 (s, 2H), 7.03 (s, 1H), 7.32 (d, 1H), 7.42 (s, 2H), 12.18 (s, 1H). | |
| | 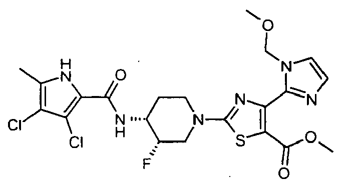 | | |
| **60** | Methyl 2-((3S,4R)-4-{[(3,4-dichloro-5-methyl-1H-pyrrol-2-yl)carbonyl]amino}-3-methoxypiperidin-1-yl)-4-isoxazol-5-yl-1,3-thiazole-5-carboxylate | MS (ES) (M+H)⁺: 514 for C₂₀H₂₁Cl₂N₅O₅S | Intermediate 59 and 3,4-dichloro-N-[(3S,4R)-3-methoxypiperidi n-4-yl]-5-methyl- 1H-pyrrole-2-carboxamide hydrochloride |
| | | NMR: 1.81 (m, 2H), 2.22 (s, 3H), 3.32 (m, 4H), 3.59 (m, 1H), 3.77 (s, 3H), 4.03 (m, 1H), 4.23 (m, 2H), 7.15 (d, 1H), 7.27 (s, 1H), 8.69 (s, 1H), 12.20 (s, 1H). | |
| | 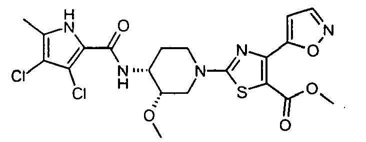 | | |
| **61** | Methyl 2-((3S,4R)-4-{[(3,4-dichloro-5-methyl-1H-pyrrol-2-yl)carbonyl]amino}-3-fluoropiperidin-1-yl)-4-isoxazol-5-yl-1,3-thiazole-5-carboxylate | MS (ES) (M+H)⁺: 503 for C₁₉H₁₈Cl₂FN₅O₄S | Intermediate 59 and 3,4-dichloro-N-[(3S,4R)-3-fluoropiperidin-4-yl]-5-methyl-1H-pyrrole-2-carboxamide hydrochloride |
| | | NMR: 1.91 (m, 2H), 2.21 (s, 3H), 3.65 (dd, 1H), 3.85 (s, 3H), 4.09 (m, 1H), 4.86 (m, 2H), 5.02 (d, 1H), 7.34 (m, 2H), 8.74 (s, 1H), 12.18 (s, 1H). | |
| | 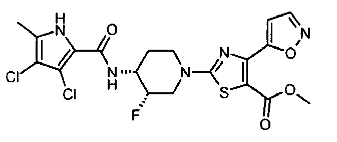 | | |
| **62** | Methyl 2-((3S,4R)-4-{[(3,4-dichloro-5-methyl-1H-pyrrol-2-yl)carbonyl]amino}-3-methoxypiperidin-1-yl)-4-(1H-pyrazol-5-yl)-1,3-thiazole-5-carboxylate | MS (ES) (M+H)⁺: 513 for C₂₀H₂₂Cl₂N₆O₄S | Intermediate 60 and 3,4-dichloro-N-[(3S,4R)-3-methoxypiperidin-4-yl]-5-methyl-1H-pyrrole-2-carboxamide hydrochloride |
| | | NMR: 1.82 (m, 2H), 2.24 (s, 3H), 3.34 (m, 4H), 3.57 (m, 1H), 3.81 (s, 3H), 4.13 (m, 1H), 4.25 (m, 2H), 7.24 (d, 1H), 7.25 (s, 1H), 7.58 (s, 1H), 12.21 (s, 1H), 13.34 (s, 1H). | |
| | 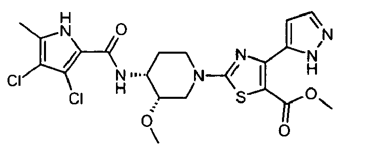 | | |
| **63** | Methyl 2-((3S,4R)-4-{[(3,4-dichloro-5-methyl-1H-pyrrol-2-yl)carbonyl]amino}-3-fluoropiperidin-1-yl)-4-(1H-pyrazol-5-yl)-1,3-thiazole-5-carboxylate | MS (ES) (M+H)⁺: 501 for C₁₉H₁₉Cl₂FN₆O₃S | Intermediate 60 and 3,4-dichloro-N-[(3S,4R)-3-fluoropiperidin-4-yl]-5-methyl-1H-pyrrole-2-carboxamide hydrochloride |
| | | NMR: 1.91 (m, 2H), 2.22 (s, 3H), 3.65 (dd, 1H), 3.84 (s, 3H), 4.07 (m, 1H), 4.86 (m, 2H), 5.03 (d, 1H), 7.32 (m, 2H), 8.71 (s, 1H), 12.19 (s, 1H). | |
| | 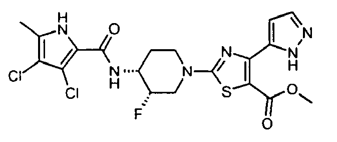 | | |
| **64** | Methyl 2-((3S,4R)-4-{[(3,4-dichloro-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylate | MS (ES) (M+H)⁺: 571 for C₂₃H₂₈Cl₂N₆O₅S | Intermediate 8 and 3,4-dichloro-N-[(3S,4R)-3-methoxypiperidin-4-yl]-5-methyl-1H-pyrrole-2-carboxamide hydrochloride |
| | | NMR: 1.82 (m, 2H), 2.22 (s, 3H), 3.22 (s, 3H), 3.34 (m, 4H), 3.58 (m, 1H), 3.69 (m, 2H), 4.02 (m, 1H), 4.34 (m, 2H), 4.77 (m, 2H), 7.23 (d, 1H), 7.31 (s, 1H), 7.52 (s, 1H), 12.22 (s, 1H). | |
| | 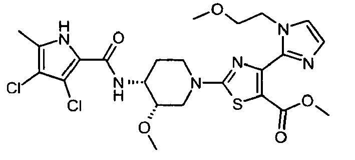 | | |
| **65** | Methyl 2-((3S,4R)-4-{[(3,4-dichloro-5-methyl-1H-pyrrol-2-yl)carbonyl]amino}-3-fluoropiperidin-1-yl)-4-[1-(2-methoxyethyl)-1H-imidazol-2-yl]-1,3-thiazole-5-carboxylate | MS (ES) (M+H)⁺: 559 for C₂₂H₂₅Cl₂FN₆O₄S | Intermediate 8 and 3,4-dichloro-N-[(3S,4R)-3-fluoropiperidin-4-yl]-5-methyl-1H-pyrrole-2-carboxamide hydrochloride |
| | | NMR: 1.91 (m, 2H), 2.22 (s, 3H), 3.21 (s, 3H), 3.34-3.46 (m, 4H), 3.52 (t, 2H), 3.48-3.86 (m, 5H), 4.02 (m, 3H), 4.22-4.52 (m, 2H), 5.01 (d, 1H), 6.95 (s, 1H), 7.28(m, 2H), 12.18 (s, 1H). | |
| | 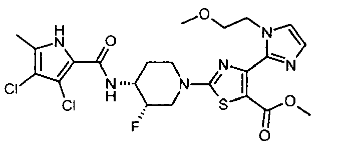 | | |
| **66** | Methyl 2-((3S,4R)-4-{[(3,4-dichloro-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylate | MS (ES) (M+H)⁺: 528 for C₂₀H₂₃Cl₂N₇O₄S | Intermediate 9 and 3,4-dichloro-N-[(3S,4R)-3-methoxypiperidin-4-yl]-5-methyl-1H-pyrrole-2-carboxamide hydrochloride |
| | | NMR: 1.81 (m, 2H), 2.22 (s, 3H), 3.34 (m, 4H), 3.57 (m, 1H), 3.71 (s, 6H), 4.01 (m, 1H), 4.28 (m, 2H), 7.15 (d, 1H), 8.04 (s, 1H), 12.23 (s, 1H). | |
| | 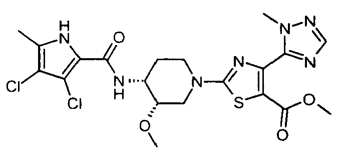 | | |
| **67** | Methyl 2-((3S,4R)-4-{[(3,4-dichloro-5-methyl-1H-pyrrol-2-yl)carbonyl]amino}-3-fluoropiperidin-1-yl)-4-(1-methyl-1H-1,2,4-triazol-5-yl)-1,3-thiazole-5-carboxylate | MS (ES) (M+H)⁺: 516 for C₁₉H₂₀Cl₂FN₇O₃S | Intermediate 9 and 3,4-dichloro-N-[(3S,4R)-3-fluoropiperidin-4-yl]-5-methyl-1H-pyrrole-2-carboxamide hydrochloride |
| | | NMR: 1.91 (m, 2H), 2.22 (s, 3H), 3.32 (m, 4H), 3.57 (m, 1H), 3.75 (s, 6H), 4.02 (m, 1H), 4.43 (m, 2H), 5.02 (d, 1H), 7.27 (d, 1H), 8.03 (s, 1H), 12.18 (s, 1H). | |
| | 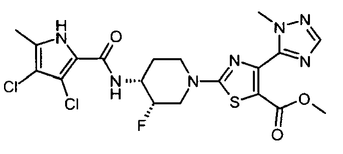 | | |
| **68** | Methyl 2-((3S,4R)-4-{[(4-eyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylate | MS (ES) (M+H)⁺: 485 for C₂₁H₂₄N₈O₄S | Intermediate 65 and Intermediate 9 |
| | | NMR: 1.64 (m, 1H), 1.87-2.04 (m, 1H), 2.33 (s, 3H), 3.33 (m, 4H), 3.58 (m, 1H), 3.71 (s, 6H), 4.01 (m, 1H), 4.18-4.36 (m, 2H), 7.26 (s, 1H), 7.91 (d, 1H), 8.04 (s, 1H), 12.33 (s, 1H). | |
| | 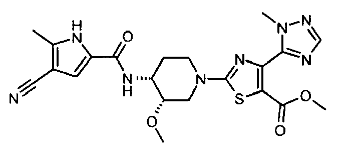 | | |
| **69** | Methyl 2-((3S,4R)-4-{[(3-chloro-4-cyano-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylate | MS (ES) (M+H)⁺: 562 for C₂₄H₂₈ClN₇O₅S | Intermediate 8 and Intermediate 64 |
| | | NMR: 1.81 (m, 2H), 2.22 (s, 3H), 3.23 (s, 3H), 3.33 (m, 4H), 3.54 (m, 2H), 3.59 (m, 4H), 3.91 (m, 1H), 4.01 (m, 2H), 4.33 (m, 2H), 6.95 (s, 1H), 7.33 (s, 1H), 7.35 (d, 1H), 12.71 (s, 1H). | |
| | 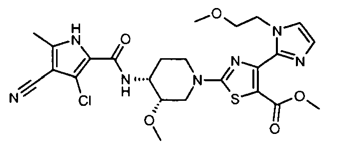 | | |
| **70** | Methyl 2-((3S,4R)-4-{[(3,4-dichloro-5-methyl-1H-pyrrol-2-yl)carbonyl]amino}-3-methoxypiperidin-1-yl)-4-(1,4,5-trimethyl-1H-imidazol-2-yl)-1,3-thiazole-5-carboxylate | MS (ES) (M+H)⁺: 555 for C₂₃H₂₈Cl₂N₆O₄S | Intermediate 11 and 3,4-dichloro-N-[(3S,4R)-3-methoxypiperidin-4-yl]-5-methyl-1H-pyrrole-2- carboxamide hydrochloride |
| | | NMR: 1.75 (m, 2H), 2.03 (s, 3H), 2.15 (s, 3H), 2.25 (s, 3H), 3.35 (m, 4H), 3.56 (m, 1H), 3.65 (s, 3H), 4.01 (m, 1H), 4.29 (m, 2H), 7.20 (d, 1H), 12.23 (s, 1H). | |
| | 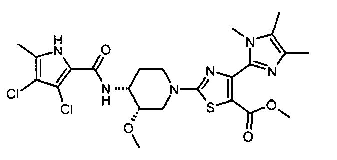 | | |
| **71** | Methyl 2'-amino-2-((3S,4R)-4-{[(3,4-dichloro-5-methyl-1H-pyrrol-2-yl)carbonyl]amino}-3-methoxypiperidin-1-yl)-4,4'-bi-1,3-thiazole-5-carboxylate | MS (ES) (M+H)⁺: 545 for C₂₀H₂₂Cl₂N₆O₄S₂ | Intermediate 55 and 3,4-dichloro-N-[(3S,4R)-3-methoxypiperidi n-4-yl]-5-methyl-1H-pyrrole-2-carboxamide hydrochloride |
| | | NMR: 1.75 (m, 2H), 2.23 (s, 3H), 3.41 (m, 4H), 3.57 (m, 1H), 3.68 (s, 3H), 4.02 (m, 1H), 4.25 (m, 2H), 7.02 (m, 2H), 7.15 (m, 2H), 12.24 (s, 1H). | |
| | 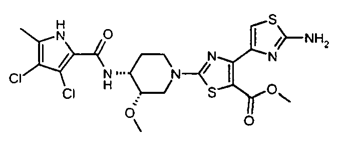 | | |
| **72** | Methyl 2-((3S,4R)-4-{[(3,4-dichloro-5-methyl-1H-pyrrol-2-yl)carbonyl]amino}-3-methoxypiperidin-1-yl)-2'-(methylamino)-4,4'-bi-1,3-thiazole-5-carboxylate | MS (ES) (M+H)⁺: 559 for C₂₁H₂₄Cl₂N₆O₄S₂ | Intermediate 56 and 3,4-dichloro-N-[(3S,4R)-3-methoxypiperidin-4-yl]-5-methyl-1H-pyrrole-2- carboxamide hydrochloride |
| | | NMR: 1.74 (m, 2H), 2.22 (s, 3H), 2.75 (d, 3H), 3.34-3.48 (m, 4H), 3.55 (m, 1H), 3.65 (s, 3H), 3.91 (m, 1H), 4.18 (m, 2H), 7.13 (s, 1H), 7.23 (d, 1H), 7.53 (m, 1H), 12.27 (s, 1H). | |
| | 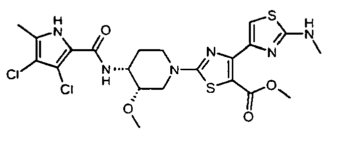 | | |
| **73** | Methyl 2-((3S,4R)-4-{[(3,4-dichloro-5-methyl-1H-pyrrol-2-yl)carbonyl]amino}-3-methoxypipcridin-1-yl)-2'-(dimethylamino)-4,4'-bi-1,3-thiazole-5-carboxylate | MS (ES) (M+H)⁺: 573 for C₂₂H₂₆Cl₂N₆O₄S₂ | Intermediate 57 and 3,4-dichloro-N-[(3S,4R)-3-methoxypiperidin-4-yl]-5-methyl-1H-pyrrole-2- carboxamide hydrochloride |
| | | NMR: 1.81 (m, 2H), 2.22 (s, 3H), 3.08 (s, 6H), 3.27-3.45 (m, 4H), 3.55 (m, 1H), 3.71 (s, 3H), 3.89 (m, 1H), 4.32 (m, 2H), 7.17 (s, 1H), 7.20 (d, 1H), 12.24 (s, 1H). | |
| | 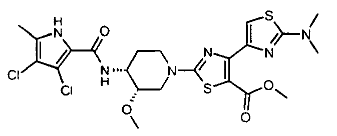 | | |
| **74** | Methyl 2'-amino-2-((3S,4R)-4-{[(3,4-dichloro-5-methyl-1H-pyrrol-2-yl)carbonyl]amino}-3-fluoropiperidin-1-yl)-4,4'-bi-1,3-thiazole-5-carboxylate | MS (ES) (M+H)⁺: 533 for C₁₉H₁₉Cl₂FN₆O₃S₂ | Intermediate 55 and 3,4-dichloro-N-[(3S,4R)-3-fluoropiperidin-4-yl]-5-methyl-1H-pyrrole-2-carboxamide hydrochloride |
| | | NMR: 1.81 (m, 2H), 2.22 (s, 3H), 3.32 (m, 4H), 3.46 (dd, 1H), 3.69 (s, 3H), 4.02 (m, 1H), 4.41 (m, 2H), 5.01 (d, 1H), 7.02 (s, 2H), 7.23 (s, 1H), 7.37 (d, 1H), 12.10 (s, 1H). | |
| | 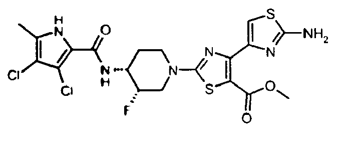 | | |
| **75** | Methyl 2-((3S,4R)-4-{[(3,4-dichloro-5-methyl-1H-pyrrol-2-yl)carbonyl]amino}-3-fluoropiperidin-1-yl-2'-(methylamino)-4,4'-bi-1,3-thiazole-5-carboxylate | MS (ES) (M+H)⁺: 547 for C₂₀H₂₁Cl₂FN₆O₃S₂ | Intermediate 56 and 3,4-dichloro-N-[(3S,4R)-3-fluoropiperidin-4-yl]-5-methyl-1H-pyrrole-2-carboxamide hydrochloride |
| | | NMR: 1.91 (m, 2H), 2.23 (s, 3H), 2.81 (d, 3H), 3.34 (m, 4H), 3.55 (dd, 1H), 3.71 (s, 3H), 4.06 (m, 1H), 4.31 (m, 2H), 5.02 (d, 1H), 7.08 (s, 2H), 7.29 (d, 1H), 7.51 (m, 1H), 12.19 (s, 1H). | |
| | 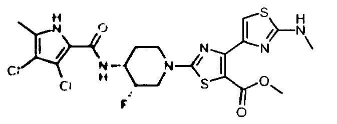 | | |
| **76** | Methyl 2-((3S,4R)-4-{[(3,4-dichloro-5-methyl-1H-pyrrol-2-yl)carbonyl]amino}-3-fluoropiperidin-1-yl)-2'-(dimethylamino)-4,4'-bi-1,3-thiazole-5-carboxylate | MS (ES) (M+H)⁺: 561 for C₂₁H₂₃Cl₂FN₆O₃S₂ | Intermediate 57 and 3,4-dichloro-N-[(3S,4R)-3-fluoropiperidin-4-yl]-5-methyl-1H-pyrrole-2-carboxamide hydrochloride |
| | | NMR: 1.82 (m, 2H), 2.22 (s, 3H), 3.03 (s, 6H), 3.34 (m, 4H), 3.57 (dd, 1H), 3.69 (s, 3H), 4.05 (m, 1H), 4.31 (m, 2H), 5.03 (d, 1H), 7.12 (s, 1H), 7.31 (d, 1H), 7.52 (m, 1H), 12.10 (s, 1H). | |
| | 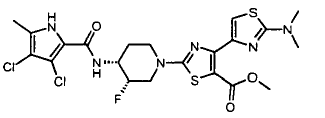 | | |
| **77** | Ethyl 2-((3S,4R)-4-{[(3,4-dichloro-5-methyl-1H-pyrrol-2-yl)carbonyl]amino}-3-methoxypiperidin-1-yl)-4-(1-methyl-1H-imidazol-4-yl)-1,3-thiazole-5-carboxylate | MS (ES) (M+H)⁺: 541 for C₂₂H₂₆Cl₂N₆O₄S | Intermediate 12 and 3,4-dichloro-N-[(3S,4R)-3-methoxypiperidi n-4-yl]-5-methyl-1H-pyrrole-2-carboxamide hydrochloride |
| | | NMR: 1.22 (t, 3H), 1.75 (m, 2H), 2.23 (s, 3H), 3.34 (m, 4H), 3.48 (m, 1H), 3.71 (s, 3H), 4.03 (m, 1H), 4.13 (m, 2H), 4.17-4.34 (m, 2H), 7.15 (s, 1H), 7.61 (s, 1H), 8.04 (s, 1H), 12.20 (s, 1H). | |
| | 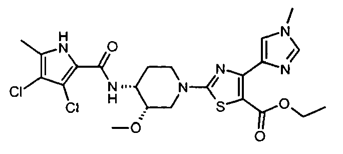 | | |
| **78** | Ethyl 2-((3S,4R)-4-{[(3,4-dichloro-5-methyl-1H-pyrrol-2-yl)carbonyl]amino}-3-fluoropiperidin-1-yl)-4-(1-methyl-1H-imidazol-4-yl)-1,3-thiazole-5-carboxylate | MS (ES) (M+H)⁺: 529 for C₂₁H₂₃Cl₂FN₆O₃S | Intermediate 12 and 3,4-dichloro-N-[(3S,4R)-3-fluoropiperidin-4-yl]-5-methyl-1H-pyrrole-2-carboxamide hydrochloride |
| | | NMR: 1.23 (t, 3H), 1.85 (m, 2H), 2.22 (s, 3H), 3.35 (m, 4H), 3.51 (m, 1H), 3.69 (s, 3H), 4.03 (m, 1H), 4.15 (m, 2H), 4.34 (m, 2H), 5.01 (d, 1H), 7.27 (d, 1H), 7.59 (s, 1H), 8.03 (s, 1H), 12.13 (s, 1H). | |
| | 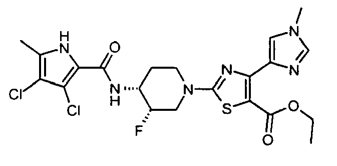 | | |
| **79** | Methyl 2-((3S,4R)-4-{[(3,4-dichloro-5-methyl-1H-pyrrol-2-yl)carbonyl]amino}-3-fluoropiperidin-1-yl)-4-(1-methyl-1H-imidazol-2-yl)-1,3-thiazole-5-carboxylate | MS (ES) (M+H)⁺: 515 for C₂₀H₂₁Cl₂FN₆O₃S | Intermediate 10 and 3,4-dichloro-N-[(3S,4R)-3-fluoropiperidin-4-yl]-5-methyl-1H-pyrrole-2-carboxamide hydrochloride |
| | | NMR: 1.91 (m, 2H), 2.22 (s, 3H), 3.34 (m, 4H), 3.58 (m, 1H), 3.55 (s, 3H), 3.65 (s, 3H), 4.10 (m, 1H), 4.28 (m, 2H), 5.02 (d, 1H), 7.03 (s, 1H), 7.34 (m, 2H), 12.18 (s, 1H). | |
| | 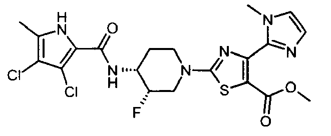 | | |
| **80** | Methyl 2-((3S,4R)-4-{[(3-chloro-4-cyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylate | MS (ES)(M+H)⁺: 519 for C₂₁H₂₃ClN₈O₄S | Intermediate 64 and Intermediate 9 |
| | | NMR: 1.81 (m, 2H), 2.34 (s, 3H), 3.32 (m, 4H), 3.58 (m, 1H), 3.65 (s, 2H), 3.70 (s, 3H), 4.01 (m, 1H), 4.32 (m, 2H), 7.31 (d, 1H), 8.04 (s, 1H), 12.76 (s, 1H). | |
| | 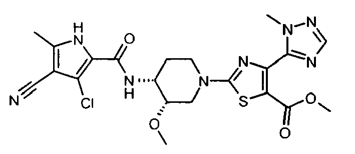 | | |
| **81** | Ethyl 2-((3S,4R)-4-{[(3,4-dichloro-5-methyl-1H-pyrrol-2-yl)carbonyl]amino}-3-methoxypiperidin-1-yl)-4-pyrimidin-4-yl-1,3-thiazole-5-carboxylate | MS (ES) (M+H)⁺: 539 for C₂₂H₂₄Cl₂N₆O₄S | 3,4-dichloro-N-[(3S,4R)-3-methoxypiperidin-4-yl]-5-methyl-1H-pyrrole-2-carboxamide hydrochloride and Intermediate 2 |
| | | NMR: 1.02 (t, 3H), 1.70 (m, 2H), 2.12 (s, 3H), 3.25 (m, 2H), 3.31 (s, 3H), 3.50 (s, 1H), 4.00 (q, 2H), 4.22 (m, 3H), 7.09 (d, 1H), 7.66 (d, 1H), 8.83 (d, 1H), 9.17 (s, 1H), 12.10 (s, 1H) | |
| | 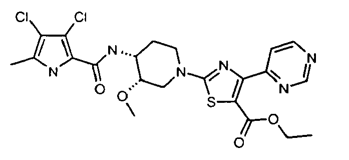 | | |
| **82** | Methyl 2-((3*S*,4*R*)-4-{[(3,4-dichloro-5-methyl-1*H*-pyrrol-2-yl)carbonyl]amino}-3-methoxypiperidin-1-yl)-4-(4-methoxypyrimidin-2-yl)-1,3-thiazole-5-carboxylate | MS (ES) (M+H)⁺: 556 for C₂₂H₂₄Cl₂N₆O₅S | 3,4-dichloro-*N*-[(3*S*,4*R*)-3-methoxypiperidin-4-yl]-5-methyl-1*H*-pyrrole-2-carboxamide hydrochloride and Intermediate 70 |
| | | | |
| | 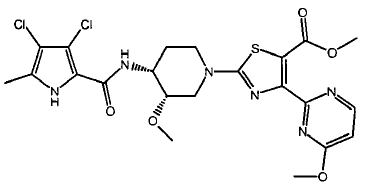 | | |
| **83** | Ethyl 2-((3*S*,4*R*)-4-{[(3,4-dichloro-5-methyl-1*H*-pyrrol-2-yl)carbonyl]amino}-3-methoxypiperidin-1-yl)-4-(1,3,4-oxadiazol-2-yl)-1,3-thiazole-5-carboxylate | MS (ES) (M+H)⁺: 529 for C₂₀H₂₂Cl₂N₆O₅S | 3,4-dichloro-*N*-[(3*S*,4*R*)-3-methoxypiperidin-4-yl]-5-methyl-1*H*-pyrrole-2-carboxamide hydrochloride and Intermediate 75 |
| | | | |
| | 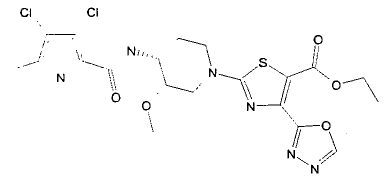 | | |
| **84** | Ethyl 2-((3*S*,4*R*)-4-{[(3,4-dichloro-5-methyl-1*H*-pyrrol-2-yl)carbonyl]amino}-3-methoxypiperidin-1-yl)-4-(3-methyl-1,2,4-oxadiazol-5-yl)-1,3-thiazole-5-carboxylate | MS (ES) (M+H)⁺: 544 for C₂ₗH₂₄Cl₂N₆O₅S | 3,4-dichloro-*N*-[(3*S*,4*R*)-3-methoxypiperidin-4-yl]-5-methyl-1*H*-pyrrole-2-carboxamide hydrochloride and Intermediate 78 |
| | | NMR (CDCl₃): 1.28 (t, 3H), 2.03 (m, 2H), 2.30 (s, 3H), 2.53 (s, 3H), 3.22 (d, 1H), 3.30 (m, 1H), 3.48 (s, 3H), 3.52 (m, 1H), 4.02 (m, 1H), 4.26 (q, 2H), 4.32 (m, 1H), 4.56 (m, 1H), 7.23 (d, 1H), 9.27 (br, 1H). | |
| | 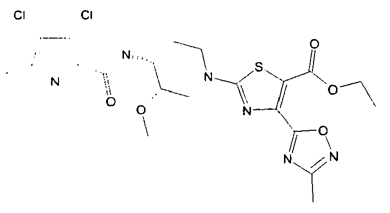 | | |
| **85** | Ethyl 2-((3*S*,4*R*)-4-{[(3,4-dichloro-5-methyl-1*H*-pyrrol-2-yl)carbonyl]amino}-3-methoxypiperidin-1-yl)-4-(5-methyl-1,3,4-oxadiazol-2-yl)-1,3-thiazole-5-carboxylate | MS (ES) (M+H)⁺: 544 for C₂₁H₂₄Cl₂N₆O₅S | 3,4-dichloro-*N*-[(3*S*,4*R*)-3-methoxypiperidin-4-yl]-5-methyl-1*H*-pyrrole-2-carboxamide hydrochloride and Intermediate 79 |
| | | NMR(CDCl₃): 1.29 (t, 3H), 2.03 (m, 2H), 2.30 (s, 3H), 2.64 (s, 3H), 3.22 (d, 1H), 3.30 (m, 1H), 3.49 (s, 3H), 3.52 (m, 1H), 4.01 (m, 1H), 4.26 (q, 2H), 4.32 (m, 1H), 4.58 (m, 1H), 7.25 (d, 1H), 9.25(br, 1H) | |
| | 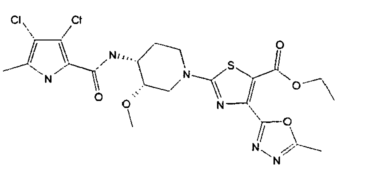 | | |

### Example 86

### Methyl 2-((3S,4R)-4-{[(3,4-dichloro-5-methyl-1H-pyrrol-2-yl)carbonyl]amino}-3-methoxypiperidin-1-yl)-4-(1H-imidazol-2-yl)-1,3-thiazole-5-carboxylate

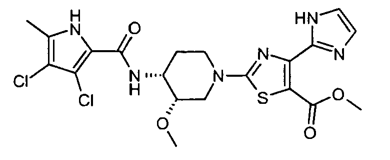

A solution of methyl 2-((3*S*,4*R*)-4-{[(3,4-dichloro-5-methyl-1*H*-pyrrol-2-yl)carbonyl]amino}-3-methoxypiperidin-1-yl)-4-[1-(methoxymethyl)-1*H*-imidazol-2-yl]-1,3-thiazolc-5-carboxylate (Example 58; 173 mg 0.31 mmol) in 3 ml 8:1 acetic acid-water was heated at 120 °C for 2 hours in a microwave reactor. Solvent was removed and the residue was partitioned between NaHCO₃ (aq) and EtOAc. The EtOAc was separated and washed with brine. Drying (MgSO₄) and removal of solvent gave a solid that was chromatographed on silica gel (100% EtOAc followed by gradient elution to 20% MeOH in EtOAc). Product (78 mg) was isolated as a solid. MS (ES) (M+H)⁺: 513 for C₂₂H₂₆Cl₂N₆O₅S; NMR: 1.83 (m, 2H), 2.24 (s, 3H), 3.34 (s, 3H), 3.45 (m, 4H), 3.59 (m, 1H), 3.82 (s, 3H), 4.02 (m, 1H), 4.33 (m, 2H), 7.15 (m, 3H), 7.41 (s, 2H), 12.20 (s, 1H), 12.77 (s, 1H).

### Example 87

The following Example was synthesized by an analogous method to Example 86 from the starting material (SM) given in the table below.

| **Ex** | **Compound** | **Data** | **SM** |
|---|---|---|---|
| **87** | Methyl 2-((3*S*,4*R*)-4-{[(3,4-dichloro-5-methyl-1*H*-pyrrol-2-yl)carbonyl]amino}-3-fluoropiperidin-1-yl)-4-(1*H*-imidazol-2-yl)-1,3-thiazole-5-carboxylate | MS (ES) (M+H)⁺: 501 for C₁₉H₁₉Cl₂FN₆O₃S | Example 59 |
| | | NMR: 1.91 (m, 2H), 2.22 (s, 3H), 3.41 (m, 4H), 3.46-3.73 (m, 2H), 3.81 (s, 3H), 4.11 (m, 1H), 4.26-4.53 (m, 2H), 5.01 (d, 1H), 7.21 (s, 2H), 7.31 (d, 1H), 12.16 (s, 1H), 12.74 (s, 1H). | |
| | 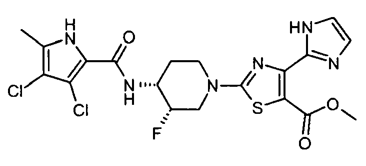 | | |

### Example 88 and Example 89

### Methyl 2-((3S,4R)-4-{[(3,4-dichloro-5-methyl-1H-pyrrol-2-yl)carbonyl]amino}-3-methoxypiperidin-1-yl)-4-(1-methyl-1H-imidazol-2-yl)-1,3-thiazole-5-carboxylate and 2-[2-((3S,4R)-4-{[(3,4-Dichloro-5-methyl-1H-pyrrol-2-yl)carbonyl]amino}-3-methoxypiperidin-1-yl)-5-(methoxycarbonyl)-1,3-thiazol-4-yl]-1,3-dimethyl-1H-imidazol-3-ium iodide

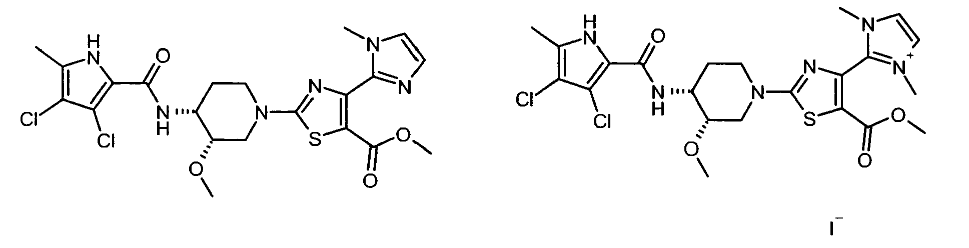

A solution of 280 mg (0.55 mmol) of methyl 2-((3*S*,4*R*)-4-{[(3,4-dichloro-5-methyl-1*H*-pyrrol-2-yl)carbonyl]amino}-3-methoxypiperidin-1-yl)-4-(1*H*-imidazol-2-yl)-1,3-thiazole-5-carboxylate (Example 86), 46 mg (0.55 mmol) NaHCO₃ and 35 µl (0.56 mmol) methyl iodide in 3 ml CH₃CN was heated at 80 °C for 30 min in a microwave reactor. The mixture was diluted with water, which was saturated with NaCl before being extracted with 4 times with DCM. The extracts were dried (MgSO₄) and concentrated, and the residue was chromatographed on silica gel (100% DCM followed by gradient elution to 15% MeOH in DCM) to afford 2 components. The second eluting component (75 mg), corresponds to the second titled compound, MS (ES) (M+H)⁺: 541 for C₂₂H₂₇Cl₂N₆O₄S; NMR: 1.81 (m, 2H), 2.22 (s, 3H), 3.43 (m, 4H), 3.54 (m, 1H), 3.66 (m, 1H), 3.71 (s, 6H), 4.01 (m, 1H), 4.31 (m, 2H), 7.15 (d, 1H), 7.87 (s, 2H), 12.20 (s, 1H). The first eluting component was chromatographed again (100% EtOAc followed by gradient elution to 20% MeOH in EtOAc) to afford 2 components with the first eluting component (70 mg) corresponding to starting material [MS (ES) (M+H)⁺: 513 for C₂₀H₂₂Cl₂N₆O₅S] and the second eluting component (103 mg) corresponding to the first titled compound, MS (ES) (M+H)⁺: 527 for C₂₁H₂₄Cl₂N₆O₄S; NMR: 1.81 (m, 2H), 2.22 (s, 3H), 3.44 (s, 3H), 3.52 (s, 3H), 3.62 (s, 3H), 4.04 (m, 1H), 4.27 (m, 2H), 7.05 (s, 1H), 7.22 (d, 1H), 7.25 (s, 1H), 12.20 (s, 1H).

### Example 90

### 2-((3S,4R)-4-{[(3,4-Dichloro-5-methyl-1H-pyrrol-2-yl)carbonyl]amino}-3-methoxypiperidin-1-yl)-4-(5-methyl-1,3,4-oxadiazol-2-yl)-1,3-thiazole-5-carboxylic acid

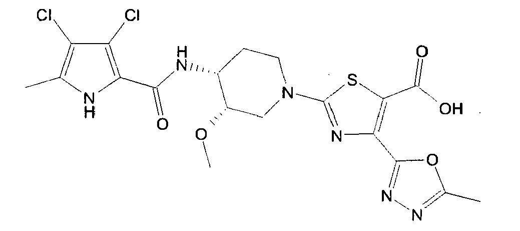

Ethyl 2-((3*S*,4*R*)-4-{[(3,4-dichloro-5-methyl-1*H*-pyrrol-2-yl)carbonyl]amino}-3-methoxypiperidin-1-yl)-4-(5-methyl-1,3,4-oxadiazol-2-yl)-1,3-thiazole-5-carboxylate (Example 85; 140 mg, 0.26 mmol) was dissolved in 1,4-dioxane/2 M NaOH (9 ml:3 ml), the mixture was stirred at room temperature for 3hrs, LC-MS showed the completion of the reaction. The reaction mixture was washed with ether (10 ml), the aqueous layer was collected and acidified to pH=3 with 2 M HCl solution. The resulting precipitate was collected by filtration and washed with H₂O, dried under high vacuum. The desired product was obtained as white solid (127 mg). MS (ES) (M+H)⁺: 516 for C₁₉H₂₀Cl₂N₆O₅S; NMR: 1.77 (m, 2H), 2.19 (s, 3H), 2.58 (s, 3H), 3.22 (d, 1H), 3.30 (m, 1H), 3.38 (s, 3H), 3.57 (m, 1H), 4.01 (m, 1H), 4.26 (q, 2H), 7.17 (d, 1H), 12.16 (s, 1H), 13.29 (br, 1H).

### Example 91

The following compound was synthesized according to the procedure described for Example 90.

| **Ex** | **Compound** | **Data** | **SM** |
|---|---|---|---|
| **91** | 2-((3*S*,4*R*)-4-{[(3,4-Dichloro-5-methyl-1*H*-pyrrol-2-yl)carbonyl]amino}-3-methoxypiperidin-1-yl)-4-(5-methyl-1,3,4-oxadiazol-2-yl)-1,3-thiazole-5-carboxylic acid | MS (ES) (M+H)⁺: 516 for C₁₉H₂₀Cl₂N₆O₅S | Example 84 |
| | | NMR: 1.77 (m, 2H), 2.19 (s, 3H), 2.44 (s, 3H), 3.30 (m, 1H), 3.37 (s, 3H), 3.40 (m, 1H), 3.57 (m, 1H), 3.97 (m, 1H), 4.27 (m, 2H), 7.17 (d, 1H), 12.16 (s, 1H), 13.35 (br, 1H). | |
| | 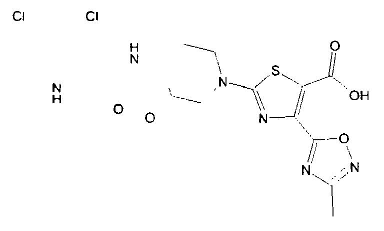 | | |

### Examples 92-108

The following compounds were synthesized according to the procedure described for Example 43.

| **Ex** | **Compound** | **Data** | **SM** |
|---|---|---|---|
| **92** | methyl 2-((3S,4R)-4-(3,4-dichloro-5-methyl-1H-pyrrole-2-carboxamido)-3-methoxypiperidin-1-yl)-4-(1-methyl-1H-1,2,3-triazol-4-yl)thiazole-5-carboxylate | MS (ES): 529 (MH⁺) for C₂₀H₂₃Cl₂N₇O₄S | 3,4-dichloro-N-[(3S,4R)-3-methoxypiperidin-4-yl]-5-methyl-1H- pyrrole-2-carboxamide hydrochloride and Intermediate 81 |
| | | ¹H-NMR: 1.77 (m, 2H); 2.19 (s, 3H); 3.27 (m, 2H); 3.39 (s, 3H); 3.58(m, 1H); 3.72 (s, 3H); 4.03(m, 1H); 4.12 (s, 3H); 4.29 (m, 2H); 7.18 (d, 1H); 8.68 (s, 1H); 12.16 (s, 1H). | |
| | 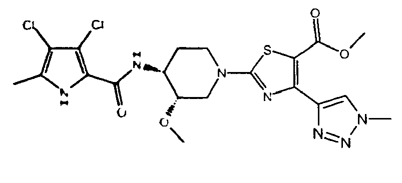 | | |
| **93** | methyl 2-((3S,4R)-4-(3,4-dichloro-5-methyl-1H-pyrrole-2-carboxamido)-3-methoxypiperidin-1-yl)-4-(3,5-dimethylpyrazin-2-yl)thiazole-5-carboxylate | MS (ES): 554 (MH⁺) for C₂₃H₂₆Cl₂N₆O₄S | 3,4-dichloro-N-[(3S,4R)-3-methoxypiperidin-4-yl]-5-methyl-1H-pyrrole-2-carboxamide hydrochloride and Intermediate 82 |
| | | ¹H-NMR: 1.77 (m, 2H); 2.19 (s, 3H); 2.31 (s, 3H); 2.46 (s, 3H); 3.27 (m, 2H); 3.37 (s, 3H); 3.58(s, 3H); 3.58 (m, 1H); 4.03(m, 1H); 4.29 (m, 2H); 7.18 (d, 1H); 8.44 (s, 1H); 12.16 (s, 1H). | |
| | 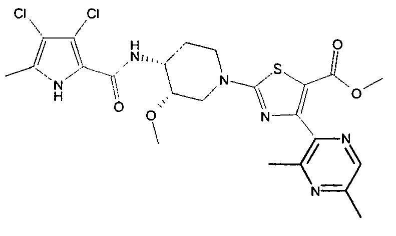 | | |
| **94** | methyl 2-((3S,4R)-4-(3,4-dichloro-5-methyl-1H-pyrrole-2-carboxamido)-3-methoxypiperidin-1-yl)-4-(6-methoxypyridin-2-yl)thiazole-5-carboxylate | MS (ES): 555 (MH⁺) for C₂₃H₂₅Cl₂N₅O₅S | 3,4-dichloro-N-[(3S,4R)-3-methoxypiperidin-4-yl]-5-methyl-1H-pyrrole-2-carboxamide hydrochloride and Intermediate 83 |
| | | ¹H-NMR: 1.77 (m, 2H); 2.19 (s, 3H); 3.30 (m, 1H); 3.40 (s, 3H); 3.40 (m, 1H); 3.58 (m, 1H); 3.63 (s, 3H); 3.81 (s, 3H); 3.94 (m, 1H); 4.29 (m, 2H); 6.83 (d, 1H); 7.17 (d, 1H); 7.22 (d, 1H); 7.75 (t, 1H); 12.16 (s, 1H). | |
| | 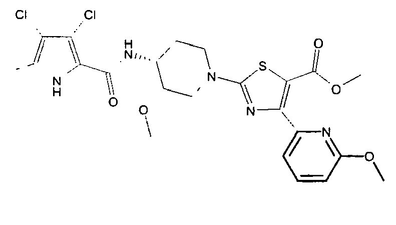 | | |
| **95** | ethyl 2-((3S,4R)-4-(3,4-dichloro-5-methyl-1H-pyrrole-2-carboxamido)-3-methoxypiperidin-1-yl)-4-(4-methoxypyridin-2-yl)thiazole-5-carboxylate | MS (ES): 569 (MH⁺) for C₂₄H₂₇Cl₂N₅O₅S | 3,4-dichloro-N-[(3S,4R)-3-methoxypiperidin-4-yl]-5-methyl-1H-pyrrole-2-carboxamide hydrochloride and Intermediate 84 |
| | | ¹H-NMR: 1.27 (t, 3H); 1.97 (m, 2H); 2.27 (s, 3H); 3.20∼3.40 (m, 4H); 3.54 (s, 3H); 3.58 (m, 1H); 4.01( s, 3H); 4.25 (q, 2H); 4.31 (m, 1H); 7.03 (d, 1H); 7.24 (d, 1H); 7.25 (d, 1H); 8.81 (s, 1H); 9.35 (s, 1H). | |
| | 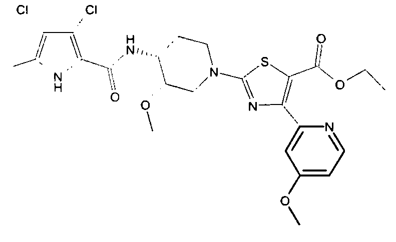 | | |
| **96** | ethyl 2-((3S,4R)-4-(3,4-dichloro-5-methyl-1H-pyrrole-2-carboxamido)-3-methoxypipcridin-1-yl)-4-(5-methylpyrazin-2-yl)thiazole-5-carboxylate | MS (ES): 554 (MH⁺) for C₂₃H₂₆Cl₂N₆O₄S | 3,4-dichloro-N-[(3S,4R)-3-methoxypiperidin-4-yl]-5-methyl-1H-pyrrole-2-carboxamide hydrochloride and Intermediate 85 |
| | | ¹H-NMR: 1.10 (t, 3H); 1.77 (m, 2H); 2.18 (s, 3H); 2.55 (s, 3H); 3.38 (s, 3H); 3.39 (m, 3H); 3.56 (m, 1H); 4.06 (q, 2H); 4.29 (m, 2H); 7.15 (d, 1H); 8.58 (s, 1H); 8.69 (s, 1H); 12.14 (s, 1H). | |
| | 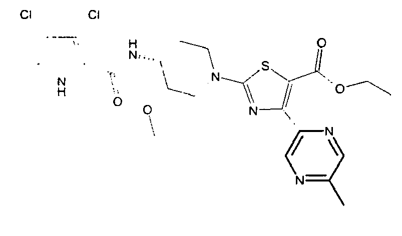 | | |
| **97** | ethyl 2-((3S,4R)-4-(3,4-dichloro-5-methyl-1H-pyrrole-2-carboxamido)-3-methoxypiperidin-1-yl)-4-(3-fluoropyridin-2-yl)thiazole-5-carboxylate | MS (ES): 557 (MH⁺) for C₂₃H₂₄Cl₂FN₅O₄S | 3,4-dichloro-N-[(3S,4R)-3-methoxypiperidin-4-yl]-5-methyl-1H-pyrrole-2-carboxamide hydrochloride and Intermediate 86 |
| | | ¹H-NMR (CDCl₃): 1.19 (t, 3H); 1.90 (m, 1H); 2.0 (m, 1H); 2.27 (s, 3H); 3.25 (m, 2H); 3.46 (s, 3H); 3.52 (m, 1H); 4.10 (m, 1H); 4.17 (q, 2H); 4.30 (m, 1H); 4.53 (m, 1H); 7.20 (d, 1H); 7.49 (dd, 1H); 7.61 (t, 1H); 8.58 (d, 1H); 9.30 (s, 1H). | |
| | 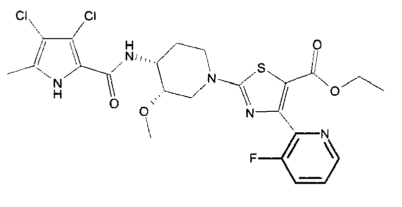 | | |
| **98** | Ethyl 2-((3S,4R)-4-(3,4-dichloro-5-methyl-1H-pyrrole-2-carboxamido)-3-fluoropiperidin-1-yl)-4-(4-methoxypyridin-2-yl)thiazole-5-carboxylate | MS (ES): 557 (MH⁺) for C₂₃H₂₄FCl₂N₅O₄S | 3,4-dichloro-N-[(3S,4R)-3-fluoropiperidin-4-yl]-5-methyl-1H-pyrrole-2-carboxamide hydrochloride and Intermediate 84 |
| | | ¹H-NMR (CDCl₃): 1.77 (m, 2H); 2.08 (m, 3H); 2.27 (s, 3H); 3.33 (m, 2H); 3.48 (m, 1H); 4.14 (s, 3H); 4.33 (q, 2H); 4.66 (m, 1H); 4.87 (m, 1H); 5.05 (m, 1H); 7.21 (d, 1H); 8.46 (s, 1H); 8.78 (d, 1H); 9.0 (d, 1H); 9.42 (s, 1H). | |
| | 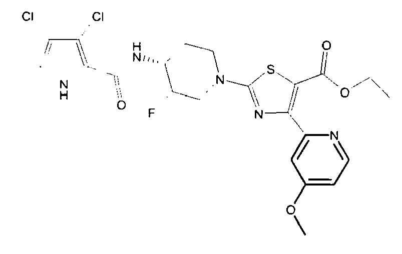 | | |
| **99** | methyl 2-((3S,4R)-4-(3,4-dichloro-5-methyl-1H-pyrrole-2-carboxamido)-3-fluoropiperidin-1-yl)-4-(1-methyl-1H-1,2,3-triazol-4-yl)thiazole-5-carboxylate | MS (ES): 517 (MH⁺) for C₁₉H₂₀Cl₂FN₇O₃S | 3,4-dichloro-N-[(3S,4R)-3-fluoropiperidin-4-yl]-5-methyl-1H-pyrrole-2-carboxamide hydrochloride and Intermediate 81 |
| | | ¹H-NMR: 1.87 (m, 2H); 2.18 (s, 3H); 3.35 (m, 2H); 3.72 (s, 3H); 4.10 (s, 3H); 4.11 (m, 1H); 4.39 (m, 2H); 4.90∼5.06 (d, 1H); 7.27 (d, 1H); 8.68 (s, 1H); 12.10 (s, 1H). | |
| | 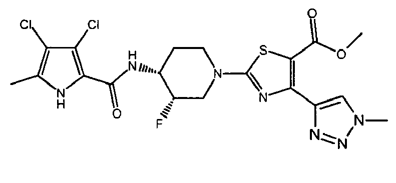 | | |
| **100** | methyl 2-((3S,4R)-4-(3,4-dichloro-5-methyl-1H-pyrrole-2-carboxamido)-3-fluoropiperidin-1-yl)-4-(6-methoxypyridin-2-yl)thiazole-5-carboxylate | MS (ES): 543 (MH⁺) for C₂₂H₂₂Cl₂FN₅SO₄S | 3,4-dichloro-N-[(3S,4R)-3-fluoropiperidin-4-yl]-5-methyl-1H-pyrrole-2-carboxamide hydrochloride and Intermediate 83 |
| | | ¹H-NMR: 1.88 (m, 2H); 2.20 (s, 3H); 3.39 (m, 1H); 3.52 (m, 1H); 3.64 (s, 3H); 3.82 (s, 3H); 4.03 (m, 1H); 4.35 (m, 2H); 4.90∼5.06 (d, 1H); 6.85 (d, 1H); 7.22 (d, 1H); 7.27 (d, 1H); 7.76 (t, 1H); 12.11 (s, 1H). | |
| | 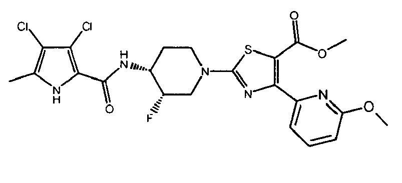 | | |
| **101** | methyl 2-((3S,4R)-4-(3,4-dichloro-5-methyl-1H-pyrrole-2-carboxamido)-3-fluoropiperidin-1-yl)-4-(6-methoxypyridin-2-yl)thiazole-5-carboxylate | MS (ES): 542 (MH⁺) for C₂₂H₂₃Cl₂FN₆O₃S | 3,4-dichloro-N-[(3S,4R)-3-fluoropiperidin-4-yl]-5-methyl-1H-pyrrole-2-carboxamide hydrochloride and Intermediate 85 |
| | | ¹H-NMR: 1.10 (t, 3H); 1.88 (m, 2H); 2.20 (s, 3H); 2.55 (s, 3H); 3.31 (m, 2H); 3.52∼4.04 ( dd, 1H); 4.08 (q, 2H); 4.35 (m, 2H); 4.90∼5.06 (d, 1H); 7.27 (d, 1H); 8.59 (s, 1H); 8.70 (s, 1H); 12.09 (s, 1H). | |
| | 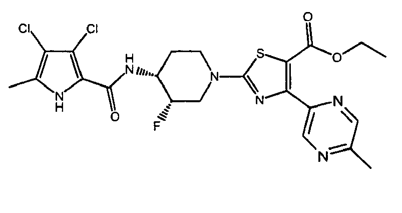 | | |
| **102** | ethyl 2-((3S,4R)-4-(3,4-dichloro-5-methyl-1H-pyrrole-2-carboxamido)-3-fluoropiperidin-1-yl)-4-(3-fluoropyridin-2-yl)thiazole-5-carboxylate | MS (ES): 545 (MH⁺) for C₂₂H₂₁Cl₂F₂N₅O₃S | 3,4-dichloro-N-[(3S,4R)-3-fluoropiperidin-4-yl]-5-methyl-1H-pyrrole-2-carboxamide hydrochloride and Intermediate 86 |
| | | ¹H-NMR CDCl3: 1.18 (t, 3H); 2.09 (m, 3H); 2.29 (s, 3H); 3.31 (m, 1H); 3.50∼3.71 (dd, 1H); 4.17 (q, 2H); 4.31 (m, 1H); 4.53 (m, 1H); 4.83∼4.99 (d, 1H); 7.02 (d, 1H); 7.41 (dd, 1H); 7.51 (t, 1H); 8.54 (d, 1H); 10.01 (s, 1H). | |
| | 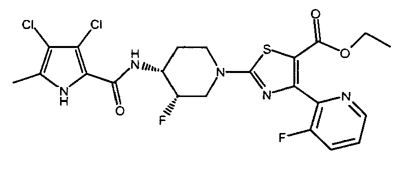 | | |
| **103** | methyl 2-((3S,4R)-4-(4-chloro-3-cyano-5-methyl-1H-pyrrole-2-carboxamido)-3-methoxypiperidin-1-yl)-4-(1-methyl-1H-1,2,3-triazol-4-yl)thiazole-5-carboxylate | MS (ES): 520 (MH⁺) for C₂₁H₂₃ClN₈O₄S | Intermediate 119 and Intermediate 81 |
| | | ¹H-NMR: 1.77 (m, 2H); 2.21 (s, 3H); 3.33 (m, 2H); 3.36 (s, 3H); 3.58(m, 1H); 3.73 (s, 3H); 4.03(m, 1H); 4.12 (s, 3H); 4.28 (m, 2H); 7.77 (d, 1H); 8.69 (s, 1H); 12.68 (s, 1H). | |
| | 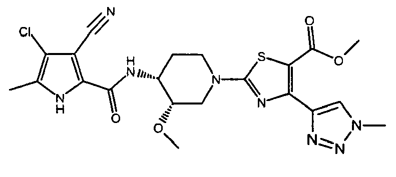 | | |
| **104** | methyl 2-((3S,4R)-4-(4-chloro-3-cyano-5-methyl-1H-pyrrole-2-carboxamido)-3-methoxypiperidin-1-yl)-4-(1-methyl-1H-1,2,4-triazol-5-yl)thiazole-5-carboxylate | MS (ES): 520 (MH⁺) for C₂₁H₂₃ClN₈O₄S | Intermediate 119 and Intermediate 9 |
| | | ¹H-NMR: 1.75 (m, 2H); 2.20 (s, 3H); 3.34 (s, 3H); 3.37 (m, 2H); 3.58(m, 1H); 3.60 (s, 3H); 3.72 (s, 3H); 3.96 (m, 1H); 4.25 (m, 2H); 7.75 (d, 1H); 8.01 (s, 1H); 12.67 (s, 1H). | |
| | 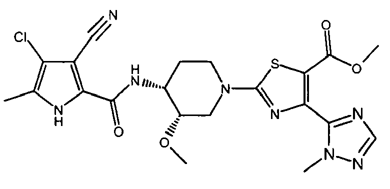 | | |
| **105** | ethyl 2-((3S,4R)-4-(4-chloro-3-cyano-5-methyl-1H-pyrrole-2-carboxamido)-3-methoxypiperidin-1-yl)-4-(pyrimidin-2-yl)thiazole-5-carboxylate | MS (ES): 531 (MH⁺) for C₂₃H₂₄ClN₇O₄S | Intermediate 119 and Intermediate 1 |
| | | ¹H-NMR: 1.08 (t, 3H); 1.77 (m, 2H); 2.21 (s, 3H); 3.33 (m, 2H); 3.35 (s, 3H); 3.58(m, 1H); 4.10 (q, 2H); 4.28 (m, 2H); 7.76 (d, 1H); 8.71 (m, 1H); 8.67 (m, 1H); 8.84 (m, 1H); 12.68 (s, 1H). | |
| | 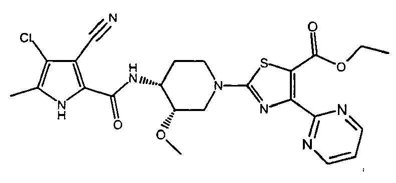 | | |
| **106** | ethyl 2-((3S,4R)4-(4-chloro-3-cyano-5-methyl-1H-pyrrole-2-carboxamido)-3-methoxypiperidin-1-yl)-4-(3-fluoropyridin-2-yl)thiazole-5-carboxylate | MS (ES): 548 (MH⁺) for C₂₄H₂₄ClFN₆O₄S | Intermediate 119 and Intermediate 86 |
| | | ¹H-NMR: 1.03 (t, 3H); 1.75 (m, 2H); 2.21 (s, 3H); 3.34 (s, 3H); 3.36 (m, 2H); 3.56 (m, 1H); 4.02 (m, 3H); 4.26 (m, 2H); 7.56 (m, 1H); 7.80 (m, 2H); 8.46 (d, 1H); 12.68 (s, 1H). | |
| | 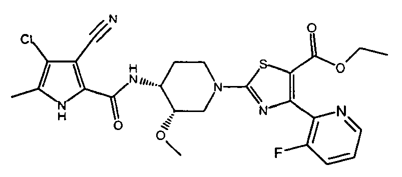 | | |
| **107** | ethyl 2-((3S,4R)-4-(4-chloro-3-cyano-5-mcthyl-1H-pyrrole-2-carboxamido)-3-methoxypiperidin-1-yl)-4-(5-methylpyrazin-2-yl)thiazole-5-carboxylate | MS (ES): 545 (MH⁺) for C₂₄H₂₆ClN₇O₄S | Intermediate 119 and Intermediate 85 |
| | 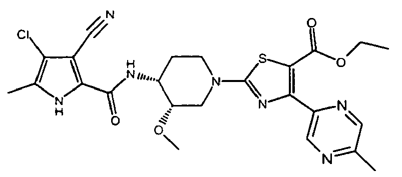 | | |
| **108** | ethyl 2-((3S,4R)-4-(4-chloro-3-cyano-5-methyl-1H-pyrrole-2-carboxamido)-3-methoxypiperidin-1-yl)-4-(1-methyl-1H-imidazol-4-yl)thiazole-5-carboxylate | MS (ES): 533 (MH⁺) for C₂₃H₂₆ClN₇O₄S | Intermediate 119 and Intermediate 12 |
| | | ¹H-NMR: 1.28 (t, 3H); 1.75 (m, 2H); 2.21 (s, 3H); 3.17 (m, 2H); 3.33 (m, 2H); 3.36 (s, 3H); 3.61 (m, 1H); 3.94 (s, 3H); 4.29 (m, 3H); 7.77 (d, 1H); 8.55 (s, 1H); 9.17 (s, 1H); 12.72 (br, 1H). | |
| | 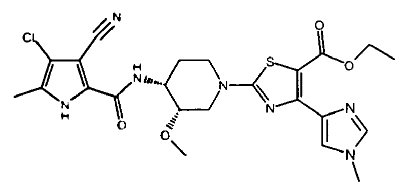 | | |

### Examples 109-125

The following compounds were synthesized according to the procedure described for Example 1.

| **Ex** | **Compound** | **Data** | **SM** |
|---|---|---|---|
| **109** | 2-((3S,4R)-4-(3,4-dichloro-5-methyl-1H-pyrrole-2-carboxamido)-3-methoxypiperidin-1-yl)-4-(1-methyl-1H-1,2,3-triazol-4-yl)thiazole-5-carboxylic acid | MS (ES): 515 (MH⁺) for C₁₉H₂₁Cl₂N₇O₄S | Example 92 |
| | | ¹H-NMR: 1.77 (m, 2H); 2.19 (s, 3H); 3.27 (m, 2H); 3.39 (s, 3H); 3.58 (m, 1H); 4.03(m, 1H); 4.15 (s, 3H); 4.29 (m, 2H); 7.18 (d, 1H);8.70 (s, 1H); 12.17 (s, 1H); 14.15 (s, br, 1H). | |
| | 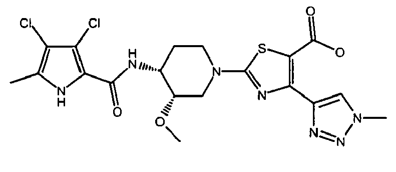 | | |
| **110** | 2-((3S,4R)-4-(3,4-dichloro-5-methyl-1H-pyrrole-2-carboxamido)-3-methoxypiperidin-1-yl)-4-(3,5-dimethylpyrazin-2-yl)thiazole-5-carboxylic acid | MS (ES): 540 (MH⁺) for C₂₂H₂₄Cl₂N₆O₄S | Example 93 |
| | | ¹H-NMR: 1.77 (m, 2H); 2.19 (s, 3H); 2.31 (s, 3H); 2.46 (s, 3H); 3.27 (m, 2H); 3.37 (s, 3H); 3.58(s, 3H); 3.58 (m, 1H); 4.03(m, 1H); 4.29 (m, 2H); 7.18 (d, 1H); 8.44 (s, 1H); 12.16 (s, 1H). | |
| | 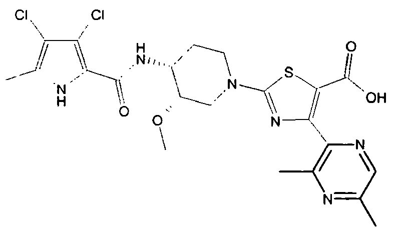 | | |
| **111** | 2-((3S,4R)-4-(3,4-dichloro-5-methyl-1H-pyrrolc-2-carboxamido)-3-methoxypiperidin-1-yl)-4-(6-methoxypyridin-2-yl)thiazole-5-carboxylic acid | MS (ES): 541 (MH⁺) for C₂₂H₂₃Cl₂N₅O₅S | Example 94 |
| | | ¹H-NMR: 1.77 (m, 2H); 2.19 (s, 3H); 3.30 (m, 1H); 3.40 (s, 3H); 3.40 (m, 1H); 3.58 (m, 1H); 3.99 (s, 3H); 4.27 (m, 2H); 4.40 (m, 1H); 7.17 (d, 1H); 7.18 (d, 1H); 7.97 (d, 1H); 8.10 (t, 1H); 12.16 (s, 1H). | |
| | 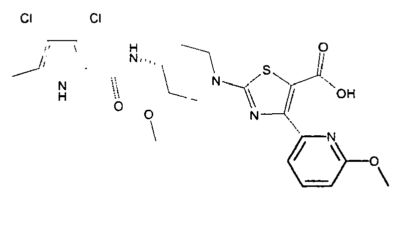 | | |
| **112** | 2-((3S,4R)-4-(3,4-dichloro-5-methyl-1H-pyrrole-2-carboxamido)-3-melhoxypiperidin-1-yl)-4-(4-methoxypyridin-2-yl)thiazole-5-carboxylic acid | MS (ES): 541 (MH⁺) for C₂₂H₂₃Cl₂N₅O₅S | Example 95 |
| | | ¹H-NMR: 1.79 (m, 2H); 2.19 (s, 3H); 3.32 (m, 2H); 3.40 (s, 3H); 3.58 (m, 1H); 3.99( s, 3H); 4.28 (m, 2H); 4.50 (m, 1H); 7.17 (d, 1H); 7.30 (d, 1H); 7.89 (s, 1H); 8.62 (d, 1H); 12.16 (s, 1H). | |
| | 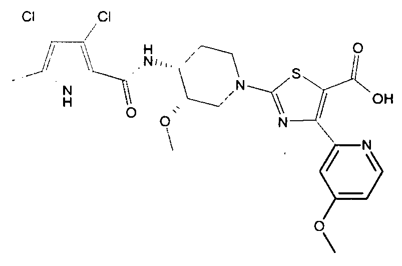 | | |
| **113** | 2-((3S,4R)-4-(3,4-dichloro-5-methyl-1H-pyrrole-2-carboxamido)-3-methoxypiperidin-1-yl)-4-(5-methylpyrazin-2-yl)thiazole-5-carboxylic acid | MS (ES): 526 (MH⁺) for C₂₁H₂₂Cl₂N₆O₄S | Example 96 |
| | | ¹H-NMR: 1.77 (m, 2H); 2.19 (s, 3H); 2.61 (s, 3H); 3.40 (s, 3H); 3.38 (m, 2H); 3.59 (m, 1H); 4.00 (m, 1H); 4.29 (m, 1H); 4.46 (m, 1H); 7.16 (d, 1H); 8.68 (s, 1H); 9.29 (s, 1H); 12.14 (s, 1H); 15.75 (s, 1H). | |
| | 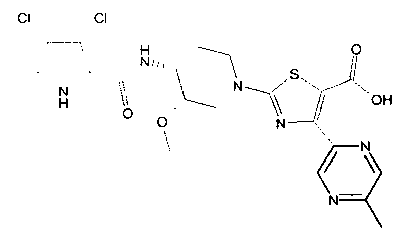 | | |
| **114** | 2-((3S,4R)-4-(3,4-dichloro-5-methyl-1H-pyrrole-2-carboxamido)-3-methoxypiperidin-1-yl)-4-(3-fluoropyridin-2-yl)thiazole-5-carboxylic acid | MS (ES): 529 (MH⁺) for C₂₁H₂₀Cl₂FN₅O₄S | Example 97 |
| | | ¹H-NMR: 1.77 (m, 2H); 2.19 (s, 3H); 3.37 (s, 3H); 3.40 (m, 2H); 3.56 (m, 1H); 3.96 (m, 1H); 4.26 (m, 2H); 7.17 (d, 1H); 7.54 (dd, 1H); 7.79 (t, 1H); 8.46 (d, 1H); 12.16 (s, 1H); 12.82 (br, 1H). | |
| | 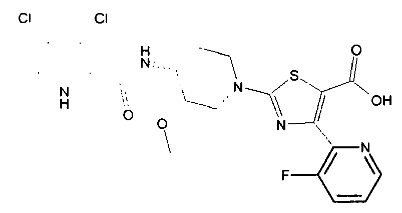 | | |
| **115** | 2-((3S,4R)-4-(3,4-dichloro-5-methyl-1H-pyrrole-2-carboxamido)-3-fluoropiperidin-1-yl)-4-(3-fluoropyridin-2-yl)thiazole-5-carboxylic acid | MS (ES): 529 (MH⁺) for C₂₁H₂₀FCl₂N₅O₄S | Example 98 |
| | | ¹H-NMR: 1.77 (m, 2H); 2.19 (s, 3H); 3.40 (m, 2H); 3.37 (s, 3H); 3.56 (m, 1H); 3.96 (m, 1H); 4.26 (m, 2H); 7.17 (d, 1H); 7.54 (t, 1H); 7.75 (t, 1H); 8.46 (s, 1H); 12.16 (s, 1H); 12.82 (br, 1H). | |
| | 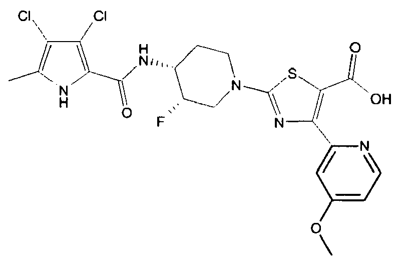 | | |
| **116** | 2-((3S,4R)-4-(3,4-dichloro-5-methyl-1H-pyrrole-2-carboxamido)-3-fluoropiperidin-1-yl)-4-(1-methyl-1H-1,2,3-triazol-4-yl)thiazole-5-carboxylic acid | MS (ES): 503 (MH⁺) for C₁₈H₁₈Cl₂FN₇O₃S | Example 99 |
| | | ¹H-NNMR: 1.88 (m, 2H); 2.19 (s, 3H); 3.27 (m, 2H); 3.35 (m, 2H); 3.53 (m, 1H); 3.65(m, 1H); 4.13 (m, 1H); 4.39 (m, 2H); 4.90∼5.06 (d, 1H); 7.29 (d, 1H);8.70 (s, 1H); 12.10 (s, 1H). | |
| | 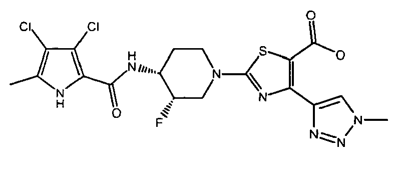 | | |
| **117** | 2-((3S,4R)-4-(3,4-dichloro-5-methyl-1H-pyrrole-2-carboxamido)-3-fluoropipcridin-1-yl)-4-(6-methoxypyridin-2-yl)thiazole-5-carboxylic acid | MS (ES): 529 (MH⁺) for C₂₁H₂₀Cl₂FN₅O₄S | Example 100 |
| | | ¹H-NMR: 1.88 (m, 2H); 2.20 (s, 3H); 3.39 (m, 2H); 3.50∼3.71 (dd, 1H); 3.97 (s, 3H); 4.14 (m, 1H); 4.43 (m, 2H); 4.92∼5.08 (d, 1H); 7.16 (d, 1H); 7.30 (d, 1H); 7.98 (d, 1H);8.10 (t, 1H); 12.12 (s, 1H). | |
| | 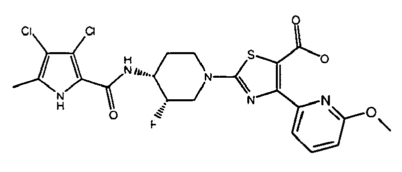 | | |
| **118** | 2-((3S,4R)-4-(3,4-dichloro-5-methyl-1H-pyrrole-2-carboxamido)-3-fluoropiperidin-1-yl)-4-(5-methylpyrazin-2-yl)thiazole-5-carboxylic acid | MS (ES): 514 (MH⁺) for C₂₀H₁₉Cl₂FN₆O₃S | Example 101 |
| | | ¹H-NMR: 1.89 (m, 2H); 2.20 (s, 3H); 2.61 (s, 3H); 3.41 (m, 1H); 3.50∼3.71 (dd, 1H); 4.11 (m, 1H); 4.43 (m, 2H); 4.92∼5.08 (d, 1H); 7.27 (d, 1H); 8.68 (s, 1H); 9.25 (s, 1H);12.10 (s, 1H); 15.64 (s, 1H). | |
| | 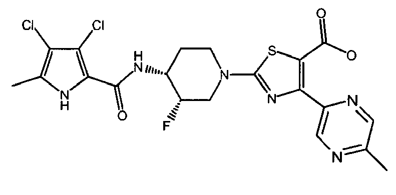 | | |
| **119** | 2-((35,4R)-4-(3,4-dichloro-5-methyl-1H-pyrrole-2-carboxamido)-3-fluoropiperidin-1-yl)-4-(3-fluoropyridin-2-yl)thiazole-5-carboxylic acid | MS (ES): 517 (MH⁺) for C₂₀H₁₇Cl₂F₂N₅O₃S | Example 102 |
| | | ¹H-NMR: 1.87 (m, 2H); 2.19 (s, 3H); 3.29 (m, 1H); 3.50∼3.71 (dd, 1H); 4.01 (m, 1H); 4.32 (m, 2H); 4.89∼5.05 (d, 1H); 7.32 (d, 1H); 7.54 (dd, 1H); 7.76 (t, 1H); 8.46 (d, 1H); 12.13 (s, 1H); 12.88 (br, 1H). | |
| | 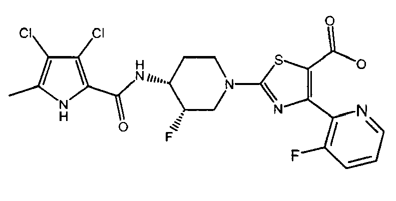 | | |
| **120** | 2-((3S,4R)-4-(4-chloro-3-cyano-5-methyl-1H-pyrrole-2-carboxamido)-3-methoxypiperidin-1-yl)-4-(1-methyl-1H-1,2,3-triazol-4-yl)thiazole-5-carboxylic acid | MS (ES): 505 (MH⁺) for C₂₀H₂₁ClN₈O₄S | Example 103 |
| | | ¹H-NMR: 1.74 (m, 2H); 2.21 (s, 3H); 3.33 (m, 2H); 3.50 (s, 3H); 3.58(m, 1H); 4.01 (m, 1H); 4.15 (s, 3H); 4.28 (m, 2H); 7.98 (d, 1H); 8.70 (s, 1H); 13.03 (s, 1H); 14.20 (br, 1H). | |
| | 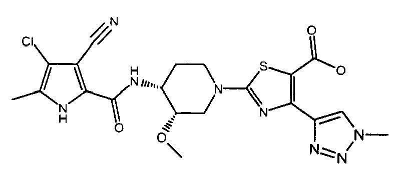 | | |
| **121** | 2-((3 S,4R)-4-(4-chloro-3-cyano-5-methyl-1H-pyrrole-2-carboxamido)-3-methoxypiperidin-l-yl)-4-(1-methyl-1H-1,2,4-triazol-5-yl)thiazole-5-carboxylic acid | MS (ES): 506 (MH⁺) for C₂₀H₂₁ClN₈O₄S | Example 104 |
| | | ¹H-NMR DMSO-D6 (DMSO-d₆) δ: 1.77 (m, 2H); 2.21 (s, 3H); 3.33 (m, 2H); 3.50 (s, 3H); 3.58(m, 1H); 4.01 (m, 1H); 4.08 (s, 3H); 4.28 (m, 2H); 8.05 (d, 1H); 8.25 (s, 1H); 13.16 (s, 1H); 15.47 (br, 1H). | |
| | 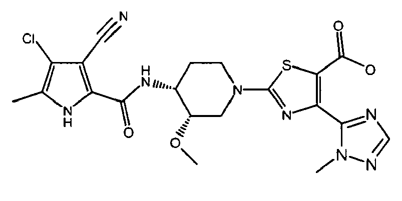 | | |
| **122** | 2-((3S,4R)-4-(4-chloro-3-cyano-5-methyl-1H-pyrrole-2-carboxamido)-3-methoxypiperidin-1-yl)-4-(pyrimidin-2-yl)thiazole-5-carboxylic acid | MS (ES): 503 (MH⁺) for C₂₁H₂₀ClN₇O₄S | Example 105 |
| | | ¹H-NMR: 1.76 (m, 2H); 2.20 (s, 3H); 3.33 (m, 2H); 3.50 (s, 3H); 3.60(m, 1H); 3.99 (m, 1H); 4.28 (m, 2H); 7.94 (d, 1H); 8.78 (s, 1H); 8.83 (s, 1H); 9.35 (s, 1H); 12.30 (s, 1H); 15.28 (br, 1H). | |
| | 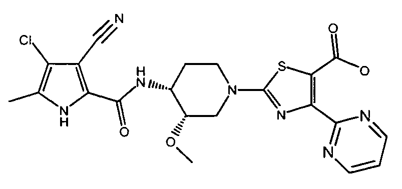 | | |
| **123** | 2-((3S,4R)-4-(4-chloro-3-cyano-5-methyl-1H-pyrrole-2-carboxamido)-3-methoxypiperidin-1-yl)-4-(3-fluoropyridin-2-yl)thiazole-5-carboxylic acid | MS (ES): 520 (MH⁺) for C₂₂H₂₀FClN₆O₄S | Example 106 |
| | | ¹H-NMR: 1.75 (m, 2H); 2.21 (s, 3H); 3.33 (m, 2H); 3.36 (s, 3H); 3.58(m, 1H); 3.98 (m, 1H); 4.26 (m, 2H); 7.54 (m, 1H); 7.78 (m, 2H); 8.46 (d, 1H); 12.76 (br, 1H). | |
| | 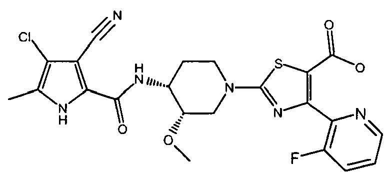 | | |
| **124** | 2-((3S,4R)-4-(4-chloro-3-cyano-5-methyl-1H-pyrrole-2-carboxamido)-3-methoxypiperidin-1-yl)-4-(5-methylpyrazin-2-yl)thiazole-5-carboxylic acid | MS (ES): 517 (MH⁺) for C₂₂H₂₂ClN₇O₄S | Example 107 |
| | 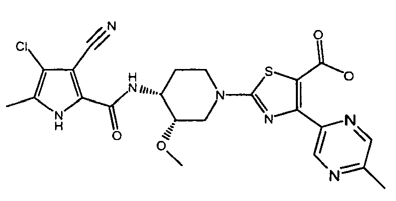 | ¹H-NMR: 1.75 (m, 2H); 2.21 (s, 3H); 2.60 (s, 3H); 3.33 (m, 2H); 3.35 (s, 3H); 3.50(m, 1H); 3.98 (m, 1H); 4.28 (m, 2H); 7.85 (d, 1H); 8.62 (s, 1H); 9.29 (s, 1H); 12.84 (s, 1H); 15.77 (br, 1H). | |
| **125** | 2-((3S,4R)-4-(4-chloro-3-cyano-5-methyl-1H-pyrrole-2-carboxamido)-3-methoxypiperidin-1-yl)-4-(1-methyl-1H-imidazol-4-yl)thiazole-5-carboxylic acid | MS (ES): 505 (MH⁺) for C₂₁H₂₂ClN₇O₄S | Example 108 |
| | | ¹H-NMR: 1.75 (m, 2H); 2.21 (s, 3H); 3.33 (m, 2H); 3.36 (s, 3H); 3.58(m, 1H); 3.82 (s, 3H); 3.98 (m, 1H); 4.26 (m, 2H); 7.82 (d, 1H); 7.93 (s, 1H); 8.37 (s, 1H); 12.76 (br, 1H). | |
| | 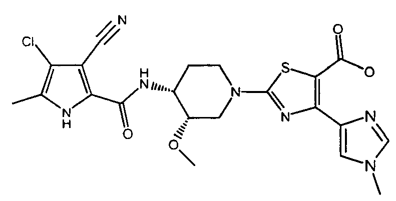 | | |

### Examples 126-140

The following compounds were synthesized according to the procedure described for Example 43.

| **Ex** | **Compound** | **Data** | **SM** |
|---|---|---|---|
| **126** | Methyl 2-[(3S,4R)-3-(benzyloxy)-4-{[(3,4-dichloro-5-methyl-1H-pyrrol-2-yl)carbonyl]amino}piperidin-1-yl]-4-(1-methyl-1H-1,2,4-triazol-5-yl)-1,3-thiazole-5-carboxylate | MS (ES): 604. (M+H). | Intermediate 103 and Intermediate 9 |
| | | ¹H NMR: 1.84 (m, 2H), 2.18 (s, 3H), 3.34 (m, 3H), 3.72 (s, 6H), 4.38 (br s, 1H), 4.53 (d, 1H), 4.76 (d, 1H), 7.13 (d, 1H), 7.32 (br s, 5H), 8.02 (s, 1H), 12.18 (s, 1H). | |
| | 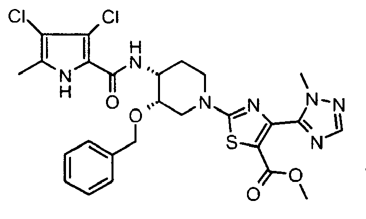 | | |
| **127** | Methyl 2-[(3S,4R)-3-(benzyloxy)-4-{[(3,4-dichloro-5-methyl-1H-pyrrol-2-yl)carbonyl]amino}piperidin-1-yl]-4-[1-(2-methoxyethyl)-1H-imidazol-2-yl]-1,3-thiazole-5-carboxylate | ¹H NMR: 1.82 (br s, 2H), 2.17 (s, 3H), 3.13 (s, 3H), 3.31 (m, 1H), 3.46 (t, 3H), 3.61 (s, 3H), 3.82 (s, 1H), 3.97 (t, 3H), 4.32 (m, 2H), 4.48 (d, 1H), 4.72 (d, 1H), 6.94 (s, 2H), 7.06 (d, 1H), 7.28 (s, 5H), 12.09 (s, 2H). | Intermediate 103 and Intermediate 8 |
| | 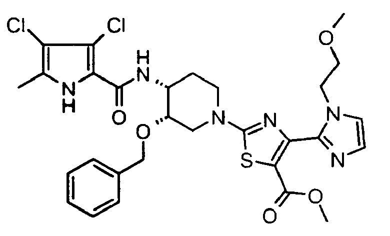 | | |
| **128** | Ethyl 2-[(3S,4R)-3-(benzyloxy)-4-{[(3,4-dichloro-5-methyl-1H-pyrrol-2-yl)carbonyl]amino}piperidin-1-yl]-4-(3-fluoropyridin-2-yl)-1,3-thiazole-5-carboxylate | MS (ES): 632 (M+H); | Intermediate 103 and Intermediate 86 |
| | | ¹H NMR: 1.15 (t, 3H), 1.87 (m, 2H), 2.19 (s, 3H), 3.47 (m, 2H), 3.87 (s, 1H), 4.08 (q, 2H), 4.38 (m, 2H), 4.49 (d, 1H), 4.74 (d, 1H), 7.15 (d, 1H), 7.26 (s, 5H), 7.64 (d, 1H), 7.78 (m, 1H), 8.48 (s, 1H), 12.18 (s, 1H). | |
| | 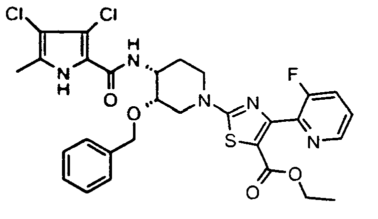 | | |
| **129** | Ethyl 2-[(3S,4R)-3-(benzyloxy)-4-{[(3,4-dichloro-5-methyl-1H-pyrrol-2-yl)carbonyl]amino}piperidin-1-yl]-4-(pyrazin-2-yl)-1,3-thiazole-5-carboxylate | MS (ES): 615 (M+H); ¹H NMR: 1.15 (t, 3H), 1.86 (m, 2H), 2.21 (s, 3H), 3.45 (d, 2H), 3.84 (s, 1H), 4.13 (q, 2H), 4.36 (m, 2H), 4.55 (d, 1H), 4.79 (d, 114), 7.16 (d, 1H), 7.25 (s, 5H), 8.65 (d, 1H), 8.72 (d, 1H), 8.75 (s, 1H), 12.16 (s, 1H). | Intermediate 103 and Intermediate 6 |
| | 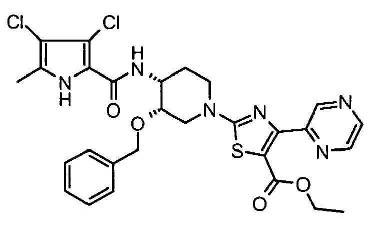 | | |
| **130** | Ethyl 2-[(3S,4R)-3-(benzyloxy)-4-{[(3,4-dichloro-5-methyl-1H-pyrrol-2-yl)carbonyl]amino}piperidin-1-yl]-4-(1H-tetrazol-5-yl)-1,3-thiazole-5-carboxylate | ¹H NMR: 1.12 (t, 3H), 1.85 (d, 2H), 2.18 (s, 3H), 3.34 (d, 1H), 3.84 (s, 1H), 4.14 (q, 2H), 4.36 (d, 1H), 4.51 (d, 1H), 4.73 (d, 1H), 7.08 (d, 1H), 7.20-7.38 (m, 5H), 12.09 (s, 1H). | Intermediate 103 and Intermediate 112 |
| | 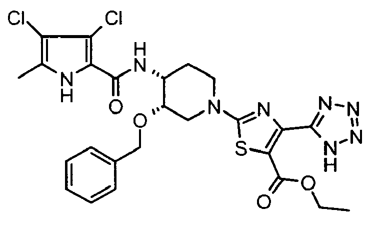 | | |
| **131** | Methyl 2-[(3S,4R)-3-(benzyloxy)-4-{[(4-chloro-5-methyl-1H-pyrrol-2-yl)carbonyl]amino}piperidin-1-yl]-4-(1-methyl-1H-1,2,4-triazol-5-yl)-1,3-thiazole-5-carboxylate | ¹H NMR: 1.68 (d, 2H), 2.19 (s, 3H), 3.72 (m, 6H), 3.21 (s, 2H), 4.26 (m, 2H), 4.51 (d, 1H), 4.68 (d, 1H), 6.92 (s, 1H), 7.21 (d, 3H), 7.31 (d, 2H), 7.74 (d, 1H), 8.12 (s, 1H), 11.62 (s, 2H). | Intermediate 104 and Intermediate 9 |
| | 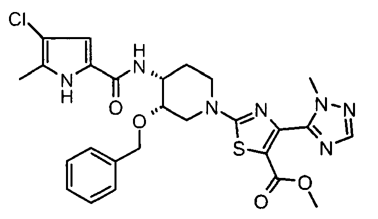 | | |
| **132** | Ethyl 2-[(3S,4R)-3-(benzyloxy)-4-{[(4-chloro-5-methyl-1H-pyrrol-2-yl)carbonyl]amino}piperidin-1-yl]-4-(1H-tetrazol-5-yl)-1,3-thiazole-5-carboxylate | ¹H NMR: 1.12 (t, 3H), 1.85 (d, 2H), 2.18 (s, 3H), 3.34 (d, 1H), 3.84 (s, 1H), 4.14 (q, 2H), 4.36 (d, 1H), 4.51 (d, 1H), 4.73 (d, 1H), 6.88 (s, 1H), 7.20-7.38 (m, 5H), 7.72 (d, 1H), 12.09 (s, 1H). | Intermediate 104 and Intermediate 112 |
| | 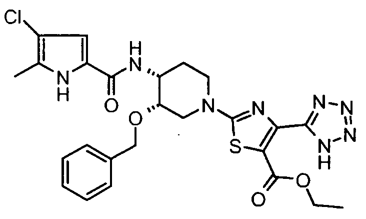 | MS (ES): 571 (M+H). | |
| **133** | Methyl 2-[(3S,4R)-3-(benzyloxy)-4-{[(4-chloro-5-methyl-1H-pyrrol-2-yl)carbonyl]amino}piperidin-1-yl]-4-[1-(2-methoxyethyl)-1H-imidazol-2-yl]-1,3-thiazole-5-carboxylate | ¹H NMR: 1.82 (br s, 2H), 2.17 (s, 3H), 3.13 (s, 3H), 3.31 (m, 1H), 3.46 (t, 3H), 3.61 (s, 3H), 3.82 (s, 1H), 3.97 (t, 3H), 4.32 (m, 2H), 4.48 (d, 1H), 4.72 (d, 1 H), 6.94 (s, 2H), 6.95 (s, 1H), 7.25 (m, 2H), 7.28 (m, 3H), 7.71 (d, 1H), 11.60 (s, 1H). | Intermediate 104 and Intermediate 8 |
| | 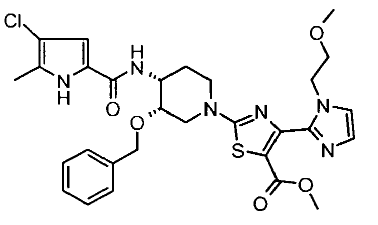 | | |
| **134** | Ethyl 2-[(3S,4R)-3-(benzyloxy)-4-{[(4-chloro-5-methyl-1H-pyrrol-2-yl)carbonyl]amino}piperidin-1-yl]-4-(3-fluoropyridin-2-yl)-1,3-thiazole-5-carboxylate | ¹H NMR:1.05 (t, 3H), 1.33 (m, 1H), 1.69 (m, 1H), 2.19 (s, 3H), 2.71 (s, 1H), 3.45 (m, 2H), 3.80 (s, 1H), 4.06 (q, 2H), 4.27 (d, 2H), 4.51 (d, 1H), 6.85 (s, 1H), 7.27 (d, 5H), 7.53 (m, 1H), 7.72 (d, 1H), 7.81 (t, 1H), 8.51 (s, 1H), 11.61 (s, 1H). | Intermediate 104 and Intermediate 86 |
| | 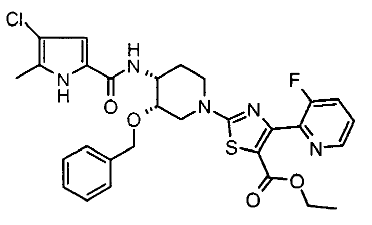 | | |
| **135** | Ethyl 2-[(3S,4R)-3-(benzyloxy)-4-{[(4-chloro-5-methyl-1H-pyrrol-2-yl)carbonyl]amino}piperidin-1-yl]-4-(pyrazin-2-yl)-1,3-thiazole-5-carboxylate | ¹H NMR: 1.14 (t, 3H), 1.64 (m, 1H), 1.95 (m, 1H), 2.19 (s, 3H), 2.71 (s, 1H), 3.42 (d, 2H), 3.80 (s, 1H), 4.15 (q, 2H), 4.35 (m, 3H), 4.48 (d, 1H), 4.56 (d, 1H), 6.85 (s, 1H), 7.24 (d, 5H), 7.76 (d, 1H), 8.75 (t, 3H), 11.60 (s, 1H). | Intermediate 104 and Intermediate 6 |
| | 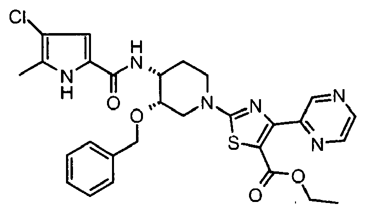 | | |
| **136** | Methyl 2-[(3S,4R)-3-(benzyloxy)-4-{[(4-chloro-3-cyano-5-methyl-1H-pyrrol-2-yl)carbonyl]amino}piperidin-yl]-4-(1-methyl-1H-1,2,4-triazol-5-yl)-1,3-thiazole-5-carboxylate | ¹H NMR: 1.86 (m, 2H), 2.21 (s, 3H), 3.58 (m, 4H), 3.72 (s, 6H), 3.85 (s, 1H), 4.38 (br s, 1H), 4.59 (d, 1H), 4.67 (d, 1H), 7.19 (s, 3H), 7.28 (s, 2H), 7.74 (d, 1H), 8.01 (s, 1H), 12.58 (s, 1H). | Intermediate 105 and Intermediate 9 |
| | 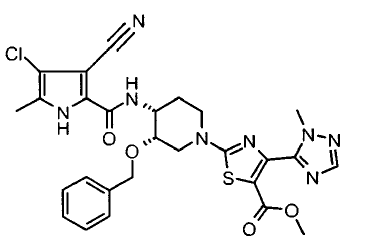 | | |
| **137** | Ethyl2-[(3S,4R)-3-(benzyloxy)-4-{[(4-chloro-3-cyano-5-methyl-1H-pyrrol-2-yl)carbonyl]amino}piperidin-1-yl]-4-(1H-tetrazol-5-yl)-1,3-thiazole-5-carboxylate | ¹H NMR: 1.12 (t, 3H), 1.85 (d, 2H), 2.18 (s, 3H), 3.34 (d, 1H), 3.84 (s, 1H), 4.14 (q, 2H), 4.36 (d, 1H), 4.51 (d, 1H), 4.73 (d, 1H), 7.20-7.38 (m, 5H), 7.72 (d, 1H), 12.09 (s, 1H). | Intermediate 105 and Intermediate 112 |
| | 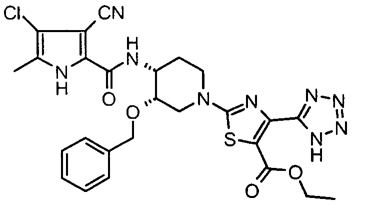 | | |
| **138** | Methyl 2-[(3S,4R)-3-(benzyloxy)-4-{[(4-chloro-3-cyano-5-methyl-1H-pyrrol-2-yl)carbonyl]amino}piperidin-1-yl]-4-[1-(2-methoxyethyl)-1H-imidazol-2-yl]-1,3-thiazole-5-carboxylate | ¹H NMR: 1.82 (br s, 2H), 2.17 (s, 3H), 3.13 (s, 3H), 3.31 (m, 1H), 3.46 (t, 3H), 3.61 (s, 3H), 3.82 (s, 1H), 3.97 (t, 3H), 4.32 (m, 2H), 4.48 (d, 1H), 4.72 (d, 1H), 6.94 (s, 2H), 7.18 (s, 2H), 7.25 (d, 3H), 7.63 (d, 1H), 12.60 (s, 1H). | Intermediate 105 and Intermediate 8 |
| | 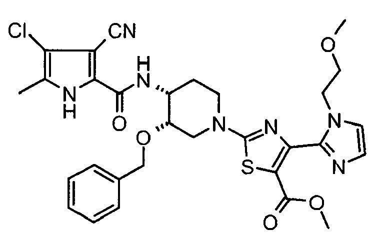 | | |
| **139** | Ethyl 2-[(3S,4R)-3-(benzyloxy)-4-{[(4-chloro-3-cyano-5-methyl-1H-pyrrol-2-yl)carbonyl]amino}piperidin-1-yl]-4-(3-fluoropyridin-2-yl)-1,3-thiazole-5-carboxylate | ¹H NMR: 1.05 (t, 3H), 1.78 (m, 1H), 1.91 (m, 1H), 2.23 (s, 3H), 3.43 (m, 2H), 3.83 (s, 1H), 4.08 (q, 2H), 4.23 (d, 2H), 4.45 (d, 1H), 4.63 (d, 1H), 7.19 (s, 3H), 7.21 (s, 2H), 7.52 (t, 1H), 7.64 (m, 1H), 7.82 (t, 1H), 8.52 (s, 1H), 12.60 (s, 1H). | Intermediate 105 and Intermediate 86 |
| | 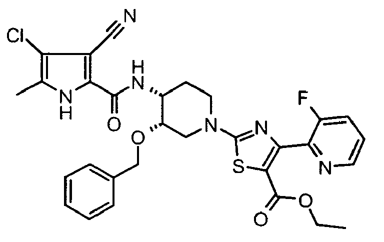 | | |
| **140** | Ethyl 2-[(3S,4R)-3-(benzyloxy)-4-{[(4-chloro-3-cyano-5-methyl-1H-pyrrol-2-yl)carbonyl]amino}piperidin-1-yl]-4-(pyrazin-2-yl)-1,3-thiazole-5-carboxylate | ¹H NMR: 1.09 (t, 3H), 1.79 (d, 1H), 2.00 (d, 1H), 2.23 (s, 3H), 3.43 (d, 2H), 3.84 (br s, 1H), 4.05 (q, 2H), 4.26 (m; 2H), 4.53 (d, 1H), 4.74 (d, 1H), 7.18 (s, 3H), 7.21 (s, 2H), 7.65 (br s, 1H), 8.79 (m, 3H), 12.61 (s, 1H). | Intermediate 105 and Intermediate 6 |
| | 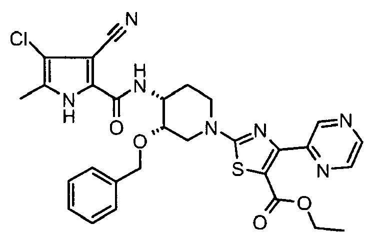 | | |

### Examples 141-155

The following compounds were synthesized according to the procedure described for Example 1.

| **Ex** | **Compound** | **Data** | **SM** |
|---|---|---|---|
| **141** | 2-[(3S,4R)-3-(benzyloxy)-4-{[(3,4-dichloro-5-methyl-1H-pyrrol-2-yl)carbonyl]ammo}piperidin-1-yl]-4-(1-methyl-1H-1,2,4-triazol-5-yl)-1,3-thiazole-5-carboxylic acid | ¹H NMR: 1.79 (m, 1H), 2.05 (m, 1H), 2.21 (s, 3H), 3.55 (m, 1H), 3.81 (s, 1H), 4.13 (s, 3H), 4.38 (br s, 1H), 4.58 (d, 1H), 4.72 (d, 1H), 7.18 (s, 3H), 7.29 (s, 3H), 7.29 (s, 3H), 7.97 (m, 1H), 8.31 (s, 1H), 12.96 (br s, 1H). | Example 126 |
| | 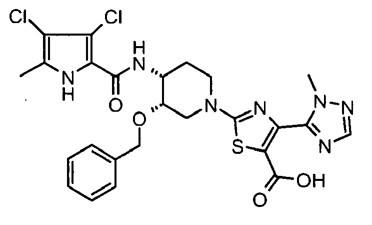 | | |
| **142** | 2-[(3S,4R)-3-(benzyloxy)-4-{[(3,4-dichloro-5-methyl-1H-pyrrol-2-yl)carbonyl]amino}pipendin-1-yl]-4-(1H-tetrazol-5-yl)-1,3-thiazole-5-carboxylic acid | ¹H NMR: δ 1.85 (d, 2H), 2.18 (s, 3H), 3.34 (d, 1H), 3.84 (s, 1H), 4.36 (d, 1H), 4.51 (d, 1H), 4.73 (d, 1H), 7.08 (d, 1H), 7.20-7.38 (m, 5H), 12.09 (s, 1H). | Example 130 |
| | 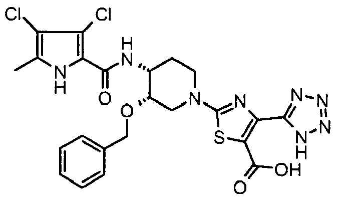 | | |
| **143** | 2-[(3S,4R)-3-(benzyloxy)-4-{[(3,4-dichloro-5-methyl-1H-pyrrol-2-yl)carbonyl]amino}piperidin-1-yl]-4-[1-(2-methoxyethyl)-1H-imidazol-2-yl]-1,3-thiazole-5-carboxylic acid | ¹H NMR: 1.82 (br s, 2H), 2.17 (s, 3H), 3.31 (m, 1H), 3.46 (t, 3H), 3.61 (s, 3H), 3.82 (s, 1H), 3.97 (t, 3H), 4.32 (m, 2H), 4.48 (d, 1H), 4.72 (d, 1H), 6.94 (s, 2H), 7.06 (d, 1H), 7.28 (s, 5H), 12.09 (s, 2H). | Example 127 |
| | 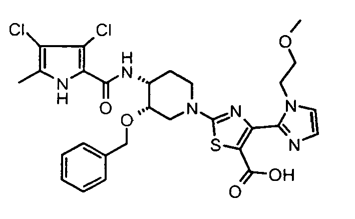 | | |
| **144** | 2-[(3S,4R)-3-(benzyloxy)-4-{[(3,4-dichloro-5-methyl-1H-pyrrol-2-yl)carbonyl]amino}piperidin-1-yl]-4-(3-fluoropyridin-2-yl)-1,3-thiazole-5-carboxylic acid | ¹H NMR: 1.81 (m, 2H), 2.19 (s, 3H), 3.42 (m, 2H), 3.81 (s, 1H), 3.95 (m, 1H), 4.38 (m, 2H), 4.47 (d, 1H), 4.76 (d, 1H), 7.13 (d, 1H), 7.26 (s, 5H), 7.59 (d, 1H), 7.85 (m, 1H), 8.49 (s, 1H), 12.12 (s, 1H), 12.84 (br s, 1H). | Example 128 |
| | 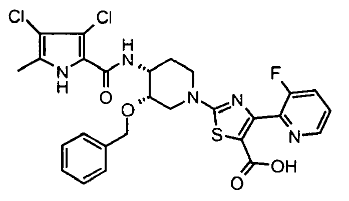 | | |
| **145** | 2-[(3S,4R)-3-(benzyloxy)-4-{[(3,4-dichloro-5-methyl-1H-pyrrol-2-yl)carbonyl]amino}piperidin-1-yl]-4-(pyrazin-2-yl)-1,3-thiazole-5-carboxylic acid | ¹H NMR: 1.82 (m, 2H), 2.18 s, 3H), 3.43 (m, 4H), 3.82 (s, 1H), 4.38 (br s, 1H), 4.51 (d, 1H), 4.76 (d, 1H), 7.23 (s, 5H), 7.13 (m, 1H), 8.83 (d, 2H), 9.24 (s, 1H), 12.01 (s, 1H). | Example 129 |
| | 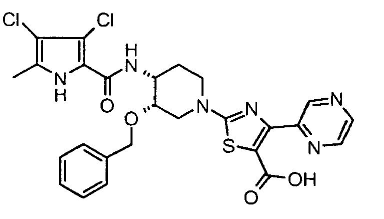 | | |
| **146** | 2-[(3S,4R)-3-(benzyloxy)-4-{[(4-chloro-5-methyl-1H-pyrrol-2-yl)carbonyl]amino}piperidin-1-yl]-4-(1-methyl-1H-1,2,4-triazol-5-yl)-1,3-thiazole-5-carboxylic acid | ¹H NMR: 1.72 (d, 1H), 2.10 (d, 1H), 2.22 (s, 3H), 3.45 (d, 2H), 3.84 (s, 1H), 4.16 (s, 3H), 4.31 (br s, 2H), 4.57 (d, 1H), 4.65 (d, 1H), 6.95 (s, 1H), 7.14 (d, 3H), 7.28 (d, 2H), 7.76 (d, 1H), 8.28 (s, 1H), 11.65 (s, 1H), 15.81 (s, 1H). | Example 131 |
| | 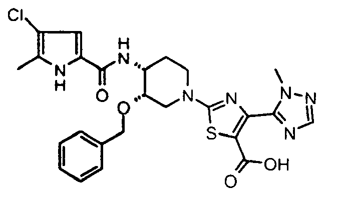 | | |
| **147** | 2-[(3S,4R)-3-(benzyloxy)-4-{[(4-chloro-5-methyl-1H-pyrrol-2-yl)carbonyl]amino}piperidin-1-yl]-4-(1H-tetrazol-5-yl)-1,3-thiazole-5-carboxylic acid | ¹H NMR: 1.85 (d, 2H), 2.18 (s, 3H), 3.34 (d, 1H), 3.84 (s, 1H), 4.36 (d, 1H), 4.51 (d, 1H), 4.73 (d, 1H), 6.88 (s, 1H), 7.20-7.38 (m, 5H), 7.72 (d, 1H), 12.09 (s, 1H). | Example 132 |
| | 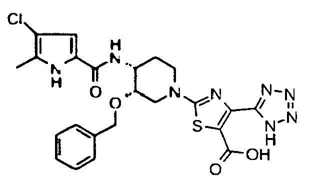 | | |
| **148** | 2-[(3S,4R)-3-(benzyloxy)-4-{[(4-chloro-5-methyl-1H-pyrrol-2-yl)carbonyl]amino}piperidin-1-yl]-4-[1-(2-methoxyethyl)-1H-imidazol-2-yl]-1,3-thiazole-5-carboxylic acid | ¹H NMR: 1.82 (br s, 2H), 2.17 (s, 3H), 3.31 (m, 1H), 3.46 (t, 3H), 3.61 (s, 3H), 3.82 (s, 1H), 3.97 (t, 3H), 4.32 (m, 2H), 4.48 (d, 1H), 4.72 (d, 1H), 6.94 (s, 2H), 6.95 (s, 1H), 7.25 (m, 2H), 7.28 (m, 3H), 7.71 (d, 1H), 11.60 (s, 1H). | Example 133 |
| | 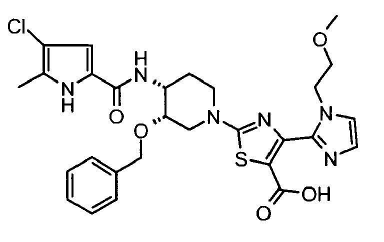 | | |
| **149** | 2-[(3S,4R)-3-(benzyloxy)-4-{[(4-chloro-5-methyl-1H-pyrrol-2-y))carbonyl]amino}piperidin-1-yl]-4-(3-fluoropyridin-2-yl)-1,3-thiazole-5-carboxylic acid | ¹H NMR: 1.67 (d, 2H), 2.18 (s, 3H), 3.45 (m, 3H), 3.79 (s, 1H), 4.02 (m, 1H), 4.38 (m, 2H), 4.51 (d, 1H), 4.71 (d, 1H), 6.91 (s, 1H), 7.31 (m, 5H), 7.59 (m, 1H), 7.81 (m, 3H), 8.51 (s, 1H), 11.61 (s, 1H), 12.82 (m, 1H). | Example 134 |
| | 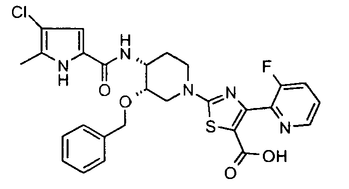 | | |
| **150** | 2-[(3S,4R)-3-(benzyloxy)-4-{[(4-chloro-5-methyl-1H-pyrrol-2-yl)carbonyl]amino}piperidin-1-yl]-4-(pyrazin-2-yl)-1,3-thiazole-5-carboxylic acid | ¹H NMR: 1.72 (d, 2H), 2.21 (s, 3H), 3.45 (m, 3H), 3.84 (s, 1H), 4.02 (br s, 1H), 4.31 (m, 1H), 4.55 (d, 1H), 4.75 (d, 1H), 6.93 (s, 1H), 7.21 (d, 5H), 7.72 (d, 1H), 8.81 (d, 2H), 9.41 (s, 1H), 11.61 (s, 1H), 15.39 (s, 1H). | Example 135 |
| | 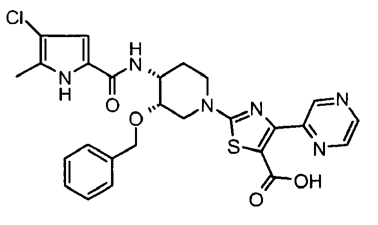 | | |
| **151** | 2-[(3S,4R)-3-(benzyloxy)-4-{[(4-chloro-3-cyano-5-methyl-1H-pyrrol-2-yl)carbonyl]amino}piperidin-1-yl]-4-(1-methyl-1H-1,2,4-triazol-5-yl)-1,3-thiazole-5-carboxylic acid | ¹H NMR: 1.86 (m, 2H), 2.21 (s, 3H), 3.52 (m, 4H), 3.79 (s, 3H), 3.94 (m, 1H), 4.39 (br s, 1H), 4.47 (d, 1H), 4.78 (d, 1H), 7.19 (d, 1H), 7.27 (d, 5H), 7.95 (s, 1H). | Example 136 |
| | 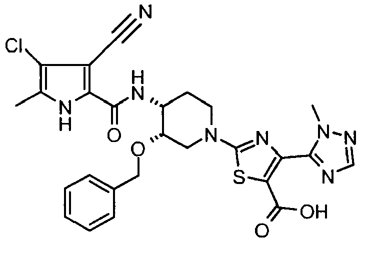 | | |
| **152** | 2-[(3S,4R)-3-(benzyloxy)-4-{[(4-chloro-3-cyano-5-methyl-1H-pyrrol-2-yl)carbonyl]amino}piperidin-1-yl]-4-(1H-tetrazol-5-yl)-1,3-thiazole-5-carboxylic acid | ¹H NMR: 1.85 (d, 2H), 2.18 (s, 3H), 3.34 (d, 1H), 3.84 (s, 1H), 4.36 (d, 1H), 4.51 (d, 1H), 4.73 (d, 1H), 7.20-7.38 (m, 5H), 7.72 (d, 1H), 12.09 (s, 1H). | Example 137 |
| | 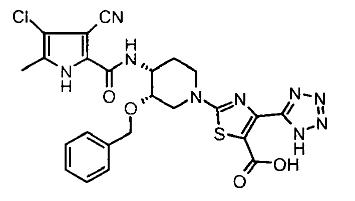 | | |
| **153** | 2-[(3S,4R)-3-(benzyloxy)-4-{[(4chloro-3-cyano-5-methyl-1H-pyrrol-2-yl)carbonyl]amino}piperidin-1-yl]-4-[1-(2-methoxyethyl)-1H-imidazol-2-yl]-1,3-thiazole-5-carboxylic acid | ¹H NMR: 1.82 (br s, 2H), 2.17 (s, 3H), 3.31 (m, 1H), 3.46 (t, 3H), 3.61 (s, 3H), 3.82 (s, 1H), 3.97 (t, 3H), 4.32 (m, 2H), 4.48 (d, 1H), 4.72 (d, 1H), 6.94 (s, 2H), 7.18 (s, 2H), 7.25 (d, 3H), 7.63 (d, 1H), 12.60 (s, 1H). | Example 138 |
| | 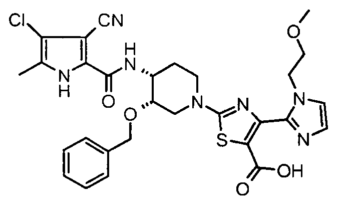 | | |
| **154** | 2-[(3S,4R)-3-(benzyloxy)-4-{[(4-chloro-3-cyano-5-methyl-1H-pyrrol-2-yl)carbonyl]amino}piperidin-1-yl]-4-(3-fluoropyridin-2-yl)-1,3-thiazole-5-carboxylic acid | ¹H NMR: 1.72 (m, 1H), 1.92 (m, 1H), 2.24 (s, 3H), 3.45 (m, 2H), 3.81 (s, 1H), 4.03 (br s, 1H), 4.32 (m, 2H), 4.54 (d, 1H), 4.63 (d, 1H), 7.18 (d, 3H), 7.23 (d, 2H), 7.52 (m, 1H), 7.87 (m, 2H), 8.49 (d, 1H), 12.73 (s, 1H). | Example 139 |
| | 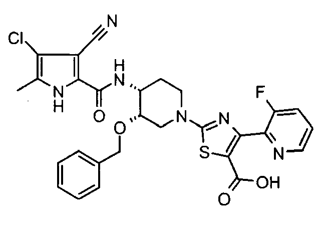 | | |
| **155** | 2-[(3S,4R)-3-(benzyloxy)-4-{[(4-chloro-3-cyano-5-methyl-1H-pyrrol-2-yl)carbonyl]amino}piperidin-1-yl]-4-(pyrazin-2-yl)-1,3-thiazole-5-carboxylic acid | ¹H NMR: 1.84 (d, 1H), 2.03 (m, 1H), 2.21 (s, 3H), 3.45 (m, 2H), 3.84 (s, 1H), 4.12 (m, 1H), 4.32 (m, 2H), 4.56 (d, 1H), 4.65 (d, 1H), 7.15 (s, 3H), 7.25 (s, 2H), 7.94 (d, 1H), 8.87 (d, 2H), 9.25 (s, 1H), 12.97 (s, 1H). | Example 140 |
| | 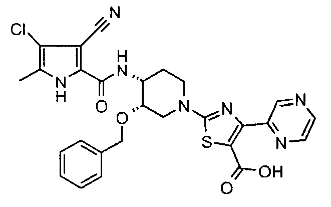 | | |

### Examples 156-158

The following compounds were synthesized according to the procedure described for Example 43.

| **Ex** | **Compound** | **Data** | S**M** |
|---|---|---|---|
| **156** | Methyl 2-[(3S,4R)-4-{[(3,4-dichlorc methyl-1H-pyrrol-2-yl)carbonyl]am 3-propoxypiperidin-1-yl]-4-(1-methy 1,2,4-triazol-5-yl)-1,3-thiazole-5-carboxylate | ¹H NMR (400 MHz, DMSO-d₆): 0.81 (t, 3H), 1.47 (m, 2H), 1.79 (br s, 2H), 2.18 (s, 3H), 3.44 (m, 3H), 3.58 (q, 2H), 3.65 (s, 3H), 3.71 (s, 3H), 3.91 (br s, 1H), 4.29 (m, 2H), 7.10 (d, 1H), 8.00 (s, 1H), 12.04 (s, 1H). | Intermediate 115 and Intermediate 9 |
| | 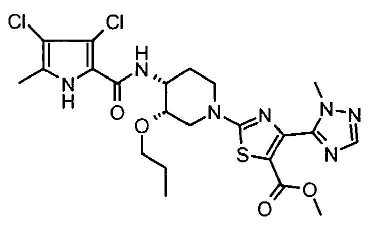 | | |
| **157** | Ethyl 2-[(3S,4R)-4-{[(3,4-dichloro-5-methyl-1H-pyrrol-2-yl)carbonyl]amino}-3-propoxypiperidin-1-yl]-4-(3-fluoropyridin-2-yl)-1,3-thiazole-5-carboxylate | ¹H NMR (400 MHz, DMSO-d₆): 0.80 (t, 3H), 1.04 (t, 3H), 1.45 (m, 2H), 1.78 (br s, 2H), 2.18 (s, 3H), 3.37 (m, 2H), 3.60 (t, 2H), 3.64 (br s, 1H), 4.05 (q, 2H), 4.29 (m, 2H), 7.09 (d, 1H), 7.56 (m, 1H), 7.80 (t, 1H), 8.45 (s, 1H), 12.14 (s, 1H). | Intermediate 115 and Intermediate 86 |
| | 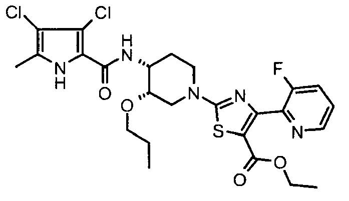 | | |
| **158** | Ethyl 2-[(3S,4R)-4-{[(3,4-dichloro-5-methyl-1H-pyrrol-2-yl)carbonyl]amino}-3-propoxypiperidin-1-yl]-4-(pyrazin-2-yl)-1,3-thiazole-5-carboxylate | ¹H NMR (400 MHz, DMSO-d₆): 0.80 (t, 3H), 1.04 (t, 3H), 1.45 (m, 2H), 1.78 (br s, 2H), 2.18 (s, 3H), 3.39 (m, 5H), 4.10 (q, 2H), 4.25 (br s, 2H), 7.11 (d, 1H), 8.69 (d, 2H), 8.81 (s, 1H), 12.14 (s, 1H). | Intermediate 115 and Intermediate 6 |
| | 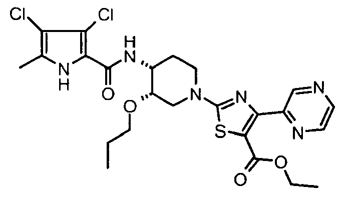 | | |

### Examples 159-161

The following compounds were synthesized according to the procedure described for Example 1.

| **Ex** | **Compound** | **Data** | **SM** |
|---|---|---|---|
| **159** | 2-[(3*S*,4*R*)-4-{[(3,4-dichloro-5-methyl-1*H*-pyrrol-2-yl)carbonyl]amino}-3-propoxypiperidin-1-yl]-4-(1-methyl-1*H*-1,2,4-triazol-5-yl)-1,3-thiazole-5-carboxylic acid | ¹H NMR (400 MHz, DMSO-d₆): 0.81 (t, 3H), 1.47 (m, 2H), 1.79 (br s, 2H), 2.18 (s, 3H), 3.44 (m, 3H), 3.58 (q, 2H), 3.65 (s, 3H), 3.91 (br s, 1H), 4.29 (m, 2H), 7.10 (d, 1H), 8.00 (s, 1H), 12.04 (s, 1H). | Example 156 |
| | 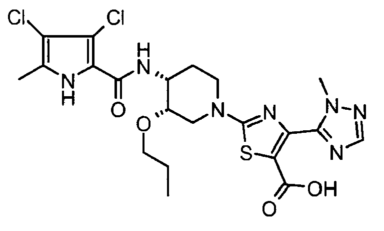 | | |
| **160** | 2-[(3*S*,4*R*)-4-{[(3,4-dichloro-5-methyl-1H-pyrrol-2-yl)carbonyl]amino}-3-propoxypiperidin-1-yl]-4-(3-fluoropyridin-2-yl)-1,3-thiazole-5-carboxylic acid | ¹H NMR (400 MHz, DMSO-d₆): 1.04 (t, 3H), 1.45 (m, 2H), 1.78 (br s, 2H), 2.18 (s, 3H), 3.37 (m, 2H), 3.60 (t, 2H), 3.64 (br s, 1H), 4.29 (m, 2H), 7.09 (d, 1H), 7.56 (m, 1H), 7.80 (t, 1H), 8.45 (s, 1H), 12.14 (s, 1H). | Example 157 |
| | 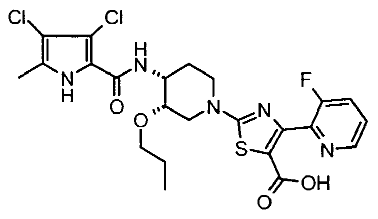 | | |
| **161** | 2-[(3S,4R)-4-{[(3,4-dichloro-5-methyl-1H-pyrrol-2-yl)carbonyl]amino}-3-propoxypiperidin-1-yl]-4-(pyrazin-2-yl)-1,3-thiazole-5-carboxylic acid | ¹H NMR (400 MHz, DMSO-d₆): 1.04 (t, 3H), 1.45 (m, 2H), 1.78 (br s, 2H), 2.18 (s, 3H), 3.39 (m, 5H), 4.25 (br s, 2H), 7.11 (d, 1H), 8.69 (d, 2H), 8.81 (s, 1H), 12.14 (s, 1H). | Example 158 |
| | 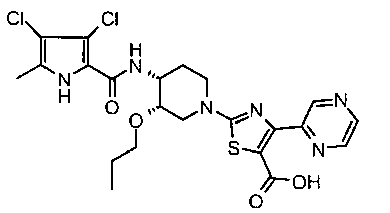 | | |

### Example 162

### 2-{(3S,4R)-4-[(4-Chloro-3-cyano-5-methyl-1H-pyrrole-2-carbonyl)-amino]-3-fluoro-piperidin-1-yl}-4-pyrazin-2-yl-thiazole-5-carboxylic acid ethyl ester

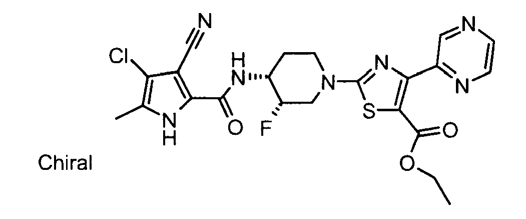

In a 10 mL round-bottomed flask, were taken 4-chloro-3-cyano-N- ((3S,4R)-3-fluoropiperidin-4-yl)-5-methyl-1H-pyrrole-2-carboxamide hydrochloride (WO 2006/087543, 107 mg, 0.33 mmol) and ethyl 2-chloro-4-(pyrazin-2-yl)thiazole-5-carboxylate (Intermediate 116, 90 mg. 0.33 mmol) in 2 mL of DMF and stirred at RT. To this diisopropylethylamine (0.174 mL, 1 mmol) was added, and the resulting reaction mixture was stirred at 60 °C for 4 hrs. The reaction mixture was monitored by LCMS and LCMS profile showed completion of reaction after 4 hr. The reaction mixture was evaporated *in vacuo* and ice cool water was added. The mixture was sonicated and the solid precipitate was filtered and dried under high vacuum to afford the title compound as solid (175 mg).
MS (ES⁺): 518 for C₂₂H₂₅ClN₇O₃S

### Examples 163-167

The following compounds were prepared in a manner analogous to Example 162 from starting materials listed and corresponding chloro thiazoles. The resulting crude material was taken for next step without further purification.

| **Ex** | **Compound** | **m/z** **(ES⁺)** | **SM** |
|---|---|---|---|
| **163** | 2-{(3S, 4R)-4-[(4-Chloro-3, 5-dimethyl-1H-pyrrole-2-carbonyl)-amino]-3-fluoro-piperidin-1-yl}-4-pyrazin-2-yl-thiazole-5-carboxylic acid ethyl ester | 507 | Intermediate 6 and Intermediate 117 |
| | 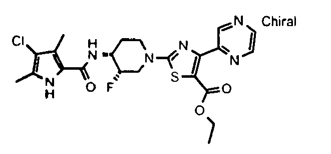 | | |
| **164** | 2-{(3S,4R)-4-[(4-chloro-3,5-dimethyl-1H-pyrrole-2carbonyl)-amino]-3-fluoro-piperidin-1-yl}4-pyrimidin-4-yl-thiazole-5-carboxylic acid ethyl ester | 507 | Intermediate 2 and Intermediate 117 |
| | 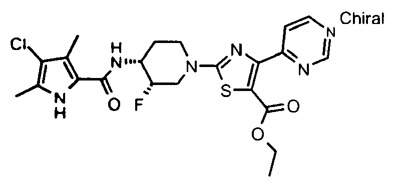 | | |
| **165** | 2-{(3S,4R)-4-[(4-[(4-Chloro-3-cyano-5-methyl-1H-pyrrole-2-carbonyl)-amino] -3-methoxy-piperidin-1-yl}-4-pyrimidin-4-yl-thiazole-5-carboxylic acid ethyl ester | 531 | Intermediate 2 and Intermediate 119 |
| | 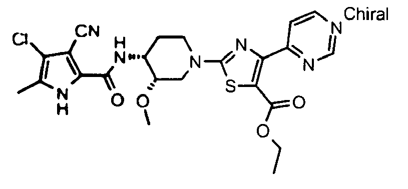 | | |
| **166** | 2-{(3S, 4R)-4-[(4-Chloro-3-cyano-5-methyl-1H-pyrrole-2-carbonyl) amino] -3-methoxy-piperidin-1-yl}-4-(1-methyl-1H-pyrazol-3-yl)-thiazole-5-carboxylic acid ethyl ester | 533 | Intermediate 60 and Intermediate 119 |
| | 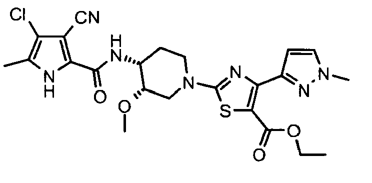 | | |
| **167** | 2-{(3S, 4R)-4-[(3,4-Dichloro-5-methyl-1H-pyrrole-2-carbonyl)-amino]-3-methoxy-piperidin-1-yl}-4-(1-methyl-1H-pyrazol-3-yl)-thiazole-5-carboxylic acid ethyl ester | 542 | Intermediate 60 and 3,4-Dichloro-5-methyl-1*H*-pyrrole-2-carboxylic acid ((3S, 4R)-3-methoxy-piperidin-4-yl)-amide (WO 2006087543) |
| | 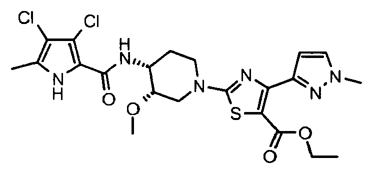 | | |

### Example 168

### 2-{(3S, 4R)-4-[(4-Chloro-3-cyano-5-methyl-1H-pyrrole-2-carbonyl)-amino]-3-fluoro-piperidin-1-yl}-4-pyrazin-2-yl-thiazole-5-carboxylic acid

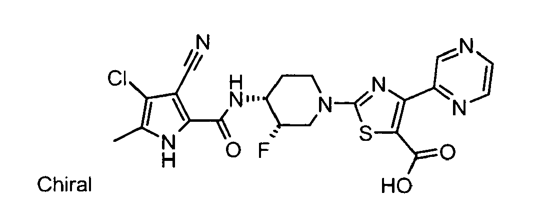

A solution of lithium hydroxide monohydrate (131 mg, 5.48 mmol) in water (1 mL) was added to a stirred solution of Ethyl 2-((3S, 4R)-4-(4-chloro-3-cyano-5-methyl-1H-pyrrole-2-carboxamido)-3-fluoropiperidin-1-yl)-4-(pyrazin-2-yl) thiazole-5-carboxylate (Example 162, 142 mg, 0.27 mmol) in THF (8 mL) and MeOH (2 mL) and the resultant solution was stirred at 60° C overnight. The reaction mixture was concentrated and dissolved in water (5 mL). The solution was acidified with 6N hydrochloric acid, and the precipitate that formed was collected, washed with water and dried under high vacuum (82 mg, 61.1%).
MS (ES⁺): 490 for C₂₀H₁₇ClN₇O₃S
H¹NMR [DMSO-d⁶] δ : 1.85-1.95(m, 2H); 2.20(s,3H); 3.50-3.60 (m,2H); 4.15 (d, 1H); 4.25-4.43 (m, 2H); 4.90-5.10 (d, 1H); 8.30(d,1H); 8.90(d,2H) ;9.30(s,1H); 13.0(bs,1H); 15.15(bs,1H);

### Examples 169-173

The following compounds were synthesized by an analogous method to Example 168 from the starting material (SM) given in the table below.

| **Ex** | **Compound** | **M/Z** | **¹HNMR (300MHz)** (δ) | **SM** |
|---|---|---|---|---|
| 169 | 2-[(3*S*,4*R*)-4-{[(4-chloro-3,5-dimethyl-1*H*-pyrrol-2-yl)carbonyl]amino}-3-fluoropiperidin-1-yl]-4-pyrazin-2-yl-1,3-thiazole-5-carboxylic acid | 479 | H¹NMR [DMSO-d⁶] δ : 1.80-1.95(m, 2H); 2.17(s,3H); 2.21(s,3H); 3.35-3.42 (m,1H); 3.50-3.70 (m,2H);4.15 (d, 1H); 4.25-4.47 (m, 2H); 4.90-5.05 (d, 1H); 7.45(d,1H); 8.85(d,2H) ;9.30(s,1H) ; 11.35(bs,1H); 15.10(bs,1H); | Example 163 |
| | 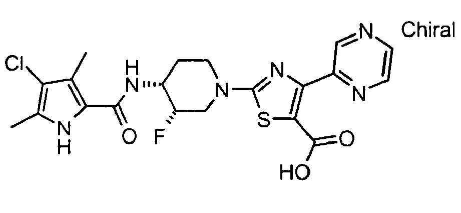 | | | |
| 170 | 2-[(3*S*,4*R*)-4-{[(4-chloro-3,5-dimethyl-1*H*-pyrrol-1-yl)carbonyl]amino}-3-fluoropiperidin-1-yl]-4-pyrimidin-4-yl-1,3-thiazole-5-carboxylic acid | 479 | H¹NMR [DMSO-d⁶) δ : 1.79-1.91(m, 2H); 2.15(d,6H); 3.35-3.42 (m,1H); 3.50-3.70 (m,2H);4.15 (d, 1H); 4.25-4.47 (m, 2H); 4.85-5.05 (d, 1H); 7.40(d,1H); 8.25(d,2H) ; 9.10(d,2H) ;9.40(s,1H) ; 11.30(bs,1H); | Example 164 |
| | 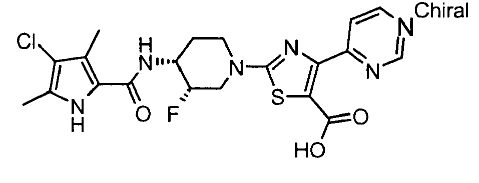 | | | |
| 171 | 2-[(3*S*,4*R*)-4-{[(4-chloro-3-cyano-5-methyl-1*H*-pyrrol-2-yl)carbonyl]amino}-3-methoxypiperidin-1-yl]-4-pyrimidin-4-yl-1,3-thiazole-5-carboxylic acid | 502 | H¹NMR [DMSO-d⁶] δ : 1.75-1.90(m, 2H); 2.20(s, 3H); 3.25 (s,3H); 3.40-3.50 (m, 2H);3.60 (s, 1H); 4. 05 (bs, 1H); 4. 25-4.47 (m, 2H); 7.75(d, 1H); 8.30(d, 2H); 9.15(d, 1H); 9.35(s, 1H) 12.65(bs, 1H); | Example 165 |
| | 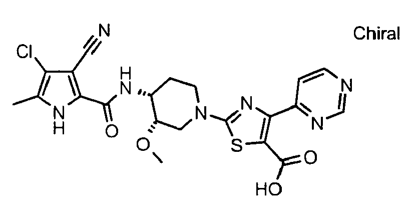 | | | |
| 172 | 1-[(3*S*,4*R*)-4-{[(4-chloro-3-cyano-5-methyl-1*H*-pyrrol-2-yl)carbonyl]amino}-3-methoxypiperidin-1-yl]-4-(1-methyl-1*H*-pyrazol-3-yl)-1,3-thiazole-5-carboxylic acid | 504 | H¹NMR [DMSO-d⁶] δ : 1.75-1.90(m, 2H); 2.20(s, 3H); 3.35 -3.50 (m, 5H);3.60 (s, 1H); 3.90-4.0 (m, 4H); 4. 25-4.45 (m, 2H); 6.85(s, 1H); 7.82(d, 1H); 7.95(s, 1H); 12.75(bs, 1H); | Example 166 |
| | 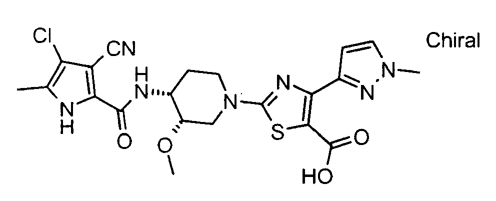 | | | |
| 173 | 2-[(3*S*,4*R*)-4-{[(3,4-dichloro-5-methyl-1*H*-pyrrol-2-yl)carbonyl]amino}-3-mcthoxypipcridin-1-yl]-4-(1-methyl-1*H*-pyrazol-3-y))-1,3-thiazole-5-carboxylic acid | 513 | H¹NMR [DMSO-d⁶] δ : 1.75-1.85(m, 2H); 2.20(s, 3H); 3.25(s, 3H); 3.30 - 3.40 (m, 2H);3.60 (s, 1H); 3.95-4.0 (m, 4H); 4. 25-4.45 (m, 2H); 6.80(s, 1H); 7.15(d, 1H); 7.95(s, 1H); 12.10(bs, 1H); 15.0(bs, 1H); | Example 167 |
| | 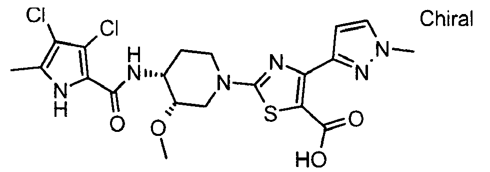 | | | |

### Preparation of Starting Materials

### Intermediate 1

### Ethyl 2-chloro-4-pyrimidin-2-yl-1,3-thiazole-5-carboxylate

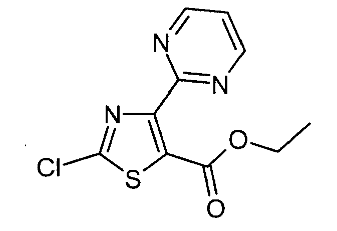

Ethyl 2-amino-4-pyrimidin-2-yl-1,3-thiazole-5-carboxylate (Intermediate 23; 0.55 g, 2.2 mmol) was suspended in glacial acetic acid (20 ml) and concentrated HCl (30 ml). The solution was cooled to 0 °C and to this a solution of sodium nitrite in water (15 ml) was added dropwise. After stirring at 0 °C for 10 mins, the reaction was slowly warmed to room temperature and stirred for 1 hour. Once complete by LCMS, a solution of urea (0.25 g) in water (10 ml) was added dropwise. After stirring at room temperature for 30 mins, solvent was removed under reduced pressure. The residue was partitioned with sat. NaHCO₃ (aq) and EtOAc. Extraction with EtOAc (x3), drying with MgSO₄ and concentrating yielded an orange oil which was used without purification (0.20 g). MS (ES) (M+H)⁺: 270 for C₁₀H₈ClN₃O₂S

### Intermediates 2-12

The following Intermediates were prepared by the procedure described in Intermediate 1 from the starting materials (SM) indicated.

| **Int** | **Compound** | **Data** | **SM** |
|---|---|---|---|
| **2** | Ethyl 2-chloro-4-pyrimidin-4-yl-1,3-thiazole-5-carboxylate | MS (ES) (M+H)⁺: 271 for C₁₀H₈ClN₃O₂S | Intermediate 24 |
| | 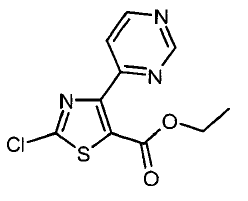 | NMR: 1.19 (t, 3H), 4.24 (q, 2H), 7.92 (d, 2H), 8.98 (d, 2H), 9.29 (s, 1H) | |
| **3** | Methyl 2'-chloro-2,4'-bi-1,3-thiazole-5'-carboxylate | MS (ES) (M+H)⁺: 261 for C₈H₅ClN₂O₂S₂ | Intermediate 25 |
| | 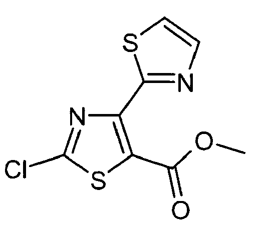 | NMR: 3.84 (s, 3H), 8.02 (m, 2H) | |
| **4** | Ethyl 4-[2,6-bis(dimethylamino)pyrimidin-4- | MS (ES) (M+H)⁺: 356 for C₁₄H₁₈CN₅O₂S | Intermediate 26 |
| | yl]-2-chloro-1,3-thiazole-5-carboxylate | NMR: 1.21 (t, 3H), 3.08 (s, 3H), 3.10 (s, 3H), 4.25 (q 2H), 6.45 (s, 1H) | |
| | 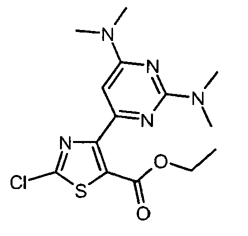 | | |
| **5** | Ethyl 2-chloro-4-(4,6-dimethoxypyrimidin-2-yl)-1,3-thiazole-5-carboxylate | MS (ES) (M+H)⁺: 330 for C₁₂H₁₂ClN₃O₄S | Intermediate 27 |
| | | NMR: 1.14 (t, 3H), 3.89 (s, 6H), 4.21 (q, 2H), 6.37 (s, 1H) | |
| | 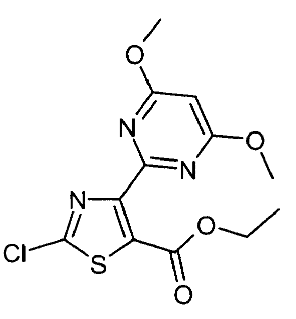 | | |
| **6** | Ethyl 2-chloro-4-pyrazin-2-yl-1,3-thiazole-5-carboxylate | MS (ES) (M+H)⁺: 270 for C₁₀H₈ClN₃O₂S | Intermediate 28 |
| | 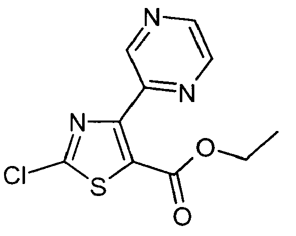 | NMR: 1.16 (t, 3H), 4.22 (q, 2H), 8.76 (m, 2H), 9.03 (s, 1H) | |
| **7** | Ethyl 4-(1,3-benzothiazol-2-yl)-2-chloro-1,3-thiazole-5-carboxylate | MS (ES) (M+H)⁺: 325 for C₁₃H₉ClN₂O₂S₂ | Intermediate 29 |
| | | NMR: 1.27 (t, 3H), 4.34 (q, 2H), 7.59 (m, 2H), 8.08 (d, 1H), 8.22 (d, 1H), | |
| | 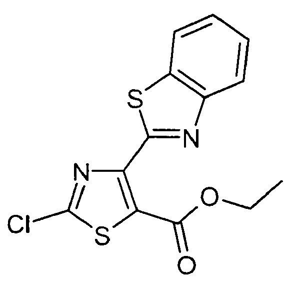 | | |
| **8** | Methyl 2-chloro-4-[1-(2-methoxycthyl)-1H-inidazol-2-yl]-1,3-thiazole-5-carboxylate | MS (ES) (M+H)⁺: 302 for C₁₁H₁₂ClN₃O₃S | Intermediate 19 |
| | | NMR: 3.34 (s, 3H), 3.62 (m, 2H), 3.81 (s, 3H), 4.22 (m, 2H), 7.24 (s, 2H). | |
| | 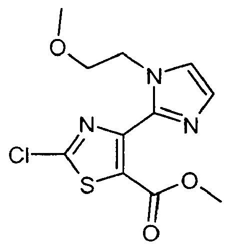 | | |
| **9** | Methyl 2-chloro-4-(1-methyl-1H-1,2,4-triazol-5-yl)-1,3-thiazole-5-carboxylate | MS (ES) (M+H)⁺: 259 for C₈H₇ClN₄O₂S | Intermediate 20 |
| | | NMR: 3.92 (s, 6H), 8.04 (s, 1H). | |
| | 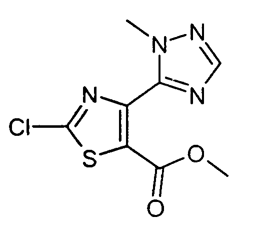 | | |
| **10** | Methyl 2-chloro-4-(1-methyl-1H-imidazol-2-yl)-1,3-thiazole-5-carboxylate | MS (ES) (M+H)⁺: 258 for C₉H₈ClN₃O₂S | Intermediate 16 |
| | | NMR: 3.73 (s, 3H), 3.81 (s, 3H), 7.03 (s, 1H), 7.21 (s, 1H). | |
| | 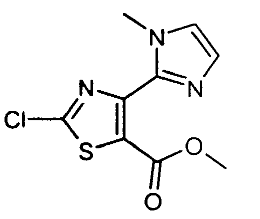 | | |
| **11** | Methyl 2-chloro-4-(1,4,5-trimethyl-1H-imidazol-2-yl)-1,3-thiazole-5-carboxylate | MS (ES) (M+H)⁺: 286 for C₁₁H₁₂ClN₃O₂S | Intermediate 21 |
| | | NMR: 2.03 (s, 3H), 2.13 (s, 3H), 3.34 (s, 3H), 3.61 (s, 3H), 7.90 (s, 2H). | |
| | 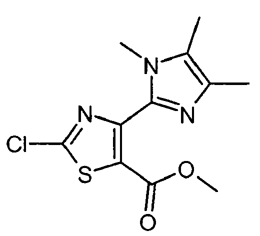 | | |
| **12** | Ethyl 2-chloro-4-(1-methyl-1H-imidazol-4-yl)-1,3-thiazole-5-carboxylate | MS (ES) (M+H)⁺: 272 for C₁₀H₁₀ClN₃O₂S | Intermediate 22 |
| | | NMR: 1.42 (t, 3H), 3.81 (s, 3H), 4.35 (q, 2H), 7.50 (s, 1H), 8.21 (s, 1H). | |
| | 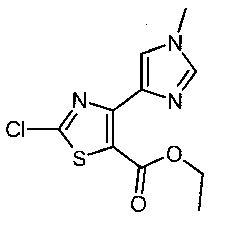 | | |

### Intermediate 13

### Ethyl 2-chloro-4-pyridin-2-yl-1,3-thiazole-5-carboxylate

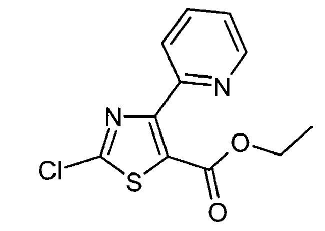

To a solution of ethyl 2-oxo-4-pyridin-2-yl-2,3-dihydro-1,3-thiazole-5-carboxylate (0.42 g, 1.7 mmol) in neat phosphorus oxychloride (5 ml) was added pyridine and the reaction was heated to reflux. After 3hrs, LCMS indicates complete conversion. The reaction mixture was concentrated to remove phosphorus oxychloride. The residue was diluted with water and extracted three times with EtOAc, dried with MgSO₄ and concentrated to a brown oil (0.35 g).
MS (ES) (M+H)⁺: 269 for C₁₁H9ClN₂O₂S; NMR: 1.16 (t, 3H), 4.21 (q, 2H), 7.52 (dd, 1H), 7.82 (d, 1H), 7.96 (t 1H), 8.65 (d, 1H)

### Intermediate 14

### Methyl 2-chloro-4-(1-{[2-(trimethylsilyl)ethoxy]methyl}-1H-imidazol-2-yl)-1,3-thiazole-5-carboxylate

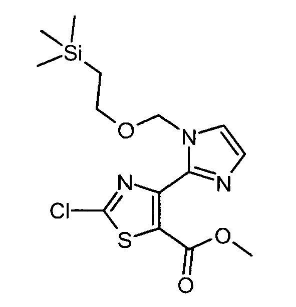

*t*-Butylnitrite (0.81 ml, 6.25 mmol) was added dropwise to a mixture of 1.5 g (4.2 mmol) methyl 2-amino-4-(1-{[2-(trimethylsilyl)ethoxy]methyl}-1*H*-imidazol-2-yl)-1,3-thiazole-5-carboxylate (Intermediate 17) and 900 mg CuCl₂ in 30 ml CH₃CN at room temperature. After stirring 1 hour, the mixture was quenched with NaHSO₃ (aq) and diluted with water before being extracted twice with EtOAc. The EtOAc extracts were washed with brine, dried (MSO₄) and concentrated to afford an oil that was chromatographed on silica gel (100% DCM followed by gradient elution to 90% EtOAc in DCM) to give 800 mg of a solid: MS (ES) (M+H)⁺: 374 for C₁₄H₂₀N₃O₃SSi; NMR: -0.13 (s, 9H), 0.75 (m, 2H), 3.32 (m, 4H), 3.57 (3, 3H), 5.32 (s, 2H), 6.04 (s, 2H).

### Intermediate 15

The following Intermediate was synthesized by an analogous method to Intermediate 14 from the starting materials (SM) given in the table below.

| **Int** | **Compound** | **Data** | **SM** |
|---|---|---|---|
| **15** | Methyl 2-chloro-4-[1-(methoxymethyl)-1*H*-imidazol-2-yl]-1,3-thiazole-5-carboxylate | MS (ES) (M+H)⁺: 269 for C₁₀H₁₀ClN₃O₃S NMR: 3.22 (s, 3H), 3.78 (s, 3H), 5.35 (s, 2H), 7.32 (s, 2H). | Intermediate 18 |
| | 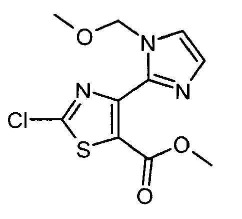 | | |

### Intermediate 16

### Methyl 2-amino-4-(1-methyl-1H-imidazol-2-yl)-1,3-thiazole-5-carboxylate

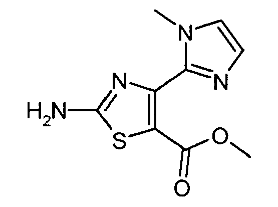

*N*-Iodosuccinimide (9.3 g, 41 mmol) was added to a mixture of 7.52 g (41 mmol) methyl 3-(1-methyl-1*H*-imidazol-2-yl)-3-oxopropanoate (Intermediate 48) and 7.5 g Amberlyst-15 resin in 400 ml EtOAc followed by stirring for 1 hour at room temperature. The resin was filtered off and rinsed with EtOAc. Solvent was removed from the filtrate and the residue was taken up in diethyl ether. Insoluble material was filtered off and rinsed with additional ether. Solvent was removed from the filtrate and the residue was dissolved in 200 ml McOH before added 4.7 g (62 mmol) thiourea. The mixture was heated at reflux for 1 hour. Solvent was removed and the residue was taken up in aqueous Na₂CO₃. Insoluble material was collected by filtration and rinsed well with water. The solids were dried *in vacuo* affording 4.51 g of product: MS (ES) (M+H)⁺: 239 for C₉H₁₀N₄O₂S; NMR: 3.48 (s, 3H), 3.57 (s, 3H), 6.90 (s, 1H), 7.12 (s, 1H), 7.98 (s, 2H).

### Intermediates 17-22

The following Intermediates were synthesized by an analogous method to Intermediate 16 from the starting materials (SM) given in the table below.

| **Int** | **Compound** | **Data** | **SM** |
|---|---|---|---|
| **17** | Methyl 2-amino-4-(1-{[2-(trimethylsilyl)ethoxy]methyl}-1H-imidazol-2-yl)-1,3-thiazole-5-carboxylate | MS (ES) (M+H)⁺: 355 for C₁₄H₂₂N₄O₃SSi | Intermediate 45 |
| | | NMR: -0.14 (s, 9H), 0.67 (m, 2H), 3.34 (m, 2H), 3.62 (s, 3H), 5.28 (s, 2H), 7.03 (s, 1H), 7.35 (s, 1H), 7.95 (s, 2H). | |
| | 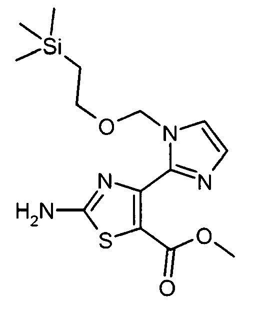 | | |
| **18** | Methyl 2-amino-4-[1-(methoxymethyl)-1H-imidazol-2-yl]-1,3-thiazole-5-carboxylate | MS (ES) (M+H)⁺: 269 for C₁₀H₁₂N₄O₃S | Intermediate 46 |
| | 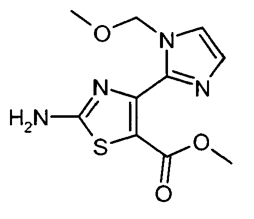 | NMR: 3.23 (s, 3H), 3.71 (s, 3H), 5.51 (s, 2H), 7.87 (s, 1H), 8.02 (s, 1H), 8.44 (s, 2H). | |
| **19** | Methyl 2-amino-4-[1-(2-methoxyethyl)-1H-imidazol-2-yl]-1 ,3-thiazole-5-carboxylate | MS (ES) (M+H)⁺: 283 for C₁₁H₁₄N₄O₃S | Intermediate 47 |
| | 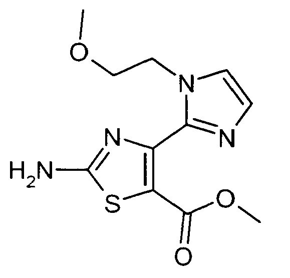 | NMR: 3.22 (s, 3H), 3.61 (m, 2H), 3.69 (s, 3H), 4.32 (m, 2H), 7.91 (s, 2H), 8.41 (s, 2H). | |
| **20** | Methyl 2-amino-4-(1-methyl-1H-1,2,4-triazol-5-yl)-1,3-thiazole-5-carboxylate | MS (ES) (M+H)⁺: 240 for C₈H₉N₅O₂S | Intermediate 44 |
| | 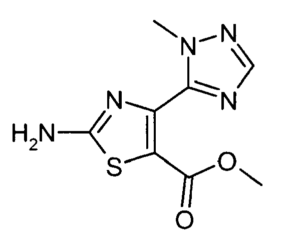 | NMR: 3.61 (s, 3H), 3.71 (s, 3H), 7.96 (s, 1H), 8.10 (s, 2H). | |
| **21** | Methyl 2-amino-4-(1,4,5-trimethyl-1H-imidazol-2-yl)-1,3-thiazole-5-carboxylate | MS (ES) (M+H)⁺: 267 for C₉H₁₀N₄O₂S | Intermediate 49 |
| | 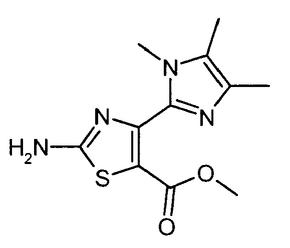 | NMR: 2.01 (s, 3H), 2.14 (s, 3H), 3.32 (s, 3H), 3.61 (s, 3H), 7.89 (s, 2H). | |
| **22** | Ethyl 2-amino-4-(1-methyl-1H-imidazol-4-yl)-1,3-thiazole-5-carboxylate | MS (ES) (M+H)⁺: 253 for C₁₀H₁₂N₄O₂S | Intermediate 43 |
| | 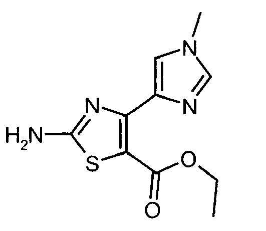 | NMR: 1.15 (t, 3H), 3.62 (s, 3H), 4.15 (q, 2H), 7.65 (s, 1H), 7.95 (s, 1H). | |

### Intermediate 23

### Ethyl 2-amino-4-pyrimidin-2-yl-1,3-thiazole-5-carboxylate

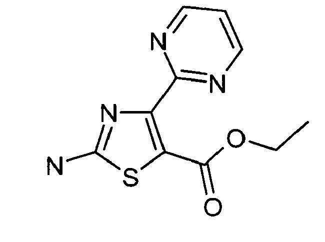

A suspension of ethyl 2-iodo-3-oxo-3-pyrimidin-2-ylpropanoate (Intermediate 30; 1.73 g, 5.4 mmol) and thiourea (0.62 g, 8.1 mmol) in EtOH was heated at reflux for 1 hour. After cooling to room temperature, the reaction was concentrated. The residue was suspended in water and basified with saturated aqueous Na₂CO₃. The resulting precipitate was filtered off and the filtrate was extracted with EtOAc (x3). The organic extracts were dried with MSO₄ and concentrated to an orange oil (0.55 g, 41 %). MS (ES) (M+H)⁺: 251 for C₁₀H₁₀N₄O₂S; NMR: 0.97 (t, 3H), 3.95 (q, 2H), 7.55 (t, 1H), 7.94 (s, 1H), 8.85 (d, 1H), 9.05 (d, 1H)

### Intermediates 24-29

The following Intermediates were prepared by the procedure described in Intermediate 23 from the starting materials (SM) indicated.

| **Int** | **Compound** | **Data** | **SM** |
|---|---|---|---|
| **24** | Ethyl 2-amino-4-pyrimidin-4-yl-1,3-thiazole-5-carboxylate | MS (ES) (M+H)⁺: 251 for C₁₀H₁₀N₄O₂S | Intermediate 31 |
| | 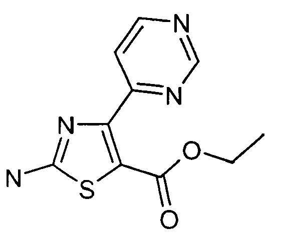 | NMR: 1.08 (t, 3H), 4.05 (q, 2H), 7.68 (d, 1H), 7.98 (s, 2H), 8.86 (d, 1H), 9.21 (s, 1H) | |
| **25** | Methyl 2'-amino-2,4'-bi-1,3-thiazole-5'-carboxylate | MS (ES) (M+H)⁺: 242 for C₈H₇N₃O₂S₂ | Intermediate 32 |
| | 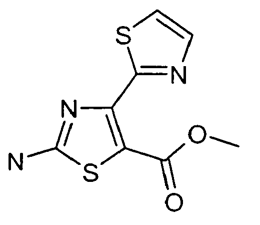 | NMR: 3.67 (s, 3H), 7.86 (d, 1H), 7.91 (d, 1H), 7.92 (s, 2H) | |
| **26** | Ethyl 2-amino-4-[2,6-bis(dimethylamino)pyrimidm-4-yl]-1,3-thiazole-5-carboxylate | MS (ES) (M+H)⁺: 337 for C₁₄H₂₀N₆O₂S | Intermediate 33 |
| | | NMR: 1.22 (t, 3H), 3.19 (s, 12H), 4.21 (q, 2H), 6.75 (s, 1H), 8.21 (s, 2H) | |
| | 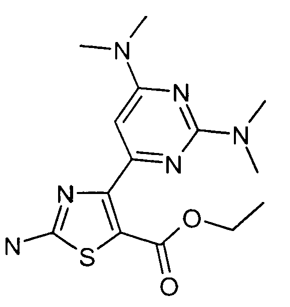 | | |
| **27** | Ethyl 2-amino-4-(4,6-dimethoxypyrimidin-2-yl)-1,3-thiazole-5-caroxylate | MS (ES) (M+H)⁺: 311 for C₁₂H₁₄N₄O₄S | Intermediate 34 |
| | | NMR: 1.02 (t, 3H), 3.86 (s, 6H), 4.03 (q, 2H), 6.27 (s, 1H), 7.94 (s, 2H) | |
| | 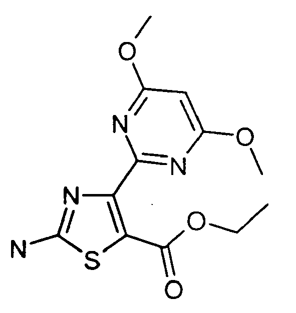 | | |
| **28** | Ethyl w-amino-4-pyrazin-2-yl-1,3-thiazole-5-carboxylate | MS (ES) (M+H)⁺: 251 for C₁₀H₁₀N₄O₂S | Intermediate 35 |
| | 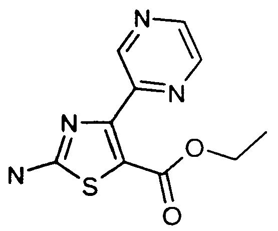 | NMR: 1.07 (t, 3H), 4.04 (q, 2H), 7.99 (s, 2H), 8.65 (d, 1H), 8.69 (d, 1H), 8.81 (s, 1H) | |
| **29** | Ethyl 2-amino-4-(1,3-benzothiazol-2-yl)-1,3-thiazole-5-carboxylate | MS (ES) (M+H)⁺: 306 for C₁₃H₁₁N₃O₂S₂ | Intermediate 36 |
| | | NMR: 1.16 (t, 3H), 4.15 (q, 2H), 7.51 (m, 2H), 8.06 (m, 3H), 8.12 (d, 1H) | |
| | 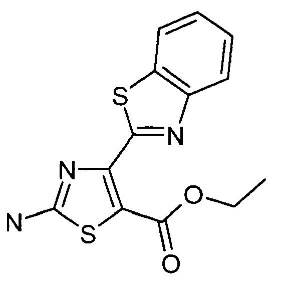 | | |

### Intermediate 30

### Ethyl 2-iodo-3-oxo-3-pyrimidin-2-ylpropanoate

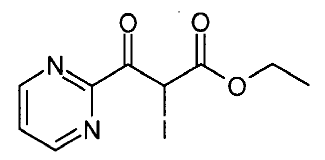

To a suspension of ethyl 3-oxo-3-pyrimidin-2-ylpropanoate (Intermediate 37; 1.19 g, 6.1 mmol) in EtOAc was added *N*-iodosuccinamide (1.38 g, 6.1 mmol) and Amberlyst-15 resin (1.19 g). After stirring at room temperature for 30 mins, LCMS shows a mixture of desired product and bis-iodinated product. (Note: workup reaction as soon as it is finished. Excessive reaction times results in decomposition). The reaction mixture was filtered to remove the Amberlyst-15 resin. The filtrate was concentrated to an orange oil which was then suspended in diethyl ether. The resulting precipitate was filtered and washed with ether. The filtrate was concentrated to an orange oil (1.73 g, 89%). MS (ES) (M+H)⁺: 321 for C₉H₉IN₂O₃

### Intermediates 31-36

The following Intermediates were prepared by the procedure described in Intermediate 30 from the starting materials (SM) indicated.

| **Int** | **Compound** | **Data** | **SM** |
|---|---|---|---|
| **31** | Ethyl 2-iodo-3-oxo-3-pyrimidin-4-ylpropanoate | MS (ES) (M+H)⁺: 321 for C₉H₉IN₂O₃ | Intermediate 38 |
| | 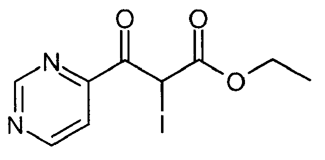 | NMR: 1.11 (t, 3H), 4.12 (q, 2H), 6.41 (s, 1H), 8.03 (d, 1H), 9.16 (d, 1H), 9.42 (s, 1H) | |
| **32** | Methyl 2-iodo-3-oxo-3-(1,3-thiazol-2-yl)propanoate | MS (ES) (M+H)⁺: 312 for C₇H₆INO₃S | Intermediate 50 |
| | 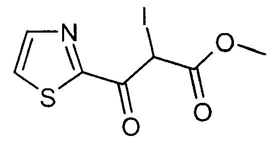 | | |
| **33** | Ethyl 3-[2,6-bis(dimethylamino)pyrimidin-4-yl]-2-iodo-3-oxopropanoate | MS (ES) (M+H)⁺: 407 for C₁₃H₁₉IN₄O₃ | Intermediate 39 |
| | | NMR: 1.19 (t, 3H), 3.06 (s, 6H), 3.17 (s, 6H), 4.09 (q, 2H), 6.41 (s, 1H) | |
| | 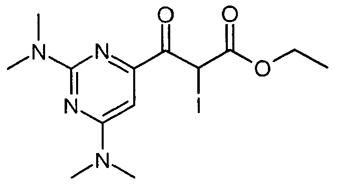 | | |
| **34** | Ethyl 3-(4,6-dimethoxypyrimidin-2-yl)-2-iodo-3-oxopropanoate | MS (ES) (M+H)⁺: 381 for C₁₁H₁₃IN₂O₅ | Intermediate 40 |
| | 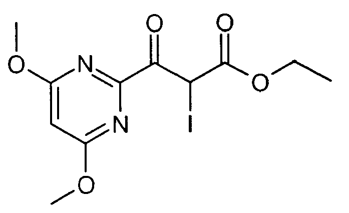 | | |
| **35** | Ethyl 2-iodo-3-oxo-3-pyrazin-2-ylpropanoate | MS (ES) (M+H)⁺: 321 for C₉H₉IN₂O₃ | Intermediate 41 |
| | 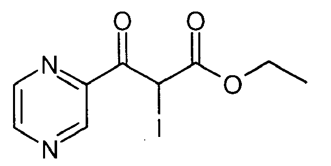 | | |
| **36** | Ethyl 3-(1,3-benzothiazol-2-yl)-2-iodo-3-oxopropanoate | MS (ES) (M+H)⁺: 376 for C₁₂H₀₁INO₃S | Intermediate 42 |
| | 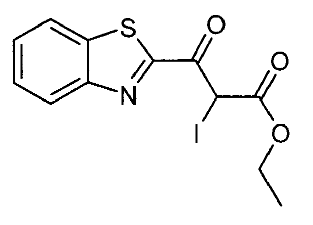 | | |

### Intermediate 37

### Ethyl 3-oxo-3-pyrimidin-2-ylpropanoate

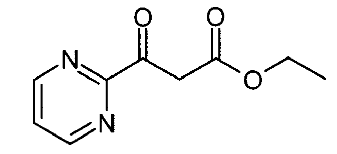

To a solution of pyrimidine-2-carboxylic acid (0.99 g, 7.98 mmol) in anhydrous THF (20 ml) was added carbonyl diimidazole (1.55 g, 9.57 mmol) and the suspension was heated at reflux for 2 hours. The mixture was then cooled to room temperature and used without workup or purification. In a separate flask, mono-ethyl malonate (0.94 ml, 7.98 mmol) was suspended in anhydrous THF (20 ml) and cooled to 0 °C. Methyl magnesium bromide (5.32 ml, 15.96 mmol, 3.0 M in diethyl ether) was added dropwise. After stirring at 0 °C for 20 mins, the crude imidazolide solution prepared earlier was added slowly. The reaction was then heated at reflux overnight. After cooling to room temperature, the reaction mixture was diluted with water and acidified with concentrated HCl to pH 5. The solution was extracted with EtOAc (x3), dried with MgSO₄ and concentrated to a yellow oil (1.19 g, 77%). NMR showed a 2:1 mixture of the keto:enol forms. MS (ES) (M+H)⁺: 195 for C₉H₁₀N₂O₃; NMR: 1.13-1.29 (t, 3H), 4.05-4.28 (q, 2H), 4.18 (s, 2H), 7.62-7.76 (t, 1H), 8.95-9.06 (d, 2H), 11.79 (s, 4H)

### Intermediates 38-43

The following Intermediates were prepared by the procedure described in Intermediate 37 from the starting materials (SM) indicated.

| **Int** | **Compound** | **Data** | **SM** |
|---|---|---|---|
| **38** | Ethyl 3-oxo-3-pyrimidin-4-ylpropanoate | MS (ES) (M+H)⁺:195 for C₉H₁₀N₂O₃ | pyrimidine-4-carboxylic acid |
| | 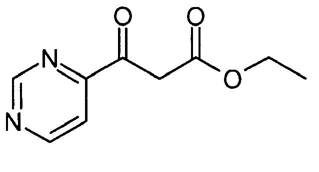 | NMR: 1.14 (t, 3H, 2/3) 1.28 (t, 3H, 1/3) 4.17 (q, 2H, 2/3) 4.19 (s, 2H), 4.25 (q, 2H, 1/3) 6.34 (s, 1H, 1/3) 7.91 (d, 1H 1/3) 7.95 (d, 1H, 2/3) 9.04 (d, 1H, 1/3) 9.15 (d, 1H, 2/3) 9.30 (s, 1H, 1/3) 9.43 (s, 1H, 2/3) 12.03 (s, 1H, 2/3) | |
| **39** | Ethyl 3-[2,6-bis(dimethylamino)pyrimidin-4-yl]-3-oxopropanoate | MS (ES) (M+H)⁺: 281 for C₁₃H₂₀N₄O₃ | 2,6-bis(dimethylami no)pyrimidine-4-carboxylic acid |
| | | NMR: 1.19 (t, 3H), 3.06 (s, 6H), 3.08 (s, 6H), 3.92 (s, 2H), 4.09 (q, 2H), 6.35 (s, 1H) | |
| | 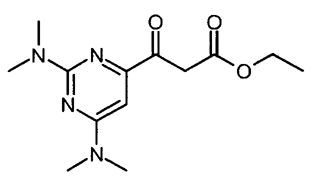 | | |
| **40** | Ethyl 3-(4,6-dimethoxypyrimidin-2-yl)-3-oxopropanoate | MS (ES) (M+H)⁺: 255 for C11H₁₄N₂O₅ | 4,6-dimethoxypyrim idine-2-carboxylic acid |
| | 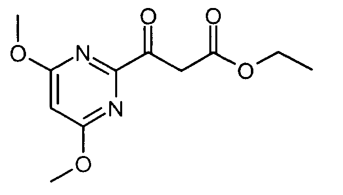 | | |
| **41** | Ethyl 3-oxo-3-pyrazin-2-ylpropanoate | MS (ES) (M+H)⁺: 195 for C₉H₁₀N₂O₃ | pyrazine-2-carboxylic acid |
| | 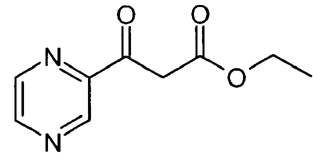 | NMR: 1.15 (t, 3H), 4.11 (q, 2H), 4.18 (s, 2H), 8.82 (s, 1H), 8.94 (s, 1H), 9.17 (s, 1H) | |
| **42** | Ethyl 3-(1,3-benzothiazol-2-yl)-3-oxopropanoate | MS (ES) (M+H)⁺: 250 for C₁₂H₁₁NO₃S | 1,3-benzothiazole-2-carboxylic acid |
| | 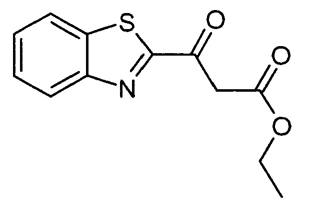 | NMR: 1.17 (t, 3H), 4.15 (q, 2H), 4.32 (s, 2H), 7.68 (m, 2H), 8.27 (m, 2H) | |
| **43** | Ethyl 3-(1-methyl-1H-imidazol-4-yl)-3-oxopropanoate | NMR: 1.25 (t, 3H), 3.72 (s, 3H), 4.01 (s, 2H), 4.18 (q, 2H), 7.43 (s, 1H), 7.57 (s, 1H). | 1-methyl-1H-imidazole-4-carboxylic acid |
| | 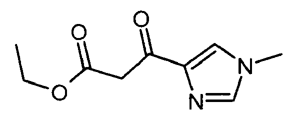 | | |

### Intermediate 44

### Methyl 3-(1-methyl-1H-1,2,4-triazol-5-yl)-3-oxopropanoate

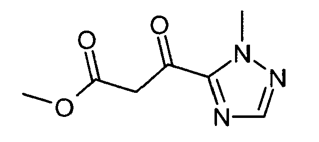

NaH (7.84 g, 196 mmol of a 60% dispersion in oil) was added portionwise to a solution of 6.18 g (34.5 mmol) of 1-(1-methyl-1*H*-1,2,4-triazol-5-yl)ethanone (Ohta, S.; Kawasaki, I.; Fukuno, A.; Yamashita, M.; Tada, T.; Kawabata, T. Chem. Pharm. Bull. (1993), 41(7), 1226-31) in 100 ml dimethylcarbonate. The mixture was heated to 90 °C for 2 hour forming a thick slurry. After cooling to room temperature, the mixture was slowly transferred to 1N HCl over ice. The pH of the mixture was brought to about 7 with NaHCO₃ before being saturated with NaCl and extracted 4 times with EtOAc. The EtOAc was dried (MgSO₄) and concentrated to give an oil that was chromatographed on silica gel (100% DCM followed by gradient elution to 50% EtOAc in DCM). Product (5.3 g) was obtained as an oil. NMR: 3.78 (s, 3H), 4.11 (s, 2H), 4.22 (s, 3H), 7.94 (s, 1H).

### Intermediates 45-50

The following Intermediates were synthesized by an analogous method to Intermediate 44 from the starting materials (SM) given in the table below.

| **Int** | **Compound** | **Data** | **SM** |
|---|---|---|---|
| **45** | Methyl 3-oxo-3-(1-{[2-(trimethylsilyl)ethoxy]methyl}-1H-imidazol-2-yl)propanoate | MS (ES) (M-H)⁻: 297 for C₁₃H₂₂NO₂Si | Intermediate 51 |
| | | NMR: -0.14 (s, 9H), 0.82 (m, 2H), 3.52 (t, 2H), 3.77 (s, 3H), 4.10 (s, 2H), 5.71 (s, 2H), 7.21 (s, 1H), 7.71 (s, 1H). | |
| | 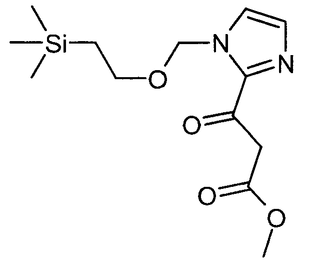 | | |
| **46** | Methyl 3-[1-(methoxymethyl)-1H-imidazol-2-yl]-3-oxopropanoate | MS (ES) (M+H)⁺: 213 for C₉H₁₂N₂O₄ | Intermediate 52 |
| | 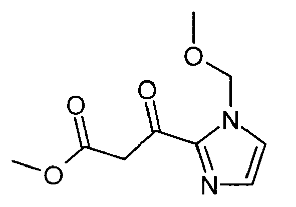 | NMR: 3.41 (s, 3H), 3.75 (s, 3H), 4.22 (s, 2H), 5.75 (s, 2H), 7.22 (s, 1H), 7.31 (s, 1H). | |
| **47** | Methyl 3-[1-(2-methoxyethyl)-1H-imidazol-2-yl]-3-oxopropanoate | MS (ES) (M+H)⁺: 227 for C₁₀H₁₄N₂O₄ | Intermediate 53 |
| | 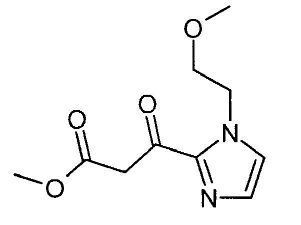 | NMR: 3.18 (s, 3H), 3.61 (m, 5H), 4.07 (s, 2H), 4.52 (m, 2H), 7.24 (s, 1H), 7.61 (s, 1H). | |
| **48** | Methyl 3-(1-methyl-1H-imidazol-2-yl)-3-oxopropanoate | MS (ES) (M+H)⁺: 183 for C₈H₁₀N₂O₃. | 1-(1-Methyl-1H-imidazol-2-yl)ethanone (Abarca- Gonzalez, B.; Jones, R. A.; Medio-Simon, M.; Quilez-Pardo, J.; Sepulveda-Arques, J.; Zaballos-Garcia, E. Synth. Comm. (1990), 20(3), 321-31). |
| | 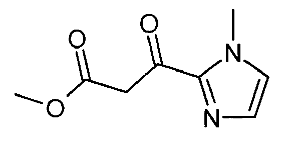 | | |
| **49** | Methyl 3-oxo-3-(1,4,5-trimethyl-1H-imidazol-2-yl)propanoate | NMR: 2.21 (s, 6H), 3.72 (s, 3H), 3.86 (s, 3H), 4.10 (s, 2H). | Intermediate 54 |
| | 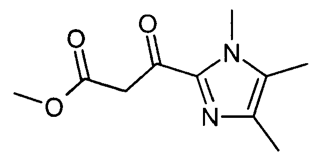 | | |
| **50** | Methyl 3-oxo-3-(1,3-thiazol-2-yl)propanoate | MS (ES) (M+H)⁺: 186 for C₇H₇NO₃S NMR: 3.65 (s, 3H), 4.22 (s, 2H), 8.18 (d, 1H), 8.29 (d, 1H) | 1-(1,3-thiazol-2-yl)ethanone |
| | 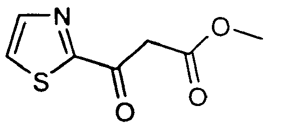 | | |

### Intermediate 51

### 1-(1-{[2-(Trimethylsilyl)ethoxy]methyl}-1H-imidazol-5-yl)ethanone

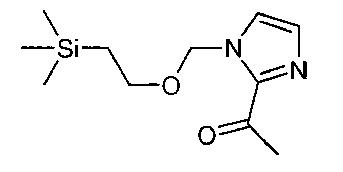

A solution of 30 ml (75 mmol) of 2.5 M *n*-butyllithium in hexanes was added slowly to a solution of 8.48 g (61.3 mmol) 1-{[2-(trimethylsilyl)ethoxy]methyl}-1*H*-imidazole (Lipshutz, B. H.: Huff. B.: Hagen, W. Tetrahedron Lett. (1988), 29(28), 3411-14) in 200 ml THF cooled in a dry ice-acetone bath. After stirring 1 hour, 8 ml (75 mmol) of *N*-methoxy-*N-*methylacetamide was added quickly, and the solution was allowed to warm to room temperature over 30 min. After quenching with aqueous NH₄Cl, the mixture was diluted with water and extracted twice with EtOAc, which was washed with brine, dried (MgSO₄) and concentrated to give an oil that was chromatographed on silica gel (100% DCM followed by gradient elution to 50% EtOAc in DCM). Product (8.5 g) was obtained as a mobile oil. NMR: -0.13 (s, 9H), 0.82 (m, 2H), 2.55 (s, 3H), 3.48 (m, 2H), 5.74 (s, 2H), 7.21 (s, 1H), 7.72 (s, 1H).

### Intermediates 52-54

The following Intermediates were synthesized by an analogous method to Intermediate 51 from the starting materials (SM) given in the table below.

| **Int** | **Compound** | **Data** | **SM** |
|---|---|---|---|
| **52** | 1-[1-(Methoxymethyl)-1H-imidazol-2-yl]ethanone | NMR: 2.72 (s, 3H), 3.35 (s, 3H), 5.75 (s, 2H), 7.22 (s, 1H), 7.34 (s, 1H). | 1-(methoxymethyl)-1H-imidazole (Manoharn, T. S.; Brown, R. S. J. Org. Chem. (1989), 54(6), 1439-42). |
| | 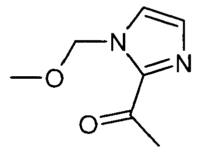 | | |
| **53** | 1-[-(2-Methoxyethyl)-1H-imidazol-2-yl]ethanone | MS (ES) (M+H)⁺: 169 for C₈H₁₂CN₂O₂ | 1-(2-Methoxyethyl}-1H-imidazole (WO 2003055876 A1) |
| | 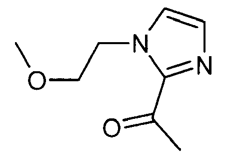 | NMR: 2.69 (s, 3H), 3.34 (s, 3H), 3.71 (m, 2H), 4.61 (m, 2H), 7.12 (s, 1H), 7.26 (s, 1H). | |
| **54** | 1-(1,4,5-Trimethyl-1H-imidazol-2-yl)ethanone | NMR: 2.15 (s, 3H), 2.22 (s, 3H), 2.57 (s, 3H), 3.88 (s, 3H), 3.34 (s, 3H), 3.71 (m, 2H). | 1,4,5-trimethyl-1H-imidazole (US 6177575 B1) |
| | 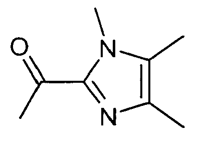 | | |

### Intermediate 55

### Methyl 2'-amino-2-chloro-4,4'-bi-1,3-thiazole-5-carboxylate

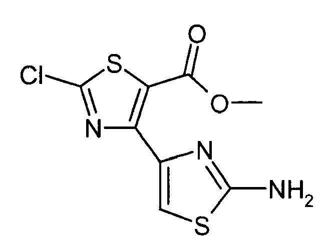

A solution of 770 mg (3.6 mmol) of methyl 2-chloro-4-(chloroacetyl)-1,3-thiazole-5-carboxylate (Intermediate 61) and 270 mg (3.6 mmol) thiourea in 2 ml MeOH was heated at reflux for 90 min. Solvent was removed, and the residue was taken up in water and treated with aqueous Na₂CO₃ precipitating solids that were collected, washed with water and dried *in vacuo* to give 670 mg of product: MS (ES) (M+H)⁺: 276 for C₈H₆ClN₃O₂S₂; NMR (CDCl₃): 3.91 (s, 3H), 5.08 (s, 2H), 7.28 (s, 1H).

### Intermediates 56-57

The following Intermediates were synthesized by an analogous method to Intermediate 55 from the starting materials (SM) given in the table below.

| **Int** | **Compound** | **Data** | **SM** |
|---|---|---|---|
| **56** | Methyl 2-chloro-2'-(methylamino)-4,4'-bi-1,3-thiazole-5-carboxylate | MS (ES) (M+H)⁺: 290 for C₉H₈CN₃O₂S₂ NMR (CDCl₃): 3.01 (d, 3H), 3.35 (s, 3H), 5.32 (s, 1H), 3.87 (s, 3H), 7.71 (s, 1H). | Intermediate 61 and methylthiourea |
| | 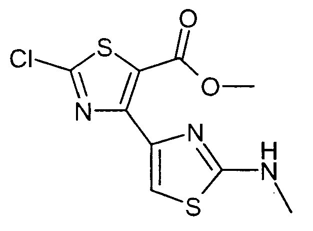 | | |
| **57** | Methyl 2-chloro-2'-(dimethylamino)-4,4'-bi-1,3-thiazole-5-carboxylate | MS (ES) (M+H)⁺: 304 for C₁₀H₁₀CN₃O₂S₂ NMR (CDCl₃): 3.11 (s, 6H), 3.91 (s, 3H), 7.43 (s, 1H). | Intermediate 61 and *N,N-*dimethylthiourea |
| | 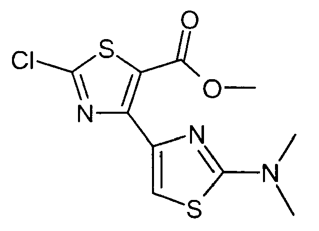 | | |

### Intermediate 58

### Methyl 2-chloro-4-[(2E)-3-(dimethylamino)prop-2-enoyl]-1,3-thiazole-5-carboxylate

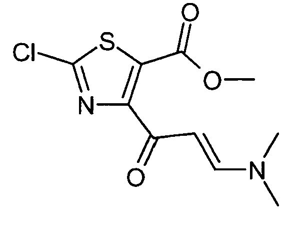

A solution of 1 g (4.55 mmol) methyl 4-acetyl-2-chloro-1,3-thiazole-5-carboxylate (WO 2006087543 A1) and 0.61 ml (4.6 mmol) dimethylformamide dimethylacetal in 4 ml toluene was heated at 100 °C for 3 hours in a microwave reactor. Solvent was removed and the residue was diluted with water, which was then saturated with NaCl. The solution was extracted 3 times with EtOAc, which was dried (MgSO₄) and concentrated. The residue was chromatographed on silica gel (100% DCM followed by gradient elution to 50% EtOAc in DCM) to give 480 mg of product as a viscous oil. NMR (CDCl₃): 2.88 (s, 3H), 3.11 (s, 3H), 3.87 (s, 3H), 5.61 (d, 1H), 7.82 (s, 1H).

### Intermediate 59

### Methyl 2-chloro-4-isoxazol-5-yl-1,3-thiazole-5-carboxylate

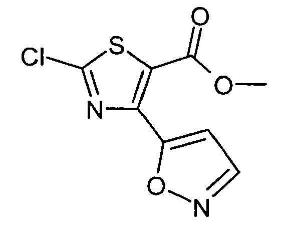

A solution of 1.18 mg (4.3 mmol) methyl 2-chloro-4-[(2*E*)-3-(dimethylamino)prop-2-enoyl]-1,3-thiazole-5-carboxylate (Intermediate 58) and 285 mg (4.1 mmol) hydroxylamine hydrochloride in 4 ml AcOH was heated at 120 °C for 90 min. Solvent was removed and the residue was partitioned between aqueous NaHCO₃ and EtOAc. The EtOAc was separated, washed with brine, dried (MSO4) and concentrated. The residue was chromatographed on silica gel (1:1 hexanes/DCM followed by gradient elution to 100% DCM) to give 450 mg of product as solid: MS (ES) (M+H)⁺: 245 for C₈H₅ClN₂O₃S; NMR: 3.91 (s, 3H), 7.17(s, 1H), 8.26 (s, I H).

### Intermediate 60

### Methyl 2-chloro-4-(1H-pyrazol-5-yl)-1,3-thiazole-5-carboxylate

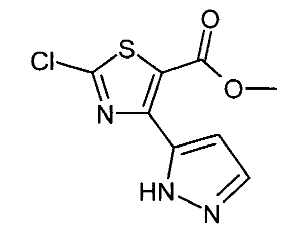

A solution of 700 mg (2.6 mmol) methyl 2-chloro-4-[(2*E*)-3-(dimethylamino)prop-2-enoyl]-1,3-thiazole-5-carboxylate (Intermediate 58) and 193 mg (2.8 mmol) hydrazine hydrochloride in 4 ml AcOH was heated at 120 °C for 1 hour. Solvent was removed and the residue was partitioned between aqueous NaHCO₃ and EtOAc. The EtOAc was separated, washed with brine, dried (MgSO₄) and concentrated. The residue was chromatographed on silica gel (100% DCM followed by gradient elution to 100% EtOAc) to give 200 mg of product as a white solid. NMR: 4.00 (s, 3H), 7.12 (s, 1H), 7.73 (s, 1H), 13.04 (s, 1H).

### Intermediate 61

### Methyl 2-chloro-4-(chloroacetyl)-1,3-thiazole-5-carboxylate

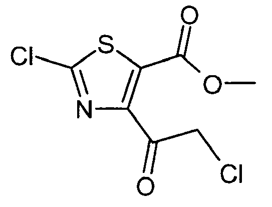

A solution of 2.0 g (9.1 mmol) of methyl 4-acetyl-2-chloro-1,3-thiazole-5-carboxylate (WO 2006087543 A1) and 6.3 g (18.2 mmol) trimethylbenzylammonium dichloroiodide in 50 ml 1,2-dichloroethane and 20 ml MeOH was heated at reflux for 3 hours. Solvent was removed, and the residue was taken up in EtOAc and washed with aqueous NaHSO₃, water and brine. Drying (MgSO₄) and removal of solvent gave 2.3 g of an oil: MS (ES) (M+H)⁺: 222 for C₇H₅Cl₂NO₃S; NMR (CDCl₃): 3.91 (s, 3H), 4.72 (s, 2H).

### Intermediate 62

### Ethyl (3S,4R)-4-{[(3-chloro-4-cyano-5-methyl-1H-pyrrol-2-yl)carbonyl]amino}-3-methoxypiperidine-1-carboxylate

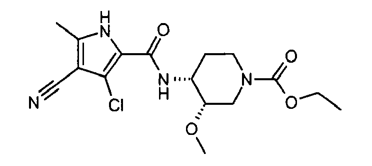

A solution of 503 mg (2.7 mmol) 3-chloro-4-cyano-5-methyl-1*H*-pyrrole-2-carboxylic acid (Intermediate 68), ethyl (3*S*,4*R*)-4-amino-3-methoxypiperidine-1-carboxylate (551 mg, 2.7 mmol) (WO 2006087543 A1), hydroxybenzotriazole (0.358 mg, 2.7 mmol) and *N-*methylmorpholine (0.58 ml, 3 mmol) in DCM (100 ml) was stirred at room temperature before adding 1-(3-dimethylaminopropyl)-3-ethylcarbodiimide hydrochloride (0.588 mg, 3 mmol). After stirring at room temperature for 4 hours, the crude reaction mixture was diluted with EtOAc and washed with saturated aqueous sodium carbonate, water, 1N HCl, water, and brine. The organic portion was then dried with MgSO₄ and concentrated. The residue was chromatographed on silica gel (100% DCM followed by gradient elution to 100% EtOAc) affording a solid that was triturated with MeOH to give 515 mg of product as a solid: MS (ES) (M+H)⁺: 367 for C₁₆H₂₁ClN₄O₄; NMR: 1.23 (t, 3H), 1.57 (m, 2H), 2.33 (s, 3H), 2.67-3.04 (m, 2H), 3.34 (m, 4H), 3.41 (m, 1H), 3.76-4.33 (m, 5H), 7.25 (d, 1H), 12.73 (s, 1H).

### Intermediate 63

The following Intermediate were synthesized by an analogous method to Intermediate 62 from the starting materials (SM) given in the table below.

| **Int** | **Compound** | **Data** | **SM** |
|---|---|---|---|
| **63** | Ethyl (3*S*,4*R*)-4-{[(4-cyano-5-methyl-1*H*-pyrrol-2-yl)carbonyl]amino}-3-methoxypiperidine-1-carboxylate | MS (ES) (M-H)⁻: 333 for C₁₆H₂₂N₄O₄ | Intermediate 69 and ethyl (3*S*,4*R*)-4-amino-3-methoxypiperidine-1-carboxylate (WO 2006087543 A1) |
| | | NMR: 1.22 (t, 3H), 1.52 (m, 1H), 1.79 (m, 1H), 2.32 (s, 3H), 2.77-3.14 (m, 2H), 3.32-3.43 (m, 4H), 3.79-4.33 (m, 5H), 7.21 (s, 1H), 7.89 (d, 1H), 12.21 (s, 1H). | |
| | 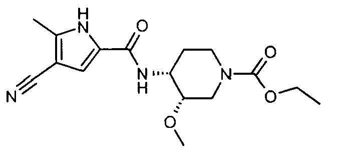 | | |

### Intermediate 64

### 3-Chloro-4-cyano-N-[(3S,4R)-3-methoxypiperidin-4-yl]-5-methyl-1H-pyrrole-2-carboxamide

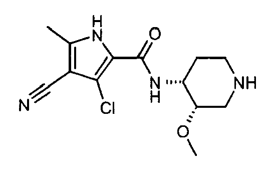

A solution of ethyl (3*S*,4*R*)-4-{[(3-chloro-4-cyano-5-methyl-1*H*-pyrrol-2-yl)carbonyl]amino}-3-methoxypiperidine-1-carboxylate (Intermediate 62; 578 mg, 1.57 mmol) and 0.83 ml (15.6 mmol) 50% NaOH in 7 ml MeOH was heated at 120 °C for 2 hours in a microwave reactor. The mixture was diluted with water and saturated with NaCl before being extracted 5 times with THF. The THF extracts were dried (MgSO₄) and concentrated to give material that was triturated with MeOH to give 215 mg of a white solid: MS (ES) (M+H)⁺: 297 for C₁₃H₁₇ClN₄O₄; NMR: 1.62 (m, 2H), 2.34 (s, 3H), 2.61 (dm, 2H), 2.91 (dm, 1H), 3.14 (dm, 1H), 3.37 (m, 4H), 7.23 (d, 1H), 7.71 (s, 1H).

### Intermediate 65

### (3S,R)-4-{[(4-Cyano-5-methyl-1H-pyrrol-2-yl)carbonyl]amino}-3-methoxypiperidinium trifluoroacetate

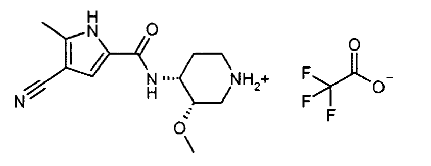

A solution of ethyl (3*S*,4*R*)-4-{[(4-cyano-5-methyl-1*H*-pyrrol-2-yl)carbonyl]amino}-3-methoxypiperidine-1-carboxylate (Intermediate 63; 340 mg, 1.0 mmol) and 0.54 ml (10 mmol) 50% NaOH in 5 ml MeOH was heated at 120 °C for 2 hours in a microwave reactor. The mixture was diluted with water and saturated with NaCl before being extracted 5 times with THF. The THF extracts were dried (MgSO₄) and concentrated to give material that was purified by reverse phase HPLC (10-20% gradient acetonitrile in water with 0.1 % TFA) to give 200 mg of product as the TFA salt: MS (ES) (M-H)⁻: 261 for C₁₆H₂₂N₄O₄; NMR: 1.71 (m, 1H), 2.04 (m, 1H), 2.34 (s, 3H), 3.02-3.34 (m, 3H), 3.41 (m, 4H), 3.52 (m, 1H), 3.61 (m, 1H), 4.18 (m, 2H), 7.21 (s, 1H), 8.05 (d, 1H), 8.25 (d, 1H), 8.78 (m, 1H), 12.21 (s, 1H).

### Intermediate 66

### Ethyl 4-cyano-5-methyl-1H-pyrrole-2-carboxylate

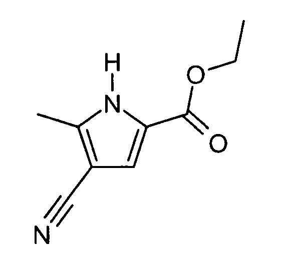

A solution of 3.13 g (20 mmol) ethyl 5-methyl-1*H*-pyrrole-2-carboxylate (Curran, T.P.; Keancy, M.T. J. Org. Chem. (1996), 61(25), 9068-9069) and 4 ml DMF in 60 ml acetonitrile was cooled in an ice water bath. Chlorosulfonyl isocyanate (2 ml, 23 mmol) was added, and the mixture was stirred overnight with warming to room temperature. Additional chlorosulfonyl isocyanate (0.4 ml, 4.6 mmol) was added, and the mixture was stirred 3 hours. Saturated aqueous Na₂CO₃ (40 ml) was added and solvent was removed. The residue was taken up in water and extracted 2 times with 1:1 EtOAc-diethyl ether with each extract being washed twice more with water and once with brine. The combined organic layers were dried (MgSO₄), and solvent was removed to give 3.5 g of a white solid: MS (ES) (M+H)⁺: 179 for C₉H₁₀N₂O₂; NMR: 1.32 (t, 3H), 2.35 (s, 3H), 4.17 (q, 1H), 7.16 (s, 1H), 12.59 (s, 1H).

### Intermediate 67

### Ethyl 3-chloro-4-cyano-5-methyl-1H-pyrrole-1-carboxylate

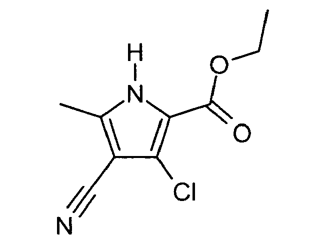

A solution of 1.72 g (11.5 mmol) of ethyl 4-cyano-5-methyl-1*H*-pyrrole-2-carboxylate (Intermediate 66) and 1.53 g (11.5 mmol) *N*-chlorosuccinimide in 20 ml DMF was heated to 70 °C in a microwave reactor for 1 hour. Solvent was removed and the residue was diluted with water and extracted twice with diethyl ether, each extract being washed 2 times more with water and once with brine. The combined organic layers were dried (MgSO₄), and solvent was removed. The residue was chromatographed on silica gel (100% DCM followed by gradient elution to 100% EtOAc) to afford 790 mg of product as a solid: MS (ES) (M-H)⁻: 211 for C₉H₉ClN₂O₂; NMR: 1.32 (t, 3H), 2.35 (s, 3H), 4.32 (q, 1H), 12.85 (s, 1H).

### Intermediate 68

### 3-Chloro-4-cyano-5-methyl-1H-pyrrole-2-carboxylic acid

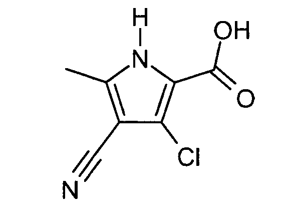

A solution of 760 mg (3.6 mmol) of ethyl 3-chloro-4-cyano-5-methyl-1*H*-pyrrole-2-carboxylate (Intermediate 67) and 3.6 ml (7.2 mmol) of 2N LiOH in 15 ml MeOH was heated at 100 °C for 3 hours in a microwave reactor. Additional 2N LiOH (1.0 ml) was added, and the mixture was heated at 100 °C for 1 hour. The mixture was diluted with water and acidified with 1N HCl before being extracted 2 times with EtOAc. The EtOAc extracts were washed with brine, dried (MgSO₄) and concentrated to give 610 mg of a solid: MS (ES) (M-H)⁻: 183 for C₇H₅ClN₂O₂; NMR: 2.31 (s, 3H), 12.72 (s, 1H), 13.31 (s, 1H).

### Intermediate 69

### 4-Cyano-5-methyl-1H-pyrrole-2-carboxylic acid

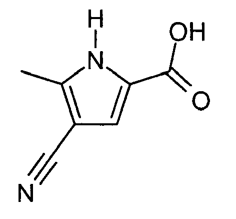

A solution of 390 mg (2.6 mmol) of ethyl 4-cyano-5-methyl-1*H*-pyrrole-2-carboxylate (Intermediate 66) and 2.6 ml (7.2 mmol) of 2N LiOH in 15 ml MeOH was heated at 100 °C for 3 hours in a microwave reactor. Additional 2N LiOH (0.5 ml) was added, and the mixture was heated at 100 °C for 1 hour. The mixture was diluted with water and acidified with 1N HCl before being extracted 2 times with EtOAc. The EtOAc extracts were washed with brine, dried (MgSO₄) and concentrated to give 317 mg of a solid: MS (ES) (M+H)⁺: 151 for C₇H₆N₂O₂; NMR: 2.31 (s, 3H), 7.01 (s, 1H), 12.47 (s, 1H), 12.75 (s, 1H).

### Intermediate 70

### Methyl 2-chloro-4-(4-methoxypyrimidin-2-yl)thiazole-5-carboxylate

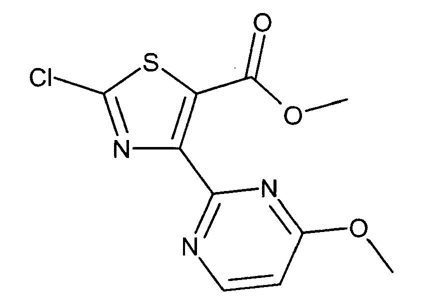

Methyl 2-amino-4-(4-methoxypyrimidin-2-yl)thiazole-5-carboxylate (Intermediate 71; 100 mg, 0.38 mmol) was suspended in 4 ml of glacial acetic acid and 6 ml of hydrochloric acid, The solution was cooled down to 0 °C and sodium nitrite (78 mg, 1.13 mmol) in 3 ml of H₂O was added dropwise. After stirring at 0 °C for 10 min, it was warmed up to room temperature and stirred for 30 min until LC-MS showed no starting material remaining. A solution of urea (50 mg) in 2 ml of H₂O was added dropwise, stirred for 20 min. Volume of the solution was then reduced under reduced pressure to ∼7 ml, Na₂CO₃ saturated aqueous solution was added slowly while stirring to neutralize the solution. The resulting liquid was extracted with EtOAc (10 mlx3), The combined EtOAc layer was washed with saturated NaHCO₃ solution and brine, dried over MgSO₄ and concentrated to an oil. This was then purified by column chromatography eluted with Hexanes/EtOAc to give the desired product as an oil (75 mg). MS (ES) (M+H)⁺: 286 for C₁₀H₈N₃O₃S; NMR (CDCl₃): 3.81 (s, 3H), 3.97 (s, 3H), 6.74 (d, 1H), 8.53 (d, 1H).

### Intermediate 71

### Methyl 2-amino-4-(4-methoxypyrimidin-2-yl)thiazole-5-carboxylate

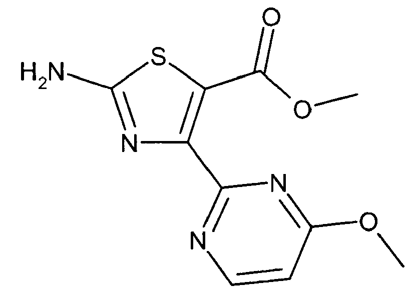

Methyl 3-(4-methoxypyrimidin-2-yl)-3-oxopropanoate (Intermediate 72; 250 mg, 1.19 mmol) was dissolved in EtOAc (10 ml), Amberlyst 15 ion-exchange resin (230 mg) and n-iodo succinimide (282 mg, 1.19 mmol) were added, and the reaction mixture was stirred at room temperature for 1 hr. The mixture was filtered and the filter cake was rinsed with MeOH, the combined filtrate was concentrated to dryness. Either was added and the resulting precipitate was filtered off, the filtrate was concentrated to an oil and dried under high vacuum. To this crude thiourea (136 mg, 1.78 mmol) and MeOH (10.00 ml) was added and the mixture was refluxed for 1.5hr, cooled down to rt and yellowish precipitate was filtered and washed with MeOH, the filter cake was washed with saturated Na₂CO₃ aqueous solution and kept as the desired product (off-white solid), the filtrate was then concentrated to dryness and was suspended into saturated Na₂CO₃ solution (10 ml), the resulting precipitate was collected by filtration. LC-MS showed both solids were the desired product. (97 mg). MS (ES) (M+H)⁺: 267 for C₁₀H₁₀N₄O₄S; NMR (CDCl₃): 3.55 (s, 3H), 3.89 (s, 3H), 6.95 (d, 1H), 7.95 (br, 2H), 8.62 (d, 1H).

### Intermediate 72

### Methyl 3-(4-methoxypyrimidin-2-yl)-3-oxopropanoate

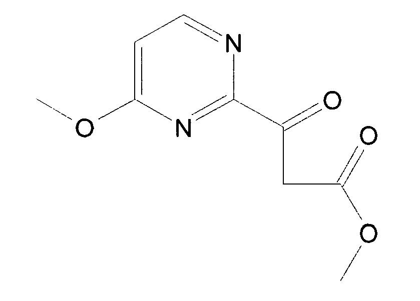

1-(4-Methoxypyrimidin-2-yl)ethanone (Intermediate 73; 462 mg, 3.04 mmol) was dissolved in dimethyl carbonate (20 ml, 237.35 mmol) and the reaction mixture was cooled down to 0 °C, sodium hydride (291 mg, 12.15 mmol) was added and the mixture was stirred at 0°c for 10 min, raised the temperature to 100 °C, refluxed for 1 hr. The mixture was cooled down to RT and was poured slowed into the ice cold 1N HCI solution (20 ml), stirred for 5 min, pH was brought back to ∼7 and extracted with EtOAc. Organic layer was washed with brine and dried over MgSO₄, concentrated to oil. Chromatography gave a mixture of 2 compounds. Purified by column eluted with DCM/EtOAc (100∼30%) gave the desired product as an oil (250 mg), it was carried over to the next step without further purification. MS (ES) (M+H)⁺: 211 for C₉H₁₀N₂O₄S.

### Intermediate 73

### 1-(4-Methoxypyrimidin-2-yl)ethanone

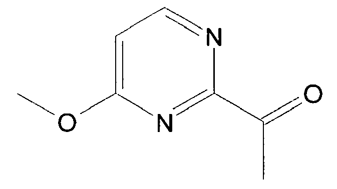

2-Iodo-4-methyoxypyrimidine (Intermediate 74; 1.62 g, 6.84 mmol) was dissolved in dry THF (20 ml), cooled down to -10 °C, i-PrMgCl (2 M in ether, 3.42 ml, 6.84 mmol) was added and the reaction mixture was stirred at 0 °C for 1 hour. *N*-Methoxy-*N*-methyl acetamide (776 mg, 7.52 mmol) was added; the mixture was slowly warmed up to room temperature over night. Water (10 ml) was added and the reaction mixture was extracted with DCM. The organic layer was dried over MgSO₄, concentrated and purified by column chromatography (Hex/EtOAc, gradient) to give the desired product as a yellowish solid (470 mg). MS (ES) (M+H)⁺: 153 for C₇H₈N₂O₂; NMR (CDCl₃): 2.77 (s, 3H), 4.10 (s, 3H), 6.89 (d, 1H), 8.62 (d, 2H).

### Intermediate 74

### 2-Iodo-4-methoxypyrimidine

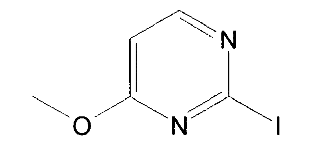

2-Chloro-4-methoxypyrinmidine (1.05 g, 7.26 mmol) was dissolved in DCM (20 ml), cooled down to -5 °C, hydrogen iodide (55%, 36 mmol) was added drop wise, the mixture was stirred at 0 °C for 4 hours then slowly warmed up to room temperature over night. The reaction mixture was cooled down to 0 °C and K₂CO₃ was added to neutralize the solution to pH=7, followed by the addition of 30% sodium meta bisulfite. This mixture was extracted with DCM (20 ml x 3). Organic layer was dried over anhydrous Na₂SO₄, concentrated to give a white solid as the desired product (1.62 g). MS (ES) (M+H)⁺: 237 for C₅H₅IN₂O; NMR (CDCl₃): 4.00 (s, 3H), 6.73 (d, 1H), 8.13 (d, 1H).

### Intermediate 75

### Ethyl 2-chloro-4-(1,3,4-oxadiazol-2-yl)thiazole-5-carboxylate

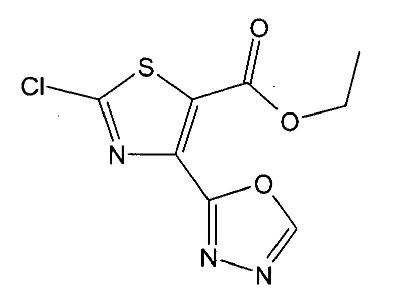

Ethyl 2-chloro-4-(2-formylhydrazinecarbonyl)thiazole-5-carboxylate (Intermediate 76; 220 mg, 0.79 mmol), phosphorus triphenyl (416 mg, 1.58 mmol), carbon tetrachloride (0.154 ml, 1.58 mmol) and DIEA (0.415 ml, 2.38 mmol) were mixed in anhydrous acetonitrile (10 ml), stirred at room temperature over night, LC-MS indicated the SM was disappeared and the desired product mass and a side product. Solvent was evaporated and the residue was purified by column chromatography (DCM/EtOAc) afforded the desired product (78 mg). MS (ES) (M+H)⁺: 260 for C₈H₆ClN₃O₃S; NMR (CDCl₃): 1.36 (t, 3H), 4.40 (q, 2H), 8.59 (s, 1H).

### Intermediate 76

### Ethyl 2-chloro-4-(2-formylhydrazinecarbonyl)thiazole-5-carboxylate

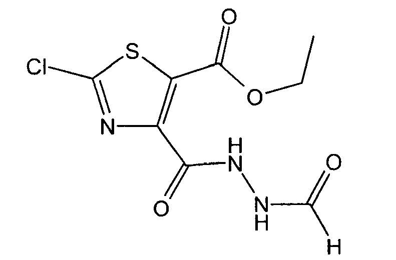

Ethyl 2-chloro-4-(chlorocarbonyl)thiazole-5-carboxylate (Intermediate 77; 300 mg, 1.18 mmol)and 2,6-lutidine (0.206 ml, 1.77 mmol) were mixed in 5 ml of dry DCM, cooled down to 0 °C, formohydrazide (73.8 mg, 1.20 mmol) was added, the reaction mixture was stirred at 0 °C for 5 min and gradually warmed up to room temperature overnight. Solvent was removed and the residual was purified by column eluted with DCM/MeOH gradient. Product was obtained as yellowish solid. (230 mg). MS (ES) (M+H)⁺: 278 for C₈H₈ClN₃O₄S; NMR (CDCl₃): 1.38 (t, 3H), 4.44 (q, 2H), 8.24 (s, 1H), 9.41 (br, 1H), 11.68 (br, 1H).

### Intermediate 77

### Ethyl 2-chloro-4-(chlorocarbonyl)thiazole-5-carboxylate

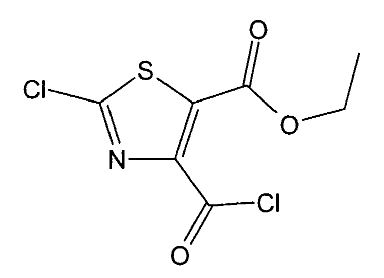

2-Chloro-5-(ethoxycarbonyl)thiazole-4-carboxylic acid (2 g, 8.49 mmol) was dissolved in dry DCM (15 ml), oxalyl dichloride (1.131 g, 8.91 mmol) was added and the mixture was cooled to 0 °C, 2 drops of DMF was added and the reaction mixture was stirred at room temperature until the bubbling stopped. Solvent was removed and the residual was dried under high vacuum over night afforded the desired product as a crystal (2.15 g). NMR (CDCl₃): 1.35 (t, 3H), 4.37 (q, 2H).

### Intermediate 78

### Ethyl 2-chloro-4-(3-methyl-1,2,4-oxadiazol-5-yl)thiazole-5-carboxylate

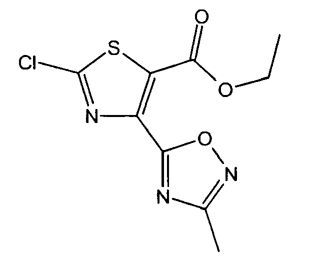

Ethyl 2-chloro-4-(chlorocarbonyl)thiazole-5-carboxylate (Intermediate 77; 300 mg, 1.18 mmol) and 2,6-lutidine (0.206 ml, 1.77 mmol) were mixed in 5 ml of dry DCM, cooled down to 0 °C, (Z)-N'-hydroxyacetimidamide (WO200032565, 91 mg, 1.18 mmol) was added, the reaction mixture was stirred at 0 °C for 5 min, DMF (2 ml) was added to assist the solvation of the SM and the reaction mixture was gradually warmed up to room temperature overnight. DCM was removed and the remaining solution was heated to 55 °C and stirred for 4 days. LC-MS showed the reaction was completed. The reaction mixture was diluted with EtOAc (20 ml), washed with water (10 mlx3), the organic phase was washed with brine and dried over MgSO₄, concentrated and purified by column chromatography (Hex/EtOAc) to give the desired product (yellowish crystal, 140 mg). MS (ES) (M+H)⁺: 274 for C₉H₈ClN₃O₃S; NMR (CDCl₃): 1.36 (t, 3H), 2.55 (s, 3H), 4.40 (q, 2H).

### Intermediate 79

### Ethyl 2-chloro-4-(5-methyl-1,3,4-oxadiazol-2-yl)thiazole-5-carboxylate

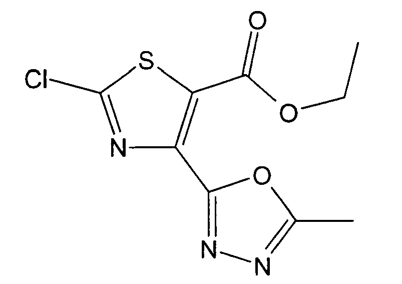

Ethyl 4-(2-acetylhydrazinecarbonyl)-2-chlorothiazole-5-carboxylate (Intermediate 80; 264 mg, 0.91 mmol), phosphorus triphenyl (475 mg, 1.81 mmol), carbon tetrachloride (0.176 ml, 1.81 mmol) and diisopropyl ethylamine (0.484 ml, 2.72 mmol) were mixed in anhydrous acetonitrile (10 ml). The mixture was stirred at room temperature over night, solvent was then evaporated. The residue was purified by column chromatography (DCM/EtOAc) to give the desired product as a solid (211 mg). MS (ES) (M+H)⁺: 274 for C₉H₈ClN₃O₃S; NMR (CDCl₃): 1.37 (t, 3H), 2.67 (s, 3H), 4.40 (q, 2H).

### Intermediate 80

### Ethyl 4-(2-acetylhydrazinecarbonyl)-2-chlorothiazole-5-carboxylate

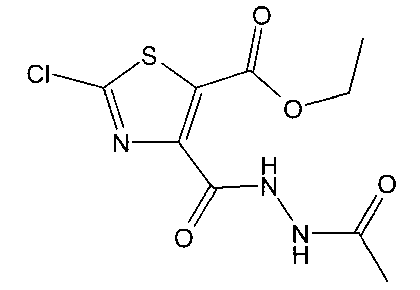

Ethyl 2-chloro-4-(chlorocarbonyl)thiazole-5-carboxylate (Intermediate 77; 300 mg, 1.18 mmol) and 2,6-lutidine (0.206 ml, 1.77 mmol) were mixed in 5 ml of dry DCM, cooled down to 0 °C, acetohydrazide (97 mg, 1.18 mmol) was added, the reaction mixture was stirred at 0 °C for 5 min and gradually warmed up to room temperature overnight. Solvent was removed and the residual was purified by column eluted with DCM/MeOH gradient. Product was obtained as yellowish solid. (264 mg). MS (ES) (M+H)⁺: 274 for C₉H₁₀ClN₃O₄S; NMR (CDCl₃): 1.39 (t, 3H), 2.12 (s, 3H), 4.45 (q, 2H), 8.55 (d, 1H), 11.66 (d, 1H).

### Intermediate 81-86

The following Intermediates were synthesized by an analogous method to Intermediate I from the starting materials (SM) given in the table below.

| **Int** | **Compound** | **Data** | **SM** |
|---|---|---|---|
| **81** | methyl 2-chloro-4-(1-methyl-1H-1,2,3-triazol-4-yl)-1,3-thiazole-5-carboxylate | MS (ES): 259 (MH⁺) for C₈H₇ClN₄O₂S | Intermediate 87 |
| | | ¹H-NMR (CDCl₃) δ: 3.9(s, 3H); 4.18 (s, 3H); 8.71(s, 1H). | |
| | 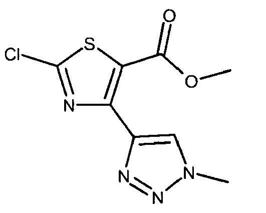 | | |
| **82** | methyl 2-chloro-4-(3,5-dimethylpyrazin-2-yl)-1,3-thiazole-5-carboxylate | MS (ES): 284 (MH⁺) for C₁₁H₁₀ClN₃O₂S | Intermediate 88 |
| | | ¹H-NMR (CDCl₃): 2.43 (s, 3H); 2.57 (s, 3H); 3.76 (s, 3H); 8.42 (s, 1H). | |
| | 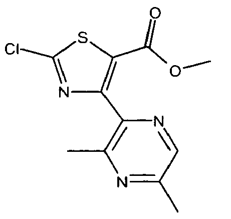 | | |
| **83** | methyl 2-chloro-4-(6-methoxypyridin-2-yl)-1,3-thiazole-5-carboxylate | MS (ES): 285 (MH⁺) for C₁₁H₉ClN₂O₃S | Intermediate 89 |
| | | ¹H-NMR (CDCl₃): 3.85 (s, 3H); 3.93 (s, 3H); 6.85 (d, 1H); 7.41 (d, 1H); 7.73 (t, 1H). | |
| | 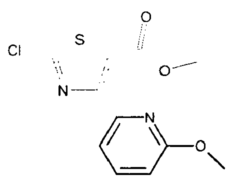 | | |
| **84** | ethyl 2-chloro-4-(4-methoxypyridin- 2-yl)-1,3-thiazole-5-carboxylate | MS (ES): 299 (MH⁺) for C₁₂H₁₁ClN₂O₃S | Intermediate 90 |
| | 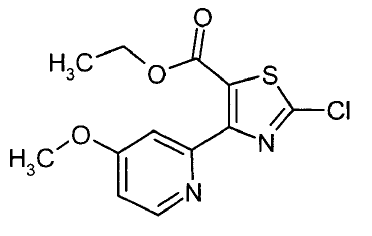 | ¹H-NMR (CDCl₃): 1.32 (t, 3H); 3.77 (s, 3H); 4.21 (q, 2H); 6.77 (dd, 1H); 7.25 (d, 1H); 8.40 (d, 1H). | |
| **85** | ethyl 2-chloro-4-(5-methylpyrazin2-yl)-1,3-thiazole-5-carboxylate | MS (ES): 284 (MH⁺) for C₁₁H₁₀ClN₃O₂S | Intermediate 91 |
| | 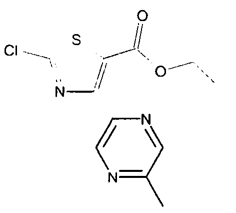 | ¹H-NMR (CDCl₃): 2.43 (s, 3H); 2.57 (s, 3H); 3.76 (s, 3H); 8.42 (s, 1H). | |
| **86** | ethyl 2-chloro-4-(3-fluoropyridin-2-yl)-1,3-thiazole-5-carboxylate | MS (ES): 287 (MH⁺) for C₁₁H₈ClFN₂O₂S | Intermediate 92 |
| | 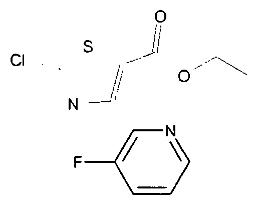 | ¹H-NMR: 1.25 (t, 3H); 4.28 (q, 2H); 7.55 (dd, 1H); 7.62 (t, 1H); 8.62 (d, 1H). | |

### Intermediate 87-92

The following Intermediates were synthesized by an analogous method to Intermediate 16 from the starting materials (SM) given in the table below.

| **Int** | **Compound** | **Data** | **SM** |
|---|---|---|---|
| **87** | 2-methyl 2-amino-4-(1-methyl-1H-1,2,3-triazol-4-yl)-1,3-thiazole-5-carboxylate | MS (ES): 240 (MH⁺) for C₈H₉N₅O₂S | Intermediate 93 |
| | | ¹H-NMR (CDCl₃): 3.80 (s, 3H); 4.18 (s, 3H); 8.16 (s, 1H). | |
| | 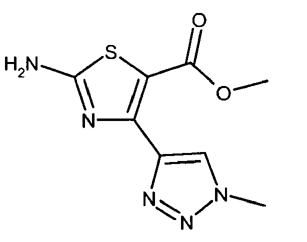 | | |
| **88** | methyl 2-amino-4-(3,5-dimethylpyrazin-2-yl)-1,3-thiazole-5-carboxylate | MS (ES): 278 (MH⁺) for C₁₁H₁₂N₄O₂S | Intermediate 94 |
| | | ¹H-NMR (CDCl₃₎: 2.49 (s, 3H); 2.59 (s, 3H); 3.71 (s, 3H); 6.90 (br, 2H); 8.44 (s, 1H). | |
| | 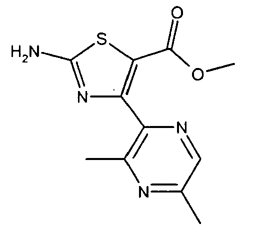 | | |
| **89** | methyl 2-amino-4-(6-methoxypyridin-2-yl)-1,3-thiazole-5-carboxylate | MS (ES): 266 (MH⁺) for C₁₁H₁₁N₃O₃S | Intermediate 95 |
| | | ¹H-NMR (CDCl₃): 3.75 (s, 3H); 3.98 (s, 3H); 6.27 (br, 2H); 6.77 (d, 1H); 7.42 (d, 1H); 7.63 (t, 1H). | |
| | 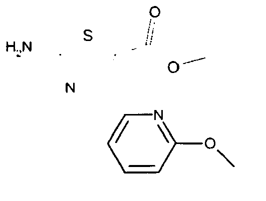 | | |
| **90** | ethyl 2-amino-4-(4-methoxypyridin-2-yl)-1,3-thiazole-5-carboxylate | MS (ES): 280 (MH⁺) for C₁₂H₁₃N₃O₃S | Intermediate 96 |
| | 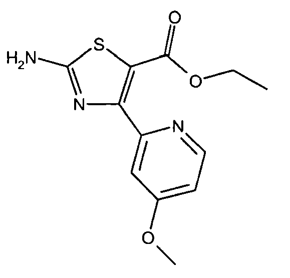 | ¹H-NMR (CDCl₃): 1.20 (t, 3H); 3.87 (s, 3H); 4.15 (q, 2H); 6.27 (br, 2H); 6.83 (dd, 1H); 7.33 (d, 1H); 8.47 (d, 1H). | |
| **91** | ethyl 2-amino-4-(5-methylpyrazin-2-yl)-1,3-thiazole-5-carboxylate | MS (ES): 278 (MH⁺) for C₁₁H₁₂N₄O₂S | Intermediate 97 |
| | 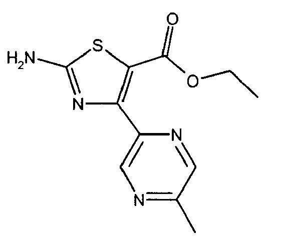 | ¹H-NMR (CDCl₃): 1.30 (t, 3H); 1.57 (s, 2H); 2.65 (s, 3H); 4.30 (q, 2H); 8.55 (s, 1H); 8.91 (s, 1H). | |
| **92** | ethyl 2-amino-4-(3-fluoropyridin-2-yl)-1,3-thiazole-5-carboxylate | MS (ES): 268 (MH⁺) for C₁₁H₁₀FN₃O₂S | Intermediate 98 |
| | 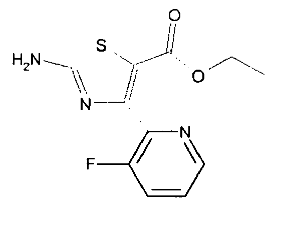 | ¹H-NMR: 1.01 (t, 3H); 3.99 (q, 2H); 7.53 (dd, 1H); 7.77 (t, 1H); 7.95 (s, 2H); 8.44 (d, 1H). | |

### Intermediate 93-98

The following intermediates were synthesized by an analogous method to Intermediate 44 from the starting materials (SM) given in the table below.

| **Int** | **Compound** | **Data** | **SM** |
|---|---|---|---|
| **93** | methyl 3-(1-methyl-1H-1,2,3-triazol-4-yl)-3-oxopropanoate | MS (ES): 184 (MH⁺) for C₇H₉N₃O₃ | 1-(1-methyl-1H-1,2,3-triazol-4-yl)ethanone Bull Soc Chim Belg [BSCBAG] 1991, 100 (4), 289-290 |
| | 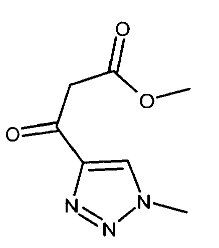 | ¹H-NMR (CDCl₃): 3.74 (s, 3H); 4.14 (s, 2H); 4.16 (s, 3H); 8.10 (s, 1H). | |
| **94** | methyl 3-(3,5-dimethylpyrazin-2-yl)-3-oxopropanoate | MS (ES): 209 (MH⁺) for C₁₀H₁₂N₂O₃ | 1-(3,5-dimethylpyrazin-2-yl)ethanone |
| | 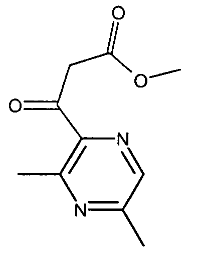 | ¹H-NMR (CDCl₃): 2.55 (s, 3H); 2.81 (s, 3H); 3.72 (s, 3H); 4.15 (s, 2H); 8.48 (s, 1H). | |
| **95** | methyl 3-(6-methoxypyridin-2-yl)-3-oxopropanoate | MS (ES): 210 (MH⁺) for C₁₀H₁₁NO₄ | 1-(6-methoxypyridin-2-yl)ethanone |
| | 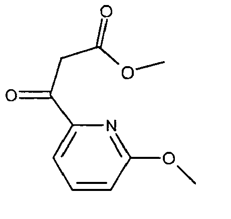 | ¹H-NMR (CDCl₃): 3.71 (s, 3H); 3.94 (s, 3H); 4.09 (s, 2H); 6.95 (d, 1H); 7.68 (d, 1H); 7.72 (t, 1H). | |
| **96** | ethyl 3-(4-methoxypyridin-2-yl)-3-oxopropanoate | MS (ES): 224 (MH⁺) for C₁₁H₁₃NO₄ | 3-(4-methoxypyridin-2-yl)ethanone |
| | 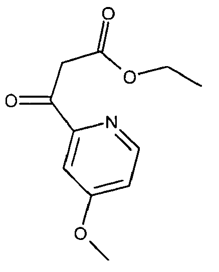 | | |
| **97** | ethyl 3-(5-methylpyrazin-2-yl)-3-oxopropanoate | MS (ES): 209 (MH⁺) for C₁₀H₁₂N₂O₃ | 1-(5-methylpyrazin-2-yl)ethanone |
| | 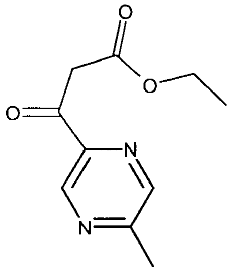 | ¹H-NMR (CDCl₃: 1.23 (t, 3H); 2.66 (s, 3H); 4.12 (s, 2H); 4.20 (q, 2H); 8.48 (s, 1H); 9.13 (s, 1H). | |
| **98** | ethyl 3-(3-fluoropyridin-2-yl)-3-oxopropanoate | MS (ES): 212 (MH⁺) for C₁₀H₁₀FNO₃ | 1-(3-fluoropyridin-2-yl)ethanone |
| | 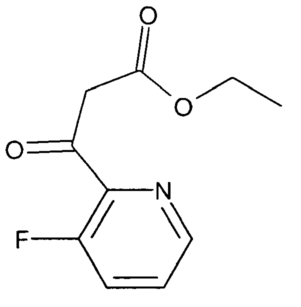 | ¹H-NMR: 1.15 (t, 3H); 3.99 (q, 2H); 4.10 (s, 2H); 7.45 (dd, 1H); 8.36 (t, 1H); 8.56 (d, 1H). | |

### Intermediate 99

### Ethyl (3S,4R)-4-amino-3-(benzyloxy)pipendine-1-carboxytate

Racemic Ethyl-4-amino-3-(benzyloxy)piperidine-1-carboxylate (WO2006087543) was separated by chiral HPLC (Chiralpak AD 5X50 cm, 20u, 85% Hexane, 15% ethanol:methanol (1:1), 0.1 % diethylamine) to give the desired product.

### Intermediate 100-102

The following Intermediates were synthesized by an analogous method to Intermediate 62 from the starting materials (SM) given in the table below.

| **Int** | **Compound** | **Data** | **SM** |
|---|---|---|---|
| **100** | Ethyl(3*S*,4*R*)-3-(benzyloxy)-4-{[(3,4- dichloro-5-methyl-1*H*-pyrrol-2-yl)carbonyl]amino}piperidine-1-carboxylate: | MS (APCI): 454 (M+H) | Intermediate 99 and 3,4-dichloro-5-methyl-1H-pyrrole-2-carboxylic acid (WO2006087543) |
| | | ¹H NMR : δ 1.22 (br s, 3H), 1.65 (m, 2H), 1.93 (s, 3H), 3.30 (m, 2H), 3.65 (br s, 1H), 4.09 (br s, 2H), 4.22 (m, 1H), 4.45 (m, 2H), 4.75 (d, 1H), 7.12 (br s, 1H), 7.35 (m, 5H), 12.16 (s, 1H). | |
| | 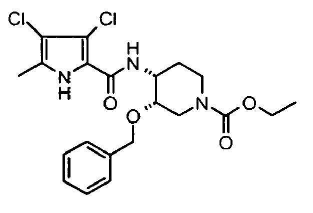 | | |
| **101** | Ethyl (3S,4R)-3-(benzyloxy)-4-{[(4-chloro-5-methyl-1H-pyrrol-2-yl)carbonyl]amino}piperidine-1-carboxylate | ¹H NMR: 1.19 (t, 3H), 1.53 (m, 1H), 1.86 (m, 1H), 2.18 (s, 1H), 3.08 (m, 2H), 3.65 (s, 1H), 4.09 (q, 4H), 4.38 (m, 3H), 4.63 (d, 2H), 6.89 (s, 1H), 7.27 (d, 5H), 7.64 (m, 1H), 11.56 (s, 1H). | Intermediate 99 and 4-chloro-5-methyl-1H-pyrrole-2-carboxylic acid (WO2006087543) |
| | 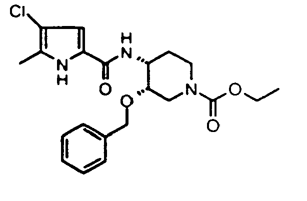 | | |
| **102** | Ethyl (3S,4R)-3-(benzyloxy)-4-{[(4-chloro-3-cyano-5-methyl-1H-pyrrol-2-yl)carbonyl]amino}piperidine -1-carboxylate | ¹H NMR: 1.21 (t, 3H), 1.61 (m, 1H), 2.21 (s, 3H), 3.04 (m, 2H), 3.68 (s, 1H), 4.06 (m, 3H), 4.24 (q, 2H), 4.48 (d, 1H), 4.64 (d, 1H), 7.29 (d, 3H), 7.38 (s, 2H), 7.62 (s, 1H), 12.61 (s, 1H). | Intermediate 99 and 4-chloro-3-cyano-5-methyl-1*H*-pyrrole-2-carboxylic acid |
| | 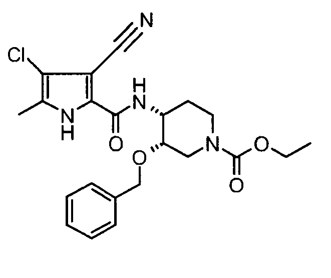 | | |

**Intermediate 103-105**

The following Intermediates were synthesized by an analogous method to Intermediate 64 from the starting materials (SM) given in the table below.

| **Int** | **Compound** | **Data** | **SM** |
|---|---|---|---|
| **103** | N-[(3S,4R)-3-(benzyloxy)piperidin-4-yl]-3,4-dichloro-5-methyl-1H-pyrrole-2-carboxamidc | MS (APCI): 382 (M+H) ¹H NMR: 2.17 (s, 3H), 2.58 (m, 2H), 2.88 (d, 1H), 3.18 (d, 1H), 3.58 (s, 1H), 4.16 (br s, 1H), 4.43 (d, 1H), 4.69 (d, 1H), 7.36 (m, 5H), 7.09 (br s, 1H), 12.09 (m, 1H). HPLC: 96.39% | Intermediate 100 |
| | 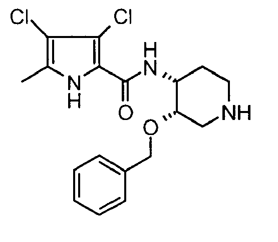 | | |
| **104** | N-[(3S,4R)-3-(benzyloxy)piperidin-4-yl]-4-chloro-5-methyl-1H-pyrrole-2-carboxamide | ¹H NMR: 1.43 (m, 1H), 1.84 (m, 1H), 2.20 (s, 3H), 2.45 (m, 1H), 2.94 (d, 2H), 3.16 (m, 1H), 3.56 (s, 1H), 4.06 (br s, 1H), 4.45 (d, 1H), 4.64 (d, 1H), 6.87 (s, 1H), 7.32 (dd, 5H), 7.59 (d, 1H), 11.62 (s, 1H). | Intermediate 101 |
| | 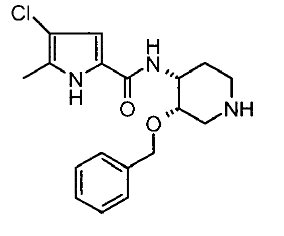 | | |
| **105** | N-[(3S,4R)-3-(benzyloxy)piperidin-4-yl]-4-chloro-3-cyano-5-methyl-1H-pyrrole-2-carboxamide | ¹H NMR: 1.64 (m, 1H), 1.78 (m, 1H), 2.19 (s, 3H), 2.78 (m, 2H), 2.98 (d, 1H), 3.61 (s, 1H), 3.27 (d, 1H), 4.19 (br s, 1H), 4.51 (d, 1H), 4.67 (d, 1H), 7.27 (m, 3H), 7.43 (d, 2H), 7.54 (d, 1H). | Intermediate 102 |
| | 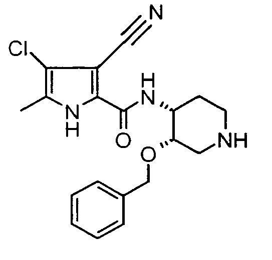 | | |

### Intermediate 106

### Ethyl 4-(benzyloxy)-3-oxobutanoate

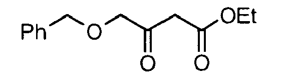

Sodium hydride (145 g, 6.06 mol) was suspended in tetrahydrofuran (3.0 L). Benzyl alcohol (328 g, 3.03 mol and ethyl 4-chloro-3-oxobutanoate (500 g, 3.03 mol) were added to the suspension and the reaction mixture was stirred at 40 °C for 2 h. The reaction mixture was cooled to room temperature, quenched with ice water and extracted with ethyl acetate. The combined organic layers were dried over anhydrous sodium sulphate and concentrated under vacuum to afford 500 g (69%) of Ethyl 4-(benzyloxy)-3-oxobutanoate.
¹H NMR (400 MHz, CDCl₃): δ 1.29 (t, 3H), 3.53 (s, 2H), 4.22 (q, 2H), 4.22 (s, 2H), 4.60 (s, 2H), 7.38 (m, 5H).

### Intermediate 107

### Ethyl 2-amino-4-[(benzyloxy)methy]-1,3-thiazole-5-carboxylate

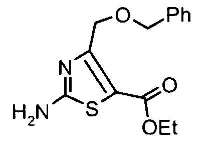

Ethyl 4-(benzyloxy)-3-oxobutanoate (Intermediate 106, 100 g, 0.42 mol) was dissolved in ethyl acetate (800 mL), amberlyst 15 ion exchange resin (100 g) and N-iodo-succinimide (104.4 g, 0.47 mol) were added, and the reaction mixture was stirred at room temperature for 1 h. The reaction mixture was filtered and rinsed with ethyl acetate and the combined filtrate was concentrated under reduced pressure. The resulting residue was partitioned between ethyl acetate and water (1:1) and the resulting organic layer was dried over sodium sulphate and concentrated to dryness to yield a solid, which was dried under high vacuum. To this residue, thiourea (48.0 g, 0.63 mol) and methanol (500 mL) were added and the reaction mixture was heated at reflux for 50 min, cooled to room temperature and the solvent was removed under reduced pressure. The resulting residue was suspended in saturated aqueous sodium bicarbonate solution (500 mL) and extracted with ethyl acetate (5 x 300 mL). The resulting organic layer was dried over sodium sulphate and concentrated under reduced pressure, diethyl ether was added, and the resulting mixture was stirred for 2 h. The solid that formed was collected by filtration and washed with diethyl ether to yield ethyl 2-amino-4-[(benzyloxy)methyl]-1,3-thiazole-5-carboxylate as a white solid 25 g (20%)
¹H NMR (400 MHz, DMSO-d₆): δ 1.23 (t, 3H), 4.19 (q, 2H), 4.51 (s, 2H), 4.72 (s, 2H), 7.35 (m, 5H), 7.80 (s, 2H).

### Intermediate 108

### Ethyl 2-amino-4-(hydroxymethyl)-1,3-thiazole-5-carboxylate

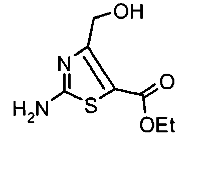

To an anisole solution of aluminium chloride (9.1 g, 68.4 mmol) was added ethyl 2-amino-4-[(benzyloxy)methyl]-1,3-thiazole-5-carboxylate (Intermediate 107, 2.0 g, 6.84 mmol) at room temperature and stirred for 3 h. The reaction mixture was cooled to 0 °C and methanol was added to the reaction mixture followed by removal of the solvent under vacuum. Ice-cold water was added to the resulting residue and stirred for ½h. The solid that formed was collected by filteration and washed with ethyl acetate to obtain Ethyl 2-amino-4-(hydroxymethyl)-1,3-thiazole-5-carboxylate (0.6 g, 47%) as solid.

¹H NMR (400 MHz, DMSO-d₆): δ 1.22 (t, 3H), 4.14 (q, 2H), 4.56 (s, 2H), 4.84 (s, 1H), 7.74 (s, 2H).

### Intermediate 109

### Ethyl 2-amino-4-formyl-1,3-thiazole-5-carboxylate

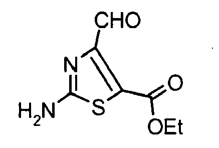

To a solution of ethyl 2-amino-4-(hydroxymethyl)-1,3-thiazole-5-carboxylate (Intermediate 108, 1.0 g, 4.9 mmol) in tetrahydrofuran (50 mL) was added manganese dioxide (6.5 g, 74.2 mmol) and stirred for 8 h at room temperature. The reaction mixture was filtered through celite bed and washed with ethyl acetate. The filtrate was concentrated under reduced pressure to give ethyl 2-amino-4-formyl-1,3-thiazole-5-carboxylate (0.7 g, 70%) as yellow solid.
¹H NMR (400 MHz, DMSO-d₆): δ 1.27 (t, 3H), 4.28 (q, 2H), 8.01 (s, 2H), 10.28 (s, 1H).

### Intermediate 110

### Ethyl 2-amino-4-cyano-1,3-thiazole-5-carboxylate

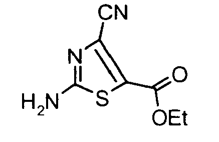

To a solution of ethyl 2-amino-4-formyl-1,3-thiazole-5-carboxylate (Intermediate 109, 7.0 g, 35.1 mmol) in tetrahydrofuran (35 mL) was added aqueous ammonia solution (50 mL) and iodine (8.89 g, 35.1 mMmol) at room temperature. The reaction mixture was stirred for 5 h and the solid that formed was collected by filtration and washed with water to afford ethyl 2-amino-4-cyano-1,3-thiazole-5-carboxylate (3.0 g; 48%) as solid
¹H NMR (400 MHz, DMSO-d₆): δ 1.27 (t, 3H), 4.26 (q, 2H), 8.31 (s, 2H).

### Intermediate 111

### Ethyl 2-chloro-4-cyano-1,3-thiazole-5-carboxylate

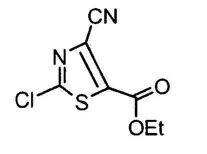

To a 0 °C suspension of ethyl 2-amino-4-cyano-1,3-thiazole-5-carboxylate (Intermediate 110, 1.0 g, 5.5 mmol) in acetic acid (3 mL) and HCI (10 mL), sodium nitrite (1.05 g, 15.1 mM) in water (10 mL) was added drop wise. After stirring at 0 °C for 10 min the reaction mixture was warmed up to room temperature and stirred for 30 min. A solution of urea (0.6 g, 10.1 mmol) in water (5 mL) was added dropwise and the reaction mixture was stirred for 10 min. The reaction mixture was neutralized with solid sodium carbonate and saturated aqueous sodium bicarbonate solution. The resulting liquid was extracted with ethyl acetate (5 x 30 mL) and the combined ethyl acetate layer was washed with saturated sodium bicarbonate solution and brine, dried over sodium sulphate and concentrated under reduced pressure to an oil, which was purified by column chromatography over silica gel to afford Ethyl 2-chloro-4-cyano-1,3-thiazole-5-carboxylate (0.6 g, 60%).
¹H NMR (400 MHz, CDCl₃): δ 1.41 (t, 3H), 4.47 (q, 2H).

### Intermediate 112

### Ethyl 2-chloro-4-(1H-tetrazol-5-yl)-1,3-thiazole-5-carboxylate

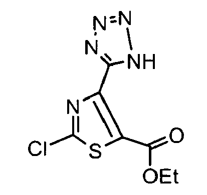

To a solution of ethyl 2-chloro-4-cyano-1,3-thiazole-5-carboxylate (Intermediate 111, 8.0 g, 37.1 mmol) in dioxane (300 mL) was added azido-trimethylsilane (21.2 g, 185.1 mmol) and dimethyl tin oxide (0.6 g, 3.7 mmol). The reaction mixture was heated to reflux for 12 h and concentrated under reduced pressure. Purification by preparative HPLC afforded ethyl 2-chloro-4-(1H-tetrazol-5-yl)-1,3-thiazole-5-carboxylate 3.0 g (31%).
¹H NMR (400 MHz, DMSO-d₆): δ 1.19 (t, 3H), 4.27 (q, 2H)

### Intermediate 113

### ethyl (3S,4R)-4-amino-3-propoxypiperidine-1-carboxylate

Racemic ethyl 4-amino-3-propoxypiperidine-1-carboxylate (WO2006087543) was separated by chiral HPLC (Chiralpak AD 5X50 cm, 20u, 85% Hexane, 15% ethanol:methanol (1:1), 0.1 % diethylamine) to give the desired product.

### Intermediate 114

The following Intermediate was synthesized by an analogous method to Intermediate 62 from the starting materials (SM) given in the table below.

| **Int** | **Compound** | **Data** | **SM** |
|---|---|---|---|
| **114** | Ethyl (3S,4R)-4-{[(3,4-dichloro-5-methyl-1H-pyrrol-2-yl)carbonyl]amino}-3-propoxypiperidine-1-carboxylate | ¹H NMR (400 MHz, DMSO-d₆): 0.86 (t, 3H), 1.18 (t, 3H), 1.50-1.52 (m, 4H), 2.18 (s, 3H), 2.95 (m, 3H), 3.29 (br s, 1H), 4.01-4.02 (q, 2H), 4.12-4.23 (m, 4H). | Intermediate 113 and ethyl (3S,4R)-3,4-dichloro-5-methyl-1H-pyrrole-2-carboxylic acid (WO2006087543) |
| | 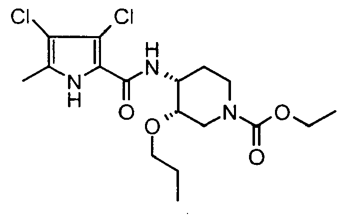 | | |

### Intermediate 115

The following Intermediate was synthesized by an analogous method to Intermediate 64 from the starting materials (SM) given in the table below.

| **Int** | **Compound** | **Data** | **SM** |
|---|---|---|---|
| **115** | 3,4-dichloro-5-methyl-N-[(3S,4R)-3-propoxypiperidin-4-yl]-1H-pyrrole-2-carboxamide | ¹H NMR (400 MHz, DMSO-d₆): 0.88 (t, 3H), 1.51-1.52 (q, 4H), 2.17 (s, 3H), 2.81-2.84 (m, 1H), 3.02-3.03 (d, 1H), 3.05-3.06 (m, 1H), 3.26 (m, 2H), 3.34 (br s, 1H), 3.49 (q, 2H), 4.06 (br s, 1H), 7.05 (d, 1H). | Intermediate 114 |
| | 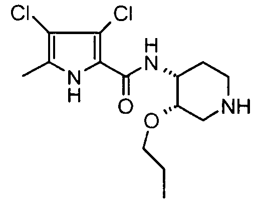 | | |

### Intermediate 116

### tert-Butyl (3S,4R)-4-{[(4-chloro-3.5-dimethyl-1H-pyrrol-2-yl)carbonyl]amino}-3-fluoropiperidine-1-carboxylate

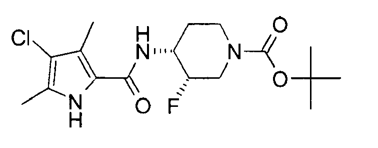

In a 100 mL round bottom flask was taken 4-chloro-3,5-dimethyl-1H-pyrrole-2-carboxylic acid (WO 2006087543,1.5 g, 8.64 mmol), HATU (3.94 g, 10.37 mmol) and DIEA (3.32 ml, 19.01 mmol in DCM (43.2 ml) to give a brown solution. To this was added (3S, 4R)-*tert-*butyl 4-amino-3-fluoropiperidine-1-carboxylate (WO 2006087543, 1.886 g, 8.64 mmol) and the mixture was allowed to stir at RT overnight. The solvent was then removed under vacuum and the residue was taken in MeOH (2 mlL) and water was added to precipitate the product. The solid thus obtained was filtered, washed with water then hexane and dried under vacuum to afford the title compound (3.00 g).
MS(ES⁺-55): 318 for C₁₃H₁₇ClFN₃O₃(M-55)

### Intermediate 117

### 4-chloro-N-[(3S,4R)-3-fluoropiperidin-4-yl]-3,5-dimethyl-1H-pyrrole-2-carboxamide

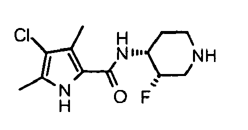

In a 50 mL round-bottomed flask was taken *tert*-Butyl (3*S*,4*R*)-4-{[(4-chloro-3,5-dimethyl-1*H*-pyrrol-2-yl)carbonyl]amino}-3-fluoropiperidine-1-carboxylate (Intermediate 116, 3.0 g, 8.02 mmol) in 4N HCl in dioxane (20.06 ml, 80.25 mmol) to give a yellow solution which was heated at 55 °C for 6.5 h. TLC and LCMS indicated completion of reaction. The reaction mixture was evaporated to dryness and methanol (15 ml) was added to the residue then the mixture was evaporated to dryness. Water (15 ml) was added to the residue and the mixture was neutralized with 14% NaOH to pH∼7.0. The solid that precipitated was dried under vacuum to afford 4-chloro-*N*-[(3*S*,4*R*)-3-fluoropiperidin-4-yl]-3,5-dimethyl-1*H*-pyrrole-2-carboxamide (1.212 g).
MS (ES⁺): 274 for C₁₂H₁₇ClFN₃O

### Intermediate 118

### Ethyl (3S,4R)-4-{[(4-chloro-3-cyano-5-methyl-1H-pyrrol-2-yl)carbonyl]amino}-3-methoxypioeridine-1-carboxylate

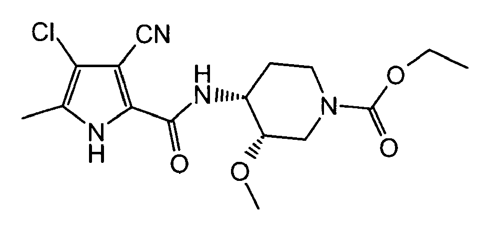

In a 50 ml round bottom flask 4-chloro-3-cyano-5-methyl-1H-pyrrole-2-carboxylic acid (WO 2006087543,540 mg, 2.93 mmol) was dissolved in CH₂Cl₂ (25 ml) and DIEA (2.55 ml, 14.63 mmol), HATU (1335 mg, 3.51 mmol) was added and stirred for 5 minutes and then (3S, 4R)-ethyl 4-amino-3-methoxypiperidine-1-carboxylate ((1R)-7,7-dimethyl-2-oxobicyclo [2.2.1] heptan-1-yl) methanesulfonate (WO2006087543, 1271 mg, 2.93 mmol) was added portion wise and the resulting mixture was stirred for overnight at RT. The progress of the reaction was monitored through LCMS which showed completion of the reaction after stirring the reaction mixture for overnight. The reaction mixture was diluted with DCM and washed with water. The organic layer was dried over sodium sulphate and concentrated under vacuum to give ethyl (3*S*,4*R*)-4-{[(4-chloro-3-cyano-5-methyl-1*H*-pyrrol-2-yl)carbonyl]amino}-3-methoxypiperidine-1-carboxylate (1000 mg)..
MS (ES⁺): 369 for C₁₆H₂₁ClN₄O₄

### Intermediate 119

### 4-Chloro-3-cyano-N-[(3S,4R)-3-methoxypiperidin-4-yl]-5-methyl-1H-pyrrole-2-carboxamide

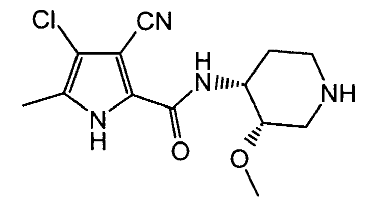

In a 250 mL round-bottomed flask (3S, 4R)-ethyl 4-(4-chloro-3-cyano-5-methyl-1*H*-pyrrole-2-carboxamido)-3-methoxypiperidine-1-carboxylate (Intermediate 118, 1 g, 2.71 mmol) was dissolved in EtOH (10 mL). NaOH (10M solution) (1.084 g, 27.11 mmol) was then added and the reaction mixture was heated to 80°C for 2 days. The progress of the reaction was monitored through LCMS. The reaction mixture was evaporated *in vacuo*, ice cold water (25 ml) was added and the mixture was neutralized with 6N HCl (pH 7), sonicated and the solid precipitate was filtered and dried under high vacuum afforded the product as pale brown solid (0.650 g).
MS (ES⁻): 297 for C₁₃H₁₇ClN₄O₂

## Claims

1. A compound of formula **(Ia):** 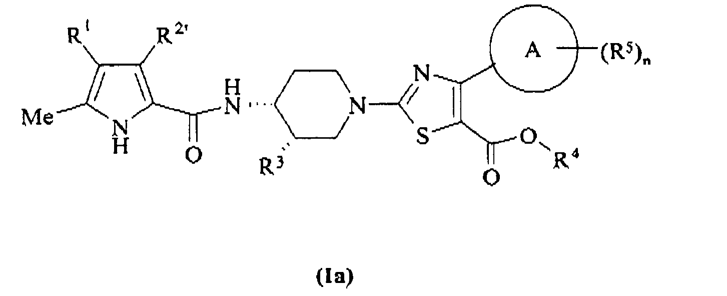
**R¹** is chloro or cyano;
**R^{2'}** is hydrogen, chloro, cyano or methyl;
**R³** is fluoro, methyl, methoxy, ethoxy, propoxy, allyloxy and benzyloxy;
**R⁴** is hydrogen or C₁₋₄alkyl;
Ring A is carbocyclyl or heterocyclyl; wherein if said heterocyclyl contains an -NH-moiety that nitrogen may be optionally substituted by a group selected from R⁶; and wherein if said heterocyclyl contains an -N= moiety that nitrogen may form a quaternary compound with a methyl group;
**R⁵** is a substituent on carbon and is selected from halo, nitro, cyano, hydroxy, amino, carboxy, carbamoyl, mercapto, sulphamoyl, sulfo, formyl, ureido, hydroxyiminomethyl, *N*-hydroxyformamido, hydrazinocarbonyl, *N*-hydroxyethanimidoyl, amino(hydroxyimino)methyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N*,*N*-(C₁₋₄alkyl)₂amino, C₁₋₄alkanoylamino, *N*-(C₁₋₄alkyl)carbamoyl, *N*,*N*-(C₁₋₄alkyl)₂carbamoyl, *N*-(C₁₋₄alkoxy)carbamoyl, *N'*-(C₁₋₄alkyl)ureido, *N*,*N*'-(C₁₋₄alkyl)₂ureido, *N-*(C₁₋₄alkyl)-*N*-(C₁₋₄alkoxy)carbamoyl, C₁₋₄alkyls(O)ₐ wherein a is 0 to 2, C₁₋₄alkoxycarbonyl, C₁₋₄alkoxycarbonylamino, *N*-(C₁₋₄alkyl)sulphamoyl, *N*,*N*-(C₁₋₄alkyl)₂sulphamoyl, C₁₋₄alkylsulphonylamino, C₁₋₄alkylsulphonylaminocarbonyl, *N*'-(C₁₋₄alkyl)hydrazinocarbonyl, *N'*,*N'*-(C₁₋₄alkyl)₂hydrazinocarbonyl, carbocyclyl-R⁷- or heterocyclyl-R⁸-; wherein R⁵ may be optionally substituted on carbon by one or more R⁹; and wherein if said heterocyclyl contains an -NH- moiety that nitrogen may be optionally substituted by a group selected from R¹⁰;
**n** is 0, 1, 2, or 3;
**R⁹** is selected from halo, nitro, cyano, hydroxy, amino, carboxy, carbamoyl, mercapto, sulphamoyl, C₁₋₄alkyl, C₂₋₄alkenyl, C₂₋₄alkynyl, C₁₋₄alkoxy, C₁₋₄alkanoyl, C₁₋₄alkanoyloxy, *N*-(C₁₋₄alkyl)amino, *N,N*-(C₁₋₄alkyl)₂amino, C₁₋₄alkanoylamino, *N*-(C₁₋₄alkyl)carbamoyl, *N*,*N*-(C₁₋₄alkyl)₂carbamoyl, C₁₋₄alkylS(O)ₐ wherein a is 0 to 2, C₁₋₄alkoxycarbonyl, *N*-(C₁₋₄alkyl)sulphamoyl, *N*,*N*-(C₁₋₄alkyl)₂sulphamoyl, C₁₋₄alkylsulphonylamino, C₁₋₄alkoxycarbonylamino, carbocyclyl-R¹¹- or heterocyclyl-R¹²-; wherein R⁹ may be optionally substituted on carbon by one or more R¹³; and wherein if said heterocyclyl contains an -NH- moiety that nitrogen may be optionally substituted by a group selected from R¹⁴;
**R⁶**, **R¹⁰** and **R¹⁴** are independently selected from C₁₋₄alkyl, C₁₋₄alkanoyl, C₁₋₄alkylsulphonyl, C₁₋₄alkoxycarbonyl, carbamoyl, *N*-(C₁₋₄alkyl)carbatnoyl, *N*,*N*-(C₁₋₄alkyl)carbamoyl, benzoyl, benzyloxycarbonyl, benzoyl and phenylsulphonyl; wherein R⁶, R¹⁰ and R¹⁴ may be independently optionally substituted on carbon by a group selected from R²⁰;
**R⁷**, **R⁸**, **R¹¹** and **R¹²** are independently selected from a direct bond, -O-, -N(R¹⁵)-, -C-(O)-, -N(R¹⁶)C(O)-, -C(O)N(R¹⁷)-, -S(O)ₚ-, -SO₂N(R¹⁸)- or -N(R¹⁹)SO₂-; wherein R¹⁵, R¹⁶, R¹⁷, R¹⁸ and R¹⁹ are independently selected from hydrogen or C₁₋₄alkyl and p is 0-2;
**R¹³** and **R²⁰** are independently selected from halo, nitro, cyano, hydroxy, trifluoromethoxy, trifluoromethyl, amino, carboxy, carbamoyl, mercapto, sulphamoyl, methyl, ethyl, ethenyl, ethynyl, methoxy, ethoxy, 2-trimethylsilylethoxy, acetyl, acetoxy, methylamino, ethylamino, dimethylamino, diethylamino, *N*-methyl-*N*-ethylamino, acetylamino, *N*-methylcarbamoyl, *N*-ethylcarbamoyl, *N*,*N*-dimethylcarbamoyl, *N*,*N*-diethylcarbamoyl, *N*-methyl-*N*-ethylcarbamoyl, methylthio, ethylthio, methylsulphinyl, ethylsulphinyl, mesyl, ethylsulphonyl, methoxycarbonyl, ethoxycarbonyl, *N*-methylsulphamoyl, *N-*ethylsulphamoyl, *N*,*N*-dimethylsulphamoyl, *N*,*N*-diethylsulphamoyl or *N*-methyl-*N*-ethylsulphamoyl;
or a pharmaceutically acceptable salt thereof.

2. The compound of Claim 1, wherein ring A is a heterocyclyl; wherein if said heterocyclyl contains an -N H- moiety that nitrogen may be optionally substituted by a group selected from R⁶; and wherein if said heterocyclyl contains an -N= moiety that nitrogen may form a quaternary compound with a methyl group; wherein:
R⁶ is C₁₋₄alkyl; wherein R⁶ may be optionally substituted on carbon by a group selected from R²⁰; and
R²⁰ is selected from methoxy or ethoxy.

3. The compound of Claim 2, wherein Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group selected from R⁶; and wherein if said imidazolyl may form a quaternary compound on an -N= moiety with a methyl, group; wherein
R⁶ is methyl or ethyl; wherein R⁶ may be optionally substituted on carbon by a group selected from R²⁰; and
R²⁰ is selected from methoxy or ethoxy.

4. The compound of Claim 3 Ring A is 1-(2-methoxyethyl)imidazol-2-yl, 1-(2-trimethylsilylethoxymethyl)imidazol-2-yl, 1-(methoxymethyl)imidazol-2-yl, 1,2,4-oxadiazol-5-yl, 1,3,4-oxadiazol-2-yl, 1,3-dimethylimidazol-2-yl, 1H-imidazol-2-yl, 1-methylimidazol-4-yl, 2H-pyrazol-3-yl, 2-methyl-1,2,4-triazol-3-yl, 2-pyridyl, benzothiazol-2-yl, isoxazol-5-yl, pyrazin-2-yl, pyrimidin-2-yl, pyrimidin-4-yl, thiazol-2-yl or thiazol-4-yl.

5. The compound of Claim 1, wherein Ring A is a carbocyclyl.

6. The compound of any one of the preceding claims wherein n is 0.

7. The compound of any one of Claims 1-5, wherein n is 1, 2, or 3 and R⁵ is selected from the group consisting of amino, N-methylamino, N,N-dimethylamino, methyl, and methoxy.

8. The compound of any one of Claims 1-5, wherein Ring A, R⁵ and n together form 1H-imidazol-2-yl, 2,6-bis(dimethylamino)pyrimidin-4-yl, 2-pyridyl, 2H-pyrazol-3-yl, pyrimidin-4-yl, isoxazol-5-yl, 1-methylimidazol-4-yl, pyrazin-2-yl, 2-aminothiazol-4-yl, 2-dimethylaminothiazol-4-yl, 2-methylaminothiazol-4-yl, 1,3-dimethylimidazol-2-yl, 1,4,5-trimethylimidazol-2-yl, 4,6-dimethoxypyrimidin-2-yl, 3-methyl-1,2,4-oxadiazol-5-yl, benzothiazol-2-yl, 4-methoxypyrimidin-2-yl, pyrimidin-2-yl, 1-methylimidazol-2-yl, 1-(2-methoxyethyl)imidazol-2-yl, 1-(methoxymethyl)imidazol-2-yl, 1-(2-trimethylsilylethoxymethyl)imidazol-2-yl, thiazol-2-yl, 2-methyl-1,2,4-triazol-3-yl, 5-methyl-1,3,4-oxadiazol-2-yl or 1,3,4-oxadiazyl-2-yl.

9. The compound of Claim 1, wherein:
R¹ is chloro or cyano;
R^{2'} is hydrogen or chloro;
R³ is fluoro or methoxy;
R⁴ is hydrogen or C₁₋₄alkyl;
Ring A is pyridyl, 2H-pyrazolyl, isoxazolyl, imidazolyl, pyrazinyl, thiazolyl, pyrimidinyl, 1,2,4-oxadiazolyl, benzothiazolyl, 1,2,4-triazolyl or 1,3,4-oxadiazolyl wherein said imidazolyl or 1,2,4-triazolyl may be optionally substituted on nitrogen by a group elected from R⁶; and wherein if said imidazolyl may form a quaternary compound on an -N= moiety with a methyl group;
R⁶ is methyl or ethyl; wherein R⁶ may be optionally substituted on carbon by a group selected from R²⁰;
R²⁰ is selected from methoxy or ethoxy;
R⁵ is a substituent on carbon and is selected from amino, C₁₋₄alkyl, C₁₋₄alkoxy, *N*-(C₁₋₄alkyl)amino or *N*,*N*-(C₁₋₄alkyl)₂amino; and
n is 0-2;
or a pharmaceutically acceptable salt thereof.

10. The compound of Claim 1, wherein:
R¹ is chloro or cyano;
R^{2'} is hydrogen or chloro;
R³ is fluoro or methoxy;
R⁴ is hydrogen, methyl or ethyl;
Ring A is 1-(2-methoxyethyl)imidazol-2-yl, 1-(2-trimethylsilylethoxymethyl)imidazol-2-yl, 1-(methoxymethyl)imidazol-2-yl, 1,2,4-oxadiazol-5-yl, 1,3,4-oxadiazol-2-yl, 1,3-dimcthylimidazol-2-yl, 1H-imidazol-2-yl, 1-methylimidazol-4-yl, 2H-pyrazol-3-yl, 2-methyl-1,2,4-triazol-3-yl, 2-pyridyl, benzothiazol-2-yl, isoxazol-5-yl, pyrazin-2-yl, pyrimidin-2-yl, pyrimidin-4-yl, thiazol-2-yl or thiazol-4-yl;
R⁵ is a substituent on carbon and is selected from amino, methyl, methoxy, methylamio or dimethylamino;
n is 0-2;
or a pharmaceutically acceptable salt thereof.

11. A compound selected from the group consisting of:
2-((3*S*,4*R*)-4-{[(3,4-Dichloro-5-methyl-1*H*-pyrrol-2-yl)carbonyl]amino}-3-methoxypiperidin-1-yl)-4-pyrimidin-2-yl-1,3-thiazole-5-carboxylic acid;
2-((3S,4R)-4-{[(3,4-Dichloro-5-methy-1H-pyrrol-2-yl)carbonyl]amino}-3-methoxypiperidin-1-yl)-4-pyrimidin-4-yl-1,3-thiazole-5-carboxylic acid;
2'-((3S,4R)-4-{[(3,4-Dichloro-5-mcthyl-1H-pyrrol-2-yl)carbonyl]amino}-3-methoxypiperidin-1-yl)-2,4'-bi-1,3-thiazole-5'-carboxylie acid;
2-((3S,4R)-4-{[(3,4-Dichloro-5-methyl-1H-pyrrol-2-yl)carbonyl]amino}-3-fluoropiperidin-1-yl)-4-pyrimidin-2-yl-1,3-thiazole-5-carrboxylic acid;
2-((3S,4R)-4-{[(3,4-Dichloro-5-methyl-1H-pyrrol-2-yl)carbonyl]amino}-3-fluoropiperidin-1-yl)-4-pyrimidin-4-yl-1,3-thiazole-5-carboxylic acid;
2'-((3S,4R)-4-{[(3,4-Dichloro-5-methyl-1H-pyrrol-2-yl)carbonyl]amino}-3-fluoropiperidin-1-yl)-2,4'-bi-1,3-thiazole-5'-carboxylic acid;
2-((3S,4R)-4-{[(3,4-Dichloro-5-methyl-1H-pyrrol-2-yl)carbonyl]amino}-3-methoxypiperidin-1-yl)-4-pyridin-2-yl-1,3-thiazole-5-carboxylic acid;
2-((3S,4R)-4-{[(3,4-Dichloro-5-methyl-1H-pyrrol-2-yl)carbonyl]amino}-3-fluoropiperidin-1-yl)-4-pyridin-2-yl-1,3-thiazole-5-carboxylic acid;
Bis(dimethylamino)pyrimidin-4-yl]-2-((3S,4R)-4-{[(3,4-dichloro-5-methyl-1H-pyrrol-2-yl)carbonyl]amino}-3-methoxypiperidin-1-yl)-1,3-thiazole-5-carboxylic acid;
Bis(dimethylamino)pyrimidin-4-yl]-2-((3S,4R)-4-{[(3,4-dichloro-5-methyl-1H-pyrrol-2-yl)carbonyl]amino}-3-fluoropiperidin-1-yl)-1,3-thiazole-5-carboxylic acid;
2-((3S,4R)-4-{[(3,A-Dichloro-5-maethyl-1H-pyrrol-2-yl)carbonyl]amino}-3-methoxypiperidin-1-yl)-4-(4,6-dimethoxypyrimidin-2-yl)-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-pyrazin-2-yl-1,3-thiazole-5-carboxylic acid;
4-(1,3-Benzothiazol-2-yl)-2-((3S,4R)-4-{[(3,4-dichloro-5-methyl-1H-pyrrol-2-yl)carbonyl]amino}-3-methoxypiperidin-1-yl)-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-pyrazin-2-yl-1,3-thiazole-s-carboxylic acid;
4-(1,3-Benzothiazol-2-yl)-2-((3S,4R)-4-{[(3,4-dichloro-5-methyl-1H-pyrrol-2-yl)carbonyl]amino}-3-fluoropiperidin-1-yl)-1,3-thiazolc-5-carboxylic acid;
2-((3S,4R)-4-{[(3,4-Dichloro-5-methyl-1H-pyrrol-2-yl)carbonyl]amino}-3-methoxypiperidin-1-yl)-4-(1-{[2-(trimethylsilyl)ethoxy]methyl}-1H-imidazol-2-yl)-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-[1-(methoxymethyl)-1H-imidazol-2-yl]-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-(1H-imidazol-2-yl)-1,3-thiazole-5-carboxylic acid;
2-((3S,4R)-4-{[(3,4-Dichloro-5-methyl-1H-pyrrol-2-yl)carbonyl]amino}-3-fluoropiperidin-1-yl)-4-[1-(methoxymethyl)-1H-imidazol-2-yl]-1,3-thiazole-5-carboxylic acid;
2-((3S,4R)-4-{[(3,4-Dichlozo-5-methyl-1H-pyrrol-2-yl)carbonyl]amino}-3-fluoropiperidin-1-yl)-4-(1H-imidazol-2-yl)-1,3-thiazole-5-carboxylic acid;
2-({3S,4R)-4-{[(3,4-Dichloro-5-methyl-1H-pyrrol-2-yl)carbonyl]amino}-3-methoxypiperidin-1-yl)-4-(1-methyl-1H-imidazol-2-yl)-1,3-thiazole-5-carboxylic acid;
2-[5-Carboxy-2-((3S,4R)-4-{[(3,4-dichloro-5-methyl-1H-pyrrol-2-yl)carbonyl]amino}-3-methoxypiperidin-1-yl)-1,3-thiazol-4-yl]-1,3-dimethyl-1H-imidazol-3-ium trifluoroacetate;
2-((3S,4R)-4-{[(3,4-Dichloro-5-methyl-1H-pyrrol-2-yl)carbonyl]amino}-3-methoxypiperidin-1-yl)-4-(1H-pyrazol-5-yl)-1,3-thiazole-5-carboxylic acid;
2-((3S,4R)-4-{[(3,4-Dichloro-5-methyl-1H-pyrrol-2-yl)carbonyl]amino}-3-fluoropiperidin-1-yl)-4-(1H-pyrazol-5-yl)-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylic acid;
2-((3*S*,4*R*)-4-{[(3,4-Dichloro-5-methyl-1*H*-pyrrol-2-yl)carbonyl]amino}-3-fluoropiperidin-1-yl)-4-[1-(2-methoxyethyl)-1*H*-imidazol-2-yl]-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-(1-methyl-1H-1,2,4-tnazol-5-yl)-1,3-thiazote-5-carboxylic acid;
2-((3S,4R)-4-{[(3,4-Dichloro-5-methyl-1H-pyrrol-2-yl)carbonyl]amino}-3-fluoropiperidin-1-yl)-4-(1-methyl-1H-1,2,4-triazol-5-yl)-1,3-thiazole-5-carboxylic acid;
2-((3S,4R)-4-{[(3-Chloro-4-cyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylic acid;
2-((3S,4R)-4-{[(3-Chloro-4-cyano-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylic acid;
2-((3S,4R)-4-{[(4-Cyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-4-(1,4,5-trimethyl-1H-imidazol-2-yl)-1,3-thiazole-5-carboxylic acid;
2'-Amino-2-((3S,4R)-4-{[(3,4-dichloro-5-methyl-1H-pyrrol-2-yl)carbonyl]amino}-3-methoxypiperidin-1-yl)-4,4'-bi-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-2'-(methylamino)-4,4'-bi-1,3-thiazole-5-carboxylic acid;
2-((3S,4R)-4-{[(3,4-Dichloro-5-methyl-1H-pyrrol-2-yl)carbonyl]amino}-3-methoxypiperidin-1-yl)-2'-(dimethylamino)-4,4'-bi-1,3-thiazole-5-caboxylic acid;
2'-Amino-2-((3S,4R)-4-{[(3,4-dichloro-5-methyl-1H-pyrrol-2-yl)carbonyl]amino}-3-fluoropiperidin-1-yl)-4,4'-bi-1,3-thiazole-5-carboxylic acid;
2-((3S,4R)-4-{[(3,4-Dichloro-5-methyl-1H-pyrrol-2-yl)carbonyl]amino}-3-fluoropiperidin-1-yl)-2'-(methylamino)-4,4'-bi-1,3-thiazole-5-carboxylic acid;
2-((3S,4R)-4-{[(3,4-Dichloro-5-methyl-1H-pyrrol-2-yl)carbonyl]amino}-3-fluoropiperidin-1-yl)-2'-(dimethylamino)-4,4'-bi-1,3-thiazole-5-carboxylic acid;
2-((3S,4R)-4-{[(3,4-Dichloro-5-methyl-1H-pyrrol-2-yl)carbonyl]amino}-3-fluoropiperidin-1-yl)-4-(1-methyl-1H-imidazol-2-yl)-1,3-thiazole-5-carboxylic acid;
2-((3S,4R)-4-{[(3,4-Dichloro-5-methyl-1H-pyrrol-2-yl)carbanyl]amino}-3-methoxypiperidin-1-yl)-4-(1-methyl-1H-imidazol-4-yl)-1,3-thiazole-5-carboxylic acid;
2-((3S,4R)-4-{[(3,4-Dichloro-5-methyl-1H-pyrrol-2-yl)carbonyl]amino}-3-fluoropipcridin-1-yl)-4-(1-methyl-1H-imidazol-4-yl)-1,3-thiazole-5-carboxylic acid;
2-((3*S*,4*R*)-4-{[(3,4-Dichloro-5-methyl-1*H*-pyrrol-2-yl)carbonyl]amino}-3-methoxypiperidin-1-yl)-4-(4-methoxypyrimidin-2-yl)-1,3-thiazole-5-carboxylic acid;
Ethyl 2-((3*S*,4*R*)-4-{[(3,4-dichloro-5-methyl-1H-pyrrol-2-yl)carbonyl]amino}-3-methoxypiperdin-1-yl)-4-pyrimidin-2-yl-1,3-thiazole-5-carboxylate;
Methyl 2'-((3S,4R)-4-{[(3,4-dichloro-5-methyl-1H-pyrrol-2-yl)carbonyl]amino}-3-methoxypiperidin-1-yl)-2,4'-bi-1,3-thiazole-5'-carboxylate;
Ethyl 2-((3S,4R)-4-{[(3,4-dichlaro-5-mcthyl-1H-pyrrol-2-yl)carbonyl]amino}-3-fluoropiperidin-1-yl)-4-pyrimidin-2-yl-1,3-thiuzole-5-carboxylate;
Ethyl 2-((3S,4R)-4-{[(3,4-dichloro-5-mathyl-1H-pyrrol-2-yl)carbonyl]amino}-3-fluoropiperidin-1-yl)-4-pyrimidin-4-yl-1,3-thiazole-5-carboxylate;
Methyl 2'-((3S,4R)-4-{[(3,4-dichloro-5-methyl-1H-pyrrol-2-yl)carbonyl]amino}-3-fluoropiperidin-1-yl)-2,4'-bi-1,3-thiazole-5'-carboxylate;
Ethyl 2-((3S,4R)-4-{[(3,4-dichloro-5-methyl-1H-pyrrol-2-yl)carbonyl]amino}-3-methoxypiperidin-1-yl)-4-pyridin-2-yl-1,3-thiazole-5-carboxylate;
Ethyl 2-((3S,4R)-4-{[(3,4-dichloro-5-methyl-1H-pyrrol-2-yl)carbonyl]amino}-3-fluoropiperidin-1-yl)-4-pyridin-2-yl-1,3-thiazole-5-carboxylate;
Ethyl 4-[2,6-his(dimethylamino)pyrimidin-4-yl]-2-((3S,4R)-4-{[(3,4-dichloro-5-methyl-1H-pyrrol-2-yl)carbonyl]amino}-3-methoxypiperidin-1-yl)-1,3-thiazole-5-carboxylate;
Ethyl 4-[2,6-bis(dimethylamino)pyrimidin-4-yl]-2-((3S,4R)-4-{[(3,4-dichloro-5-methyl-1H-pyrrol-2-yl)carbonyl]amino}-3-fluoropiperidin-1-yl)-1,3-thiazole-5-carboxylate;
Ethyl 2-((3S,4R)-4-{[(3,4-dichloro-5-methyl-1H-pyrrol-2-yl)carbonyl]amino}-3-methoxypiperidin-1-yl)-4-(4,6-dimethoxypyrimidin-2-yl)-1,3-thiazole-5-carboxylate;
Ethyl 2-((3S,4R)-4-{[(3,4-dichloro-5-methyl-1H-pyrrol-2-yl)carbonyl]amino}-3-methoxypiperidin-1-yl)-4-pyrazin-2-yl-1,3-thiazole-5-carboxylate;
Ethyl 4-(1,3-benzothiazol-2-yl)-2-((3S,4R)-4-{[(3,4-dichloro-5-methyl-1H-pyrrol-2-yl)carbonyl]amino}-3-methoxypiperidin-1-yl)-1,3-thiazole-5-carboxylate;
Ethyl 2-((3S,4R)-4-{[(3,4-dichloro-5-methyl-1H-pyrrol-2-yl)carbonyl]amino}-3-fluoropiperidin-1-yl)-4-pyrazin-2-yl-1,3-thiazole-5-carboxylate;
Ethyl 4-(1,3-benzothiazol-2-yl)-2-((3S,4R)-4-{[(3,4-dichloro-5-methyl-1H-pyrrol-2-yl)carbonyl]amino}-3-fluoropiperidin-1-yl)-1,3-thiazole-5-carboxylate;
Methyl 2-((3S,4R)-4-{[(3,4-dichloro-5-methyl-1H-pyrrol-2-yl)carbonyl]amino}-3-methoxypipcridin-1-yl)-4-(1-{[2-(trimethylsilyl)ethoxy]methyl}-1H-imidatol-2-yl)-1,3-thiazole-5-carboxylate;
Methyl 2-((3S,4R)-4-{[(3,4-dichloro-5-methyl-1H-pyrral-2-yl)carbonyl]amino}-3-methoxypiperidin-1-yl)-4-[1-(methoxymethyl)-1H-imidazol-2-yl]-1,3-thiazole-5-carboxylate;
Methyl 2-((3S,4R)-4-{[(3,4-dichloro-5-methyl-1H-pyrrol-2-yl)carbonyl]amino}-3-fluoropiperidin-1-yl)-4-[1-(methoxymethyl)-1H-imidazol-2-yl]-1,3-thiazole-5-carboxylate;
Methyl 2-((3S,4R)-4-{[(3,4-dichloro-5-methyl-1H-pyrrol-2-yl)carbonyl]amino}-3-methoxypiperidin-1-yl)-4-isoxazol-5-yl-1,3-thiazole-5-carboxylate;
Methyl 2-((3S,4R)-4-{[(3,4-dichlaro-5-methyl-1H-pyrrol-2-yl)carbonyl]amino}-3-fluoropiperidin-1-yl)-4-isoxazol-5-yl-1,3-thiazole-5-carboxylate;
Methyl 2-((3S,4R)-4-{[(3,4-dichloro-5-methyl-1H-pyrrol-2-yl)carbonyl]amino}-3-methoxypiperidin-1-yl)-4-(1H-pyrazol-5-yl)-1,3-thiazole-5-carboxylate;
Methyl 2-((3S,4R)-4-{[(3,4-dichloro-5-methyl-1H-pyrrol-2-yl)carbonyl]amino}-3-fluoropiperidin-1-yl)-4-(1H-pyrazol-5-yl)-1,3-thiazole-5-carboxylate;
Methyl 2-((3S,4R)-4-{[(3,4-dichloro-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylate;
Methyl 2-((3S,4R)-4-{[(3,4-dichloro-5-methyl-1H-pyrrol-2-yl)carbonyl]amino}-3-fluoropiperidin-1-yl)-4-[1-(2-methoxyethyl)-1H-imidazol-2-yl]-1,3-thiazole-s-carboxylate;
Methyl 2-((3S,4R)-4-{[(3,4-dichloro-5-methyl-1H-pyrrol-2-yl)carbonyl]amino}-3-methoxypiperidin-1-yl)-4-(1-methyl-1H-1,2,4-triazol-5-yl)-1,3-thiazolc-5-carboxylate;
Methyl 2-((3S,4R)-4-{[(3,4-dichlora-5-methyl-1H-pyrrol-2-yl)carbonyl]amino}-3-fluoropiperidin-1-yl)-4-(1-methyl-1H-1,2,4-triazol-5-yl)-1,3-thiazole-5-carboxylate;
Methyl 2-((3S,4R)-4-{[(4-cyano-5-methyl-1H-pyrrol-2-yl)carbonyl]amino}-3-methoxypiperidin-1-yl)-4-(1-methyl-1H-1,2,4-triazol-5-yl)-1,3-thiazole-5-carboxylate;
Methyl 2-((3S,4R)-4-{[(3-chloro-4-cyano-5-methyl-1H-pyrrol-2-yl)carbonyl]amino}-3-methoxypiperidin-1-yl)-4-[1-(2-methoxyethyl)-1H-imidazol-2-yl]-1,3-thiazole-5-carboxylate;
Methyl 2-((3S,4R)-4-{[(3,4-dichloro-5-methyl-1H-pyrrol-2-yl)carbonyl]amino}-3-methoxypiperidin-1-yl)-4-(1,4,5-trimethyl-1H-imidazol-2-yl)-1,3-thiazole-5-carboxylate;
Methyl 2'-amino-2-((3S,4R)-4-{[(3,4-dichloro-5-methyl-1H-pyrrol-2-yl)carbonyl]amino}-3-methoxypiperidin-1-yl)-4,4'-bi-1,3-thiazole-5-carboxylate;
Methyl 2-((3S,4R)-4-{[(3,4-dxchloro-5-methyl-1H-pyrrol-2-yl)carbonyl]amino}-3-methoxypiperidin-1-yl)-2'-(methylamino)-4,4'-bi-1,3-thiazole-5-carboxylate;
Methyl 2-((3S,4R)-4-{[(3,4-dichloro-5-methyl-1H-pyrrol-2-yl)carbonyl]amino}-3-methoxypiperidin-1-yl)-2'-(dimethylamino)-4,4'-bi-1,3-thiazole-5-carboxylate;
Methyl 2'-amino-2-((3S,4R)-4-{[(3,4-dichloro-5-methyl-1H-pyrrol-2-yl)carboztyl]amino}-3-fluoropiperidin-1-yl)-4,4'-bi-1,3-thiazole-5-carboxylate;
Methyl 2-((3S,4R)-4-{[(3,4-dichloro-5-methyl-1H-pyrrol-2-yl)carbonyl]amino}-3-fluoropiperidin-1-yl)-2'-(methylamino)-4,4'-bi-1,3-thiazole-5-carboxylate;
Methyl 2-((3S,4R)-4-{[(3,4-dichloro-5-methyl-1H-pyrrol-2-yl)carbonyl]amino}-3-fluoropiperidin-1-yl)-2'-(dimethylamino)-4,4'-bi-1,3-thiazole-5-carboxylate;
Ethyl 2-((3S,4R)-4-{[(3,4-dichloro-5-methyl-1H-pyrrol-2-yl)carbonyl]amino}-3-methoxypiperidin-1-yl)-4-(1-methyl-1H-imidazol-4-yl)-1,3-thiazole-5-carboxylate;
Ethyl 2-((3S,4R)-4-{[(3,4-dichloro-5-methyl-1H-pyrrol-2-yl)carbonyl]amino}-3-fluoropiperidin-1-yl)-4-(1-methyl-1H-imidazol-4-yl)-1,3-thiazole-5-carboxylate;
Methyl 2-((3S,4R)-4-{[(3,4-dichloro-5-methyl-1H-pyrrol-2-yl)carbonyl]amino}-3-fluoropiperidin-1-yl)-4-(1-methyl-1H-imidazol-2-yl)-1,3-thiazole-5-carboxylate;
Methyl 2-((3S,4R)-4-{[(3-chloro-4-cyano-5-methyl-1H-pyrrol-2-yl)carbonyl]amino}-3-methoxypiperidin-1-yl)-4-(1-methyl-1H-1,2,4-triaool-5-yl)-1,3-thiazole-5-carboxylate;
Ethyl 2-((3S,4R)-4-{[(3,4-dichloro-5-methyl-1H-pyrrol-2-yl)carbonyl]amino}-3-methoxypiperidin-1-yl)-4-pyrimidin-4-yl-1,3-thiazole-5-carboxylate;
Methyl 2-((3*S*,4*R*)-4-{[(3,4-dichloro-5-methyl-1*H*-pyrrol-2-yl)carbonyl]amino}-3-methoxypiperidin-1-yl)-4-(4-methoxypylimidin-2-yl)-1,3-thiazole-5-carboxylate;
Ethyl 2-((3*S*,4*R*)-4-{[(3,4-dichloro-5-methyl-1*H*-pyrrol-2-yl)carbonyl]amino}-3-methoxypiperidin-1-yl)-4-(1,3,4-oxadiazol-2-yl)-1,3-thiazole-5-carboxylate;
Ethyl 2-((3*S*,4*R*)-4-{[(3,4-dichloro-5-methyl-1*H*-pyrrol-2-yl)carbonyl]amino}-3-methoxypiperidin-1-yl)-4-(3-methyl-1,2,4-oxadiazol-5-yl)-1,3-thiazole-5-carboxylate;
Ethyl 2-((3*S*,4*R*)-4-{[(3,4-dichloro-5-methyl-1*H*-pyrrol-2-yl)carbonyl]amino}-3-methoxypiperidin-1-yl)-4-(5-methyl-1,3,4-oxadiazol-2-yl)-1,3-thiazole-5-carboxylate;
Methyl 2-((3*S*,4*R*)-4-{[(3,4-dichloro-5-methyl-1*H*-pyrrol-2-yl)carbonyl]amino}-3-methoxypiperidin-1-yl)-4-(1*H*-imidazol-2-yl)-1,3-thiazole-5-carboxylate;
Methyl 2-((3*S*,4*R*)-4-{[(3,4-dichloro-5-methyl-1*H*-pyrrol-2-yl)carbonyl]amino}-3-fluoropiperidin-1-yl)-4-(1*H*-imidazol-2-yl)-1,3-thiazole-5-carboxylate;
Methyl 2-((3*S*,4*R*)-4-{[(3,4-dichloro-5-methyl-1*H*-pyrrol-2-yl)carbonyl]amino}-3-methoxypiperidin-1-yl)-4-(1-methyl-1*H*-imidazol-2-yl)-1,3-thiazole-5-carboxylate;
2-[2-((3*S*,4*R*)-4-{[(3,4-Dichloro-5-methyl-1*H*-pyrrol-2-yl)carbonyl]amino}-3-methoxypiperidin-1-yl)-5-(methoxycarbonyl)-1,3-thiazol-4-yl]-1,3-dimethyl-1*H*-imidazol-3-ium iodide;
2-((3*S*,4*R*)-4-{[(3,4-Dichloro-5-methyl-1*H-*pyrrol-2-yl)carbonyl]amino}-3-methoxypiperidin-1-yl)-4-(5-methyl-1,3,4-oxadiazol-2-yl)-1,3-thiazole-5-carboxylic acid;
2-((3*S*,4*R*)-4-{[(3,4-Dichloro-5-methyl-1*H*-pyrrol-2-yl)carbonyl]amino}-3-methoxypiperidin-1-yl)-4-(5-methyl-1,3,4-oxadiazol-2-yl)-1,3-thiazole-5-carboxylic acid;
Methyl 2-((3S,4R)-4-(3,4-dichloro-5-methyl-1H-pyrrole-2-carboxamido)-3-methoxypiperidin-1-yl)-4-(1-methyl-1H-1,2,3-triazol-4-yl)thiazole-5-carboxylate;
Methyl 2-((3S,4R)-4-(3,4-dichloro-5-methyl-1H-pyrrole-2-carboxamido)-3-methoxypiperidin-1-yl)-4-(3,5-dimethylpyrazin-2-yl)thiazole-5-carboxylate;
Methyl 2-((3S,4R)-4-(3,4-dichloro-5-methyl-1H-pyrrole-2-carboxamido)-3-methoxypiperidin-1-yl)-4-(6-methoxypyridin-2-yl)thiazole-5-carboxylate;
Ethyl 2-((3S,4R)-4-(3,4-dichloro-5-methyl-1H-pyrrole-2-carboxamido)-3-methoxypiperidin-1-yl)-4-(4-methoxypyridin-2-yl)thiazole-5-carboxylate;
Ethyl 2-((3S,4R)-4-(3,4-dichloro-5-methyl-1H-pyrrole-2-carboxamido)-3-methoxypiperidin-1-yl)-4-(5-methylpyrazin-2-yl)thiazole-5-carboxylate;
Ethyl 2-((3S,4R)-4-(3,4-dichloro-5-methyl-1H-pyrrole-2-carboxamido)-3-methoxypipeidin-1-yl)-4-(3-fluoropyridin-2-yl)thiazole-5-carboxylate;
Ethyl 2-((3S,4R)-4-(3,4-dichloro-5-methyl-1H-pyrrole-2-carboxamido)-3-fluoropiperidin-1-yl)-4-(4-methoxypyridin-2-yl)thiazole-5-carboxylate;
Methyl 2-((3S,4R)-4-(3,4-dichloro-5-methyl-1H-pyrrole-2-carboxamido)-3-fluoropiperidin-1-yl)-4-(1-methyl-1H-1,2,3-triazol-4-yl)thiazole-5-carboxylate;
Methyl 2-((3S,4R)-4-(3,4-dichloro-5-mcthyl-1H-pyrrole-2-carboxamido)-3-fluoropiperidin-1-yl)-4-(6-methoxypyridin-2-yl)thiazole-5-carboxylate;
Methyl 2-((3S,4R)-4-(3,4-dichloro-5-methyl-1H-pyrrole-2-carboxamido)-3-fluoropiperidin-1-yl)-4-(6-methoxypyridin-2-yl)thiazole-5-carboxylate;
Ethyl 2-((3S,4R)-4-(3,4-dichloro-5-methyl-1H-pyrrole-2-carboxamido)-3-fluoropiperidin-1-yl)-4-(3-fluoropyridin-2-yl)thiazole-5-carboxylate;
Ethyl 2-((3S,4R)-4-(3,4-dichloro-5-methyl-1-H-pyrrolc-2-carboxamido)-3-fluoropiperidin-1-yl)-4-(4-methoxypyridin-2-yl)thiazole-5-carboxylate;
Methyl 2-((3S,4R)-4-(4-chloro-3-cyano-5-methyl-1H-pyrrole-2-carboxarraido)-3-methoxypiperidin-1-yl)-4-(1-methyl-1H-1,2,3-triazol-4-yl)thiazole-5-carboxylate;
Methyl 2-((3S,4R)-4-(4-chloro-3-cyano-5-methyl-1H-pyrrole-2-carboxamido)-3-methoxypiperidin-1-yl)-4-(1-methyl-1H-1,2,4-triazol-5-yl)thiazole-5-carboxylate;
Ethyl 2-((3S,4R)-4-(4-chloro-3-cyano-5-methyl-1H-pyrrole-2-carboxamido)-3-methoxypiperidin-1-yl)-4-(pyrimidin-2-yl)thiazole-5-carboxylate;
Ethyl 2-((3S,4R)-4-(4-chloro-3-cyano-5-methyl-1H-pyrrole-2-carboxamido)-3-methoxypiperidin-1-yl)-4-(3-fluoropyridin-2-yl)thiazole-5-carboxylate;
Ethyl 2-((3S,4R)-4-(4-chloro-3-cyano-5-methyl-1H-pyrrole-2-carboxamido)-3-methoxypiperidin-1-yl)-4-(5-methylpyrazin-2-yl)thiazole-5-carboxylate;
Ethyl 2-((3S,4R)-4-(4-chloro-3-cyano-5-methyl-1H-pyrrole-2-carboxamido)-3-methoxypiperidin-1-yl)-4-(1-mtethyl-1H-imidazol-4-yl)thiazole-5-carboxylate;
2-((3S,4R)-4-(3,4-Dichloro-5-methyl-1H-pyrrole-2-carboxamido)-3-methoxypiperidin-1-yl)-4-(1-methyl-1H-1,2,3-triazol-4-yl)thiazole-5-carboxylic acid;
2-((3S,4R)-4-(3,4-Dichloro-5-methyl-1H-pyrrole-2-carboxamido)-3-methoxypiperidin-1-yl)-4-(3,5-dimethylpyrazin-2-yl)thiazole-5-carboxylic acid;
2-((3S,4R)-4-(3,4-Dichloro-5-methyl-1H-pyrrole-2-carboxamido)-3-methoxypiperidin-1-yl)-4-(6-methoxypyridin-2-yl)thiazolc-5-carboxylic acid;
2-((3S,4R)-4-(3,4-Dichloro-5-methyl-1H-pyrrole-2-carboxamido)-3-methoxypiperidin-1-yl)-4-(4-methoxypyridin-2-yl)thiazole-5-carboxylic acid;
2-((3S,4R)-4-(3,4-Dichloro-5-methyl-1H-pyrrolc-2-carboxamido)-3-methoxypiperidin-1-yl)-4-(5-methylpyrazin-2-yl)thiazole-5-carboxylic acid;
2-((3S,4R)-4-(3,4-Dichloro-5-methyl-1H-pyrrole-2-carboxamido)-3-methoxypiperidin-1-yl)-4-(3-fluoropyridin-2-yl)thiazole-5-carboxylic acid;
2-((3S,4R)-4-(3,4-Dichloro-5-methyl-1H-pyrrole-2-carboxamido)-3-fluoropiperidin-1-yl)-4-(3-fluorvpyridin-2-yl)thiazole-5-carbyxylic acid;
2-((3S,4R)-4-(3,4-Diehloro-5-methyl-1H-pyrrole-2-carboxamido)-3-fluoropiperidin-1-yl)-4-(1-methyl-1H-1,2,3-triazol-4-yl)thiazole-5-carboxylic acid;
2-((3S,4R)-4-(3,4-Dichloro-5-methyl-1H-pyrrole-2-carboxamido)-3-fluoropiperidin-1-yl)-4-(6-methoxypyridin-2-yl)thiazole-5-carboxylic acid;
2-((3S,4R)-4-(3,4-Dichloro-5-methyl-1H-pyrrole-2-carboxamido)-3-fluoropiperidin-1-yl)-4-(5-methylpyrazin-2-yl)thiazole-5-carboxylic acid;
2-((3S,4R)4-(3,4-Dichloro-5-methyl-1H-pyrrole-2-carboxamido)-3-fluoropiperidin-1-yl)-4-(3-fluoropyridin-2-yl)thiazole-5-carboxylic acid;
2-((3S,4R)-4-(3,4-Diehloro-5-methyl-1H-pyrrolc-2-carboxamido)-3-fluoropiperidin-1-yl)-4-(4-methoxypyridin-2-yl)thiazole-5-carboxylic acid;
2-((3S,4R)-4-(4-Chloro-3-cyano-5-methyl-1H-pyrrole-2-carboxamido)-3-methoxypiperidin-1-yl)-4-(1-methyl-1H-1,2,3-thiazol-4-yl)thiazole-5-carboxylic acid;
2-((3S,4R)-4-(4-Chloro-3-cyano-5-methyl-1H-pyrrole-2-carboxamido)-3-methoxypiperidin-1-yl)-4-(1-methyl-1H-1,2,4-triazol-5-yl)thiazole-5-carboxylic acid;
2-((3S,4R)-4-(4-Chloro-3-cyano-5-methyl-1H-pyrrole-2-carboxamido)-3-methoxypiperidin-1-yl)-4-(pyrimidin-2-yl)thiazole-5-carboxylic acid;
2-((3S,4R)-4-(4-Chloro-3-cyano-5-methyl-1H-pyrrole-2-carboxamido)-3-methoxypiperidin-1-yl)-4-(3-fluoropyridin-2-yl)thiazole-5-carboxylic acid;
2-((3S,4R)-4-(4-Chloro-3-cyano-5-methyl-1H-pyrrole-2-carboxamido)-3-methoxypiperidin-1-yl)-4-(5-methylpyrazin-2-yl)thiazole-5-carboxylic acid;
2-((3S,4R)-4-(4-Chloro-3-cyano-5-methyl-1H-pyrrole-2-carboxamido)-3-methoxypiperidin-l-yl)-4-(1-methyl-1H-imidazol-4-yl)thiazole-5-carboxylic acid;
2-{(3S, 4R)-4-[(4-Chloro-3-cyano-5-methyl-1H-pyrrole-2-carbonyl)-amino]-3-fluoro-piperidin-1-yl}-4-pyrazin-2-yl-thiazole-5-carboxylic acid ethyl ester;
2-{(3S, 4R)-4-[(4-Chloro-3,5-dimethyl-1H-pyrrole-2-carbonyl)-amino]-3-fluoro-piperidin-l-yl}-4-pyrazin-2-yl-thiazole-5-carboxylic acid ethyl ester;
2-{(3S, 4R)-4-[(4-chloro-3,5-dimethyl-1H-pyrrole-2carbonyl)-amino]-3-fluoro-piperidin-1-yl}-4-pyrimidin-4-yl-thiazole-5-carboxylic acid ethyl ester;
2-{(3S, 4R)-4-[(4-Chloro-3-cyano-5-methyl-1H-pyrrole-2-carbonyl)-amino]-3-methoxy-piperidin-1-yl}-4-pyrimidin-4-yl-thiazole-5-carboxylic acid ethyl ester;
2-{(3S, 4R)-4-[(4-Chloro-3-cyano-5-methyl-1H-pyrrole-2-carbonyl)amino]-3-methoxy-piperidin-1-yl}-4-(1-methyl-1H-pyrazol-3-yl)-thiazole-5-carboxylic acid ethyl ester;
2-{(3S, 4R)-4-[(3,4-Dichloro-5-methyl-1H-pyrrole-2-carbonyl)-amino]-3-methoxy-piperidin-1-yl}-4-(1-methyl-1H-pyrazol-3-yl)-thiazole-5-carboxylic acid ethyl ester;
2-{(3S, 4R)-4-[(4-Chloro-3-cyano-5-methyl-1H-pyrrole-2-carbonyl)-amino]-3-fluoro-piperidin-1-yl}-4-pyrazin-2-yl-thiazole-5-carboxylic acid;
2-[(3*S*,4*R*)-4-{[(4-chloro-3,5-dimethyl-1*H*-pyrrol-2-yl)carbonyl]amino}-3-fluoropiperidin-1-yl]-4-pyrazin-2-yl-1,3-thiazole-5-carboxylic acid;
2-[(3*S*,4*R*)-4-{[(4-chloro-3,5-dimethyl-1*H*-pyrrol-2-yl)carbonyl]amino}-3-fluoropiperidin-1-yl]-4-pyrimidin-4-yl-1,3-thiazole-5-carboxylic acid;
2-[(3*S*,4*R*)-4-{[(4-chioro-3-cyano-5-methyl-1H-pyrrol-2-yl)carbonyl]amino}-3-methoxypiperidin-1-yl]-4-pyrimidin-4-yl-1,3-thiazole-5-carboxylic acid;
2-[(3*S*,4*R*)-4-{[(4-chloro-3-cyano-5-methyl-1*H*-pyrrol-2-yl)carbonyl]amino}-3-methoxypiperidin-1-yl]-4-(1-methyl-1H-pyrazol-3-yl)-1,3-thiazole-5-carboxylic acid;
2-[(3*S*,4*R*)-4-{[(3,4-dichloro-5-methyl-1*H*-pyrrol-2-yl)carbonyl]amino}-3-methoxypiperidin-1-yl]-4-(1-methyl-1*H*-pyrazol-3-yl)-1,3-thiazole-5-carboxylic acid;
or a pharmaceutically acceptable salt thereof.

12. A compound of any one of Claims 1-11, or a pharmaceutically acceptable salt thereof, for use in production of an anti-bacterial effect in a warm-blooded animal.

13. A compound of any one of Claims 1-11, or a pharmaceutically acceptable salt thereof, for use in inhibition of bacterial DNA gyrase and/or topoisomerase IV in a warm-blooded animal.

14. A compound of any one of Claims 1-11, or a pharmaceutically acceptable salt thereof, for use in the treatment of a bacterial infection in a warm-blooded animal.

15. A compound of any one of Claims 1-11, or a pharmaceutically acceptable salt thereof, for use in the treatment of community-acquired pneumoniae, hospital-acquired pneumoniae, skin and skin structure infections, acute exacerbation of chronic bronchitis, acute sinusitis, acute otitis media, catheter-related sepsis, febrile neutropenia, osteomyelitis, endocarditis, urinary tract infections, Penicillin-resistant Streptococcus pneumoniae, methicillin-resistant Staphylococcus aureus, methicillin-resistant Staphylococcus epidermidis or Vancomycin-Resistant Enterococci.

## Patentansprüche

1. Verbindung der Formel (Ia): 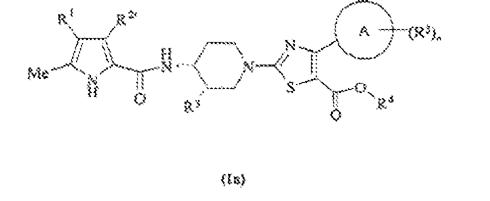 worin
**R¹** für Chlor oder Cyano steht;
**R^{2'}** für Wasserstoff, Chlor, Cyano oder Methyl steht;
**R³** für Fluor, Methyl, Methoxy, Ethoxy, Propoxy, Allyloxy und Benzyloxy steht;
**R⁴** für Wasserstoff oder C₁₋₄-Alkyl steht;
**Ring A** für Carbocyclyl oder Heterocyclyl steht; wobei dann, wenn das Heterocyclyl eine -NH-Gruppierung enthält, dieser Stickstoff gegebenenfalls durch eine unter R6 ausgewählte Gruppe substituiert sein kann; und wobei dann, wenn das Heterocyclyl eine -N=-Gruppierung enthält, dieser Stickstoff mit einer Methylgruppe eine quartäre Verbindung bilden kann;
**R⁵** für einen Substituenten an Kohlenstoff steht und unter Halogen, Nitro, Cyano, Hydroxy, Amino, Carboxy, Carbamoyl, Mercapto, Sulfamoyl, Sulfo, Formyl, Ureido, Hydroxyiminomethyl, *N*-Hydroxyformamido, Hydrazinocarbonyl, *N*-Hydroxyethanimidoyl, Amino(hydroxyimino)methyl, C₁₋₄-Alkyl, C₂₋₄-Alkenyl, C₂₋₄-Alkinyl, C₁₋₄-Alkoxy, C₁₋₄-Alkanoyl, C₁₋₄-Alkanoyloxy, *N-*(C₁₋₄-Alkyl)amino, *N,N-*(C₁₋₄-Alkyl)₂amino, C₁₋₄-Alkanoylamino, *N*-(C₁₋₄-Alkyl) carbamoyl, *N,N-*(C₁₋₄-Alkyl)₂carbamoyl, *N*-(C₁₋₄-Alkoxy)carbamoyl, *N*'-(C₁₋₄-Alkyl)ureido, *N*',*N*'-(C₁₋₄-Alkyl)₂ureido, *N*-(C₁₋₄-Alkyl)-*N-*(C₁₋₄-alkoxy)carbamoyl, C₁₋₄-Alkyl-S(O)ₐ, worin a für 0 bis 2 steht, C₁₋₄-Alkoxycarbonyl, C₁₋₄-Alkyoxycarbonylamino, *N*-(C₁₋₄-Alkyl) sulfamoyl, *N*,*N*-(C₁₋₄-Alkyl)₂sulfamoyl, C₁₋₄-Alkylsulfonylamino, C₁₋₄-Alkylsulfonylaminocarbonyl, *N*'-(C₁₋₄-Alkyl)hydrazinocarbonyl, *N',N*'-(C₁₋₄-Alkyl)₂hydrazinocarbonyl, Carbocyclyl-R⁷- oder Heterocyclyl-R⁸- ausgewählt ist; wobei R⁵ gegebenenfalls an Kohlenstoff durch eine oder mehrere Gruppen R⁹ substituiert sein kann und wobei dann, wenn das Heterocyclyl eine -NH-Gruppierung enthält, dieser Stickstoff gegebenenfalls durch eine unter R¹⁰ ausgewählte Gruppe substituiert sein kann;
**n** für 0, 1, 2 oder 3 steht;
**R⁹** unter Halogen, Nitro, Cyano, Hydroxy, Amino, Carboxy, Carbamoyl, Mercapto, Sulfamoyl, C₁₋₄-Alkyl, C₂₋₄ -Alkenyl, C₂₋₄-Alkinyl, C₁₋₄-Alkoxy, C₁₋₄-Alkanoyl, C₁₋₄-Alkanoyloxy, *N*-(C₁₋₄-Alkyl) amino, *N*,*N*-(C₁₋₄-Alkyl)₂amino, C₁₋₄-Alkanoylamino, *N*-(C₁₋₄-Alkyl) carbamoyl, *N*,*N*-(C₁₋₄-Alkyl)₂carbamoyl, C₁₋₄-Alkyl-S(O)ₐ, worin a für 0 bis 2 steht, C₁₋₄-Alkoxycarbonyl, *N*-(C₁₋₄-Alkyl)sulfamoyl, *N*,*N-*(C₁₋₄-Alkyl)₂sulfamoyl, C₁₋₄-Alkylsulfonylamino, C₁₋₄-Alkoxycarbonylamino, Carbocyclyl-R¹¹- oder Heterocyclyl-R¹²- ausgewählt ist; wobei R⁹ gegebenenfalls an Kohlenstoff durch eine oder mehrere Gruppen R¹³ substituiert sein kann und wobei dann, wenn das Heterocyclyl eine -NH-Gruppierung enthält, dieser Stickstoff gegebenenfalls durch eine unter R¹⁴ ausgewählte Gruppe substituiert sein kann;
**R⁶, R¹⁰** und **R¹⁴** unabhängig voneinander unter C₁₋₄-Alkyl, C₁₋₄-Alkanoyl, C₁₋₄-Alkylsulfonyl, C₁₋₄-Alkoxycarbonyl, Carbamoyl, *N*-(C₁₋₄-Alkyl)carbamoyl, *N*,*N-*(C₁₋₄-Alkyl)carbamoyl, Benzyl, Benzyloxycarbonyl, Benzoyl und Phenylsulfonyl ausgewählt sind; wobei R⁶, R¹⁰ und R¹⁴ gegebenenfalls an Kohlenstoff durch eine unter R²⁰ ausgewählte Gruppe substituiert sein können;
**R⁷, R⁸, R¹¹** und **R¹²** unabhängig voneinander unter einer direkten Bindung, -O-, -N(R¹⁵)-, -C(O)-, -N(R¹⁶)C(O)-, -C(O)N(R¹⁷)-, -S(O)ₚ-, -SO₂N(R¹⁸)- oder -N(R¹⁹)SO₂- ausgewählt sind; wobei R¹⁵, R¹⁶, R¹⁷, R¹⁸ und R¹⁹ unabhängig voneinander unter Wasserstoff oder C₁₋₄-Alkyl ausgewählt sind und p für 0-2 steht;
**R¹³** und **R²⁰** unabhängig voneinander unter Halogen, Nitro, Cyano, Hydroxy, Trifluormethoxy, Trifluormethyl, Amino, Carboxy, Carbamoyl, Mercapto, Sulfamoyl, Methyl, Ethyl, Ethenyl, Ethinyl, Methoxy, Ethoxy, 2-Trimethylsilylethoxy, Acetyl, Acetoxy, Methylamino, Ethylamino, Dimethylamino, Diethylamino, *N*-Methyl-*N-*ethylamino, Acetylamino, *N*-Methylcarbamoyl, *N-*Ethylcarbamoyl, *N,N*-Dimethylcarbamoyl, *N,N-*Diethylcarbamoyl, *N*-Methyl-*N*-ethylcarbamoyl, Methylthio, Ethylthio, Methylsulfinyl, Ethylsulfinyl, Mesyl, Ethylsulfonyl, Methoxycarbonyl, Ethoxycarbonyl, *N*-Methylsulfamoyl, *N*-Ethylsulfamoyl, *N,N-*Dimethylsulfamoyl, *N,N*-Diethylsulfamoyl oder *N*-Methyl-*N*-ethylsulfamoyl ausgewählt sind;
oder ein pharmazeutisch annehmbares Salz davon.

2. Verbindung nach Anspruch 1, worin Ring A für Heterocyclyl steht; wobei dann, wenn das Heterocyclyl eine -NH-Gruppierung enthält, dieser Stickstoff gegebenenfalls durch eine unter R⁶ ausgewählte Gruppe substituiert sein kann; und wobei dann, wenn das Heterocyclyl eine -N=-Gruppierung enthält, dieser Stickstoff mit einer Methylgruppe eine quartäre Verbindung bilden kann; wobei:
R⁶ für C₁₋₄-Alkyl steht; wobei R⁶ gegebenenfalls an Kohlenstoff durch eine unter R²⁰ ausgewählte Gruppe substituiert sein kann; und
R²⁰ unter Methoxy oder Ethoxy ausgewählt ist.

3. Verbindung nach Anspruch 2, wobei Ring A für Pyridyl, 2H-Pyrazolyl, Isoxazolyl, Imidazolyl, Pyrazinyl, Thiazolyl, Pyrimidinyl, 1,2,4-Oxadiazolyl, Benzothiazolyl, 1,2,4-Triazolyl oder 1,3,4-Oxadiazolyl steht, wobei das Imidazolyl oder 1,2,4-Triazolyl gegebenenfalls an Stickstoff durch eine unter R⁶ ausgewählte Gruppe substituiert sein kann; und wobei das Imidazolyl an einer -N=-Gruppierung mit einer Methylgruppe eine quartäre Verbindung bilden kann; wobei
R⁶ für Methyl oder Ethyl steht; wobei R⁶ gegebenenfalls an Kohlenstoff durch eine unter R²⁰ ausgewählte Gruppe substituiert sein kann; und
R²⁰ unter Methoxy oder Ethoxy ausgewählt ist.

4. Verbindung nach Anspruch 3, worin Ring A für 1-(2-Methoxyethyl)imidazol-2-yl, 1-(2-Trimethylsilylethoxymethyl)imidazol-2-yl, 1-(Methoxymethyl)imidazol-2-yl, 1,2,4-Oxadiazol-5-yl, 1,3,4-Oxadiazol-2-yl, 1,3-Dimethylimidazol-2-yl, 1H-Imidazol-2-yl, 1-Methylimidazol-4-yl, 2H-Pyrazol-3-yl, 2-Methyl-1,2,4-Triazol-3-yl, 2-Pyridyl, Benzothiazol-2-yl, Isoxazol-5-yl, Pyrazin-2-yl, Pyrimidin-2-yl, Pyrimidin-4-yl, Thiazol-2-yl oder Thiazol-4-yl steht.

5. Verbindung nach Anspruch 1, worin Ring A für Carbocyclyl steht.

6. Verbindung nach einem der vorhergehenden Ansprüche, worin n für 0 steht.

7. Verbindung nach einem der Ansprüche 1-5, worin n für 1, 2 oder 3 steht und R⁵ aus der Gruppe bestehend aus Amino, N-Methylamino, N,N-Dimethylamino, Methyl und Methoxy ausgewählt ist.

8. Verbindung nach einem der Ansprüche 1-5, worin Ring A, R⁵ und n zusammen 1H-Imidazol-2-yl, 2,6-Bis(dimethylamino)pyrimidin-4-yl, 2-Pyridyl, 2H-Pyrazol-3-yl, Pyrimidin-4-yl, Isoxazol-5-yl, 1-Methylimidazol-4-yl, Pyrazin-2-yl, 2-Aminothiazol-4-yl, 2-Dimethylaminothiazol-4-yl, 2-Methylaminothiazol-4-yl, 1,3-Dimethylimidazol-2-yl, 1,4,5-Trimethylimidazol-2-yl, 4,6-Dimethoxypyrimidin-2-yl, 3-Methyl-1,2,4-oxadiazol-5-yl, Benzothiazol-2-yl, 4-Methoxypyrimidin-2-yl, Pyrimidin-2-yl, 1-Methylimidazol-2-yl, 1-(2-Methoxyethyl)imidazol-2-yl, 1-(Methoxymethyl)imidazol-2-yl, 1-(2-Trimethylsilylethoxymethyl)imidazol-2-yl, Thiazol-2-yl, 2-Methyl-1,2,4-triazol-3-yl, 5-Methyl-1,3,4-Oxadiazol-2-yl oder 1,3,4-Oxadiazol-2-yl bilden.

9. Verbindung nach Anspruch 1, worin:
R¹ für Chlor oder Cyano steht;
R² für Wasserstoff oder Chlor steht;
R³ für Fluor oder Methoxy steht;
R⁴ für Wasserstoff oder C₁₋₄-Alkyl steht;
Ring A für Pyridyl, 2H-Pyrazolyl, Isoxazolyl, Imidazolyl, Pyrazinyl, Thiazolyl, Pyrimidinyl, 1,2,4-Oxadiazolyl, Benzothiazolyl, 1,2,4-Triazolyl oder 1,3,4-Oxadiazolyl steht, wobei das Imidazolyl oder 1,2,4-Triazolyl gegebenenfalls an Stickstoff durch eine unter R⁶ ausgewählte Gruppe substituiert sein kann; und wobei das Imidazolyl an einer -N=-Gruppierung mit einer Methylgruppe eine quartäre Verbindung bilden kann;
R⁶ für Methyl oder Ethyl steht; wobei R⁶ gegebenenfalls an Kohlenstoff durch eine unter R²⁰ ausgewählte Gruppe substituiert sein kann;
R²⁰ unter Methoxy oder Ethoxy ausgewählt ist;
R⁵ für einen Substituenten an Kohlenstoff steht und unter Amino, C₁₋₄-Alkyl, C₁₋₄-Alkoxy, *N*-(C₁₋₄-Alkyl)amino oder *N,N-*(C₁₋₄-Alkyl)₂amino ausgewählt ist und
n für 0-2 steht;
oder ein pharmazeutisch annehmbares Salz davon.

10. Verbindung nach Anspruch 1, worin:
R¹ für Chlor oder Cyano steht;
R² für Wasserstoff oder Chlor steht;
R³ für Fluor oder Methoxy steht;
R⁴ für Wasserstoff, Methyl oder Ethyl steht;
Ring A für 1-(2-Methoxyethyl)imidazol-2-yl, 1-(2-Trimethylsilylethoxymethyl)imidazol-2-yl, 1-(Methoxymethyl)imidazol-2-yl, 1,2,4-Oxadiazol-5-yl, 1,3,4-Oxadiazol-2-yl, 1,3-Dimethylimidazol-2-yl, 1H-Imidazol-2-yl, 1-Methylimidazol-4-yl, 2H-Pyrazol-3-yl, 2-Methyl-1,2,4-triazol-3-yl, 2-Pyridyl, Benzothiazol-2-yl, Isoxazol-5-yl, Pyrazin-2-yl, Pyrimidin-2-yl, Pyrimidin-4-yl, Thiazol-2-yl oder Thiazol-4-yl steht;
R⁵ für einen Substituenten an Kohlenstoff steht und unter Amino, Methyl, Methoxy, Methylamino oder Dimethylamino ausgewählt ist;
n für 0-2 steht;
oder ein pharmazeutisch annehmbares Salz davon.

11. Verbindung, ausgewählt aus der Gruppe bestehend aus:
2-((3*S*,4*R*)-4-{[(3,4-Dichlor-5-methyl-1*H*-pyrrol-2-yl)carbonyl]amino}-3-methoxypiperidin-1-yl)-4-pyrimidin-2-yl-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-pyrimidin-4-yl-1,3-thiazol-5-carbonsäure;
2'-((3S,4R)-4-{[(3,4-Dichlor-5-methyl-1H-pyrrol-2-yl)carbonyl]amino}-3-methoxypiperidin-1-yl)-2,4'-bi-1,3-thiazol-5'-carbonsäure;
2-((3S,4R)-4-{[(3,4-Dichlor-5-methyl-1H-pyrrol-2-yl)carbonyl]amino}-3-fluorpiperidin-1-yl)-4-pyrimidin-2-yl-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-pyrimidin-4-yl-1,3-thiazol-5-carbonsäure;
2'-((3S,4R)-4-{[(3,4-Dichlor-5-methyl-1H-pyrrol-2-yl)carbonyl]amino}-3-fluorpiperidin-1-yl)-2,4'-bi-1,3-thiazol-5'-carbonsäure;
2-((3S,4R)-4-{[(3,4-Dichlor-5-methyl-1H-pyrrol-2-yl)carbonyl]amino}-3-methoxypiperidin-1-yl)-4-pyridin-2-yl-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-pyridin-2-yl-1,3-thiazol-5-carbonsäure;
Bis(dimethylamino)pyrimidin-4-yl]-2-((3S,4R)-4-{[(3,4-dichlor-5-methyl-1H-pyrrol-2-yl)carbonyl]amino}-3-methoxypiperidin-1-yl)-1,3-thiazol-5-carbonsäure;
Bis(dimethylamino)pyrimidin-4-yl]-2-((3S,4R)-4-{[(3,4-dichlor-5-methyl-1H-pyrrol-2-yl)carbonyl]amino}-3-fluorpiperidin-1-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(4,6-dimethoxypyrimidin-2-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-pyrazin-2-yl-1,3-thiazol-5-carbonsäure;
4-(1,3-Benzothiazol-2-yl)-2-((3S,4R)-4-{[(3,4-dichlor-5-methyl-1H-pyrrol-2-yl)carbonyl]amino}-3-methoxypiperidin-1-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-pyrazin-2-yl-1,3-thiazol-5-carbonsäure;
4-(1,3-Benzothiazol-2-yl)-2-((3S,4R)-4-{[(3,4-dichlor-5-methyl-1H-pyrrol-2-yl)carbonyl]amino}-3-fluorpiperidin-1-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(1-{[2-(trimethylsilyl)ethoxy]methyl}1H-imidazol-2-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-[1-(methoxymethyl)-1H-imidazol-2-yl]-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(1H-imidazol-2-yl)-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-[1-(methoxymethyl)-1H-imidazol-2-yl]-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-(1H-imidazol-2-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(1-methyl-1H-imidazol-2-yl)-1,3-thiazol-5-carbonsäure;
2-[5-Carboxy-2-((3S,4R)-4-{[(3,4-dichlor-5-methyl-1H-pyrrol-2-yl)carbonyl]amino}-3-methoxypiperidin-1-y1)-1,3-thiazol-4-yl]-1,3-dimethyl-1H-imidazol-3-iumtrifluoracetat;
2-((3S,4R)-4-{[(3,4-Dichlor-5-methyl-1H-pyrrol-2-yl)carbonyl]amino}-3-methoxypiperidin-1-yl)-4-(1H-pyrazol-5-yl)-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-(1H-pyrazol-5-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-[1-(2-methoxyethyl)-1H-imidazol-2-yl]-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-[1-(2-methoxyethyl)-1H-imidazol-2-yl]-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(1-methyl-1H-1,2,4-triazol-5-yl)-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-(1-methyl-1H-1,2,4-triazol-5-yl)-1,3-thiazol-5-carbonsäure;
2-((3S,4R)-4-{[(3-Chlor-4-cyano-5-methyl-1H-pyrrol-2-yl)carbonyl]amino}-3-methoxypiperidin-1-yl)-4-(1-methyl-1H-1,2,4-triazol-5-yl)-1,3-thiazol-5-carbonsäure;
2-((3S,4R)-4-{[(3-Chlor-4-cyano-5-methyl-1H-pyrrol-2-yl)carbonyl]amino}-3-methoxypiperidin-1-yl)-4-[1-(2-methoxyethyl)-1H-imidazol-2-yl]-1,3-thiazol-5-carbonsäure;
2-((3S,4R)-4-{[(4-Cyano-5-methyl-1H-pyrrol-2-yl)carbonyl]amino}-3-methoxypiperidin-1-yl)-4-(1-methyl-1H-1,2,4-triazol-5-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(1,4,5-trimethyl-1H-imidazol-2-yl)-1,3-thiazol-5-carbonsäure;
2'-Amino-2-((3S,4R)-4-{[(3,4-dichlor-5-methyl-1H-pyrrol-2-yl)carbonyl]amino}-3-methoxypiperidin-1-yl)-4,4'-bi-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-2'(methylamino)-4,4'-bi-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-2'(dimethylamino)-4,4'-bi-1,3-thiazol-5-carbonsäure;
2'-Amino-2-((3S,4R)-4-{[(3,4-dichlor-5-methyl-1H-pyrrol-2-yl)carbonyl]amino}-3-fluorpiperidin-1-yl)-4,4'-bi-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-2'(methylamino)-4,4'-bi-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-2'(dimethylamino)-4,4'-bi-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-(1-methyl-1H-imidazol-2-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(1-methyl-1H-imidazol-4-yl)-1,3-thiazol-5-carbonsäure;
2-((3S,4R)-4-{[(3,4-Dichlor-5-methyl-1H-pyrrol-2-yl)carbonyl]amino}-3-fluorpiperidin-1-yl)-4-(1-methyl-1H-imidazol-4-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(4-methoxypyrimidin-2-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-pyrimidin-2-yl-1,3-thiazol-5-carbonsäureethylester;
2'-((3S,4R)-4-{[(3,4-Dichlor-5-methyl-1H-pyrrol-2-yl)carbonyl]amino}-3-methoxypiperidin-1-yl)-2,4'-bi-1,3-thiazol-5'-carbonsäuremethylester;
2-((3S,4R)-4-{[(3,4-Dichlor-5-methyl-1H-pyrrol-2-yl)carbonyl]amino}-3-fluorpiperidin-1-yl)-4-pyrimidin-2-yl-1,3-thiazol-5-carbonsäureethylester;
2-((3S,4R)-4-{[(3,4-Dichlor-5-methyl-1H-pyrrol-2-yl)carbonyl]amino}-3-fluorpiperidin-1-yl)-4-pyrimidin-4-yl-1,3-thiazol-5-carbonsäureethylester;
2'-((3S,4R)-4-{[(3,4-Dichlor-5-methyl-1H-pyrrol-2-yl)carbonyl]amino}-3-fluorpiperidin-1-yl)-2,4'-bi-1,3-thiazol-5'-carbonsäuremethylester;
2-((3S,4R)-4-{[(3,4-Dichlor-5-methyl-1H-pyrrol-2-yl)carbonyl]amino}-3-methoxypiperidin-1-yl)-4-pyridin-2-yl-1,3-thiazol-5-carbonsäureethylester;
2-((3S,4R)-4-{[(3,4-Dichlor-5-methyl-1H-pyrrol-2-yl)carbonyl]amino}-3-fluorpiperidin-1-yl)-4-pyridin-2-yl-1,3-thiazol-5-carbonsäureethylester;
4-[2,6-Bis(dimethylamino)pyrimidin-4-yl]-2-((3S,4R)-4-{[(3,4-dichlor-5-methyl-1H-pyrrol-2-yl)carbonyl]amino}-3-methoxypiperidin-1-yl)-1,3-thiazol-5-carbonsäureethylester;
4-[2,6-Bis(dimethylamino)pyrimidin-4-yl]-2-((3S,4R)-4-{[(3,4-dichlor-5-methyl-1H-pyrrol-2-yl)carbonyl]amino}-3-fluorpiperidin-1-yl)-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-(4,6-dimethoxypyrimidin-2-yl)-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-pyrazin-2-yl-1,3-thiazol-5-carbonsäureethylester;
4-(1,3-Benzothiazol-2-yl)-2-((3S,4R)-4-{[(3,4-dichlor-5-methyl-1H-pyrrol-2-yl)carbonyl]amino}-3-methoxypiperidin-1-yl)-1,3-thiazol-5-carbonsäureethylester;
2-((3S,4R)-4-{[(3,4-Dichlor-5-methyl-1H-pyrrol-2-yl)carbonyl]amino}-3-fluorpiperidin-1-yl)-4-pyrazin-2-yl-1,3-thiazol-5-carbonsäureethylester;
4-(1,3-Benzothiazol-2-yl)-2-((3S,4R)-4-{[(3,4-dichlor-5-methyl-1H-pyrrol-2-yl)carbonyl]amino}-3-fluorpiperidin-1-yl)-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-(1-{[2-(trimethylsilyl)ethoxy]methyl}-1H-imidazol-2-yl)-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-[1-(methoxymethyl)-1H-imidazol-2-yl]-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-4-[1-(methoxymethyl)-1H-imidazol-2-yl]-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-isoxazol-5-yl-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-4-isoxazol-5-yl-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-(1H-pyrazol-5-yl)-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-4-(1H-pyrazol-5-yl)-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-[1-(2-methoxyethyl)-1H-imidazol-2-yl]-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-4-[1-(2-methoxyethyl)-1H-imidazol-2-yl]-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-(1-methyl-1H-1,2,4-triazol-5-yl)-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-4-(1-methyl-1H-1,2,4-triazol-5-yl)-1,3-thiazol-5-carbonsäuremethylester;
2-((3S,4R)-4-{[(4-Cyano-5-methyl-1H-pyrrol-2-yl)carbonyl]amino}-3-methoxypiperidin-1-yl)-4-(1-methyl-1H-1,2,4-triazol-5-yl)-1,3-thiazol-5-carbonsäuremethylester;
2-((3S,4R)-4-{[(3-Chlor-4-cyano-5-methyl-1H-pyrrol-2-yl)carbonyl]amino}-3-methoxypiperidin-1-yl)-4-[1(2-methoxyethyl)-1H-imidazol-2-yl]-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-(1,4,5-trimethyl-1H-imidazol-2-yl)-1,3-thiazol-5-carbonsäuremethylester;
2'-Amino-2-((3S,4R)-4-{[(3,4-dichlor-5-methyl-1H-pyrrol-2-yl)carbonyl]amino}-3-methoxypiperidin-1-yl)-4,4'-bi-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-2'-(methylamino)-4,4'-bi-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-2'-(dimethylamino)-4,4'-bi-1,3-thiazol-5-carbonsäuremethylester;
2'-Amino-2-((3S,4R)-4-{[(3,4-dichlor-5-methyl-1H-pyrrol-2-yl)carbonyl]amino}-3-fluorpiperidin-1-yl)-4,4'-bi-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-2'-(methylamino)-4,4'-bi-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-2'-(dimethylamino)-4,4'-bi-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-(1-methyl-1H-imidazol-4-yl)-1,3-thiazol-5-carbonsäureethylester;
2-((3S,4R)-4-{[(3,4-Dichlor-5-methyl-1H-pyrrol-2-yl)carbonyl]amino}-3-fluorpiperidin-1-yl)-4-(1-methyl-1H-imidazol-4-yl)-1,3-thiazol-5-carbonsäureethylester;
2-((3S,4R)-4-{[(3,4-Dichlor-5-methyl-1H-pyrrol-2-yl)carbonyl]amino}-3-fluorpiperidin-1-yl)-4-(1-methyl-1H-imidazol-2-yl)-1,3-thiazol-5-carbonsäuremethylester;
2-((3S,4R)-4-{[(3-Chlor-4-cyano-5-methyl-1H-pyrrol-2-yl)carbonyl]amino}-3-methoxypiperidin-1-yl)-4-(1-methyl-1H-1,2,4-triazol-5-yl)-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-pyrimidin-4-yl-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-(4-methoxypyrimidin-2-yl)-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-(1,3,4-oxadiazol-2-yl)-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-(3-methyl-1,2,4-oxadiazol-5-yl)-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-(5-methyl-1,3,4-oxadiazol-2-yl)-1,3-thiazol-5-carbonsäureethylester;
2-((3S,4R)-4-{[(3,4-Dichlor-5-methyl-1H-pyrrol-2-yl)carbonyl]amino}-3-methoxypiperidin-1-yl)-4-(1H-imidazol-2-yl)-1,3-thiazol-5-carbonsäuremethylester;
2-((3S,4R)-4-{[(3,4-Dichlor-5-methyl-1H-pyrrol-2-yl)carbonyl]amino}-3-fluorpiperidin-1-yl)-4-(1H-imidazol-2-yl)-1,3-thiazol-5-carbonsäuremethylester;
2-((3S,4R)-4-{[(3,4-Dichlor-5-methyl-1H-pyrrol-2-yl)carbonyl]amino}-3-methoxypiperidin-1-yl)-4-(1-methyl-1H-imidazol-2-yl)-1,3-thiazol-5-carbonsäuremethylester;
2-[2-((3S,4R)-4-{[(3,4-Dichlor-5-methyl-1H-pyrrol-2-yl)carbonyl]amino}-3-methoxypiperidin-1-yl)-5-(methoxycarbonyl)-1,3-thiazol-4-yl]-1,3-dimethyl-1H-imidazol-3-iumiodid;
2-((3S,4R)-4-{[(3,4-Dichlor-5-methyl-1H-pyrrol-2-yl)carbonyl]amino}-3-methoxypiperidin-1-yl)-4-(5-methyl-1,3,4-oxadiazol-2-yl)-1,3-thiazol-5-carbonsäure;
2-((3S,4R)-4-{[(3,4-Dichlor-5-methyl-1H-pyrrol-2-yl)carbonyl]amino}-3-methoxypiperidin-1-yl)-4-(5-methyl-1,3,4-oxadiazol-2-yl)-1,3-thiazol-5-carbonsäure;
2-((3S,4R)-4-(3,4-Dichlor-5-methyl-1H-pyrrol-2-carboxamido)-3-methoxypiperidin-1-yl)-4-(1-methyl-1H-1,2,3-triazol-4-yl)thiazol-5-carbonsäuremethylester;
2-((3S,4R)-4-(3,4-Dichlor-5-methyl-1H-pyrrol-2-carboxamido)-3-methoxypiperidin-1-yl)-4-(3,5-dimethylpyrazin-2-yl)thiazol-5-carbonsäuremethylester;
2-((3S,4R)-4-(3,4-Dichlor-5-methyl-1H-pyrrol-2-carboxamido)-3-methoxypiperidin-1-yl)-4-(6-methoxypyridin-2-yl)thiazol-5-carbonsäuremethylester;
2-((3S,4R)-4-(3,4-Dichlor-5-methyl-1H-pyrrol-2-carboxamido)-3-methoxypiperidin-1-yl)-4-(4-methoxypyridin-2-yl)thiazol-5-carbonsäureethylester;
2-((3S,4R)-4-(3,4-Dichlor-5-methyl-1H-pyrrol-2-carboxamido)-3-methoxypiperidin-1-yl)-4-(5-methylpyrazin-2-yl)thiazol-5-carbonsäureethylester;
2-((3S,4R)-4-(3,4-Dichlor-5-methyl-1H-pyrrol-2-carboxamido)-3-methoxypiperidin-1-yl)-4-(3-fluorpyridin-2-yl)thiazol-5-carbonsäureethylester;
2-((3S,4R)-4-(3,4-Dichlor-5-methyl-1H-pyrrol-2-carboxamido)-3-fluorpiperidin-1-yl)-4-(4-methoxypyridin-2-yl)thiazol-5-carbonsäureethylester;
2-((3S,4R)-4-(3,4-Dichlor-5-methyl-1H-pyrrol-2-carboxamido)-3-fluorpiperidin-1-yl)-4-(1-methyl-1H-1,2,3-triazol-4-yl)thiazol-5-carbonsäuremethylester;
2-((3S,4R)-4-(3,4-Dichlor-5-methyl-1H-pyrrol-2-carboxamido)-3-fluorpiperidin-1-yl)-4-(6-methoxypyridin-2-yl)thiazol-5-carbonsäuremethylester;
2-((3S,4R)-4-(3,4-Dichlor-5-methyl-1H-pyrrol-2-carboxamido)-3-fluorpiperidin-1-yl)-4-(6-methoxypyridin-2-yl)thiazol-5-carbonsäuremethylester;
2-((3S,4R)-4-(3,4-Dichlor-5-methyl-1H-pyrrol-2-carboxamido)-3-fluorpiperidin-1-yl)-4-(3-fluorpyridin-2-yl)thiazol-5-carbonsäureethylester;
2-((3S,4R)-4-(3,4-Dichlor-5-methyl-1H-pyrrol-2-carboxamido)-3-fluorpiperidin-1-yl)-4-(4-methoxypyridin-2-yl)thiazol-5-carbonsäureethylester;
2-((3S,4R)-4-(4-Chlor-3-cyano-5-methyl-1H-pyrrol-2-carboxamido)-3-methoxypiperidin-1-yl)-4-(1-methyl-1H-1,2,3-triazol-4-yl)thiazol-5-carbonsäuremethylester;
2-((3S,4R)-4-(4-Chlor-3-cyano-5-methyl-1H-pyrrol-2-carboxamido)-3-methoxypiperidin-1-yl)-4-(1-methyl-1H-1,2,4-triazol-5-yl)thiazol-5-carbonsäuremethylester;
2-((3S,4R)-4-(4-Chlor-3-cyano-5-methyl-1H-pyrrol-2-carboxamido)-3-methoxypiperidin-1-yl)-4-(pyrimidin-2-yl)thiazol-5-carbonsäureethylester;
2-((3S,4R)-4-(4-Chlor-3-cyano-5-methyl-1H-pyrrol-2-carboxamido)-3-methoxypiperidin-1-yl)-4-(3-fluorpyridin-2-yl)thiazol-5-carbonsäureethylester;
2-((3S,4R)-4-(4-Chlor-3-cyano-5-methyl-1H-pyrrol-2-carboxamido)-3-methoxypiperidin-1-yl)-4-(5-methylpyrazin-2-yl)thiazol-5-carbonsäureethylester;
2-((3S,4R)-4-(4-Chlor-3-cyano-5-methyl-1H-pyrrol-2-carboxamido)-3-methoxypiperidin-1-yl)-4-(1-methyl-1H-imidazol-4-yl)thiazol-5-carbonsäureethylester;
2-((3S,4R)-4-(3,4-Dichlor-5-methyl-1H-pyrrol-2-carboxamido)-3-methoxypiperidin-1-yl)-4-(1-methyl-1H-1,2,3-triazol-4-yl)thiazol-5-carbonsäure;
2-((3S,4R)-4-(3,4-Dichlor-5-methyl-1H-pyrrol-2-carboxamido)-3-methoxypiperidin-1-yl)-4-(3,5-dimethylpyrazin-2-yl)thiazol-5-carbonsäure;
2-((3S,4R)-4-(3,4-Dichlor-5-methyl-1H-pyrrol-2-carboxamido)-3-methoxypiperidin-1-yl)-4-(6-methoxypyridin-2-yl)thiazol-5-carbonsäure;
2-((3S,4R)-4-(3,4-Dichlor-5-methyl-1H-pyrrol-2-carboxamido)-3-methoxypiperidin-1-yl)-4-(4-methoxypyridin-2-yl)thiazol-5-carbonsäure;
2-((3S,4R)-4-(3,4-Dichlor-5-methyl-1H-pyrrol-2-carboxamido)-3-methoxypiperidin-1-yl)-4-(5-methylpyrazin-2-yl)thiazol-5-carbonsäure;
2-((3S,4R)-4-(3,4-Dichlor-5-methyl-1H-pyrrol-2-carboxamido)-3-methoxypiperidin-1-yl)-4-(3-fluorpyridin-2-yl)thiazol-5-carbonsäure;
2-((3S,4R)-4-(3,4-Dichlor-5-methyl-1H-pyrrol-2-carboxamido)-3-fluorpiperidin-1-yl)-4-(3-fluorpyridin-2-yl)thiazol-5-carbonsäure;
2-((3S,4R)-4-(3,4-Dichlor-5-methyl-1H-pyrrol-2-carboxamido)-3-fluorpiperidin-1-yl)-4-(1-methyl-1H-1,2,3-triazol-4-yl)thiazol-5-carbonsäure;
2-((3S,4R)-4-(3,4-Dichlor-5-methyl-1H-pyrrol-2-carboxamido)-3-fluorpiperidin-1-yl)-4-(6-methoxypyridin-2-yl)thiazol-5-carbonsäure;
2-((3S,4R)-4-(3,4-Dichlor-5-methyl-1H-pyrrol-2-carboxamido)-3-fluorpiperidin-1-yl)-4-(5-methylpyrazin-2-yl)thiazol-5-carbonsäure;
2-((3S,4R)-4-(3,4-Dichlor-5-methyl-1H-pyrrol-2-carboxamido)-3-fluorpiperidin-1-yl)-4-(3-fluorpyridin-2-yl)thiazol-5-carbonsäure;
2-((3S,4R)-4-(3,4-Dichlor-5-methyl-1H-pyrrol-2-carboxamido)-3-fluorpiperidin-1-yl)-4-(4-methoxypyridin-2-yl)thiazol-5-carbonsäure;
2-((3S,4R)-4-(4-Chlor-3-cyano-5-methyl-1H-pyrrol-2-carboxamido)-3-methoxypiperidin-1-yl)-4-(1-methyl-1H-1,2,3-triazol-4-yl)thiazol-5-carbonsäure;
2-((3S,4R)-4-(4-Chlor-3-cyano-5-methyl-1H-pyrrol-2-carboxamido)-3-methoxypiperidin-1-yl)-4-(1-methyl-1H-1,2,4-triazol-5-yl)thiazol-5-carbonsäure;
2-((3S,4R)-4-(4-Chlor-3-cyano-5-methyl-1H-pyrrol-2-carboxamido)-3-methoxypiperidin-1-yl)-4-(pyrimidin-2-yl)thiazol-5-carbonsäure;
2-((3S,4R)-4-(4-Chlor-3-cyano-5-methyl-1H-pyrrol-2-carboxamido)-3-methoxypiperidin-1-yl)-4-(3-fluorpyirdin-2-yl)thiazol-5-carbonsäure;
2-((3S,4R)-4-(4-Chlor-3-cyano-5-methyl-1H-pyrrol-2-carboxamido)-3-methoxypiperidin-1-yl)-4-(5-methylpyrazin-2-yl)thiazol-5-carbonsäure;
2-((3S,4R)-4-(4-Chlor-3-cyano-5-methyl-1H-pyrrol-2-carboxamido)-3-methoxypiperidin-1-yl)-4-(1-methyl-1H-imidazol-4-yl)thiazol-5-carbonsäure;
2-{(3S,4R)-4-[(4-Chlor-3-cyano-5-methyl-1H-pyrrol-2-carbonyl)amino]-3-fluorpiperidin-1-yl}-4-pyrazin-2-ylthiazol-5-carbonsäureethylester;
2-{(3S,4R)-4-[(4-Chlor-3,5-dimethyl-1H-pyrrol-2-carbonyl)amino]-3-fluorpiperidin-1-yl}-4-pyrazin-2-ylthiazol-5-carbonsäureethylester;
2-{(3S,4R)-4-[(4-Chlor-3,5-dimethyl-1H-pyrrol-2-carbonyl)amino]-3-fluorpiperidin-1-yl}-4-pyrimidin-4-ylthiazol-5-carbonsäureethylester;
2-{(3S,4R)-4-[(4-Chlor-3-cyano-5-methyl-1H-pyrrol-2-carbonyl)amino]-3-methoxypiperidin-1-yl}-4-pyrimidin-4-ylthiazol-5-carbonsäureethylester;
2-{(3S,4R)-4-[(4-Chlor-3-cyano-5-methyl-1H-pyrrol-2-carbonyl)amino]-3-methoxypiperidin-1-yl}-4-(1-methyl-1H-pyrazol-3-yl)thiazol-5-carbonsäureethylester;
2-{(3S,4R)-4-[(3,4-Dichlor-5-methyl-1H-pyrrol-2-carbonyl)amino]-3-methoxypiperidin-1-yl}-4-(1-methyl-1H-pyrazol-3-yl)thiazol-5-carbonsäureethylester;
2-{(3S,4R)-4-[(4-Chlor-3-cyano-5-methyl-1H-pyrrol-2-carbonyl)amino]-3-fluorpiperidin-1-yl}-4-pyrazin-2-ylthiazol-5-carbonsäure;
2-[(3S,4R)-4-{[(4-Chlor-3,5-dimethyl-1H-pyrrol-2-yl)carbonyl]amino}-3-fluorpiperidin-1-yl]-4-pyrazin-2-yl-1,3-thiazol-5-carbonsäure;
2-[(3S,4R)-4-{[(4-Chlor-3,5-dimethyl-1H-pyrrol-2-yl)carbonyl]amino}-3-fluorpiperidin-1-yl]-4-pyrimidin-4-yl-1,3-thiazol-5-carbonsäure;
2-[(3S,4R)-4-{[(4-Chlor-3-cyano-5-methyl-1H-pyrrol-2-yl)carbonyl]amino}-3-methoxypiperidin-1-yl]-4-pyrimidin-4-yl-1,3-thiazol-5-carbonsäure;
2-[(3S,4R)-4-{[(4-Chlor-3-cyano-5-methyl-1H-pyrrol-2-yl)carbonyl]amino}-3-methoxypiperidin-1-yl]-4-(1-methyl-1H-pyrazol-3-yl)-1,3-thiazol-5-carbonsäure;
2-[(3S,4R)-4-{[(3,4-Dichlor-5-methyl-1H-pyrrol-2-yl)carbonyl]amino}-3-methoxypiperidin-1-yl]-4-(1-methyl-1H-pyrazol-3-yl)-1,3-thiazol-5-carbonsäure;
oder ein pharmazeutisch annehmbares Salz davon.

12. Verbindung nach einem der Ansprüche 1-11 oder ein pharmazeutisch annehmbares Salz davon zur Verwendung bei der Hervorrufung einer antibakteriellen Wirkung bei einem Warmblüter.

13. Verbindung nach einem der Ansprüche 1-11 oder ein pharmazeutisch annehmbares Salz davon zur Verwendung bei der Inhibierung von bakterieller DNA-Gyrase und/oder Topoisomerase IV bei einem Warmblüter.

14. Verbindung nach einem der Ansprüche 1-11 oder ein pharmazeutisch annehmbares Salz davon zur Verwendung bei der Behandlung einer bakteriellen Infektion bei einem Warmblüter.

15. Verbindung nach einem der Ansprüche 1-11 oder ein pharmazeutisch annehmbares Salz davon zur Verwendung bei der Behandlung von ambulant erworbenen Lungenentzündungen, im Krankenhaus erworbenen Lungenentzündungen, Haut- und Hautstrukturinfektionen, akuter Verschlimmerung von chronischer Bronchitis, akuter Sinusitis, akuter Mittelohrentzündung, katheterbedingter Sepsis, febriler Neutropenie, Osteomyelitis, Endocarditis, Harnwegsinfektionen, penicillinresistentem Streptococcus pneumoniae, methicillinresistentem Staphylococcus aureus, methicillinresistentem Staphylococcus epidermidis oder vancomycinresistenten Enterokokken.

## Revendications

1. Composé de Formule (**Ia**) : 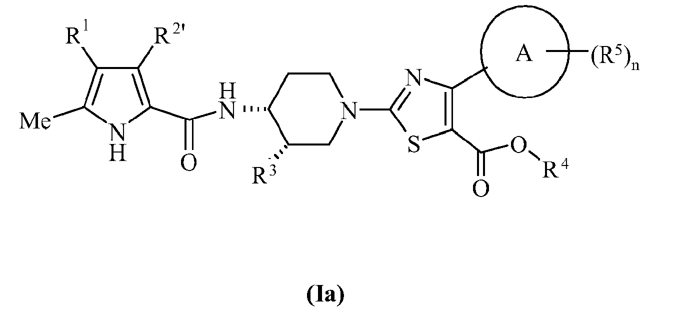
**R¹** représente un groupement chloro ou cyano ;
**R^{2'}** représente un atome d'hydrogène ou un groupement chloro, cyano ou méthyle ;
**R³** représente un groupement fluoro, méthyle, methoxy, éthoxy, propoxy, allyloxy ou benzyloxy ;
**R⁴** représente un atome d'hydrogène ou un groupement alkyle en C₁₋₄ ;
le **Cycle A** représente un groupement carbocyclyle ou hétérocyclyle ; où, lorsque ledit groupement hétérocyclyle comporte une fonction -NH-, cet atome d'azote est éventuellement substitué par un groupement choisi parmi R⁶ ; et où, lorsque ledit groupement hétérocyclyle comprend une fonction -N=, cet atome d'azote peut former un composé quaternaire avec un groupement méthyle ;
**R⁵** est un substituant d'un atome de carbone et peut être choisi parmi les groupements halogéno, nitro, cyano, hydroxy, amino, carboxy, carbamoyle, mercapto, sulfamoyle, sulfo, formyle, uréido, hydroxyiminométhyle, *N*-hydroxyformamido, hydrazinocarbonyle, *N*-hydroxyéthanimidoyle, amino(hydroxyimino)méthyle, alkyle en C₁₋₄, alcényle en C₂₋₄, alcynyle en C₂₋₄, alkoxy en C₁₋₄, alcanoyle en C₁₋₄, alcanoyloxy en C₁₋₄, *N*-(alkyle en C₁₋₄)amino, *N,N-*(alkyle en C₁₋₄)₂amino, (alcanoyle en C₁₋₄)-amino, *N*-(alkyle en C₁₋₄)carbamoyle, *N,N*-(alkyle en C₁₋₄)₂carbamoyle, *N-*(alkoxy en C₁₋₄)carbamoyle, *N*'-(alkyle en C₁₋₄)uréido, *N'*,*N'*-(alkyle en C₁₋₄)₂uréido, *N*-(alkyle en C₁₋₄)-*N-*(alkoxy en C₁₋₄)carbamoyle, (alkyle en C₁₋₄)-S(O)ₐ où a est compris entre 0 et 2, (alkoxy en C₁₋₄)-carbonyle, (alkoxy en C₁₋₄)-carbonylamino, *N*-(alkyle en C₁₋₄ )sulfamoyle, *N,N*-(alkyle en C₁₋₄)₂sulfamoyle, (alkyle en C₁₋₄)-sulfonylamino, (alkyle en C₁₋₄)-sulfonylaminocarbonyle, *N'*-(alkyle en C₁₋₄ )hydrazinocarbonyle, *N',N'*-(alkyle en C₁₋₄ )₂hydrazinocarbonyle, carbocyclyl-R⁷- ou hétérocyclyl-R⁸- ; où R⁵ est éventuellement substitué au niveau de l'atome de carbone par un ou plusieurs radicaux R⁹ ; et où, si ledit groupement hétérocyclyle comporte une fonction -NH-, cet atome d'azote est éventuellement substitué par un groupement choisi parmi R¹⁰ ;
**n** est égal à 0, 1, 2 ou 3 ;
**R⁹** est choisi parmi les groupements halogéno, nitro, cyano, hydroxy, amino, carboxy, carbamoyle, mercapto, sulfamoyle, alkyle en C₁₋₄, alcényle en C₂₋₄, alcynyle en C₂₋₄, alkoxy en C₁₋₄, alcanoyle en C₁₋₄, alcanoyloxy en C₁₋₄ , *N*-(alkyle en C₁₋₄)amino, *N,N-*(alkyle en C₁₋₄)₂amino, (alcanoyle en C₁₋₄)-amino, *N*-(alkyle en C₁₋₄)carbamoyle, *N,N*-(alkyle en C₁₋₄)₂carbamoyle, (alkyle en C₁₋₄)-S(O)ₐ, où a est compris entre 0 et 2, (alkoxy en C₁₋₄ )carbonyle, *N*-(alkyle en C₁₋₄)sulfamoyle, *N,N*-(alkyle en C₁₋₄)₂sulfamoyle, (alkyle en C₁₋₄)-sulfonylamino, (alkoxy en C₁₋₄)-carbonylamino, carbocyclyl-R¹¹- ou hétérocyclyl-R¹²- _{;} où R⁹ est éventuellement substitué au niveau de l'atome de carbone par un ou plusieurs radicaux R¹³ ; et où, lorsque ledit groupement hétérocyclyle comporte une fonction -NH-, cet atome d'azote est éventuellement substitué par un groupement choisi parmi R¹⁴ _{;}
**R⁶, R¹⁰** et **R¹⁴** sont indépendamment choisis parmi les groupements alkyle en C₁₋₄, alcanoyle en C₁₋₄, (alkyle en C₁₋₄)-sulfonyle, (alkoxy en C₁₋₄)-carbonyle, carbamoyle, *N*-(alkyle en C₁₋₄) carbamoyle, *N,N-*(alkyle en C₁₋₄)-carbamoyle, benzyle, benzyloxycarbonyle, benzoyle et phénylsulfonyle ; où R⁶, R¹⁰ et R¹⁴ sont indépendamment éventuellement substitués au niveau de l'atome de carbone par un groupement choisi parmi R²⁰;
**R⁷, R⁸, R¹¹** et **R¹²** sont indépendamment choisis parmi une liaison directe et les groupements -O-, -N(R¹⁵)-, -C(O)-, -N(R¹⁶)C(O)-, -C(O)N(R¹⁷)-, -S(O)ₚ-, -SO₂N(R¹⁸)- or - N(R¹⁹)SO₂- ; où R¹⁵, R¹⁶, R¹⁷, R¹⁸ et R¹⁹ sont indépendamment choisis parmi l'atome d'hydrogène et les groupements alkyle en C₁₋₄ et p est compris entre 0 et 2 ;
**R¹³** et **R²⁰** sont indépendamment choisis parmi les groupements halogéno, nitro, cyano, hydroxy, trifluorométhoxy, trifluorométhyle, amino, carboxy, carbamoyle, mercapto, sulfamoyle, méthyle, éthyle, éthényle, éthynyle, méthoxy, éthoxy, 2-triméthylsilyléthoxy, acétyle, acétoxy, méthylamino, éthylamino, diméthylamino, diéthylamino, *N*-méthyl-*N-*éthylamino, acétylamino, N-méthylcarbamoyle, *N-*éthylcarbamoyle, *N,N*-diméthylcarbamoyle, *N,N-*diéthylcarbamoyle, *N*-méthyl-*N*-éthylcarbamoyle, méthylthio, éthylthio, méthylsulfinyle, éthylsulfinyle, mésyle, éthylsulfonyle, méthoxycarbonyle, éthoxycarbonyle, *N*-méthylsulfamoyle, *N*-éthylsulfamoyle, *N,N*-diméthylsulfamoyle, *N,N*-diéthylsulfamoyle ou *N-*méthyl-*N*-éthylsulfamoyle ;
ou l'un de ses sels de qualité pharmaceutique.

2. Composé conforme à la Revendication 1, où le cycle A représente un groupement hétérocyclyle ; où, lorsque ledit groupement hétérocyclyle comporte une fonction -NH-, cet atome d'azote est éventuellement substitué par un groupement choisi parmi R⁶ ; et où, lorsque ledit groupement hétérocyclyle comporte une fonction -N=, cet atome d'azote peut former un composé quaternaire avec un groupement méthyle ; où :
R⁶ représente un groupement alkyle en C₁₋₄ ; où R⁶ est éventuellement substitué au niveau de l'atome de carbone par un groupement choisi parmi R²⁰ ; et
R²⁰ est choisi parmi les groupements méthoxy et éthoxy.

3. Composé conforme à la Revendication 2, où le Cycle A représente un groupement pyridyle, 2H-pyrazolyle, isoxazolyle, imidazolyle, pyrazinyle, thiazolyle, pyrimidinyle, 1,2,4-oxadiazolyle, benzothiazolyle, 1,2,4-triazolyle ou 1,3,4-oxadiazolyle, où ledit groupement imidazolyle ou 1,2,4-triazolyle est éventuellement substitué au niveau de l'atome d'azote par un groupement choisi parmi R⁶ ; et où ledit groupement imidazolyle peut former un composé quaternaire au niveau d'une fonction -N= avec un groupement méthyle ; où
R⁶ représente un groupement méthyle ou éthyle ; où R⁶ est éventuellement substitué au niveau de l'atome de carbone par un groupement choisi parmi R²⁰ ; et
R²⁰ est choisi parmi les groupements méthoxy et éthoxy.

4. Composé conforme à la Revendication 3 où le Cycle A représente un groupement 1-(2-méthoxyéthyl)imidazol-2-yle, 1-(2-triméthylsilyléthoxyméthyl)imidazol-2-yle, 1-(méthoxyméthyl)imidazol-2-yle, 1,2,4-oxadiazol-5-yle, 1,3,4-oxadiazol-2-yle, 1,3-diméthylimidazol-2-yle, 1*H-*imidazol-2-yle, 1-méthylimidazol-4-yle, 2*H*-pyrazol-3-yle, 2-méthyl-1,2,4-triazol-3-yle, 2-pyridyle, benzothiazol-2-yle, isoxazol-5-yle, pyrazin-2-yle, pyrimidin-2-yle, pyrimidin-4-yle, thiazol-2-yle ou thiazol-4-yle.

5. Composé conforme à la Revendication 1, où le Cycle A représente un groupement carbocyclyle.

6. Composé conforme à l'une quelconque des revendications précédentes, où n est égal à 0.

7. Composé conforme à l'une quelconque des Revendications 1 à 5, où n est égal à 1, 2 ou 3 et R⁵ est choisi dans le groupe constitué par les groupements amino, *N*-méthylamino, *N,N*-diméthylamino, méthyle et méthoxy.

8. Composé conforme à l'une quelconque des Revendications 1 à 5, où le Cycle A, R⁵ et n forment ensemble un groupement 1*H*-imidazol-2-yle, 2,6-bis(diméthylamino)pyrimidin-4-yle, 2-pyridyle, 2*H-*pyrazol-3-yle, pyrimidin-4-yle, isoxazol-5-yle, 1-méthylimidazol-4-yle, pyrazin-2-yle, 2-aminothiazol-4-yle, 2-diméthylaminothiazol-4-yle, 2-méthylaminothiazol-4-yle, 1,3-diméthylimidazol-2-yle, 1,4,5-triméthylimidazol-2-yle, 4,6-diméthoxypyrimidin-2-yle, 3-méthyl-1,2,4-oxadiazol-5-yle, benzothiazol-2-yle, 4-méthoxypyrimidin-2-yle, pyrimidin-2-yle, 1-méthylimidazol-2-yle, 1-(2-méthoxyéthyl)imidazol-2-yle, 1-(méthoxyméthyl)imidazol-2-yle, 1-(2-triméthylsilyléthoxyméthyl)imidazol-2-yle, thiazol-2-yle, 2-méthyl-1,2,4-triazol-3-yle, 5-méthyl-1,3,4-oxadiazol-2-yle ou 1,3,4-oxadiazol-2-yle.

9. Composé conforme à la Revendication 1, où :
R¹ représente un groupement chloro ou cyano ;
R²' représente un atome d'hydrogène ou un groupement chloro ;
R³ représente un groupement fluoro ou méthoxy ;
R⁴ représente un atome d'hydrogène ou un groupement alkyle en C₁₋₄ ;
le Cycle A représente un groupement pyridyle, 2*H-*pyrazolyle, isoxazolyle, imidazolyle, pyrazinyle, thiazolyle, pyrimidinyle, 1,2,4-oxadiazolyle, benzothiazolyle, 1,2,4-triazolyle ou 1,3,4-oxadiazolyle, où ledit groupement imidazolyle ou 1,2,4-triazolyle est éventuellement substitué au niveau de l'atome d'azote par un groupement choisi parmi R⁶ ; et
où ledit groupement imidazolyle peut former un composé quaternaire au niveau d'une fonction -N= avec un groupement méthyle ;
R⁶ représente un groupement méthyle ou éthyle ; où R⁶ est éventuellement substitué au niveau de l'atome de carbone par un groupement choisi parmi R²⁰;
R²⁰ est choisi parmi les groupements méthoxy et éthoxy ;
R⁵ est un substituant d'un atome de carbone et est choisi parmi les groupements amino, alkyle en C₁₋₄,
alkoxy en C₁₋₄, *N*-(alkyle en C₁₋₄)amino ou *N,N-*(alkyle en C₁₋₄)₂amino ; et
n est compris entre 0 et 2 ;
ou l'un de ses sels de qualité pharmaceutique.

10. Composé conforme à la Revendication 1, où :
R¹ représente un groupement chloro ou cyano ;
R^{2'} représente un atome d'hydrogène ou un groupement chloro ;
R³ représente un groupement fluoro ou méthoxy ;
R⁴ représente un atome d'hydrogène ou un groupement méthyle ou éthyle ;
le Cycle A représente un groupement 1-(2-méthoxyéthyl)imidazol-2-yle, 1-(2-triméthylsilyléthoxyméthyl)imidazol-2-yle, 1-(méthoxyméthyl)imidazol-2-yle, 1,2,4-oxadiazol-5-yle, 1,3,4-oxadiazol-2-yle, 1,3-diméthylimidazol-2-yle, 1*H-*imidazol-2-yle, 1-méthylimidazol-4-yle, 2*H*-pyrazol-3-yle, 2-méthyl-1,2,4-triazol-3-yle, 2-pyridyle, benzothiazol-2-yle, isoxazol-5-yle, pyrazin-2-yle, pyrimidin-2-yle, pyrimidin-4-yle, thiazol-2-yle ou
thiazol-4-yle ;
R⁵ est un substituant d'un atome de carbone et est choisi parmi les groupements amino, méthyle, méthoxy,
méthylamino et diméthylamino ;
n est compris entre 0 et 2 ;
ou l'un de ses sels de qualité pharmaceutique.

11. Composé choisi dans le groupe constitué par les composés suivants :
Acide 2-((3*S*,4*R*)-4-{[(3,4-dichloro-5-méthyl-1*H*-pyrrol-2-yl)carbonyl]amino}-3-méthoxypipéridin-1-yl)-4-pyrimidin-2-yl-1,3-thiazole-5-carboxylique ;
Acide 2-((3S,4R)-4-{[(3,4-dichloro-5-méthyl-1H-pyrrol-2-yl)carbonyl]amino}-3-méthoxypipéridin-1-yl)-4-pyrimidin-4-yl-1,3-thiazole-5-carboxylique ;
Acide 2'-((3S,4R)-4-{[(3,4-dichloro-5-méthyl-1H-pyrrol-2-yl)carbonyl]amino}-3-méthoxypipéridin-1-yl)-2,4'-bi-1,3-thiazole-5'-carboxylique ;
Acide 2-((3S,4R)-4-{[(3,4-dichloro-5-méthyl-1H-pyrrol-2-yl)carbonyl]amino}-3-fluoropipéridin-1-yl)-4-pyrimidin-2-yl-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-pyrimidin-4-yl-1,3-thiazole-5-carboxylique ;
Acide 12'-((3*S*,4*R*)-4-{[(3,4-dichloro-5-méthyl-1*H*-pyrrol-2-yl)carbonyl]amino}-3-fluoropipéridin-1-yl)-2,4'-bi-1,3-thiazole-5'-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-pyridin-2-yl-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-pyridin-2-yl-1,3-thiazole-5-carboxylique ;
Acide bis(diméthylamino)pyrimidin-4-yl]-2-((3*S*,4*R*)-4-{[(3,4-dichloro-5-méthyl-1*H*-pyrrol-2-yl)carbonyl]amino}-3-méthoxypipéridin-1-yl)-1,3-thiazole-5-carboxylique ;
Acide bis(diméthylamino)pyrimidin-4-yl]-2-((3*S*,4*R*)-4-{[(3,4-dichloro-5-méthyl-1*H*-pyrrol-2-yl)carbonyl]amino}-3-fluoropipéridin-1-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-(4,6-diméthoxypyrimidin-2-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-pyrazin-2-yl-1,3-thiazole-5-carboxylique ;
Acide 4-(1,3-benzothiazol-2-yl)-2-((3*S*,4*R*)-4-{[(3,4-dichloro-5-méthyl-1*H*-pyrrol-2-yl)carbonyl]amino}-3-méthoxypipéridin-1-yl)-1,3-thiazole-5-carboxylique ;
Acide 2-((3S,4R)-4-{[(3,4-dichloro-5-méthyl-1*H*-pyrrol-2-yl)carbonyl]amino}-3-méthoxypipéridin-1-yl)-4-pyrazin-2-yl-1,3-thiazole-5-carboxylique ;
Acide 4-(1,3-benzothiazol-2-yl)-2-((3*S*,4*R*)-4-{[(3,4-dichloro-5-méthyl-1*H*-pyrrol-2-yl)carbonyl]amino}-3-fluoropipéridin-1-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-((1-{[2-(triméthylsilyl)éthoxy]méthyl}-1*H*-imidazol-2-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-[1-(méthoxyméthyl)-1*H*-imidazol-2-yl]-1,3-thiazole-5-carboxylique;
Acide 2-((3*S,*4*R*)-4-{[(3,4-dichloro-5-méthyl-1*H*-pyrrol-2-yl)carbonyl]amino}-3-méthoxypipéridin-1-yl)-4-(1*H-*imidazol-2-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-[1-(méthoxyméthyl)-1*H*-imidazol-2-yl]-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-(1*H-*imidazol-2-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-(1-méthyl-1*H*-imidazol-2-yl)-1,3-thiazole-5-carboxylique ;
Trifluoroacétate de 2-[5-carboxy-2-((3*S*,4*R*)-4-{[(3,4-dichloro-5-méthyl-1*H*-pyrrol-2-yl)carbonyl]amino}-3-méthoxypipéridin-1-yl)-1,3-thiazol-4-yl]-1,3-diméthyl-1*H*-imidazol-3-ium ;
Acide 2-((3*S*,4*R*)-4-{[(3,4-dichloro-5-méthyl-1*H*-pyrrol-2-yl)carbonyl]amino}-3-méthoxypipéridin-1-yl)-4-(1*H-*pyrazol-5-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-(1*H-*pyrazol-5-yl)-1,3-thiazole-5-carboxylique ;
Acide 2-((3*S*,4*R*)-4-{[(3,4-dichloro-5-méthyl-1H-pyrrol-2-yl)carbonyl]amino}-3-méthoxypiperidin-1-yl)-4-[1-(2-méthoxyéthyl)-1*H*-imidazol-2-yl]-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-[1-(2-méthoxyéthyl)-1*H*-imidazol-2-yl]-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-(1-méthyl-1*H*-1,2,4-triazol-5-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-(1-méthyl-1*H*-1,2,4-triazol-5-yl)-1,3-thiazole-5-carboxylique ;
Acide 2-((3*S*,4*R*)-4-{[(3-chloro-4-cyano-5-méthyl-1*H-*pyrrol-2-yl)carbonyl]amino}-3-méthoxypipéridin-1-yl)-4-(1-méthyl-1*H*-1,2,4-triazol-5-yl)-1,3-thiazole-5-carboxylique ;
Acide 2-((3*S*,4*R*)-4-{[(3-chloro-4-cyano-5-méthyl-1*H-*pyrrol-2-yl)carbonyl]amino}-3-méthoxypipéridin-1-yl)-4-[1-(2-méthoxyéthyl)-1*H*-imidazol-2-yl]-1,3-thiazole-5-carboxylique ;
Acide 2-((3*S*,4*R*)-4-{[(4-cyano-5-méthyl-1*H*-pyrrol-2-yl)carbonyl]amino}-3-méthoxypipéridin-1-yl)-4-(1-méthyl-1H-1,2,4-triazol-5-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-(1,4,5-triméthyl-1*H*-imidazol-2-yl)-1,3-thiazole-5-carboxylique ;
Acide 2'-amino-2-((3*S*,4*R*)-4-{[(3,4-dichloro-5-méthyl-1*H*-pyrrol-2-yl)carbonyl]amino}-3-méthoxypipéridin-1-yl)-4,4'-bi-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-2'-(méthylamino)-4,4'-bi-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-2'-(diméthylamino)-4,4'-bi-1,3-thiazole-5-carboxylique ;
Acide 2'-amino-2-((3*S*,4*R*)-4-{[(3,4-dichloro-5-méthyl-1*H*-pyrrol-2-yl)carbonyl]amino}-3-fluoropipéridin-1-yl)-4,4'-bi-1,3-thiazole-5-carboxylique ;
Acide 2-((3*S*,4*R*)-4-{[(3,4-dichloro-5-méthyl-1H-pyrrol-2-yl)carbonyl]amino}-3-fluoropipéridin-1-yl)-2'-(méthylamino)-4,4'-bi-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-2'-(diméthylamino)-4,4'-bi-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-(1-méthyl-1H-imidazol-2-yl)-1,3-thiazole-5-carboxylique ;
Acide 2-((3*S*,4*R*)-4-{[(3,4-dichloro-5-méthyl-1H-pyrrol-2-yl)carbonyl]amino}-3-méthoxypipéridin-1-yl)-4-(1-méthyl-1H-imidazol-4-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-fluoropipéridin-1-yl)-4-(1-méthyl-1*H*-imidazol-4-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-(4-méthoxypyrimidin-2-yl)-1,3-thiazole-5-carboxylique ;
2-((3*S*,4*R*)-4-{[(3,4-dichloro-5-méthyl-1*H*-pyrrol-2-yl)carbonyl]amino}-3-méthoxypipéridin-1-yl)-4-pyrimidin-2-yl-1,3-thiazole-5-carboxylate d'éthyle ;
2'-((3*S*,4*R*)-4-{[(3,4-dichloro-5-méthyl-1*H*-pyrrol-2-yl)carbonyl]amino}-3-méthoxypipéridin-1-yl)-2,4'-bi-1,3-thiazole-5'-carboxylate de méthyle ;
2-((3*S*,4*R*)-4-{[(3,4-dichloro-5-méthyl-1*H*-pyrrol-2-yl)carbonyl]amino}-3-fluoropipéridin-1-yl)-4-pyrimidin-2-yl-1,3-thiazole-5-carboxylate d'éthyle ;
2-((3*S*,4*R*)-4-{[(3,4-dichloro-5-méthyl-1*H*-pyrrol-2-yl)carbonyl]amino}-3-fluoropipéridin-1-yl)-4-pyrimidin-4-yl-1,3-thiazole-5-carboxylate d'éthyle ;
2'-((3*S*,4*R*)-4-{[(3,4-dichloro-5-méthyl-1*H*-pyrrol-2-yl)carbonyl]amino}-3-fluoropipéridin-1-yl)-2,4'-bi-1,3-thiazole-5'-carboxylate de méthyle ;
2-((3*S*,4*R*)-4-{[(3,4-dichloro-5-méthyl-1*H*-pyrrol-2-yl)carbonyl]amino}-3-méthoxypipéridin-1-yl)-4-pyridin-2-yl-1,3-thiazole-5-carboxylate d'éthyle ;
2-((3*S*,4*R*)-4-{[(3,4-dichloro-5-méthyl-1*H*-pyrrol-2-yl)carbonyl]amino}-3-fluoropipéridin-1-yl)-4-pyridin-2-yl-1,3-thiazole-5-carboxylate d'éthyle ;
4-[2,6-bis(diméthylamino)pyrimidin-4-yl]-2-((3*S*,4*R*)-4-{[(3,4-dichloro-5-méthyl-1*H*-pyrrol-2-yl)carbonyl]amino}-3-méthoxypipéridin-1-yl)-1,3-thiazole-5-carboxylate d'éthyle ;
4-[2,6-bis(diméthylamino)pyrimidin-4-yl]-2-((3*S*,4*R*)-4-{[(3,4-dichloro-5-méthyl-1*H*-pyrrol-2-yl)carbonyl]amino}-3-fluoropipéridin-1-yl)-1,3-thiazole-5-carboxylate d'éthyle ;
2-((3*S*,4*R*)-4-{[(3,4-dichloro-5-méthyl-1*H*-pyrrol-2-yl)carbonyl]amino}-3-méthoxypipéridin-1-yl)-4-(4,6-diméthoxypyrimidin-2-yl)-1,3-thiazole-5-carboxylate d'éthyle ;
2-((3*S*,4*R*)-4-{[(3,4-dichloro-5-méthyl-1*H*-pyrrol-2-yl)carbonyl]amino}-3-méthoxypipéridin-1-yl)-4-pyrazin-2-yl-1,3-thiazole-5-carboxylate d'éthyle ;
4-(1,3-benzothiazol-2-yl)-2-((3*S*,4*R*)-4-{[(3,4-dichloro-5-méthyl-1H-pyrrol-2-yl)carbonyl]amino}-3-méthoxypipéridin-1-yl)-1,3-thiazole-5-carboxylate d'éthyle ;
2-((3*S*,4*R*)-4-{[(3,4-dichloro-5-méthyl-1*H*-pyrrol-2-yl)carbonyl]amino}-3-fluoropipéridin-1-yl)-4-pyrazin-2-yl-1,3-thiazole-5-carboxylate d'éthyle ;
4-(1,3-benzothiazol-2-yl)-2-((3*S*,4*R*)-4-{[(3,4-dichloro-5-méthyl-1*H*-pyrrol-2-yl)carbonyl]amino}-3-fluoropipéridin-1-yl)-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-méthoxypipéridin-1-yl)-4-((1-{[2-(triméthylsilyl)éthoxy]méthyl}-1*H*-imidazol-2-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-[1-(méthoxyméthyl)-1*H*-imidazol-2-yl]-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-isoxazol-5-yl-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-4-isoxazol-5-yl-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1*H-*pyrazol-5-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-4-(1*H-*pyrazol-5-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-[1-(2-méthoxyéthyl)-1*H*-imidazol-2-yl]-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-4-[1-(2-méthoxyéthyl)-1*H*-imidazol-2-yl]-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1-méthyl-1*H*-1,2,4-triazol-5-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-4-(1-méthyl-1*H*-1,2,4-triazol-5-yl)-1,3-thiazole-5-carboxylate de méthyle ;
2-((3*S*,4*R*)-4-{[(4-Cyano-5-méthyl-1*H*-pyrrol-2-yl)carbonyl]amino}-3-méthoxypipéridin-1-yl)-4-(1-méthyl-1*H*-1,2,4-triazol-5-yl)-1,3-thiazole-5-carboxylate de méthyle ;
2-((3*S*,4*R*)-4-{[(3-Chloro-4-cyano-5-méthyl-1*H*-pyrrol-2-yl)carbonyl]amino}-3-méthoxypipéridin-1-yl)-4-[1-(2-méthoxyéthyl)-1*H*-imidazol-2-yl]-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1,4,5-triméthyl-1*H*-imidazol-2-yl)-1,3-thiazole-5-carboxylate de méthyle ;
2'-Amino-2-((3*S*,4*R*)-4-{[(3,4-dichloro-5-méthyl-1*H-*pyrrol-2-yl)carbonyl]amino}-3-méthoxypipéridin-1-yl)-4,4'-bi-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-2'-(méthylamino)-4,4'-bi-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-2'-(diméthylamino)-4,4'-bi-1,3-thiazole-5-carboxylate de méthyle ;
2'-Amino-2-((3*S*,4*R*)-4-{[(3,4-dichloro-5-méthyl-1H-pyrrol-2-yl)carbonyl]amino}-3-fluoropipéridin-1-yl)-4,4'-bi-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-2'-(méthylamino)-4,4'-bi-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-2'-(diméthylamino)-4,4'-bi-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1-méthyl-1*H*-imidazol-4-yl)-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-fluoropipéridin-1-yl)-4-(1-méthyl-1*H*-imidazol-4-yl)-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-fluoropipéridin-1-yl)-4-(1-méthyl-1H-imidazol-2-yl)-1,3-thiazole-5-carboxylate de méthyle ;
2-((3*S*,4*R*)-4-{[(3-Chloro-4-cyano-5-méthyl-1*H*-pyrrol-2-yl)carbonyl]amino}-3-méthoxypipéridin-1-yl)-4-(1-méthyl-1*H*-1,2,4-triazol-5-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-pyrimidin-4-yl-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-méthoxypipéridin-1-yl)-4-(4-méthoxypyrimidin-2-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1,3,4-oxadiazol-2-yl)-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-méthoxypipéridin-1-yl)-4-(3-méthyl-1,2,4-oxadiazol-5-yl)-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-méthoxypipéridin-1-yl)-4-(5-méthyl-1,3,4-oxadiazol-2-yl)-1,3-thiazole-5-carboxylate d'éthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1H-imidazol-2-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-fluoropipéridin-1-yl)-4-(1*H-*imidazol-2-yl)-1,3-thiazole-5-carboxylate de méthyle ;
2-((3*S*,4*R*)-4-{[(3,4-Dichloro-5-méthyl-1*H*-pyrrol-2-yl)carbonyl]amino}-3-méthoxypipéridin-1-yl)-4-(1-méthyl-1*H*-imidazol-2-yl)-1,3-thiazole-5-carboxylate de méthyle ;
Iodure de 2-[2-((3*S*,4*R*)-4-{[(3,4-dichloro-5-méthyl-1*H-*pyrrol-2-yl)carbonyl]amino}-3-méthoxypipéridin-1-yl)-5-(méthoxycarbonyl)-1,3-thiazol-4-yl]-1,3-diméthyl-1*H-*imidazol-3-ium ;
Acide 2-((3*S*,4*R*)-4-{[(3,4-dichloro-5-méthyl-1*H*-pyrrol-2-yl)carbonyl]amino}-3-méthoxypipéridin-1-yl)-4-(5-méthyl-1,3,4-oxadiazol-2-yl)-1,3-thiazole-5-carboxylique ;
Acide 2-((3*S*,4*R*)-4-{[(3,4-dichloro-5-méthyl-1*H*-pyrrol-2-yl)carbonyl]amino}-3-méthoxypipéridin-1-yl)-4-(5-méthyl-1,3,4-oxadiazol-2-yl)-1,3-thiazole-5-carboxylique ;
2-((3*S*,4*R*)-4-(3,4-Dichloro-5-méthyl-1*H*-pyrrole-2-carboxamido)-3-méthoxypipéridin-1-yl)-4-(1-méthyl-1*H-*1,2,3-triazol-4-yl)thiazole-5-carboxylate de méthyle ;
2-((3*S*,4*R*)-4-(3,4-Dichloro-5-méthyl-1*H*-pyrrole-2-carboxamido)-3-méthoxypipéridin-1-yl)-4-(3,5-diméthylpyrazin-2-yl)thiazole-5-carboxylate de méthyle ;
2-((3*S*,4*R*)-4-(3,4-Dichloro-5-méthyl-1*H*-pyrrole-2-carboxamido)-3-méthoxypipéridin-1-yl)-4-(6-méthoxypyridin-2-yl)thiazole-5-carboxylate de méthyle ;
2-((3*S*,4*R*)-4-(3,4-Dichloro-5-méthyl-1*H*-pyrrole-2-carboxamido)-3-méthoxypipéridin-1-yl)-4-(4-méthoxypyridin-2-yl)thiazole-5-carboxylate d'éthyle ;
2-((3*S*,4*R*)-4-(3,4-Dichloro-5-méthyl-1*H*-pyrrole-2-carboxamido)-3-méthoxypipéridin-1-yl)-4-(5-méthylpyrazin-2-yl)thiazole-5-carboxylate d'éthyle ;
2-((3*S*,4*R*)-4-(3,4-Dichloro-5-méthyl-1*H*-pyrrole-2-carboxamido)-3-méthoxypipéridin-1-yl)-4-(3-fluoropyridin-2-yl)thiazole-5-carboxylate d'éthyle ;
2-((3*S*,4*R*)-4-(3,4-Dichloro-5-méthyl-1*H*-pyrrole-2-carboxamido)-3-fluoropipéridin-1-yl)-4-(4-méthoxypyridin-2-yl)thiazole-5-carboxylate d'éthyle ;
2-((3*S,*4*R*)-4-(3,4-Dichloro-5-méthyl-1*H*-pyrrole-2-carboxamido)-3-fluoropipéridin-1-yl)-4-(1-méthyl-1*H-*1,2,3-triazol-4-yl)thiazole-5-carboxylate de méthyle ;
2-((3*S*,4*R*)-4-(3,4-Dichloro-5-méthyl-1*H*-pyrrole-2-carboxamido)-3-fluoropipéridin-1 -yl)-4-(6-méthoxypyridin-2-yl)thiazole-5-carboxylate de méthyle ;
2-((3*S*,4*R*)-4-(3,4-Dichloro-5-méthyl-1*H*-pyrrole-2-carboxamido)-3-fluoropipéridin-1-yl)-4-(6-méthoxypyridin-2-yl)thiazole-5-carboxylate de méthyle ;
2-((3*S*,4*R*)-4-(3,4-Dichloro-5-méthyl-1*H*-pyrrole-2-carboxamido)-3-fluoropipéridin-1-yl)-4-(3-fluoropyridin-2-yl)thiazole-5-carboxylate d'éthyle ;
2-((3*S*,4*R*)-4-(3,4-Dichloro-5-méthyl-1*H*-pyrrole-2-carboxamido)-3-fluoropipéridin-1-yl)-4-(4-méthoxypyridin-2-yl)thiazole-5-carboxylate d'éthyle ;
2-((3*S*,4*R*)-4-(4-Chloro-3-cyano-5-méthyl-1*H*-pyrrole-2-carboxamido)-3-méthoxypipéridin-1-yl)-4-(1-méthyl-1*H-*1,2,3-triazol-4-yl)thiazole-5-carboxylate de méthyle ;
2-((3*S*,4*R*)-4-(4-Chloro-3-cyano-5-méthyl-1*H*-pyrrole-2-carboxamido)-3-méthoxypipéridin-1-yl)-4-(1-méthyl-1*H-*1,2,4-triazol-5-yl)thiazole-5-carboxylate de méthyle ;
2-((3*S*,4*R*)-4-(4-Chloro-3-cyano-5-méthyl-1*H*-pyrrole-2-carboxamido)-3-méthoxypipéridin-1-yl)-4-(pyrimidin-2-yl)thiazole-5-carboxylate d'éthyle ;
2-((3*S*,4*R*)-4-(4-Chloro-3-cyano-5-méthyl-1*H*-pyrrole-2-carboxamido)-3-méthoxypipéridin-1-yl)-4-(3-fluoropyridin-2-yl)thiazole-5-carboxylate d'éthyle ;
2-((3*S*,4*R*)-4-(4-chloro-3-cyano-5-méthyl-1*H*-pyrrole-2-carboxamido)-3-méthoxypipéridin-1-yl)-4-(5-méthylpyrazin-2-yl)thiazole-5-carboxylate d'éthyle ;
2-((3*S*,4*R*)-4-(4-Chloro-3-cyano-5-méthyl-1*H*-pyrrole-2-carboxamido)-3-méthoxypipéridin-1-yl)-4-(1-méthyl-1*H-*imidazol-4-yl)thiazole-5-carboxylate d'éthyle ;
Acide 2-((3*S*,4*R*)-4-(3,4-dichloro-5-méthyl-1*H*-pyrrole-2-carboxamido)-3-méthoxypipéridin-1-yl)-4-(1-méthyl-1*H-*1,2,3-triazol-4-yl)thiazole-5-carboxylique ;
Acide 2-((3*S*,4*R*)-4-(3,4-dichloro-5-méthyl-1*H*-pyrrole-2-carboxamido)-3-méthoxypipéridin-1-yl)-4-(3,5-diméthylpyrazin-2-yl)thiazole-5-carboxylique ;
Acide 2-((3*S*,4*R*)-4-(3,4-dichloro-5-méthyl-1*H*-pyrrole-2-carboxamido)-3-méthoxypipéridin-1-yl)-4-(6-méthoxypyridin-2-yl)thiazole-5-carboxylique ;
Acide 2-((3*S*,4*R*)-4-(3,4-dichloro-5-méthyl-1*H*-pyrrole-2-carboxamido)-3-méthoxypipéridin-1-yl)-4-(4-méthoxypyridin-2-yl)thiazole-5-carboxylique ;
Acide 2-((3*S*,4*R*)-4-(3,4-dichloro-5-méthyl-1*H*-pyrrole-2-carboxamido)-3-méthoxypipéridin-1-yl)-4-(5-méthylpyrazin-2-yl)thiazole-5-carboxylique ;
Acide 2-((3*S*,4*R*)-4-(3,4-dichloro-5-méthyl-1*H*-pyrrole-2-carboxamido)-3-méthoxypipéridin-1-yl)-4-(3-fluoropyridin-2-yl)thiazole-5-carboxylique ;
Acide 2-((3*S*,4*R*)-4-(3,4-dichloro-5-méthyl-1*H*-pyrrole-2-carboxamido)-3-fluoropipéridin-1-yl)-4-(3-fluoropyridin-2-yl)thiazole-5-carboxylique ;
Acide 2-((3*S*,4*R*)-4-(3,4-dichloro-5-méthyl-1*H*-pyrrole-2-carboxamido)-3-fluoropipéridin-1-yl)-4-(1-méthyl-1*H-*1,2,3-triazol-4-yl)thiazole-5-carboxylique ;
Acide 2-((3*S*,4*R*)-4-(3,4-dichloro-5-méthyl-1*H*-pyrrole-2-carboxamido)-3-fluoropipéridin-1-yl)-4-(6-méthoxypyridin-2-yl)thiazole-5-carboxylique ;
Acide 2-((3*S*,4*R*)-4-(3,4-dichloro-5-méthyl-1*H*-pyrrole-2-carboxamido)-3-fluoropipéridin-1-yl)-4-(5-méthylpyrazin-2-yl)thiazole-5-carboxylique ;
Acide 2-((3*S*,4*R*)-4-(3,4-dichloro-5-méthyl-1*H*-pyrrole-2-carboxamido)-3-fluoropipéridin-1-yl)-4-(3-fluoropyridin-2-yl)thiazole-5-carboxylique ;
Acide 2-((3*S*,4*R*)-4-(3,4-dichloro-5-méthyl-1*H*-pyrrole-2-carboxamido)-3-fluoropipéridin-1-yl)-4-(4-méthoxypyridin-2-yl)thiazole-5-carboxylique ;
Acide 2-((3*S*,4*R*)-4-(4-chloro-3-cyano-5-méthyl-1*H-*pyrrole-2-carboxamido)-3-méthoxypipéridin-1-yl)-4-(1-méthyl-1*H*-1,2,3-triazol-4-yl)thiazole-5-carboxylique ;
Acide 2-((3*S*,4*R*)-4-(4-chloro-3-cyano-5-méthyl-1*H-*pyrrole-2-carboxamido)-3-méthoxypipéridin-1-yl)-4-(1-méthyl-1*H*-1,2,4-triazol-5-yl)thiazole-5-carboxylique ;
Acide 2-((3*S*,4*R*)-4-(4-chloro-3-cyano-5-méthyl-1*H-*pyrrole-2-carboxamido)-3-méthoxypipéridin-1-yl)-4-(pyrimidin-2-yl)thiazole-5-carboxylique ;
Acide 2-((3*S*,4*R*)-4-(4-chloro-3-cyano-5-méthyl-1*H-*pyrrole-2-carboxamido)-3-méthoxypipéridin-1-yl)-4-(3-fluoropyridin-2-yl)thiazole-5-carboxylique ;
Acide 2-((3*S*,4*R*)-4-(4-chloro-3-cyano-5-méthyl-1*H-*pyrrole-2-carboxamido)-3-méthoxypipéridin-1-yl)-4-(5-méthylpyrazin-2-yl)thiazole-5-carboxylique ;
Acide 2-((3*S*,4*R*)-4-(4-chloro-3-cyano-5-méthyl-1*H-*pyrrole-2-carboxamido)-3-méthoxypipéridin-1-yl)-4-(1-méthyl-1*H*-imidazol-4-yl)thiazole-5-carboxylique ;
2-{(3*S*,4*R*)-4-[(4-Chloro-3-cyano-5-méthyl-1*H*-pyrrole-2-carbonyl)-amino]-3-fluoro-pipéridin-1-yl}-4-pyrazin-2-yl-thiazole-5-carboxylate d'éthyle ;
2-{(3*S*,4*R*)-4-[(4-Chloro-3,5-diméthyl-1*H*-pyrrole-2-carbonyl)-amino]-3-fluoro-pipéridin-1-yl}-4-pyrazin-2-yl-thiazole-5-carboxylate d'éthyle ;
2-{(3*S*,4*R*)-4-[(4-Chloro-3,5-diméthyl-1*H*-pyrrole-2-carbonyl)-amino]-3-fluoro-pipéridin-1-yl}-4-pyrimidin-4-yl-thiazole-5-carboxylate d'éthyle ;
2-{(3*S*,4*R*)-4-[(4-Chloro-3-cyano-5-méthyl-1*H*-pyrrole-2-carbonyl)-amino]-3-méthoxy-pipéridin-1-yl}-4-pyrimidin-4-yl-thiazole-5-carboxylate d'éthyle ;
2-{(3*S*,4*R*)-4-[(4-Chloro-3-cyano-5-méthyl-1*H*-pyrrole-2-carbonyl)amino]-3-méthoxy-pipéridin-1-yl}-4-(1-méthyl-1*H*-pyrazol-3-yl)-thiazole-5-carboxylate d'éthyle ;
2-{(3*S*,4*R*)-4-[(3,4-Dichloro-5-méthyl-1*H*-pyrrole-2-carbonyl)-amino]-3-méthoxy-pipéridin-1-yl}-4-(1-méthyl-1*H*-pyrazol-3-yl)-thiazole-5-carboxylate d'éthyle ;
Acide 2-{(3*S*,4*R*)-4-[(4-chloro-3-cyano-5-méthyl-1*H-*pyrrole-2-carbonyl)-amino]-3-fluoro-pipéridin-1-yl}-4-pyrazin-2-yl-thiazole-5-carboxylique ;
Acide 2-[(3*S*,4*R*)-4-{[(4-chloro-3,5-diméthyl-1*H*-pyrrol-2-yl)carbonyl]amino}-3-fluoropipéridin-1-yl]-4-pyrazin-2-yl-1,3-thiazole-5-carboxylique ;
Acide 2-[(3*S*,4*R*)-4-{[(4-chloro-3,5-diméthyl-1*H*-pyrrol-2-yl)carbonyl]amino}-3-fluoropipéridin-1-yl]-4-pyrimidin-4-yl-1,3-thiazole-5-carboxylique ;
Acide 2-[(3*S*,4*R*)-4-{[(4-chloro-3-cyano-5-méthyl-1*H-*pyrrol-2-yl)carbonyl]amino}-3-méthoxypipéridin-1-yl]-4-pyrimidin-4-yl-1,3-thiazole-5-carboxylique ;
Acide 2-[(3*S*,4*R*)-4-{[(4-chloro-3-cyano-5-méthyl-1*H-*pyrrol-2-yl)carbonyl]amino}-3-méthoxypipéridin-1-yl]-4-(1-méthyl-1*H*-pyrazol-3-yl)-1,3-thiazole-5-carboxylique ;
Acide 2-[(3*S*,4*R*)-4-{[(3,4-dichloro-5-méthyl-1*H*-pyrrol-2-yl)carbonyl]amino}-3-méthoxypipéridin-1-yl]-4-(1-méthyl-1*H*-pyrazol-3-yl)-1,3-thiazole-5-carboxylique ;
ou l'un de ses sels de qualité pharmaceutique.

12. Composé conforme à l'une quelconque des Revendications 1 à 11, ou l'un de ses sels de qualité pharmaceutique, pour emploi dans l'obtention d'un effet antibactérien chez un animal à sang chaud.

13. Composé conforme à l'une quelconque des Revendications 1 à 11, ou l'un de ses sels de qualité pharmaceutique, pour emploi dans l'inhibition d'une topoisomérase IV et/ou d'une ADN gyrase bactérienne chez un animal à sang chaud.

14. Composé conforme à l'une quelconque des Revendications 1 à 11, ou l'un de ses sels de qualité pharmaceutique, pour emploi dans le traitement d'une infection bactérienne chez un animal à sang chaud.

15. Composé conforme à l'une quelconque des Revendications 1 à 11, ou l'un de ses sels de qualité pharmaceutique, pour emploi dans le traitement d'une affection parmi les suivantes : pneumonie communautaire, pneumonie nosocomiale, infections cutanées et de la structure cutanée, exacerbation aiguë de la bronchite chronique, sinusite chronique, otite moyenne aiguë, sepsie liée au cathéter, neutropénie fébrile, ostéomyélite, endocardite, infections de l'appareil urinaire, Streptococcus pneumoniae résistante à la pénicilline, Staphylococcus aureus résistante à la méthiciline, Staphylococcus epidermidis résistante à la méthiciline ou Enterococci résistante à la vancomycine.
